# EUROPEAN PATENT APPLICATION

(11) **EP 4 650 347 A1**
(43) Date of publication of application: **19.11.2025**
(21) Application number: 24741355.2
(22) Date of filing: 12.01.2024
(51) Int. Cl.: C07D 213/69, A61K 31/44, A61P 29/00

(54) **PHENYL-SUBSTITUTED HETEROARYL COMPOUND AND PHARMACEUTICAL COMPOSITION COMPRISING SAME, PREPARATION METHOD THEREFOR AND USE THEREOF**

(30) Priority: 13.01.2023 CN 202310056683; 14.06.2023 CN 202310705177; 05.01.2024 CN 202410020993
(71) Applicant: Shanghai Meiyue Biotech Development Co., Ltd., Shanghai 200120 (CN)
(72) Inventor: LUAN, Linbo, Shanghai 200120 (CN); HUANG, Nianfeng, Shanghai 200120 (CN); YAO, Yuanshan, Shanghai 200120 (CN)
(74) Representative: Dai, Simin
(86) International application number: PCT/CN2024/071949
(87) International publication number: WO 2024/149359

(57) **Abstract**

The present disclosure relates to a phenyl-substituted heteroaryl compound and a use thereof. Specifically, provided is a phenyl-substituted heteroaryl compound represented by formula (I), which can be used for preparing a drug, especially preparing a drug for preventing and/or treating p38 MAPK MK2 pathway-mediated diseases or disorders. Each group in formula (I) is as defined in the description.

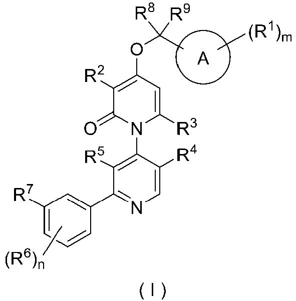

## Description

The present invention claims:
the priority of the prior application filed with the China National Intellectual Property Administration on January 13, 2023, with the patent application number of 202310056683.1, entitled "phenyl-substituted heteroaryl compound and pharmaceutical composition comprising same, preparation method therefor and use thereof";
the priority of the prior application filed with the China National Intellectual Property Administration on June 14, 2023, with the patent application number of 202310705177.0, entitled "phenyl-substituted heteroaryl compound and pharmaceutical composition comprising same, preparation method therefor and use thereof";
the priority of the prior application filed with the China National Intellectual Property Administration on January 05, 2024, with the patent application number of 202410020993.2, entitled "phenyl-substituted heteroaryl compound and pharmaceutical composition comprising same, preparation method therefor and use thereof";
the contents of the above prior applications are incorporated herein by reference in their entireties.

### TECHNICAL FIELD

The present disclosure belongs to the field of medicine, and specifically relates to a phenyl-substituted heteroaryl compound, a pharmaceutical composition comprising the same, a preparation method therefor, and a use thereof.

### BACKGROUND

Biological signal transduction involves specific protein-protein interactions and post-translational modifications, regulating genetic and epigenetic processes in response to internal and external environments. Mitogen-activated protein kinase (MAPK) is a group of serine-threonine protein kinases that can be activated by various intracellular and extracellular stressors, serving as important mediators for transmitting signals from the cell surface to the cell nucleus. Stress factors include cytokines, neurotransmitters, hormones, cellular stress, cell adhesion, *etc.*

As a subfamily of the MAPK family, p38 MAPK responds to extracellular signals and inflammatory cytokines. After being activated, p38 MAPK phosphorylates and activates various downstream protein kinases and transcription factors, thereby exerting complex biological effects. p38 MAPK includes four members, namely p38α, p38β, p38γ, and p38δ. Among them, p38α is considered to play an important role in the signaling pathways of inflammatory processes, while the biological functions of other isoforms remain incompletely discovered, though they exhibit pleiotropic effects. Studies have shown that p38β plays an important role in cellular protection mechanisms, while the mitogen-activated protein kinase MKK3 (MAP Kinase Kinase 3) mediates the effect of p38δ on the proliferation and survival of late-stage colorectal cancer (CRC) cells. As an attractive target in the field of drug development, p38 MAPK has multiple inhibitor drugs in clinical research, but none have been approved for marketing to date. According to publicly available information, some candidate compounds failed during the clinical research phase. Therefore, developing a safe and effective p38 MAPK inhibitor is currently the primary challenge in drug development in this field.

p38 MAPK can regulate over 60 substrates and performs different physiological functions [Cell 2013(152), 924]. Therefore, selectively inhibiting the activation of downstream effectors of p38 MAPK is a primary strategy to avoid side effects or insufficient efficacy resulting from the overall inhibition of p38 MAPK. MAPK-activated protein kinase 2 (MK2) is a direct downstream substrate of p38 MAPK and can be activated by p38α and p38β. As the first discovered substrate of p38 MAPK, MK2 can regulate the expression of inflammatory factors at both transcriptional and post-transcriptional levels, thereby playing an important role in the modulation of various inflammatory diseases. Studies have shown that MK2 can increase the expression of inflammatory factors such as TNF-α, IL-6, IL-8, and COX-2 by stabilizing the AU-rich elements of mRNA. In a postoperative ileus mouse model [The Journal of Surgical Research 2013(185), 102], the MK2 inhibitor can reduce the expression of inflammatory factors such as MIP-1α, TNF-α, IL-6, and IL-1β. At the same time, decreased infiltration of polymorphonuclear leukocytes, mast cells, and mononuclear macrophages is observed, along with improved contractile function of intestinal smooth muscle. In the collagen-induced arthritis (CIA) model in mice [Journal of Immunology 2006(177), 1913], MK2 gene knockout can reduce the incidence of collagen-induced arthritis. Compared with wild-type mice, MK2-/- and MK2+/- mice exhibit decreased incidence and severity of collagen-induced arthritis, along with varying reductions in the expression of inflammatory cytokines TNF-α and IL-6. In the MK2 knockout mouse model of hypercholesterolemia [Circ Res 2007(101), 1104], lipid deposition and macrophage in the aorta are reduced, along with decreased expression of inflammatory cytokines such as VCAM-1 and MCP-1. Additionally, studies have shown that inhibiting MK2 can be used in the development of anti-tumor drugs [Cancer Cell 2007(11), 175]. Therefore, it is necessary to find new small molecule inhibitors that selectively modulate the p38 MAPK/MK2 pathway. By inhibiting p38 MAPK-dependent MK2 activity while preserving the selectivity of other p38 MAPK substrates (such as ATF2 and MK5), these inhibitors can maintain therapeutic efficacy while improving drug safety.

### SUMMARY

The present disclosure provides a compound represented by formula (I) or a pharmaceutically acceptable salt thereof, 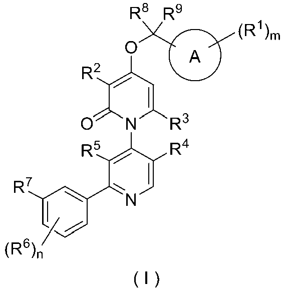 wherein
ring A is phenyl or 5- to 6-membered heteroaryl;
each R¹ is the same or different, and independently selected from the group consisting of H, halogen, C₁₋₆ alkyl, C₁₋₆ haloalkyl, C₁₋₆ alkoxy, C₁₋₆ haloalkoxy, C₁₋₆ hydroxyalkyl, cyano, hydroxyl, amino, nitro, and 3- to 8-membered cycloalkyl;
R² is selected from the group consisting of H, halogen, and C₁₋₆ alkyl;
R³ is selected from the group consisting of H, halogen, C₁₋₆ alkyl, and C₁₋₆ haloalkyl;
R⁴ is selected from the group consisting of H, halogen, cyano, hydroxyl, amino, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
R⁵ is selected from the group consisting of H, halogen, and C₁₋₆ alkyl;
each R⁶ is the same or different, and independently selected from the group consisting of H, halogen, cyano, hydroxyl, amino, C₁₋₆ alkyl, C₁₋₆ haloalkyl, C₁₋₆ alkoxy, C₁₋₆ haloalkoxy, C₁₋₆ hydroxyalkyl, -C(O)OR^{e}, -C(O)NR^{g}R^{h}, -CCO)R^{f}, -S(O)₂R^{f}, and 3- to 8-membered cycloalkyl; R⁷ is selected from the group consisting of H, halogen, 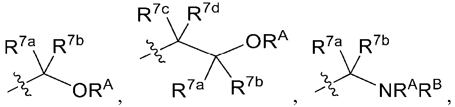 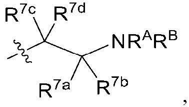 -C(O)OR^{e}, -C(O)NR^{g}R^{h}, -C(O)R^{f}, and -S(O)₂R^{f};
R^{A} and R^{B} are the same or different, and each independently selected from the group consisting of H, C₁₋₆ alkyl, and C₁₋₆ haloalkyl;
R^{7a} and R^{7b} are the same or different, and each independently selected from the group consisting of H, D, C₁₋₆ alkyl, C₁₋₆ haloalkyl, C₁₋₆ hydroxyalkyl, -C(O)OR^{e}, -C(O)NR^{g}R^{h}, -C(O)R^{f}, -S(O)₂R^{f}, and 3- to 8-membered cycloalkyl;
alternatively, R^{7a} and R^{7b} together with the carbon to which they are attached form a 3- to 8-membered cycloalkyl or 3- to 8-membered heterocyclyl ring, wherein the 3- to 8-membered cycloalkyl or 3- to 8-membered heterocyclyl is optionally substituted by one or more substituents selected from the group consisting of halogen and C₁₋₆ alkyl;
R^{7c} and R^{7d} are the same or different, and each independently selected from the group consisting of H, D (deuterium), halogen, cyano, hydroxyl, amino, oxo (=O), C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
alternatively, R^{7c} and R^{7d} together with the carbon to which they are attached form a 3- to 8-membered cycloalkyl or 3- to 8-membered heterocyclyl ring, wherein the 3- to 8-membered cycloalkyl or 3- to 8-membered heterocyclyl is optionally substituted by one or more substituents selected from the group consisting of halogen and C₁₋₆ alkyl;
R^{e} is selected from the group consisting of H, C₁₋₆ alkyl, C₁₋₆ haloalkyl, and 3- to 8-membered cycloalkyl;
R^{g} and R^{h} are the same or different, and each independently selected from the group consisting of H, C₁₋₆ alkyl, hydroxyl, C₁₋₆ alkoxy, and 3- to 8-membered cycloalkyl, wherein the C₁₋₆ alkyl, C₁₋₆ alkoxy, and 3- to 8-membered cycloalkyl are optionally substituted by one or more substituents selected from the group consisting of halogen, cyano, hydroxyl, amino, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
alternatively, R^{g} and R^{h} together with the N to which they are attached form a 3- to 6-membered heterocyclyl ring, wherein the 3- to 6-membered heterocyclyl is optionally substituted by one or more substituents selected from the group consisting of halogen, hydroxyl, C₁₋₆ alkyl, and C₁₋₆ haloalkyl;
R^{f} is selected from the group consisting of H, C₁₋₆ alkyl, C₁₋₆ haloalkyl, C₁₋₆ hydroxyalkyl, and 3- to 8-membered cycloalkyl;
alternatively, R⁶ and R⁷ together with the atom to which they are attached form a 3- to 8-membered heterocyclyl ring, wherein the 3- to 8-membered heterocyclyl is optionally substituted by one or more substituents selected from the group consisting of hydroxyl, oxo, C₁₋₆ alkoxy, C₁₋₆ alkyl, C₁₋₆ haloalkyl, and C₁₋₆ haloalkoxy;
R⁸ and R⁹ are the same or different, and each independently selected from the group consisting of H, D, halogen, C₁₋₆ alkyl, and C₁₋₆ haloalkyl;
alternatively, R⁸ and R⁹ together with the atom to which they are attached form a 3- to 6-membered cycloalkyl ring, wherein the 3- to 6-membered cycloalkyl is optionally substituted by one or more substituents selected from the group consisting of halogen, hydroxyl, C₁₋₆ alkyl, and C₁₋₆ haloalkyl;
n is selected from the group consisting of 0, 1, 2, 3, and 4;
m is selected from the group consisting of 0, 1, 2, 3, 4, and 5.

In some embodiments, the present disclosure provides the compound represented by formula (I) or the pharmaceutically acceptable salt thereof, wherein R⁷ is selected from the group consisting of H, halogen, 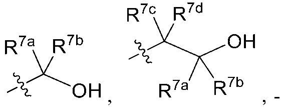 C(O)OR^{e}, -C(O)NR^{g}R^{h}, -C(O)R^{f}, and -S(O)₂R^{f};
R^{7a} and R^{7b} are the same or different, and each independently selected from the group consisting of H, D, C₁₋₆ alkyl, C₁₋₆ haloalkyl, C₁₋₆ hydroxyalkyl, and 3- to 8-membered cycloalkyl;
alternatively, R^{7a} and R^{7b} together with the carbon to which they are attached form a 3- to 8-membered cycloalkyl or 3- to 8-membered heterocyclyl ring, wherein the 3- to 8-membered cycloalkyl or 3- to 8-membered heterocyclyl is optionally substituted by one or more substituents selected from the group consisting of halogen and C₁₋₆ alkyl;
R^{7c} and R^{7d} are the same or different, and each independently selected from the group consisting of H, D (deuterium), halogen, cyano, hydroxyl, amino, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
alternatively, R^{7c} and R^{7d} together with the carbon to which they are attached form a 3- to 8-membered cycloalkyl or 3- to 8-membered heterocyclyl ring, wherein the 3- to 8-membered cycloalkyl or 3- to 8-membered heterocyclyl is optionally substituted by one or more substituents selected from the group consisting of halogen and C₁₋₆ alkyl;
R^{e} is selected from the group consisting of H, C₁₋₆ alkyl, C₁₋₆ haloalkyl, and 3- to 8-membered cycloalkyl;
R^{g} and R^{h} are the same or different, and each independently selected from the group consisting of H, C₁₋₆ alkyl, hydroxyl, C₁₋₆ alkoxy, and 3- to 8-membered cycloalkyl, wherein the C₁₋₆ alkyl, C₁₋₆ alkoxy, and 3- to 8-membered cycloalkyl are optionally substituted by one or more substituents selected from the group consisting of halogen, cyano, hydroxyl, amino, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
alternatively, R^{g} and R^{h} together with the N to which they are attached form a 3- to 6-membered heterocyclyl ring, wherein the 3- to 6-membered heterocyclyl is optionally substituted by one or more substituents selected from the group consisting of halogen, hydroxyl, C₁₋₆ alkyl, and C₁₋₆ haloalkyl;
R^{f} is selected from the group consisting of H, C₁₋₆ alkyl, C₁₋₆ haloalkyl, C₁₋₆ hydroxyalkyl, and 3- to 8-membered cycloalkyl.

In some embodiments, the present disclosure provides the compound represented by formula (I) or the pharmaceutically acceptable salt thereof, wherein R⁶ and R⁷ together with the atom to which they are attached form a 3- to 8-membered heterocyclyl ring, and the 3- to 8-membered heterocyclyl is optionally substituted by one or more substituents selected from the group consisting of hydroxyl, oxo, C₁₋₆ alkyl, and C₁₋₆ haloalkyl.

In some embodiments, the present disclosure provides the compound represented by formula (I) or the pharmaceutically acceptable salt thereof, wherein ring A is pyridinyl.

In some embodiments, the present disclosure provides the compound represented by formula (I) or the pharmaceutically acceptable salt thereof, which is a compound represented by formula (II) or a pharmaceutically acceptable salt thereof, 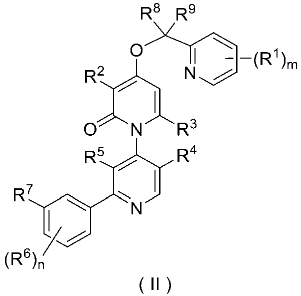 wherein
the definitions of R¹, R², R³, R⁴, R⁵, R⁶, R⁷, R⁸, R⁹, n, and m are as described in the compound represented by formula (I).

In some embodiments, the present disclosure provides the compound represented by formula (I) or formula (II) or the pharmaceutically acceptable salt thereof, which is a compound represented by formula (II-1) or formula (II-2) or a pharmaceutically acceptable salt thereof, 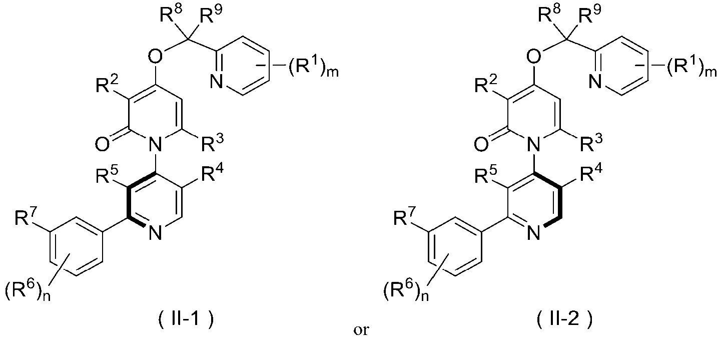 wherein
the definitions of R¹, R², R³, R⁴, R⁵, R⁶, R⁷, R⁸, R⁹, n, and m are as described in the compound represented by formula (I). In some embodiments, the present disclosure provides compounds represented by formula (I), formula (II), formula (II-1), and formula (II-2), or pharmaceutically acceptable salts thereof, wherein R⁶ and R⁷ are any one of the following schemes:
scheme I:
   each R⁶ is the same or different, and independently selected from the group consisting of H, halogen, cyano, hydroxyl, amino, C₁₋₆ alkyl, C₁₋₆ haloalkyl, C₁₋₆ alkoxy, C₁₋₆ haloalkoxy, C₁₋₆ hydroxyalkyl, -C(O)NR^{g}R^{h}, -S(O)₂R^{f}, and 3- to 8-membered cycloalkyl; R^{g} and R^{h} are the same or different, and each independently selected from the group consisting of H, C₁₋₆ alkyl, C₁₋₆ haloalkyl, and 3- to 8-membered cycloalkyl; R^{f} is selected from the group consisting of C₁₋₆ alkyl, C₁₋₆ haloalkyl, C₁₋₆ hydroxyalkyl, and 3- to 8-membered cycloalkyl;
   R⁷ is 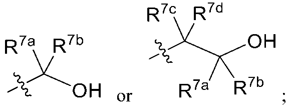 R^{7a} and R^{7b} are the same or different, and each independently selected from the group consisting of H, D, C₁₋₆ alkyl, C₁₋₆ haloalkyl, C₁₋₆ hydroxyalkyl, and 3- to 8-membered cycloalkyl;
   R^{7c} and R^{7d} are the same or different, and each independently selected from the group consisting of H, D, halogen, cyano, hydroxyl, amino, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
   scheme II:
      each R⁶ is the same or different, and independently selected from the group consisting of H, halogen, cyano, hydroxyl, amino, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
      R⁷ is selected from the group consisting of -C(O)OR^{e}, -C(O)NR^{g}R^{h}, -C(O)R^{f}, and -S(O)₂R^{f};
      R^{e} is H or C₁₋₆ alkyl;
      R^{g} and R^{h} are the same or different, and each independently selected from the group consisting of H, C₁₋₆ alkyl, hydroxyl, C₁₋₆ alkoxy, and 3- to 8-membered cycloalkyl, wherein the C₁₋₆ alkyl, C₁₋₆ alkoxy, and 3- to 8-membered cycloalkyl are optionally substituted by one or more substituents selected from the group consisting of halogen, cyano, hydroxyl, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
      alternatively, R^{g} and R^{h} together with the N to which they are attached form a 3- to 6-membered heterocyclyl ring, wherein the 3- to 6-membered heterocyclyl is optionally substituted by one or more substituents selected from the group consisting of halogen, hydroxyl, C₁₋₆ alkyl, and C₁₋₆ haloalkyl;
      R^{f} is selected from the group consisting of C₁₋₆ alkyl, C₁₋₆ haloalkyl, C₁₋₆ hydroxyalkyl, and 3- to 8-membered cycloalkyl;
      preferably,
      each R⁶ is the same or different, and independently selected from the group consisting of H and halogen;
      R⁷ is selected from the group consisting of -C(O)(CH₂)pOH, -C(O)NR^{g}R^{h}, and -S(O)₂R^{f}; p is a positive integer from 0 to 5;
      R^{g} and R^{h} are the same or different, and each independently selected from the group consisting of H, hydroxyl, C₁₋₆ alkyl, and C₁₋₆ alkoxy, wherein the C₁₋₆ alkyl and C₁₋₆ alkoxy are optionally substituted by one or more substituents selected from the group consisting of halogen and hydroxyl;
      alternatively, R^{g} and R^{h} together with the N to which they are attached form a 3- to 6-membered heterocyclyl ring, wherein the 3- to 6-membered heterocyclyl is optionally substituted by one or more substituents selected from the group consisting of halogen, hydroxyl, and C₁₋₆ alkyl;
      R^{f} is C₁₋₆ alkyl or 3- to 8-membered cycloalkyl;
      scheme III:
         R⁶ is selected from the group consisting of -C(O)(CH₂)pOH, -C(O)NR^{g}R^{h}, and -S(O)₂R^{f}; p is a positive integer from 0 to 5; n is 1;
         R^{g} and R^{h} are the same or different, and each independently selected from the group consisting of H, hydroxyl, C₁₋₆ alkyl, and C₁₋₆ alkoxy, wherein the C₁₋₆ alkyl and C₁₋₆ alkoxy are optionally substituted by one or more substituents selected from the group consisting of halogen and hydroxyl;
         alternatively, R^{g} and R^{h} together with the N to which they are attached form a 3- to 6-membered heterocyclyl ring, wherein the 3- to 6-membered heterocyclyl is optionally substituted by one or more substituents selected from the group consisting of halogen, hydroxyl, and C₁₋₆ alkyl;
         R^{f} is C₁₋₆ alkyl or 3- to 8-membered cycloalkyl;
         R⁷ is H or halogen.

In some embodiments, the present disclosure provides compounds represented by formula (I), formula (II), formula (II-1), and formula (II-2) or pharmaceutically acceptable salts thereof, wherein each R⁶ is the same or different, and independently selected from the group consisting of H, halogen, cyano, hydroxyl, amino, C₁₋₆ alkyl, C₁₋₆ haloalkyl, C₁₋₆ alkoxy, C₁₋₆ haloalkoxy, C₁₋₆ hydroxyalkyl, -C(O)NR^{g}R^{h}, -S(O)₂R^{f}, and 3- to 8-membered cycloalkyl; R^{g} and R^{h} are the same or different, and each independently selected from the group consisting of H, C₁₋₆ alkyl, C₁₋₆ haloalkyl, and 3- to 8-membered cycloalkyl; R^{f} is selected from the group consisting of C₁₋₆ alkyl, C₁₋₆ haloalkyl, C₁₋₆ hydroxyalkyl, and 3- to 8-membered cycloalkyl;
R⁷ is 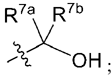 R^{7a} and R^{7b} are the same or different, and each independently selected from the group consisting of H, D, C₁₋₆ alkyl, and
-CONHR^{7e}; R^{7e} is H or C₁₋₆ alkyl.

In some embodiments, the present disclosure provides compounds represented by formula (I), formula (II), formula (II-1), and formula (II-2) or pharmaceutically acceptable salts thereof, wherein each R⁶ is the same or different, and independently selected from the group consisting of H, halogen, cyano, hydroxyl, amino, C₁₋₆ alkyl, C₁₋₆ haloalkyl, -C(O)NR^{g}R^{h}, -S(O)₂R^{f}, and 3- to 8-membered cycloalkyl; R^{g} and R^{h} are the same or different, and each is independently H or C₁₋₆ alkyl; R^{f} is selected from the group consisting of C₁₋₆ alkyl and 3- to 8-membered cycloalkyl;
R⁷ is 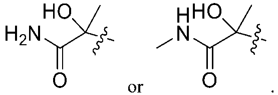
In some embodiments, the present disclosure provides compounds represented by formula (I), formula (II), formula (II-1), and formula (II-2) or pharmaceutically acceptable salts thereof, wherein R⁷ is

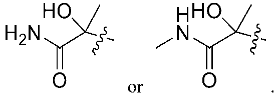

In some embodiments, the present disclosure provides compounds represented by formula (I), formula (II), formula (II-1), and formula (II-2) or pharmaceutically acceptable salts thereof, wherein the ring formed by R⁶ and R⁷ together with the carbon to which they are attached is 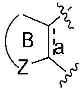 (the a end represents being fused with a benzene ring); Z is NH, O, or S; ring B is optionally substituted by one or more substituents selected from the group consisting of hydroxyl, oxo, and C₁₋₆ alkyl.

In some embodiments, the present disclosure provides compounds represented by formula (I), formula (II), formula (II-1), and formula (II-2) or pharmaceutically acceptable salts thereof, wherein the ring formed by R⁶ and R⁷ together with the carbon to which they are attached is 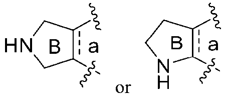 (the a end represents being fused with a benzene ring), and ring B is optionally substituted by one or more substituents selected from the group consisting of hydroxyl, oxo, and methyl.

In some embodiments, the present disclosure provides compounds represented by formula (I), formula (II), formula (II-1), and formula (II-2) or pharmaceutically acceptable salts thereof, wherein the ring formed by R⁶ and R⁷ together with the carbon to which they are attached is 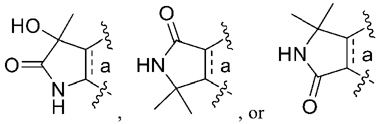 (the a end represents being fused with a benzene ring).

In some embodiments, the present disclosure provides compounds represented by formula (I), formula (II), formula (II-1), and formula (II-2) or pharmaceutically acceptable salts thereof, wherein R⁷ is selected from the group consisting of H, F, and Cl.

In some embodiments, the present disclosure provides compounds represented by formula (I), formula (II), formula (II-1), and formula (II-2) or pharmaceutically acceptable salts thereof, wherein R⁷ is selected from the group consisting of

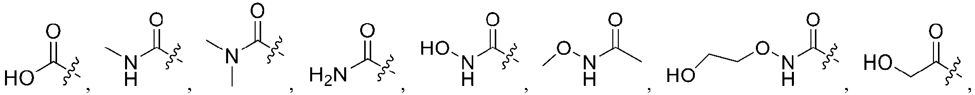

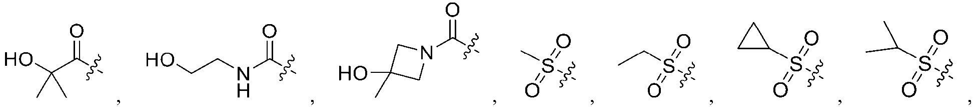

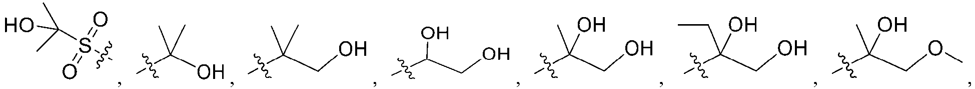

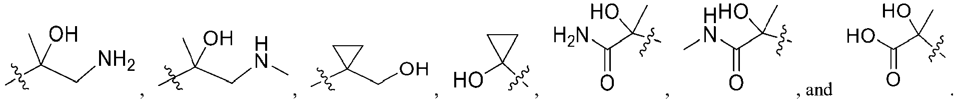

In some embodiments, the present disclosure provides compounds represented by formula (I), formula (II), formula (II-1), and formula (II-2) or pharmaceutically acceptable salts thereof, wherein R⁷ is selected from the group consisting of

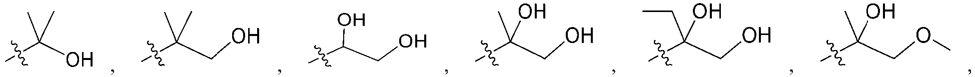

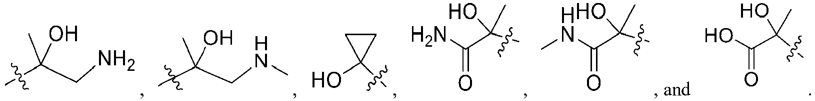

In some embodiments, the present disclosure provides compounds represented by formula (I), formula (II), formula (II-1), and formula (II-2) or pharmaceutically acceptable salts thereof, wherein R⁶ is selected from the group consisting of H, halogen, cyano, hydroxyl, C₁₋₆ alkyl, C₁₋₆ haloalkyl, C₁₋₆ alkoxy, C₁₋₆ haloalkoxy, C₁₋₆ hydroxyalkyl, -C(O)NR^{g}R^{h}, -S(O)₂R^{f}, and 3- to 8-membered cycloalkyl; R^{g} and R^{h} are the same or different, and each independently selected from the group consisting of H, C₁₋₆ alkyl, C₁₋₆ haloalkyl, and 3- to 8-membered cycloalkyl; R^{f} is selected from the group consisting of C₁₋₆ alkyl, C₁₋₆ haloalkyl, C₁₋₆ hydroxyalkyl, and 3- to 8-membered cycloalkyl.

In some embodiments, the present disclosure provides compounds represented by formula (I), formula (II), formula (II-1), and formula (II-2) or pharmaceutically acceptable salts thereof, wherein R⁶ is selected from the group consisting of H, halogen, cyano, hydroxyl, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl.

In some embodiments, the present disclosure provides compounds represented by formula (I), formula (II), formula (II-1), and formula (II-2) or pharmaceutically acceptable salts thereof, wherein R⁶ is H or halogen.

In some embodiments, the present disclosure provides compounds represented by formula (I), formula (II), formula (II-1), and formula (II-2) or pharmaceutically acceptable salts thereof, wherein n is 0, 1, or 2.

In some embodiments, the present disclosure provides compounds represented by formula (I), formula (II), formula (II-1), and formula (II-2) or pharmaceutically acceptable salts thereof, wherein 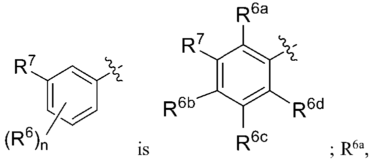 R^{6b}, R^{6c}, and R^{6d} are the same or different, and each independently selected from the group consisting of H, halogen, cyano, hydroxyl, C₁₋₆ alkyl, C₁₋₆ haloalkyl, C₁₋₆ alkoxy, C₁₋₆ haloalkoxy, C₁₋₆ hydroxyalkyl, -C(O)OH, -C(O)NR^{g}R^{h}, -S(O)₂R^{f}, and 3- to 8-membered cycloalkyl; R^{g} and R^{h} are the same or different, and each independently selected from the group consisting of H, C₁₋₆ alkyl, C₁₋₆ haloalkyl, and 3- to 8-membered cycloalkyl; R^{f} is selected from the group consisting of C₁₋₆ alkyl, C₁₋₆ haloalkyl, C₁₋₆ hydroxyalkyl, and 3- to 8-membered cycloalkyl; R⁷ is defined as above; alternatively, R^{6b} and R⁷ together with the atom to which they are attached form a 3- to 8-membered heterocyclyl ring, wherein the 3- to 8-membered heterocyclyl is optionally substituted by one or more substituents selected from the group consisting of hydroxyl, oxo, C₁₋₆ alkyl, and C₁₋₆ haloalkyl, and R^{6a}, R^{6c}, and R^{6d} are defined as above.

In some embodiments, the present disclosure provides compounds represented by formula (I), formula (II), formula (II-1), and formula (II-2) or pharmaceutically acceptable salts thereof, wherein 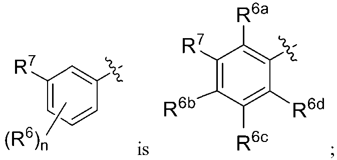 R^{6a}, R^{6b}, R^{6c}, and R^{6d} are the same or different, and each independently selected from the group consisting of H, halogen, cyano, hydroxyl, C₁₋₆ alkyl, C₁₋₆ haloalkyl, C₁₋₆ alkoxy, C₁₋₆ haloalkoxy, C₁₋₆ hydroxyalkyl, -C(O)OH, -C(O)NR^{g}R^{h}, -S(O)₂R^{f}, and 3- to 8-membered cycloalkyl; R^{g} and R^{h} are the same or different, and each independently selected from the group consisting of H, C₁₋₆ alkyl, C₁₋₆ haloalkyl, and 3- to 8-membered cycloalkyl; R^{f} is selected from the group consisting of C₁₋₆ alkyl, C₁₋₆ haloalkyl, C₁₋₆ hydroxyalkyl, and 3- to 8-membered cycloalkyl; R⁷ is defined as above.

In some embodiments, the present disclosure provides compounds represented by formula (I), formula (II), formula (II-1), and formula (II-2) or pharmaceutically acceptable salts thereof, wherein 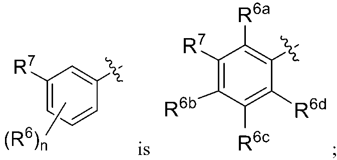 R^{6a} and R^{6b} are the same or different, and each independently selected from the group consisting of H, halogen, cyano, hydroxyl, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl; R^{6c} and R^{6d} are the same or different, and each is independently H, halogen, or C₁₋₆ alkyl; R⁷ is defined as above.

In some embodiments, the present disclosure provides compounds represented by formula (I), formula (II), formula (II-1), and formula (II-2), wherein each R⁶ is the same or different, and independently selected from the group consisting of H, halogen, cyano, hydroxyl, amino, C₁₋₆ alkyl, C₁₋₆ haloalkyl, -C(O)NR^{g}R^{h}, -S(O)₂R^{f}, and 3- to 8-membered cycloalkyl; R^{g} and R^{h} are the same or different, and each is independently H or C₁₋₆ alkyl; R^{f} is selected from the group consisting of C₁₋₆ alkyl and 3- to 8-membered cycloalkyl;
R⁷ is selected from the group consisting of 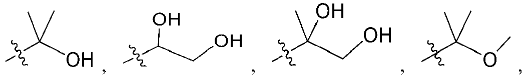 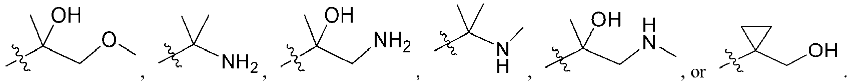
In some embodiments, the present disclosure provides compounds represented by formula (I), formula (II), formula (II-1), and formula (II-2), wherein each R⁶ is the same or different, and independently selected from the group consisting of H, halogen, cyano, hydroxyl, amino, C₁₋₆ alkyl, C₁₋₆ haloalkyl, -C(O)NR^{g}R^{h}, -S(O)₂R^{f}, and 3- to 8-membered cycloalkyl; R^{g} and R^{h} are the same or different, and each is independently H or C₁₋₆ alkyl; R^{f} is selected from the group consisting of C₁₋₆ alkyl and 3- to 8-membered cycloalkyl;
R⁷ is

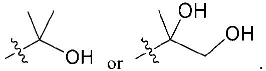

In some embodiments, the present disclosure provides compounds represented by formula (I), formula (II), formula (II-1), and formula (II-2), wherein R⁷ is selected from the group consisting of

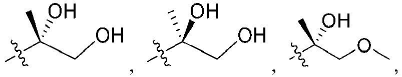

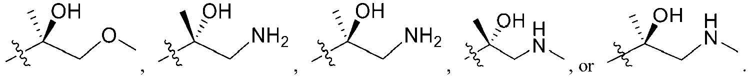

In some embodiments, the present disclosure provides compounds represented by formula (I), formula (II), formula (II-1), and formula (II-2), wherein R⁷ is

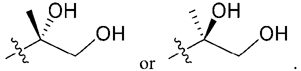

In some embodiments, the present disclosure provides compounds represented by formula (I), formula (II), formula (II-1), and formula (II-2), wherein R² is halogen or C₁₋₆ alkyl.

In some embodiments, the present disclosure provides compounds represented by formula (I), formula (II), formula (II-1), and formula (II-2), wherein R² is halogen.

In some embodiments, the present disclosure provides compounds represented by formula (I), formula (II), formula (II-1), and formula (II-2), wherein R² is selected from the group consisting of Cl, Br, F, and methyl. In some embodiments, the present disclosure provides compounds represented by formula (I), formula (II), formula (II-1), and formula (II-2), wherein R³ is C₁₋₆ alkyl or C₁₋₆ haloalkyl.

In some embodiments, the present disclosure provides compounds represented by formula (I), formula (II), formula (II-1), and formula (II-2), wherein R³ is methyl.

In some embodiments, the present disclosure provides compounds represented by formula (I), formula (II), formula (II-1), and formula (II-2), wherein R⁴ is selected from the group consisting of H, halogen, C₁₋₆ alkyl, C₁₋₆ alkoxy, and C₁₋₆ haloalkyl.

In some embodiments, the present disclosure provides compounds represented by formula (I), formula (II), formula (II-1), and formula (II-2), wherein R⁴ is selected from the group consisting of H, methyl, methoxy, and Cl.

In some embodiments, the present disclosure provides compounds represented by formula (I), formula (II), formula (II-1), and formula (II-2), wherein R⁴ is selected from the group consisting of methyl and Cl.

In some embodiments, the present disclosure provides compounds represented by formula (I), formula (II), formula (II-1), and formula (II-2), wherein R⁵ is H, halogen, or C₁₋₆ alkyl.

In some embodiments, the present disclosure provides compounds represented by formula (I), formula (II), formula (II-1), and formula (II-2), wherein R⁵ is selected from the group consisting of H, F, or methyl.

In some embodiments, the present disclosure provides compounds represented by formula (I), formula (II), formula (II-1), and formula (II-2), wherein R⁸ and R⁹ are the same or different, and each is independently H or D.

In some embodiments, the present disclosure provides compounds represented by formula (I), formula (II), formula (II-1), and formula (II-2), wherein R¹ is the same or different, and each independently selected from the group consisting of H, halogen, C₁₋₆ alkyl, and C₁₋₆ haloalkyl.

In some embodiments, the present disclosure provides compounds represented by formula (I), formula (II), formula (II-1), and formula (II-2), wherein R¹ is halogen.

In some embodiments, the present disclosure provides compounds represented by formula (I), formula (II), formula (II-1), and formula (II-2), wherein R¹ is F.

In some embodiments, the present disclosure provides compounds represented by formula (II), formula (II-1), and formula (II-2), wherein 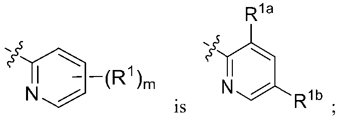 R^{1a} and R^{1b} are the same or different, and each is independently halogen.

In some embodiments, the present disclosure provides compounds represented by formula (II), formula (II-1), and formula (II-2), wherein

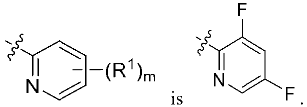

In some embodiments, exemplary specific compounds of compounds represented by formula (I), formula (II), formula (II-1), or formula (II-2) include, but are not limited to, the structures listed in Table A below:

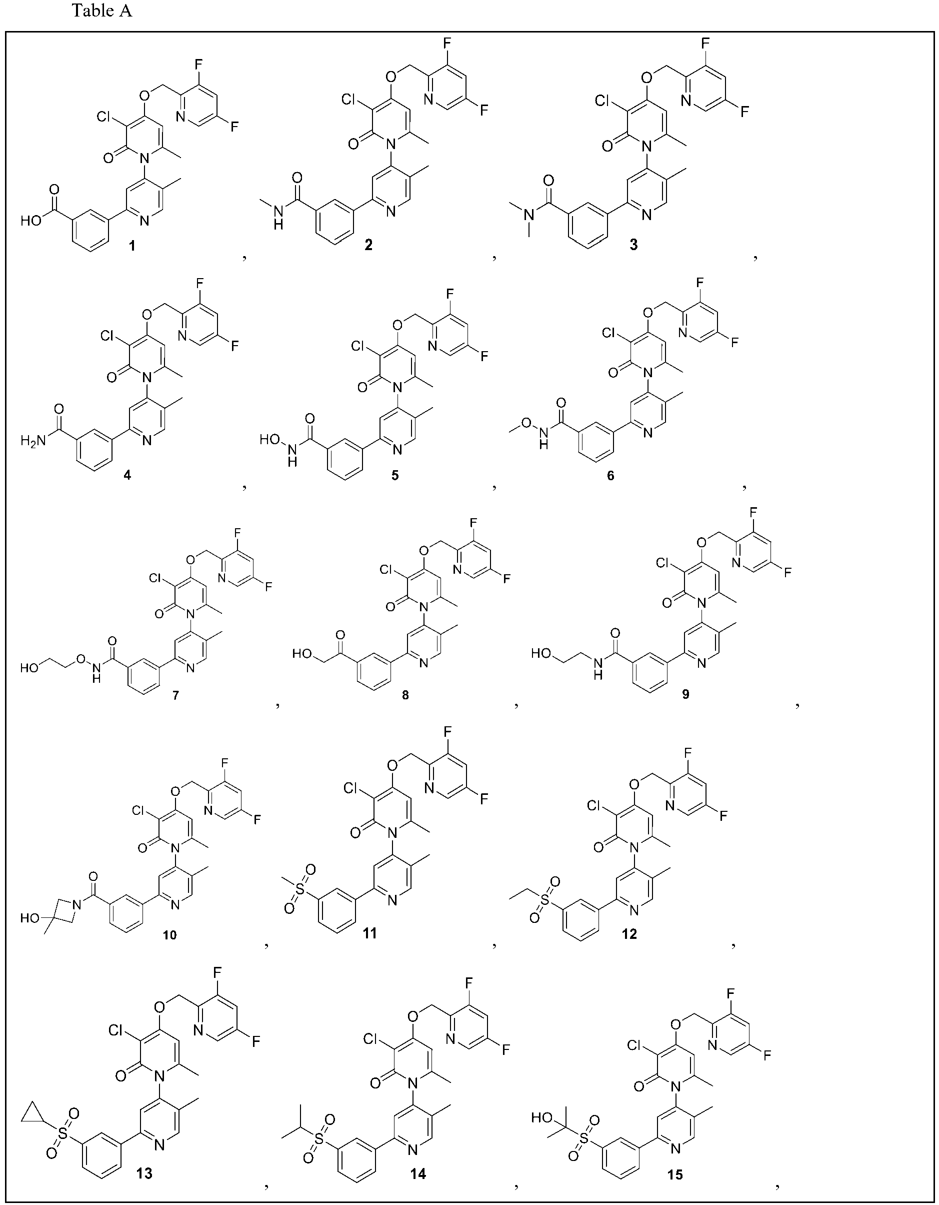

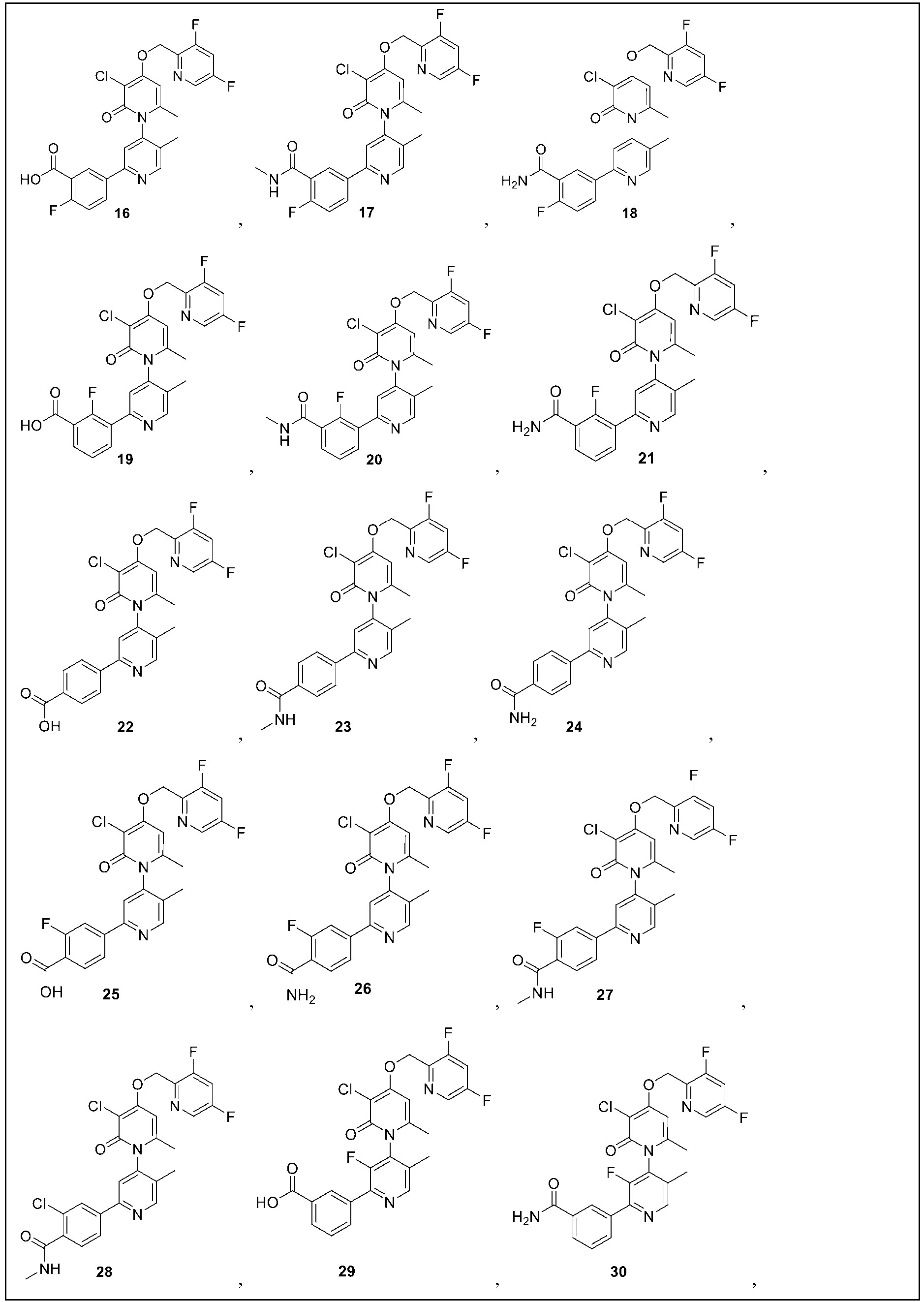

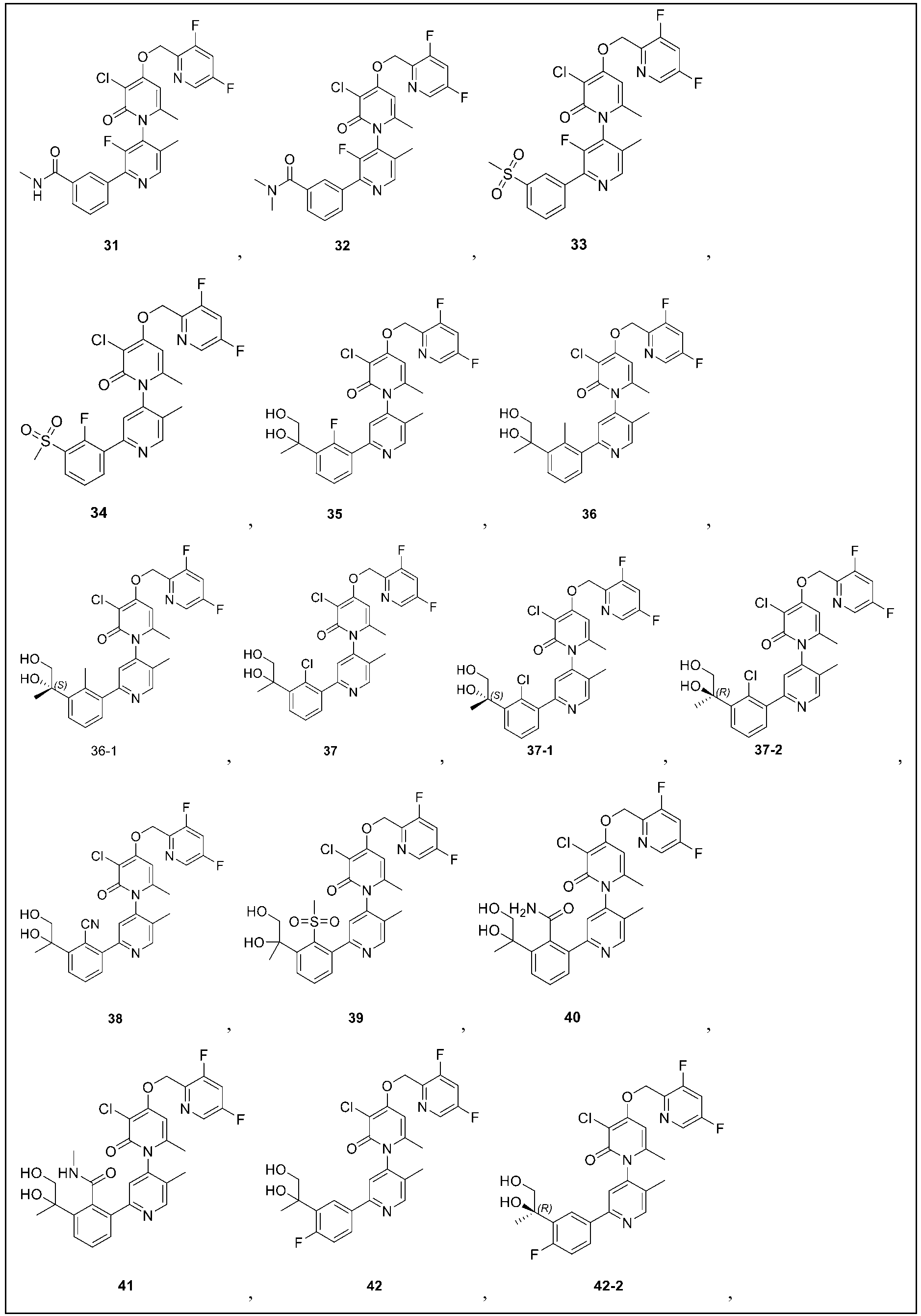

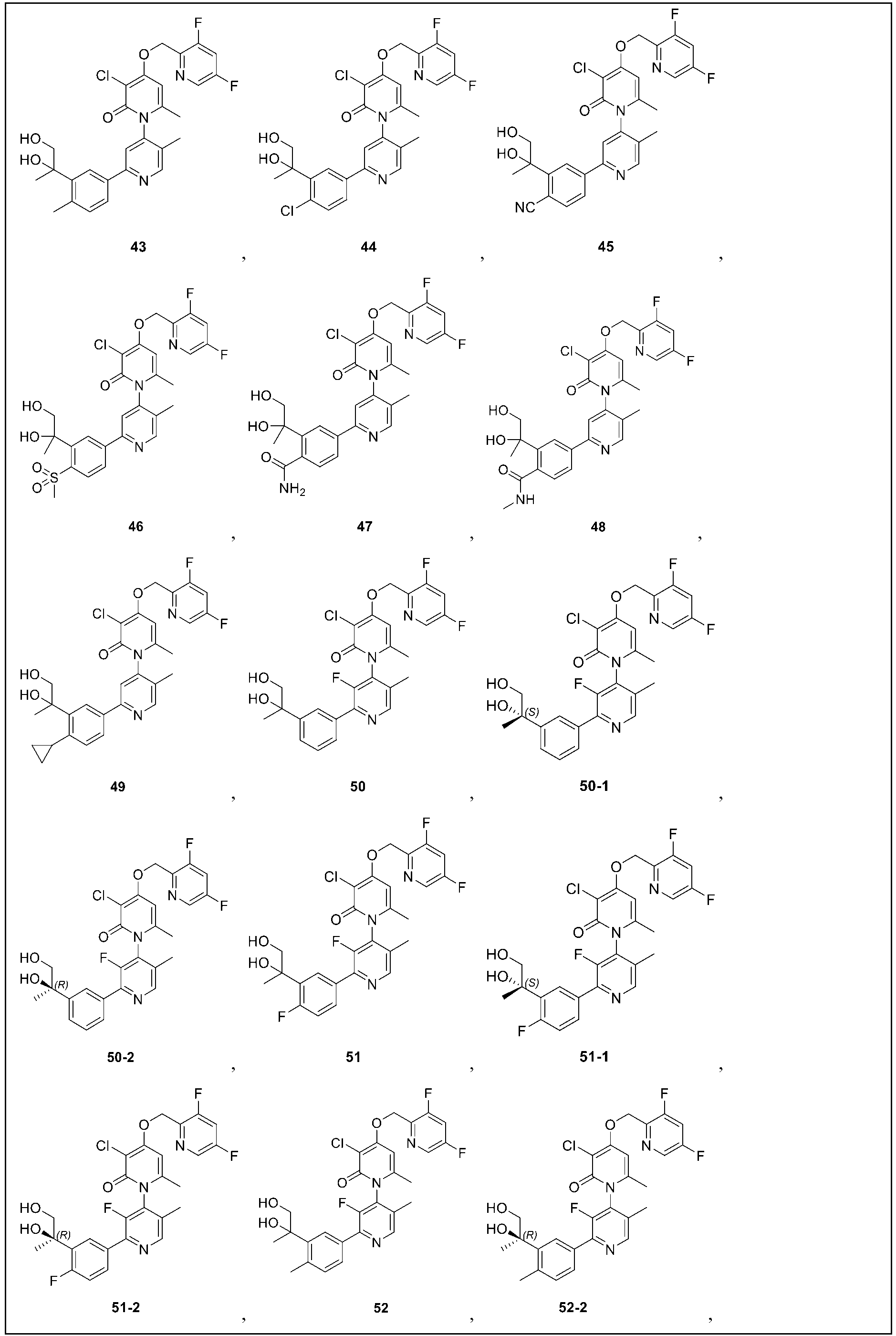

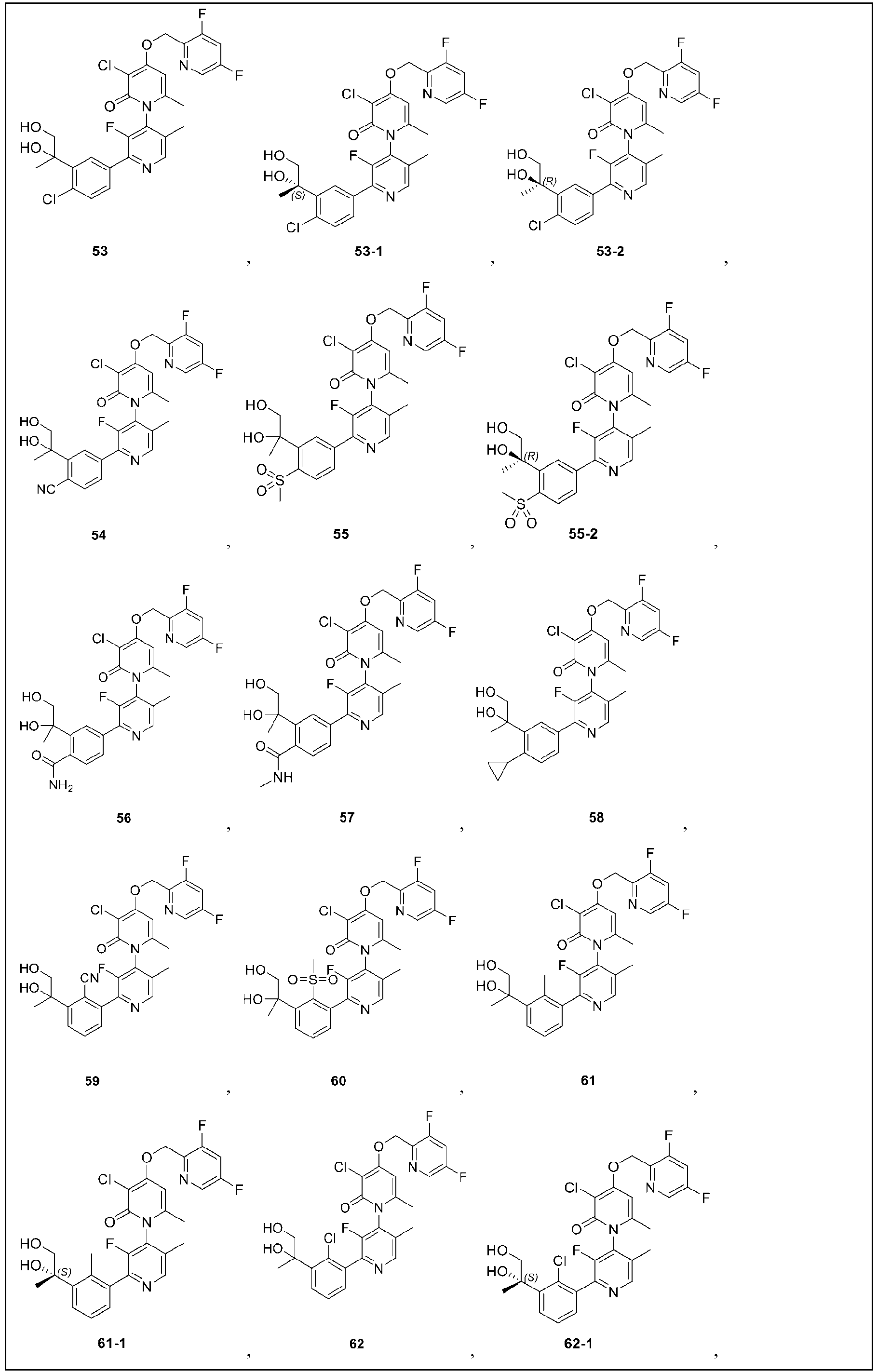

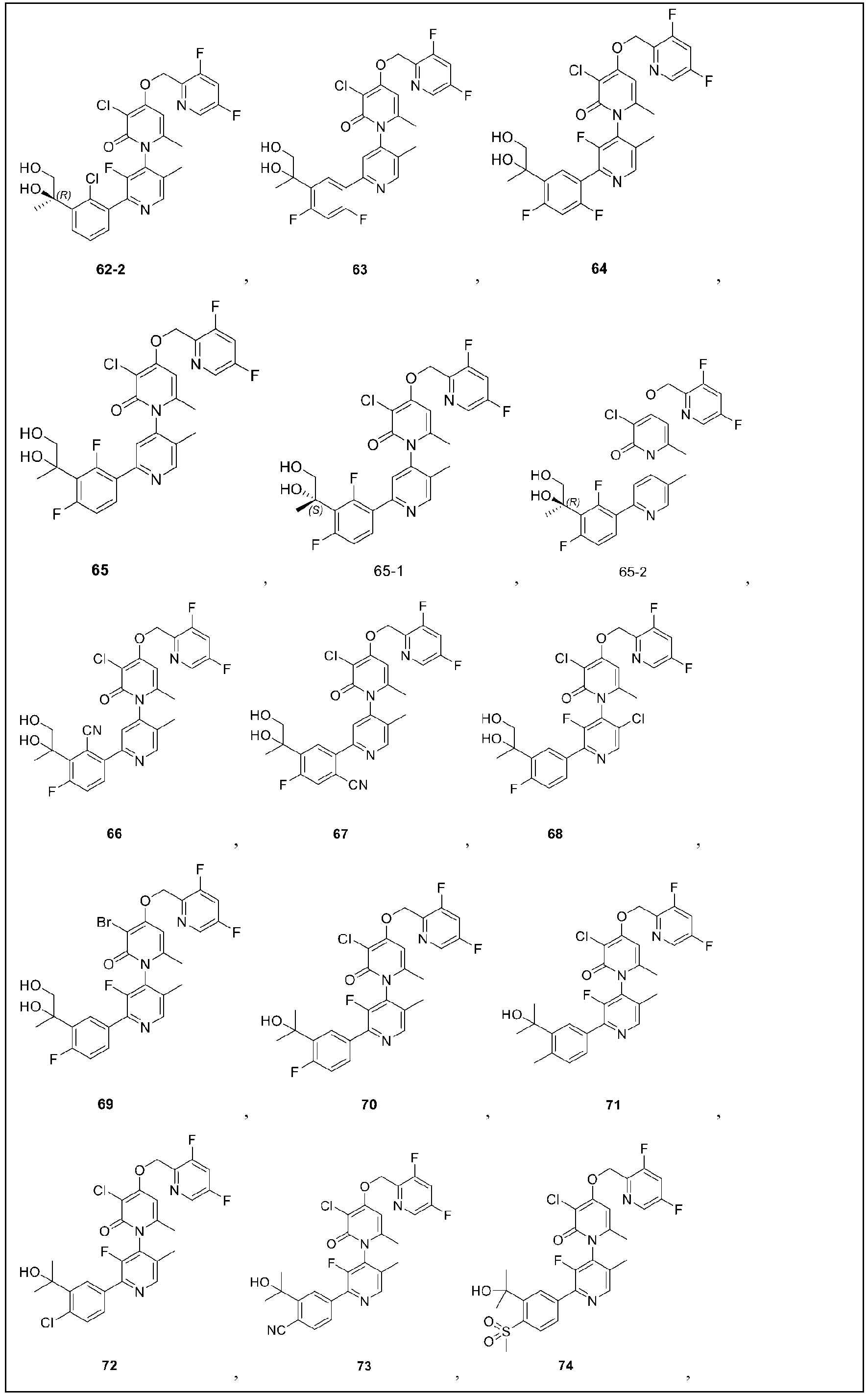

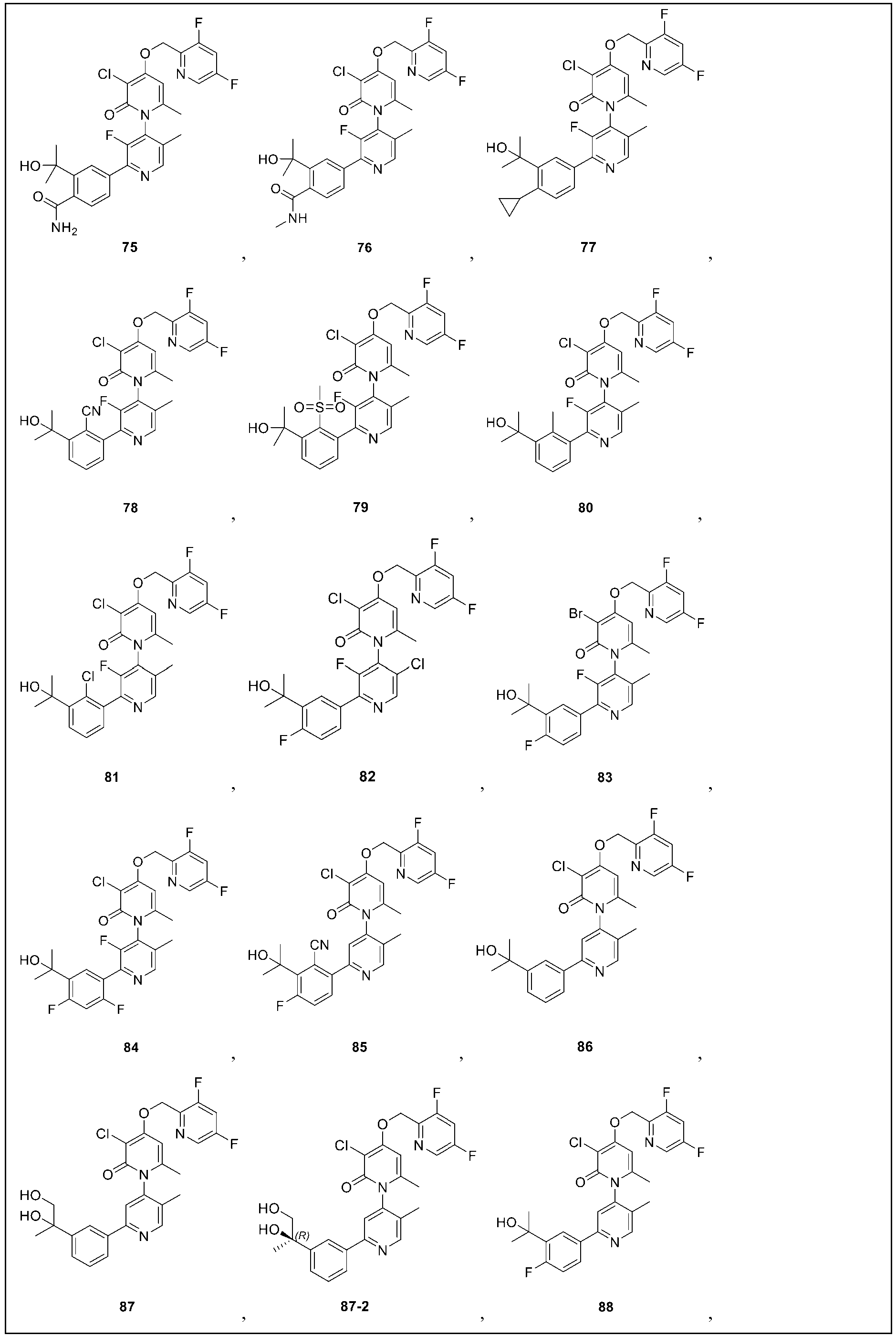

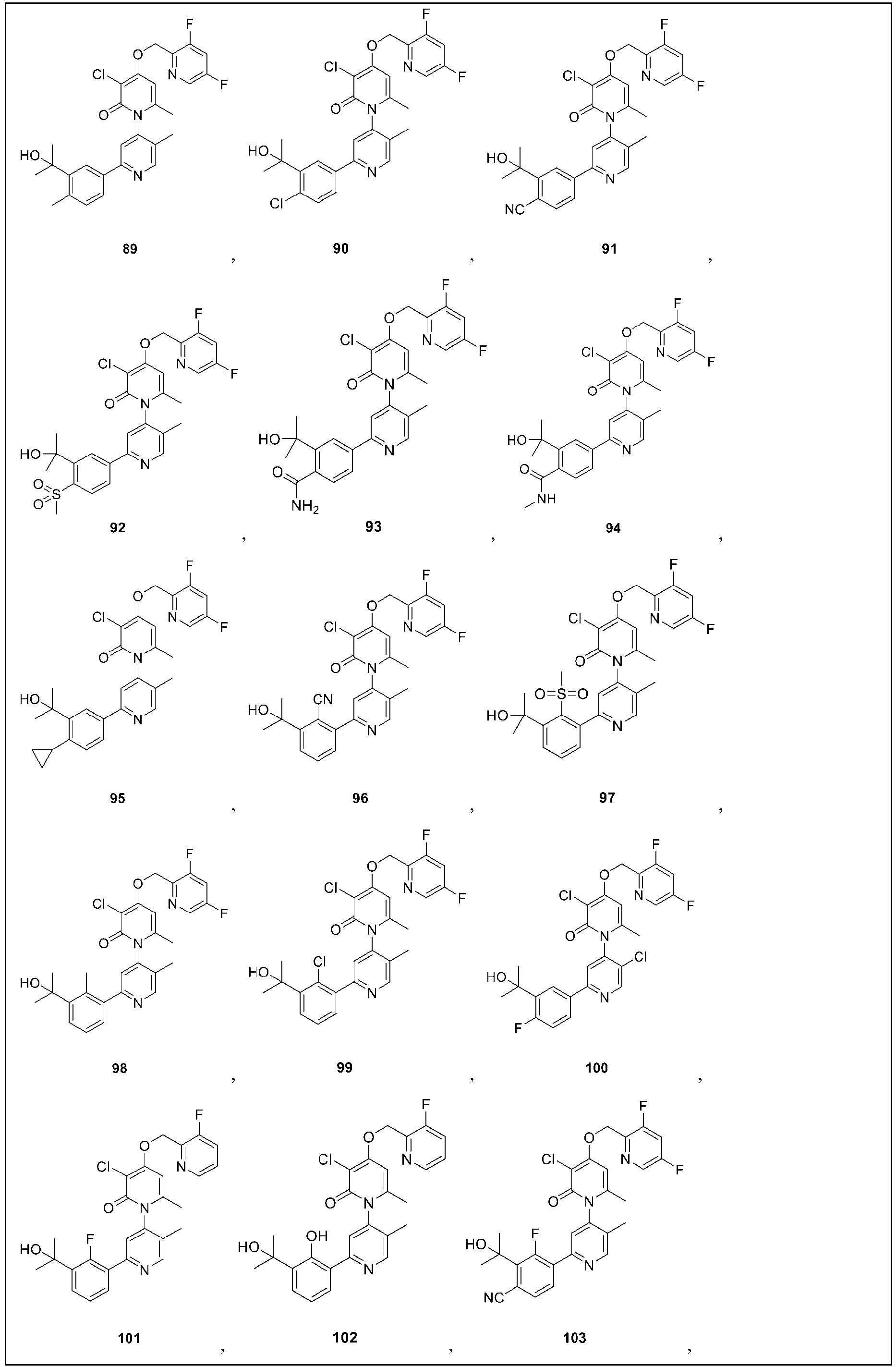

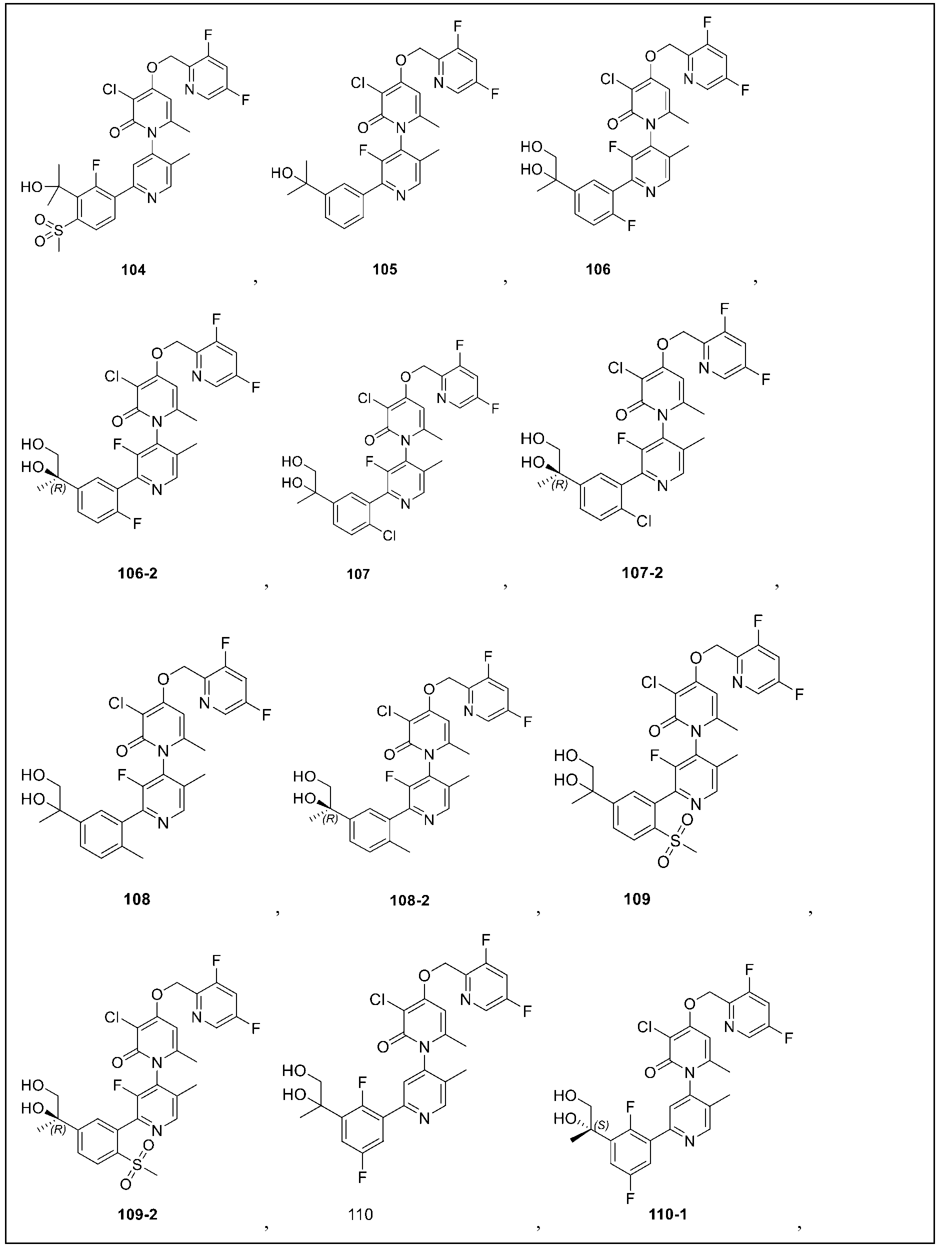

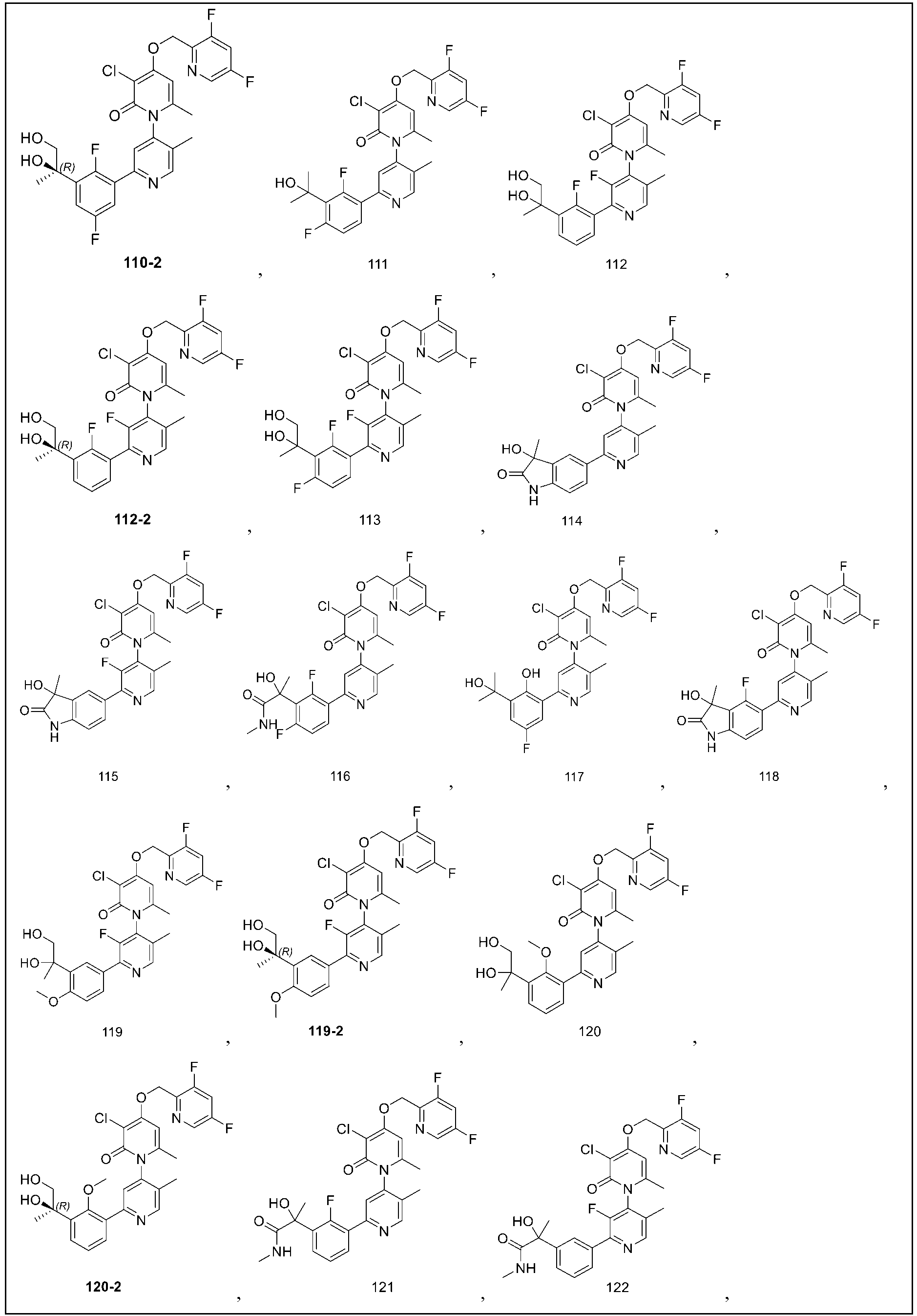

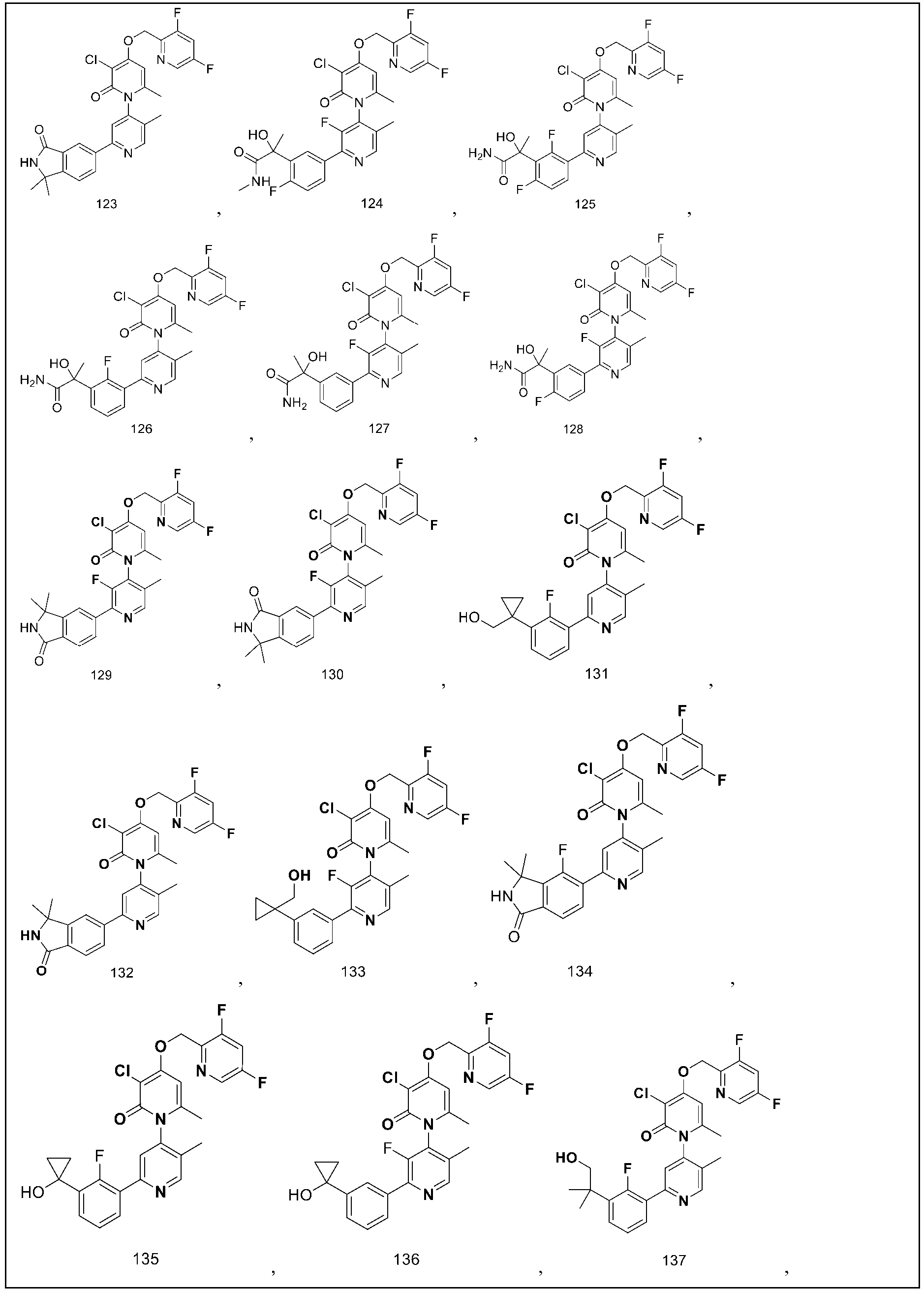

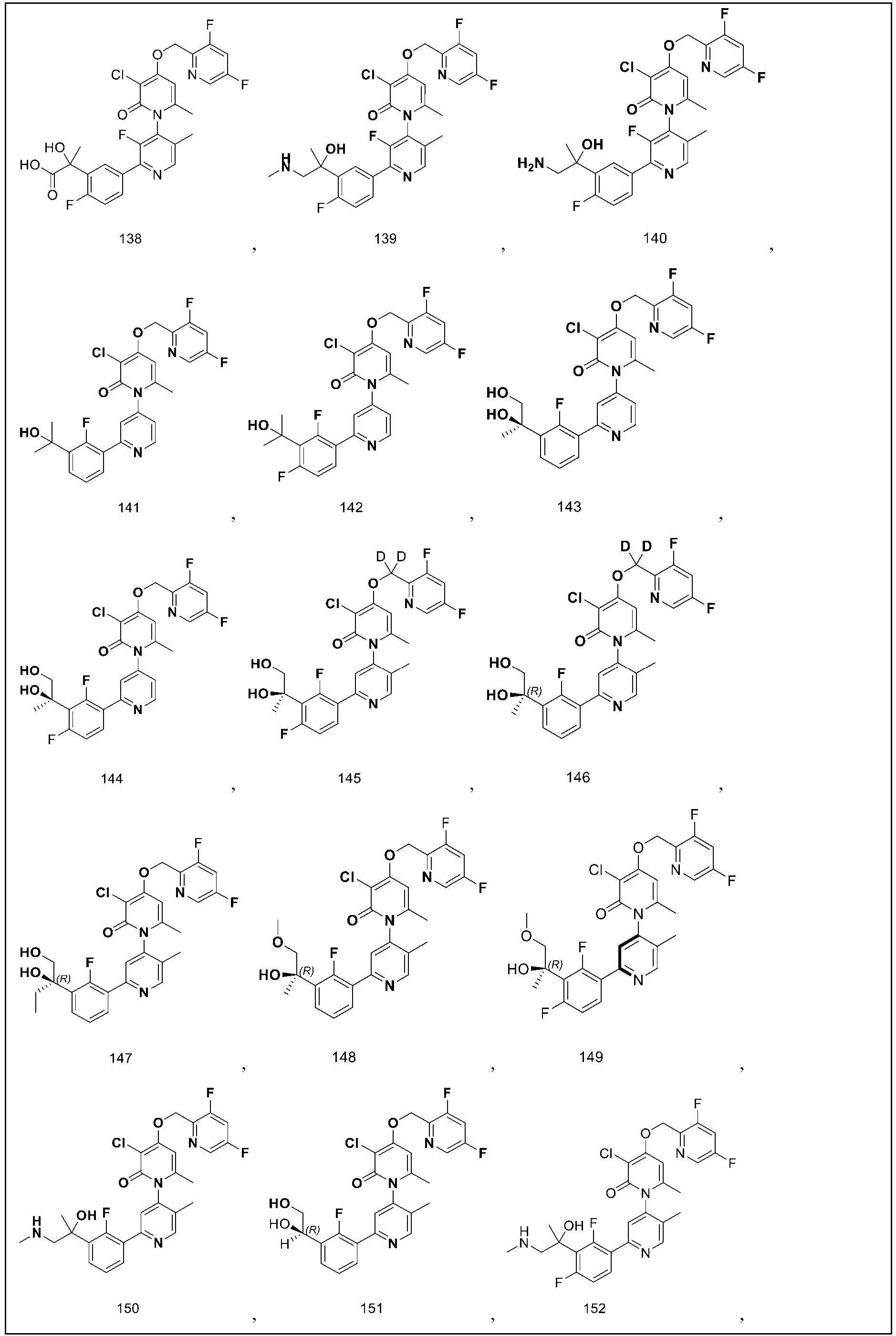

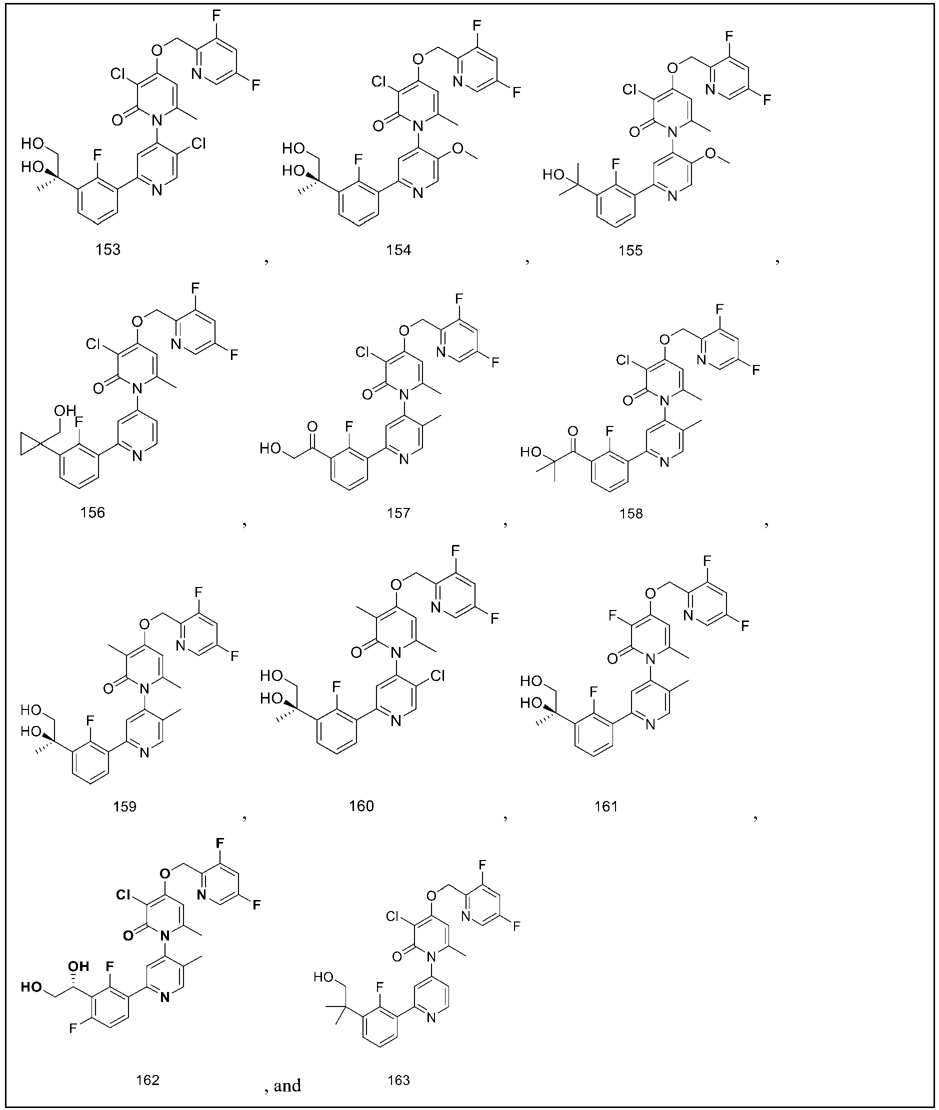

In some embodiments, exemplary specific compounds of compounds represented by formula (I), formula (II), formula (II-1), or formula (II-2) also include, but are not limited to, the structures listed in Table B below:

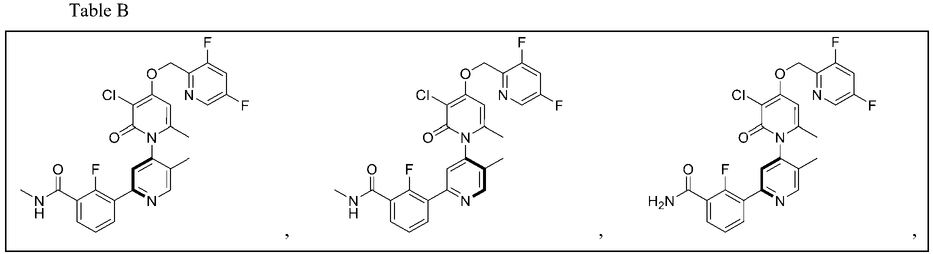

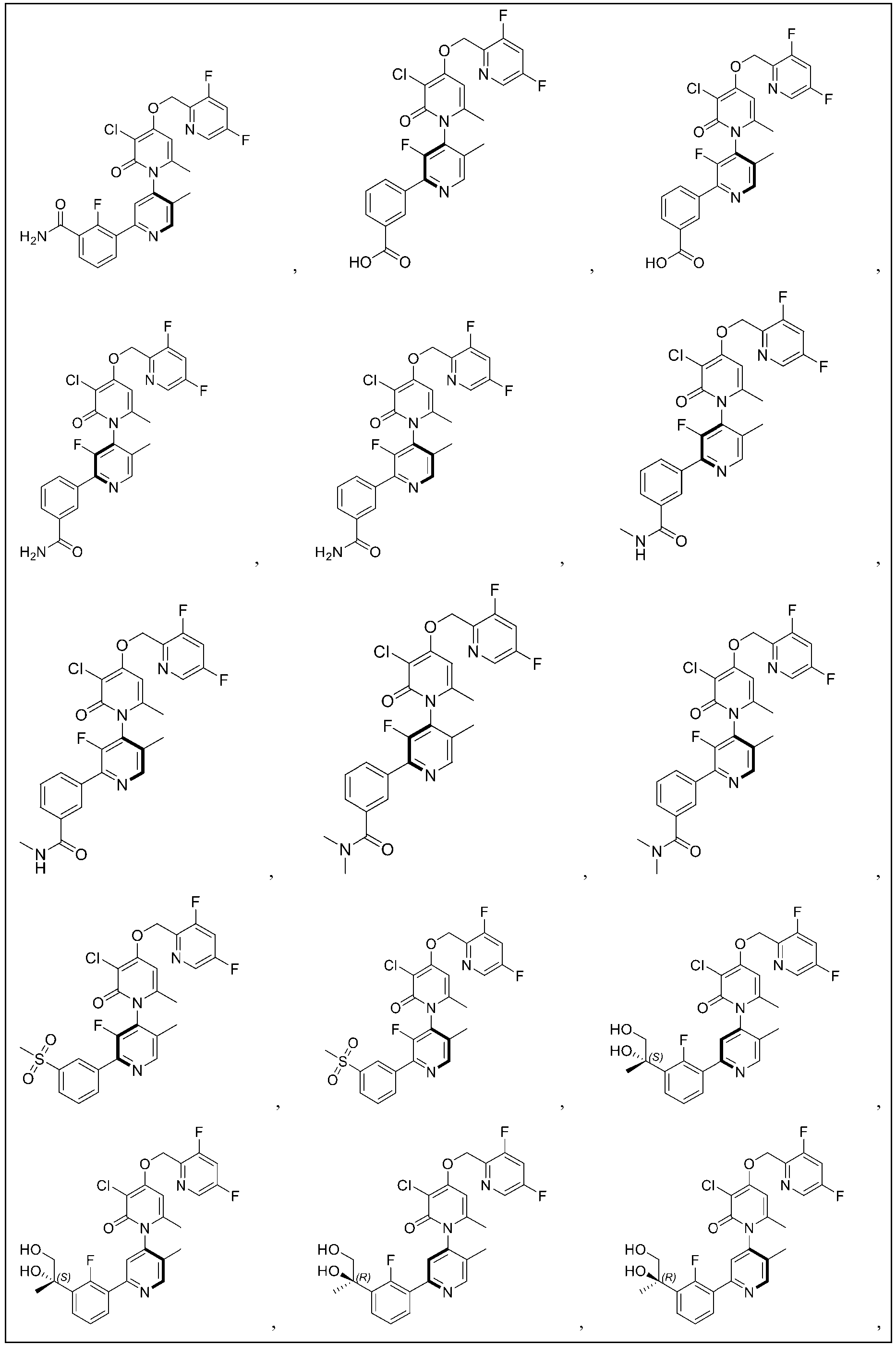

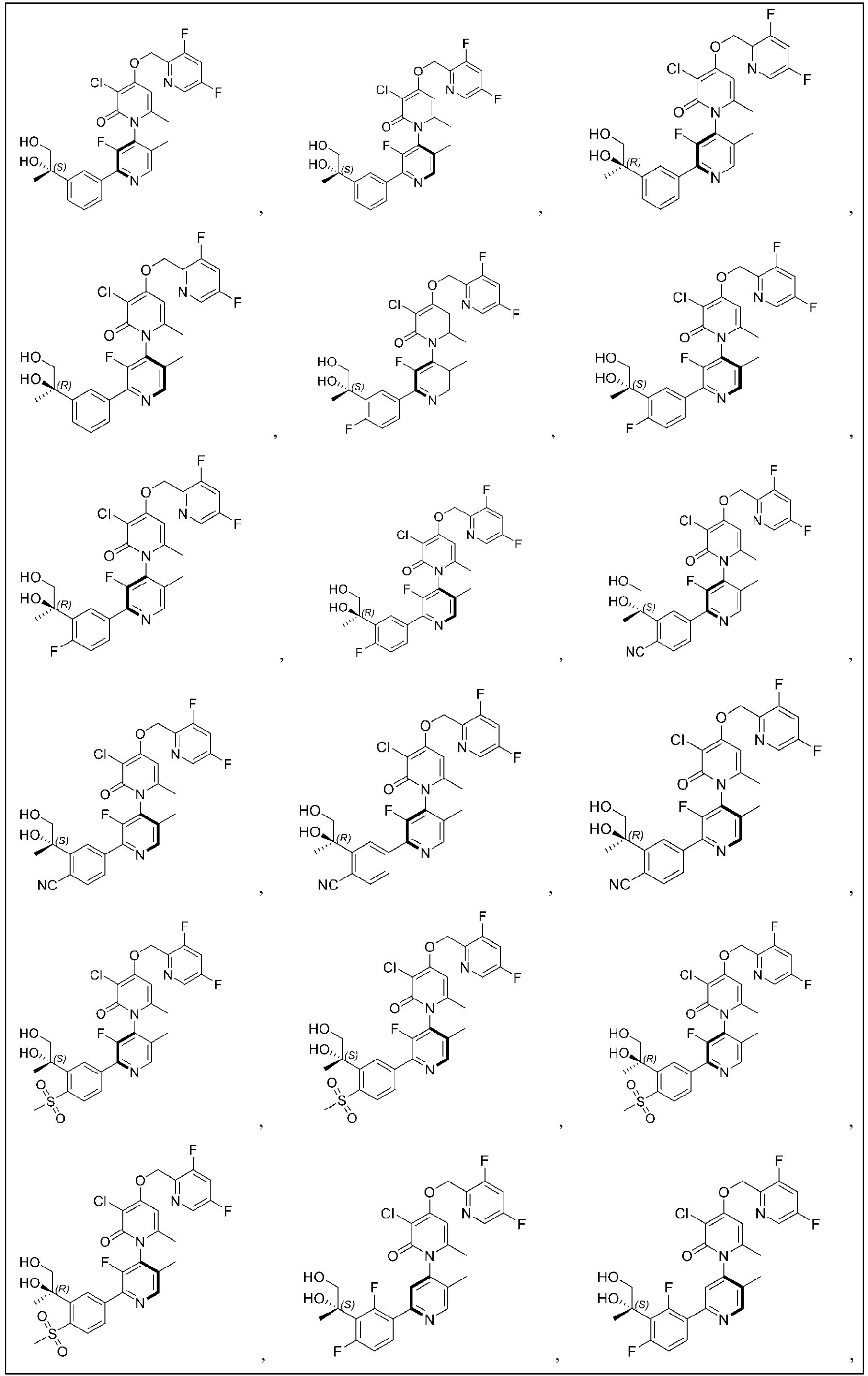

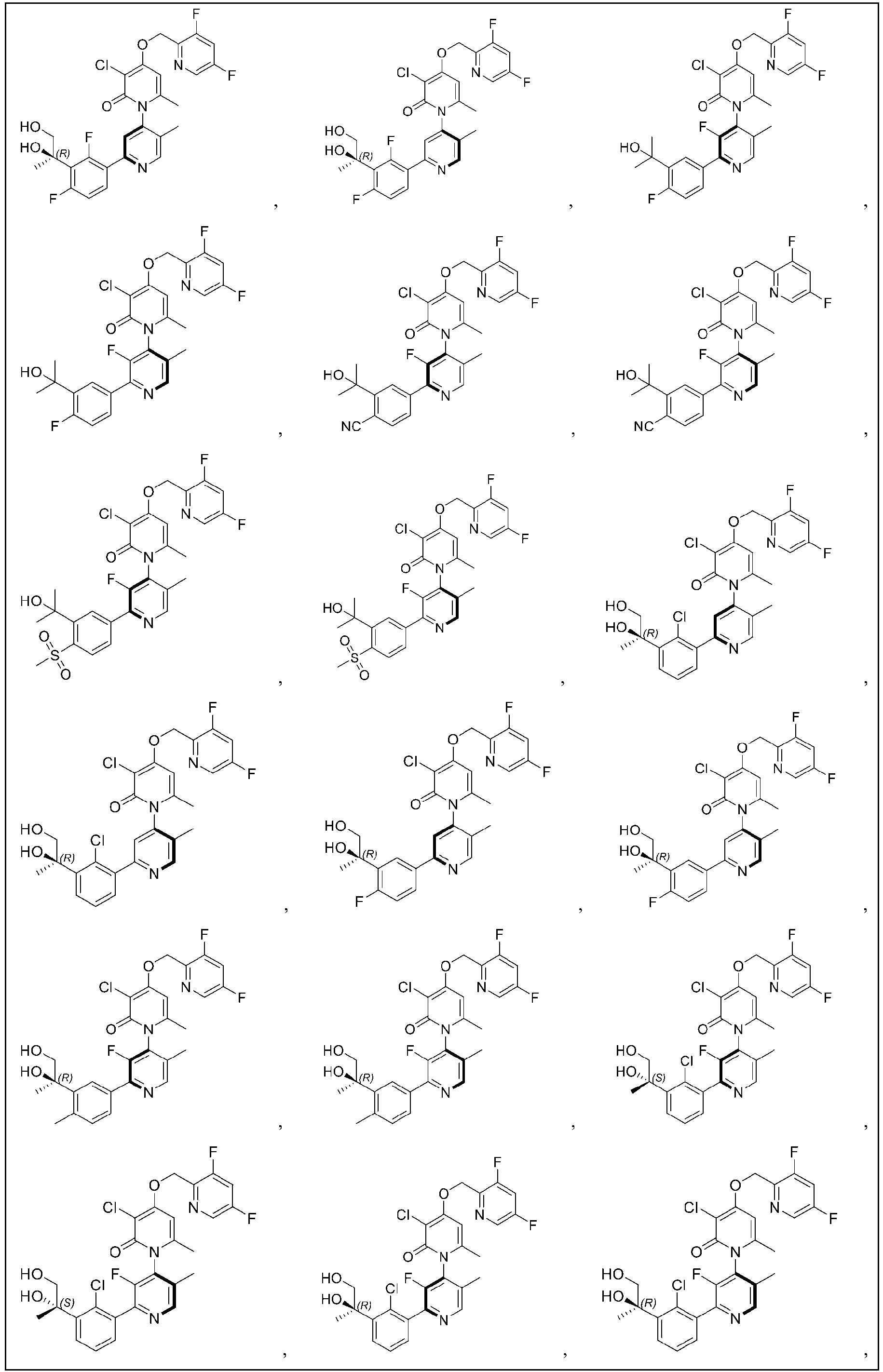

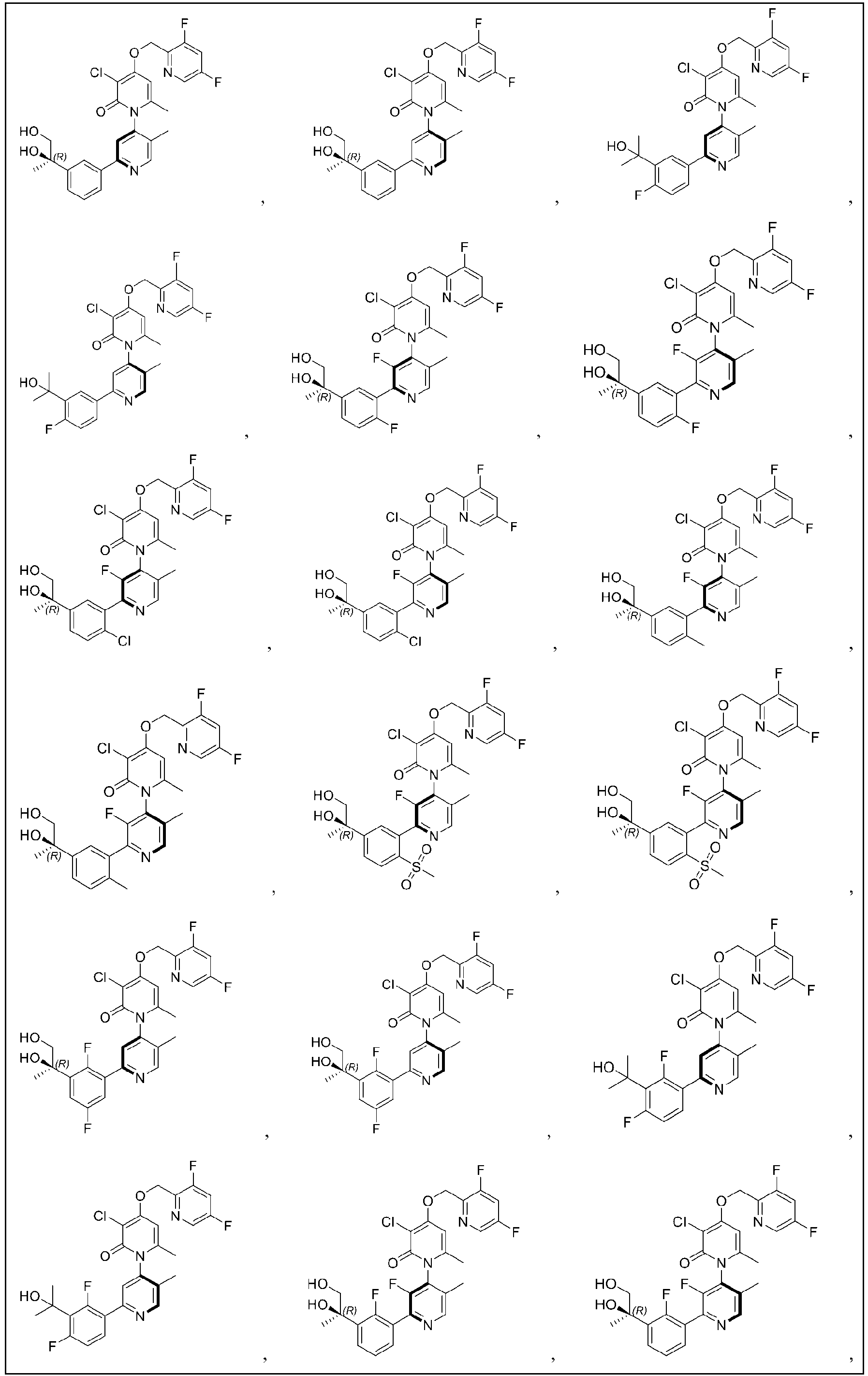

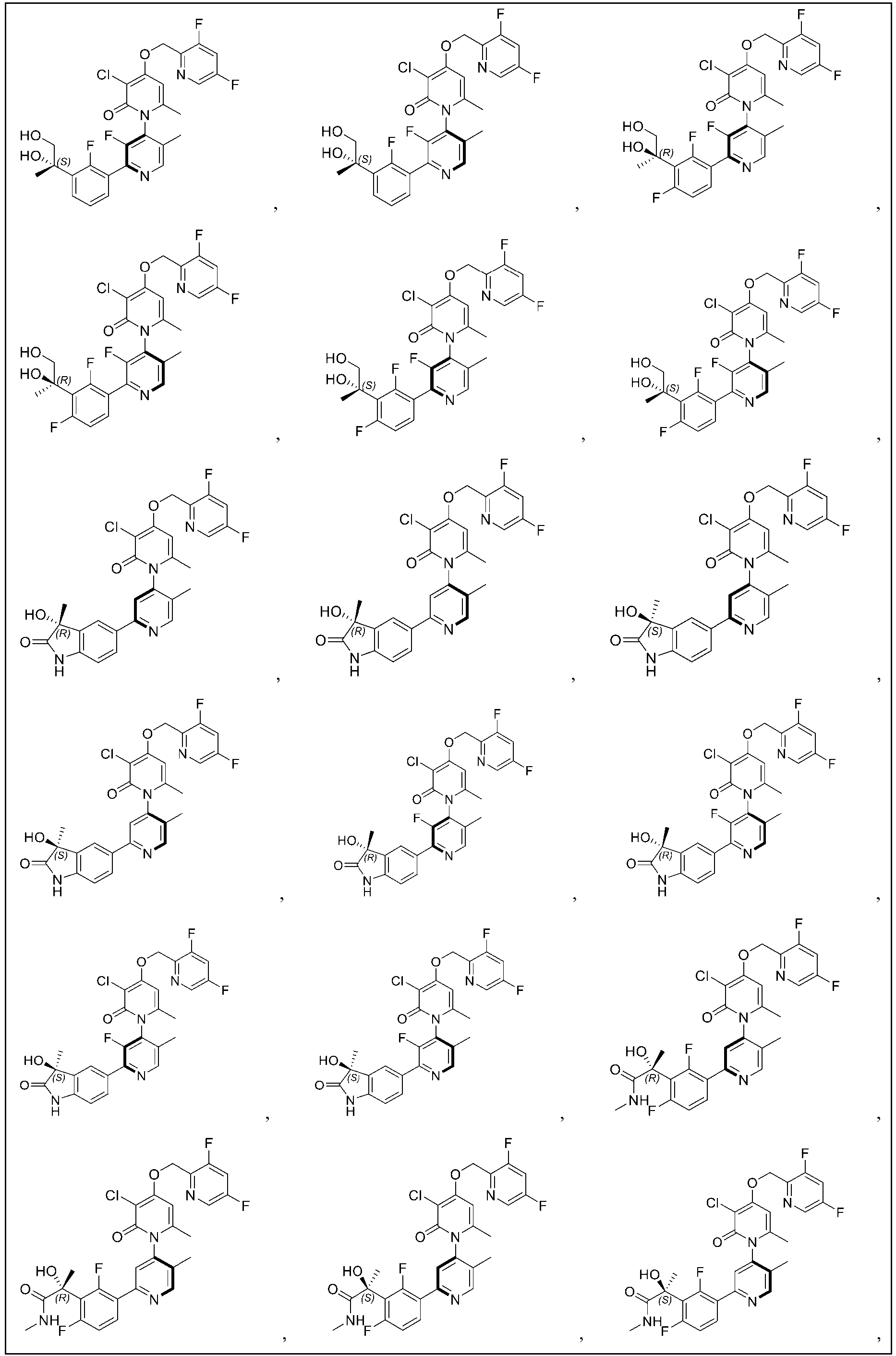

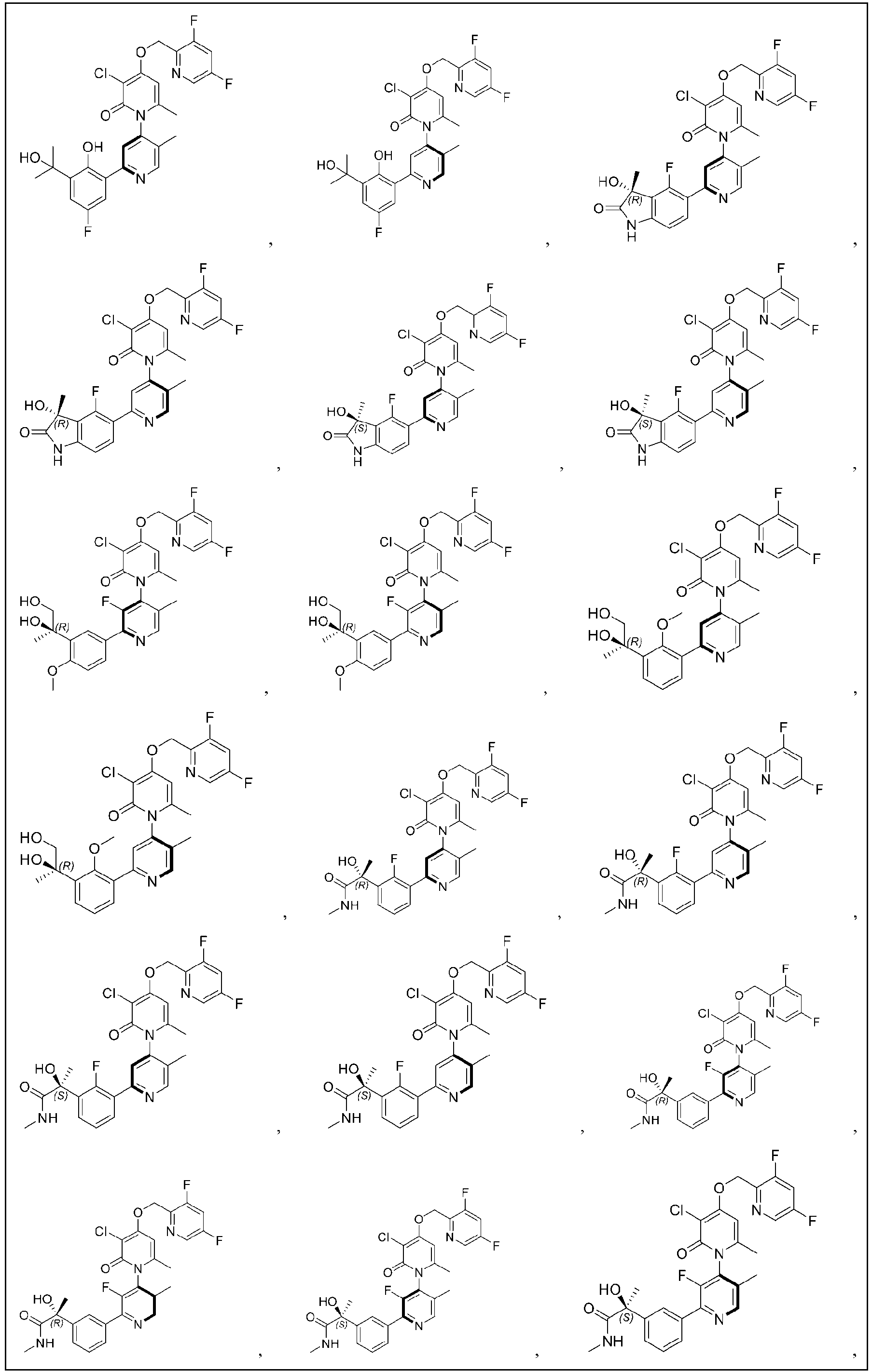

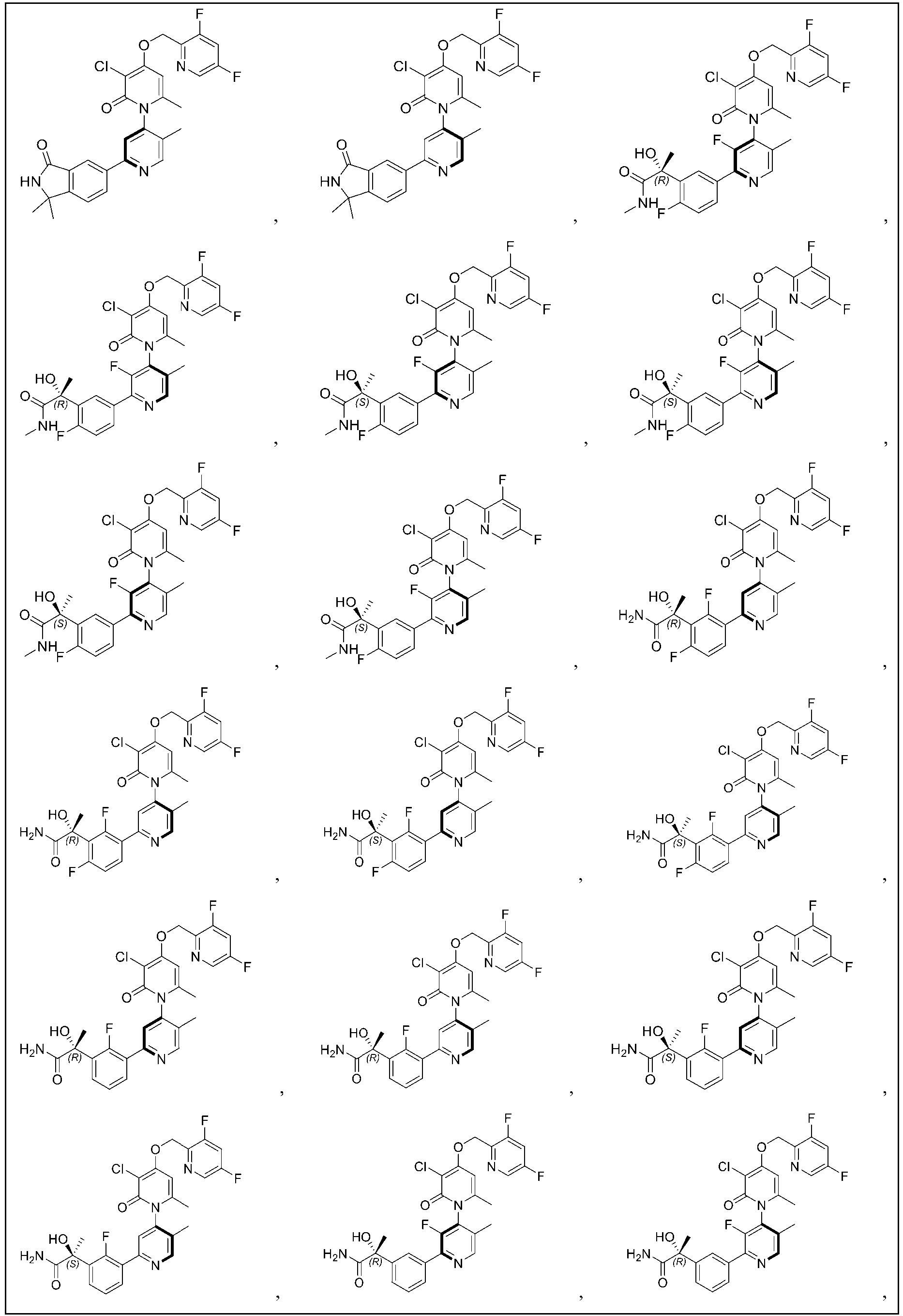

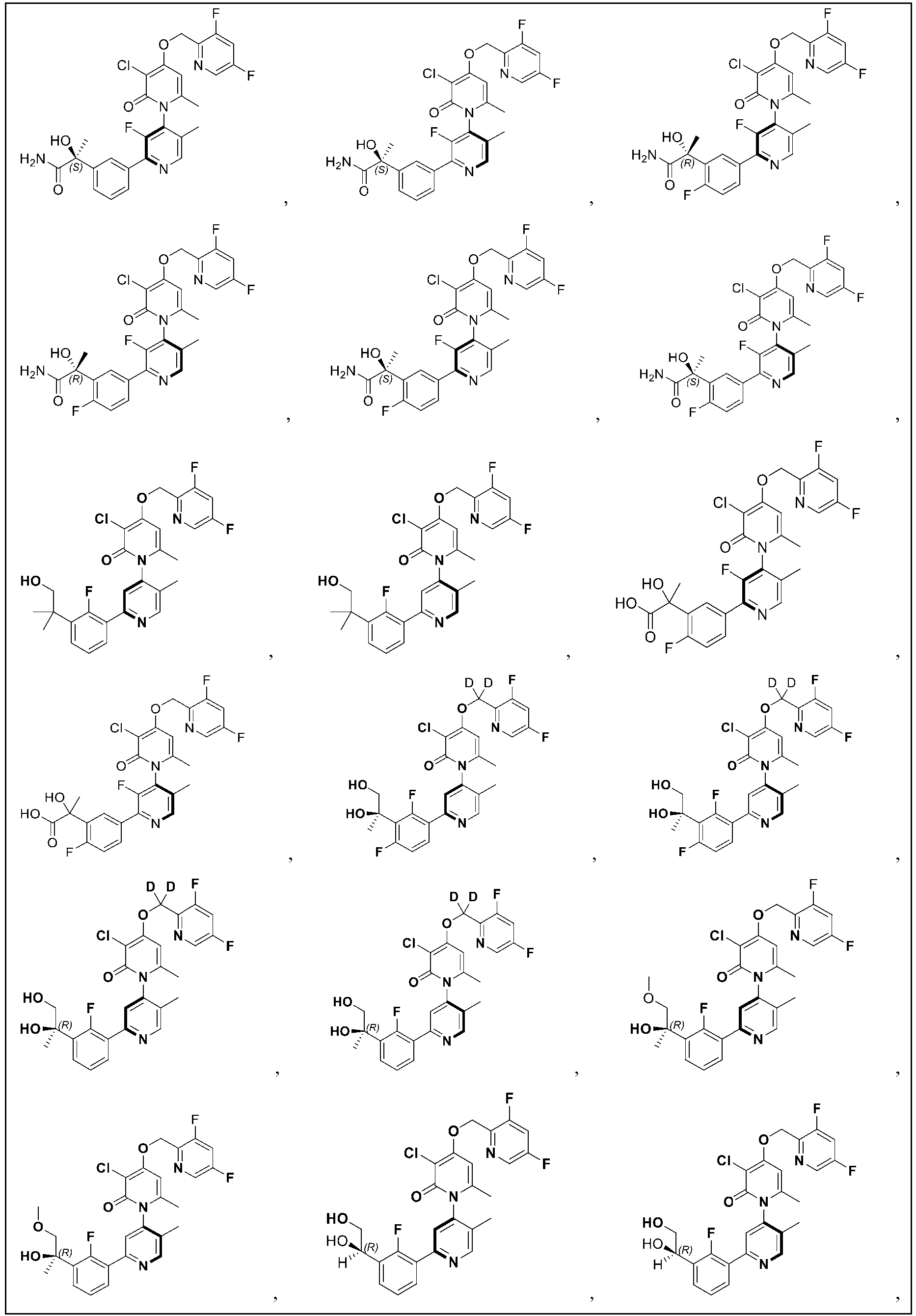

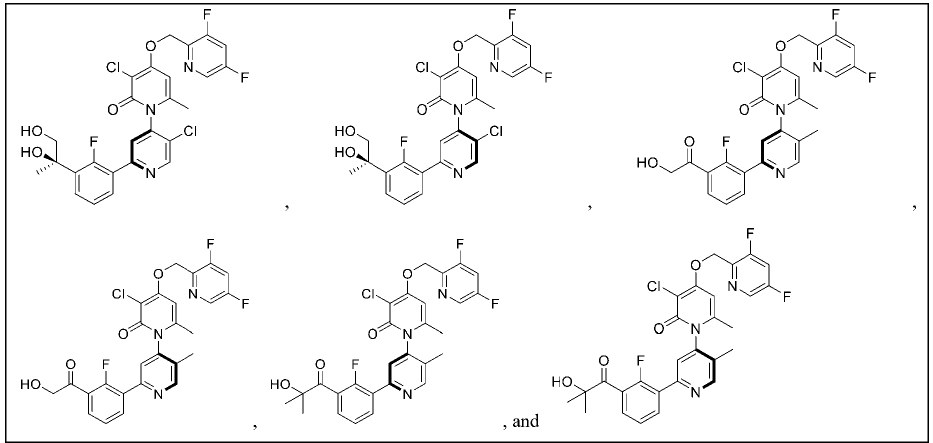

In another aspect of the present disclosure, the present disclosure provides an isotopically labeled compound of the compound represented by formula (I), formula (II), formula (II -1), formula (II-2), Table, A or Table B, wherein the isotopic labeling is preferably deuterium (D or ²H) substitution for hydrogen (¹H).

On the other hand, the present disclosure provides a method for preparing a compound represented by formula (I) or a pharmaceutically acceptable salt thereof, comprising the following steps: 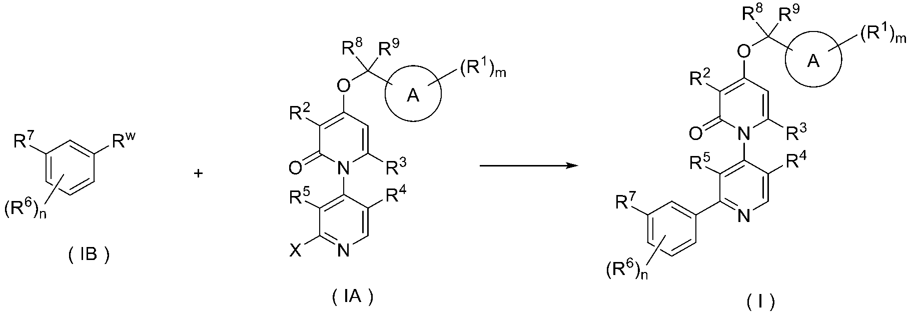
carrying out a coupling reaction on a compound represented by formula (IA) and a compound represented by formula (IB) in the presence of a catalyst to obtain the compound represented by formula (I) or a pharmaceutically acceptable salt thereof,
wherein X is halogen; preferably Cl;
R^{w} is -B(OH)₂ or 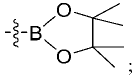
the definitions of ring A, R¹, R², R³, R⁴, R⁵, R⁶, R⁷, R⁸, R⁹, n, and m are as described in formula (I).

With reference to the method for preparing the compound represented by formula (I) or a pharmaceutically acceptable salt thereof, by replacing the compound represented by formula (IA) with a compound represented by formula (IIA) and then performing a coupling reaction with a compound represented by formula (IB), the compound represented by formula (II) or a pharmaceutically acceptable salt thereof can be obtained, and the structural formula of formula (IIA) is as follows: 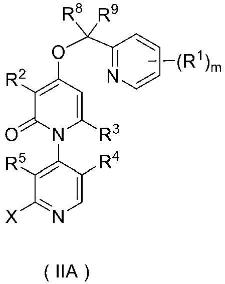 each group in the structural formula has the definitions as described above.

On the other hand, the present disclosure provides a pharmaceutical composition comprising at least one therapeutically effective amount of the compound or a pharmaceutically acceptable salt thereof, and one or more pharmaceutically acceptable excipients.

In another aspect, the present disclosure also provides a use of the compound represented by formula (I), formula (II), formula (II-1), formula (II-2), Table A, or Table B, or a pharmaceutically acceptable salt thereof, or the pharmaceutical composition comprising the same, in the manufacture of a medicament of a p38 kinase inhibitor.

The present disclosure also provides a use of the compound represented by formula (I), formula (II), formula (II-1), formula (II-2), Table A, or Table B, or a pharmaceutically acceptable salt thereof, or the pharmaceutical composition comprising the same, in the manufacture of a medicament for the prevention and/or treatment of a disease or disorder mediated by p38 kinase; preferably, the disease or disorder mediated by p38 kinase is a disease associated with p38 MAPK/MK2 pathway.

The present disclosure also provides a use of the compound represented by formula (I), formula (II), formula (II-1), formula (II-2), Table A, or Table B, or a pharmaceutically acceptable salt thereof, or the pharmaceutical composition comprising the same, in the manufacture of a medicament for the prevention and/or treatment of autoimmune diseases, inflammatory diseases, cardiovascular diseases, central nervous system diseases, and cancer; preferably, in the manufacture of a medicament for the prevention and/or treatment of arthritis, psoriasis, systemic lupus erythematosus, diabetes, leukemia, lymphoma, atherosclerosis, and Alzheimer's disease.

The present disclosure also provides a method for inhibiting p38 kinase, comprising administering to a patient in need thereof a therapeutically effective amount of the compound represented by formula (I), formula (II), formula (II-1), formula (11-2), Table A, or Table B, or a pharmaceutically acceptable salt thereof, or an isotopically labeled compound thereof, or the pharmaceutical composition comprising the same.

The present disclosure also provides a method for preventing and/or treating a disease or disorder mediated by p38 kinase, comprising administering to a patient in need thereof a therapeutically effective amount of the compound represented by formula (I), formula (II), formula (II-1), formula (II-2), Table A, or Table B, or a pharmaceutically acceptable salt thereof, or an isotopically labeled compound thereof, or the pharmaceutical composition comprising the same; preferably, the disease or disorder mediated by p38 kinase is a disease associated with p38 MAPK/MK2 pathway.

The present disclosure also provides a method for preventing and/or treating autoimmune diseases, inflammatory diseases, cardiovascular diseases, central nervous system diseases, and cancer, comprising administering to a patient in need thereof a therapeutically effective amount of the compound represented by formula (I), formula (II), formula (II-1), formula (II-2), Table A, or Table B, or a pharmaceutically acceptable salt thereof, or the pharmaceutical composition comprising the same.

The present disclosure also provides a method for preventing and/or treating arthritis, psoriasis, systemic lupus erythematosus, diabetes, leukemia, lymphoma, atherosclerosis, and Alzheimer's disease, comprising administering to a patient in need thereof a therapeutically effective amount of the compound represented by formula (I), formula (II), formula (II-1), formula (II-2), Table A, or Table B, or a pharmaceutically acceptable salt thereof, or the pharmaceutical composition comprising the same.

The present disclosure also provides the compound represented by formula (I), formula (II), formula (II-1), formula (II-2), Table A, or Table B, or a pharmaceutically acceptable salt thereof, or the pharmaceutical composition, for use as a medicament.

The present disclosure also provides the compound represented by formula (I), formula (II), formula (II-1), formula (II-2), Table A, or Table B, or a pharmaceutically acceptable salt thereof, or the pharmaceutical composition, for use as a p38 kinase inhibitor.

The present disclosure also provides the compound represented by formula (I), formula (II), formula (II-1), formula (II-2), Table A, or Table B, or a pharmaceutically acceptable salt thereof, or the pharmaceutical composition containing the same, for use in the prevention and/or treatment of a disease or disorder mediated by p38 kinase; preferably, the disease or disorder mediated by p38 kinase is a disease associated with p38 MAPK/MK2 pathway.

The present disclosure also provides the compound represented by formula (I), formula (II), formula (II-1), formula (II-2), Table A, or Table B, or a pharmaceutically acceptable salt thereof, or the pharmaceutical composition comprising the same, for use in the prevention and/or treatment of autoimmune diseases, inflammatory diseases, cardiovascular diseases, central nervous system diseases, and cancer.

The present disclosure also provides the compound represented by formula (I), formula (II), formula (II-1), formula (II-2), Table A, or Table B, or a pharmaceutically acceptable salt thereof, or the pharmaceutical composition comprising the same, for use in the prevention and/or treatment of arthritis, psoriasis, systemic lupus erythematosus, diabetes, leukemia, lymphoma, atherosclerosis, and Alzheimer's disease.

The disease mediated by p38 kinase of the present disclosure is selected from the group consisting of autoimmune diseases, inflammatory diseases, cardiovascular diseases, central nervous system diseases, and cancer.

The disease mediated by p38 kinase of the present disclosure is selected from the group consisting of arthritis, psoriasis, systemic lupus erythematosus, diabetes, leukemia, lymphoma, atherosclerosis, and Alzheimer's disease.

In some embodiments, the unit dose of the pharmaceutical composition is 0.001 mg to 1000 mg.

In some embodiments, based on the total weight of the composition, the pharmaceutical composition comprises 0.01-99.99% of the compound represented by formula (I), formula (II), formula (II-1), formula (II-2), Table A, or Table B, or a pharmaceutically acceptable salt thereof, or an isotopically labeled compound thereof. In some embodiments, the pharmaceutical composition comprises 0.1-99.9% of the compound represented by formula (I), formula (II), formula (II-1), formula (II-2), Table A, or Table B, or a pharmaceutically acceptable salt thereof, or an isotopically labeled compound thereof. In some embodiments, the pharmaceutical composition comprises 0.5%-99.5% of the compound represented by formula (I), formula (II), formula (II-1), formula (II-2), Table A, or Table B, or a pharmaceutically acceptable salt thereof, or an isotopically labeled compound thereof. In some embodiments, the pharmaceutical composition comprises 1%-99% of the compound represented by formula (I), formula (II), formula (II-1), formula (II-2), Table A, or Table B, or a pharmaceutically acceptable salt thereof, or an isotopically labeled compound thereof.

In some embodiments, based on the total weight of the composition, the pharmaceutical composition comprises 0.01%-99.99% of one or more pharmaceutically acceptable excipients. In some embodiments, the pharmaceutical composition comprises 0.1%-99.9% of one or more pharmaceutically acceptable excipients. In some embodiments, the pharmaceutical composition comprises 1%-99% of one or more pharmaceutically acceptable excipients.

When used as a medicament, the compound of the present disclosure may be administered in the form of a pharmaceutical composition. These compositions can be prepared in a manner well-known in the pharmaceutical field and may be administered via various routes, depending on whether local or systemic treatment is required and the area being treated. These compositions can be administered locally (*e.g*., transdermal, dermal, ocular, and mucosal routes including intranasal, vaginal, and rectal delivery), pulmonarily (*e.g*., via inhalation or insufflation of powders or aerosols, including by nebulizer; intratracheal, intranasal), orally, or parenterally. Parenteral administration includes intravenous, intra-arterial, subcutaneous, intraperitoneal, or intramuscular injection or infusion; or intracranial administration such as intrathecal or intraventricular administration. These compositions can be administered parenterally in a single large dose or via a continuous infusion pump.

In the preparation of the compositions of the present disclosure, the active ingredient is typically mixed with excipients, and the compositions may be in the following forms: tablets, pills, powders, lozenges, sachets, cachets, elixirs, suspensions, emulsions, solutions, syrups, aerosols (solid or dissolved in a liquid vehicle), ointments containing, for example, up to 10% by weight of the active compound, soft and hard gelatin capsules, suppositories, sterile injectable solutions, and sterile packaged powders.

The "excipients" of the present disclosure refer to components other than the active ingredients, such as diluents, fillers, absorbents, wetting agents, binders, disintegrants, and lubricants.

On the other hand, the pharmaceutically acceptable salts of the compounds of the present disclosure may be inorganic salts or organic salts. If these compounds have a basic center, they can form acid addition salts; if these compounds have an acidic center, they can form base addition salts; and if these compounds include both an acidic center (*e.g*., carboxyl) and a basic center (*e.g*., amino), they may also form inner salts.

On the other hand, the compounds of the present disclosure may exist in specific geometric or stereoisomeric forms. For example, *cis* and *trans* isomers, (-)- and (+)- enantiomers, (*R*)- and (*S*)- enantiomers, diastereomers, (*D*)-isomers, (*L*)-isomers, racemic mixtures and other mixtures, as well as enantiomerically or diastereomerically enriched mixtures, all of which fall within the scope of the present disclosure. Additional asymmetric carbon atoms may be present in substituents such as alkyl. All these isomers and their mixtures are included within the scope of the present disclosure.

In the chemical structures of the compounds of the present disclosure, the bond " 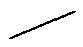 " indicates unspecified configuration, " 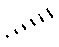 " or " 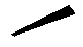 " indicates absolute configuration, *i.e.,* if chiral isomers exist in the chemical structure, the bond " 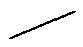 " may be " 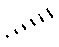 " or " 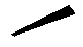 ", or simultaneously include both configurations " 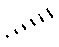 " and " 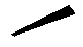 ", and " 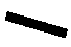 " indicates the presence of axial chirality.

The bond " 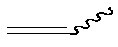 " indicates an unspecified configuration, including the *cis* (*E*) or *trans* (*Z*) configuration.

Additionally, the compounds and intermediates of the present disclosure may also exist in various tautomeric forms, all of which are encompassed within the scope of the present disclosure. "Tautomers" refer to structural isomers with different energies that can interconvert via low-energy barriers. For example, proton tautomers (also known as proton-transfer tautomers) include interconversion through proton migration, such as keto-enol isomerization, imine-enamine isomerization, and lactam-lactim isomerization. All tautomeric forms of all compounds of the present disclosure are within the scope of the present disclosure. The name of a compound designated by a single method does not exclude any tautomers.

The present disclosure also includes isotopically labeled compounds of the present disclosure, which are identical in structure to those described herein, except that one or more atoms are replaced by atoms having an atomic weight or mass number different from the atomic weight or mass number typically found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl, respectively. All isotopic variations of the compounds of the present disclosure, whether radioactive or not, are encompassed within the scope of the present disclosure.

Unless otherwise indicated, when a position is specifically designated as deuterium (D), it shall be understood to have a deuterium abundance at least 1000 times greater than the natural abundance of deuterium (which is 0.015%) (*i.e.*, at least 10% deuterium incorporation). The abundance of deuterium in the compound in the example is greater than the natural abundance of deuterium, which can be at least 1000 times the natural abundance of deuterium, at least 2000 times the natural abundance of deuterium, at least 3000 times the natural abundance of deuterium, at least 4000 times the natural abundance of deuterium, at least 5000 times the natural abundance of deuterium, at least 6000 times the natural abundance of deuterium, or even higher. Each available hydrogen atom attached to a carbon atom can be independently replaced by a deuterium atom. A person skilled in the art can synthesize deuterated forms of the compounds by referring to relevant literature. In the preparation of deuterated forms of compounds, commercially available deuterated starting materials can be used, or they can be synthesized using conventional techniques with deuterated reagents, including but not limited to deuterated borane, trideuterated borane tetrahydrofuran solution, deuterated lithium aluminum hydride, deuterated iodoethane, and deuterated iodomethane.

The "therapeutically effective amount" of the present disclosure refers to the amount of an active compound or medicament sought by researchers, veterinarians, physicians, or other clinicians to elicit a biological or medical response in tissues, systems, animals, individuals, or humans, including one or more of the following: (1) Disease prevention: for example, preventing a disease, disorder, or condition in individuals susceptible to the disease, disorder, or condition but who have not yet experienced or exhibited the pathology or symptoms of the disease. (2) Disease inhibition: for example, suppressing a disease, disorder, or condition in individuals currently experiencing or exhibiting pathology or symptoms of the disease, disorder, or condition (i.e., halting further progression of the pathology and/or symptoms). (3) Disease alleviation: for example, alleviating a disease, disorder, or condition in individuals presently experiencing or exhibiting pathology or symptoms of the disease, disorder, or condition (*i.e*., reversing the pathology and/or symptoms). For a medicament or pharmacologically active agent, a "therapeutically effective amount" refers to a sufficient amount of the medicament or agent that is non-toxic but can achieve the desired effect. The determination of an effective amount varies from person to person, depending on the recipient's age and general condition, as well as the specific active substance. The appropriate effective amount in individual cases can be determined by a person skilled in the art through routine testing.

The term "pharmaceutically acceptable" refers to compounds, materials, compositions, and/or dosage forms that are, within the scope of sound medical judgment, suitable for contact with patient tissues without excess toxicity, irritation, allergic reactions, or other problems or complications, and possess a reasonable benefit/risk ratio while being effective for the intended use.

The "patient" of the present disclosure refers to any animal, including mammals, preferably mice, rats, other rodents, rabbits, dogs, cats, pigs, cattle, sheep, horses, or primates, and most preferably humans.

### Beneficial Effects

The present disclosure provides a small-molecule phenyl-substituted heteroaryl compound, which can serve as a p38 MAPK/MK2 pathway inhibitor, such as the compound represented by formula (I), formula (II), formula (II-1), formula (11-2), Table A, or Table B with phenyl-substituted heteroaryl structures. These compounds or pharmaceutical compositions can be effectively used to treat or prevent diseases mediated by p38 MAPK/MK2 pathway. These compounds inhibit p38 MAPK-dependent MK2 activity while maintaining selectivity for ATF2 and MK5. At the same time, these compounds exhibit good *in vivo* pharmacokinetic properties.

### BRIEF DESCRIPTION OF THE DRAWINGS

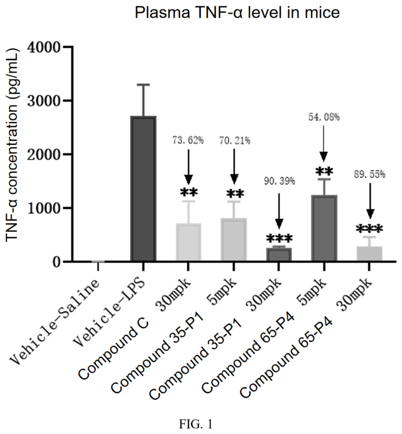

FIG. 1 shows the plasma TNF-α level in mice after treatment with various compounds of the present disclosure and control compounds.

### Term Definitions and Descriptions

Unless stated to the contrary, the terms used in the specification and claims have the following meanings.

The term "alkyl" refers to a saturated aliphatic hydrocarbon group, which is a straight or branched chain group containing 1 to 20 carbon atoms, preferably an alkyl group containing 1 to 12 (*e.g.,* 1, 2, 3, 4, 5, 6, 7, 8, 9, 10, 11, and 12) carbon atoms, and more preferably an alkyl group containing 1 to 6 carbon atoms (C₁₋₆ alkyl). Non-limiting examples of alkyl groups include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, various branched isomers thereof, and the like. The alkyl group may be substituted or unsubstituted.

The term "alkoxy" refers to -O-(alkyl), wherein the definition of alkyl is as described herein. Preferably an alkoxy group containing 1 to 12 (*e.g.,* 1, 2, 3, 4, 5, 6, 7, 8, 9, 10, 11, and 12) carbon atoms (C₁₋₁₂ alkoxy), more preferably an alkoxy group containing 1 to 6 carbon atoms (C₁₋₆ alkoxy). Non-limiting examples of alkoxy groups include methoxy, ethoxy, propoxy, and butoxy. The alkoxy group may be substituted or unsubstituted.

The term "cycloalkyl" refers to a saturated or partially unsaturated monocyclic or polycyclic hydrocarbon substituent, and the cycloalkyl ring contains 3 to 20 carbon atoms, preferably 3 to 12 carbon atoms or 3 to 8 (*e.g.*, 3, 4, 5, 6, 7, and 8) carbon atoms, more preferably 3 to 6 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, *etc.*; polycyclic cycloalkyl groups include spiro, fused, and bridged cycloalkyl groups.

The term "spirocycloalkyl" refers to a 5- to 20-membered polycyclic group, where each monocycle in the system shares one carbon atom (referred to as the spiro atom), and may contain one or more double bonds. Preferably a 6- to 14-membered spirocycloalkyl group, more preferably a 7- to 10-membered (*e.g*., 7-, 8-, 9- or 10-membered) spirocycloalkyl group. Spirocycloalkyl can be divided into monocyclic, bicyclic, or polycyclic spirocycloalkyl groups according to the number of spiro atoms shared between rings, preferably monocyclic and bicyclic. More preferably, a 3-membered/5-membered, 3-membered/6-membered, 4-membered/4-membered, 4-membered/5-membered, 4-membered/6-membered, 5-membered/5-membered, or 5-membered/6-membered monospiroalkyl group. Non-limiting examples of spirocycloalkyl groups include:

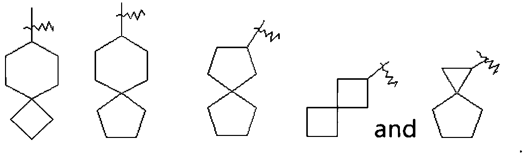

The term "fused cycloalkyl" refers to an all-carbon 5- to 20-membered polycyclic group, where each ring in the system shares an adjacent pair of carbon atoms with other rings in the system, and one or more rings may contain one or more double bonds. Preferably a 6- to 14-membered fused cycloalkyl group, more preferably a 7- to 10-membered (*e.g*., 7-, 8-, 9- or 10-membered) fused cycloalkyl group. Based on the number of constituent rings, the fused cycloalkyl group can be divided into bicyclic, tricyclic, tetracyclic, or polycyclic fused cycloalkyl groups, preferably bicyclic or tricyclic, and more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered, and 6-membered/6-membered bicyclic fused cycloalkyl groups. Non-limiting examples of fused cycloalkyl groups include:

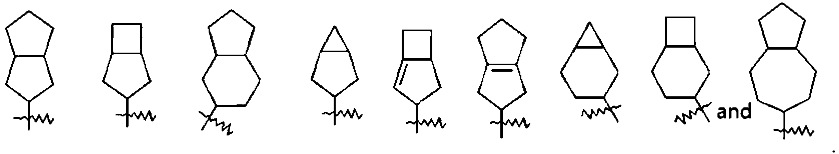

The term "bridged cycloalkyl" refers to an all-carbon 5- to 20-membered polycyclic group, where any two rings share two carbon atoms that are not directly connected, and which may contain one or more double bonds. Preferably a 6- to 14-membered bridged cycloalkyl group, more preferably a 7- to 10-membered (*e.g*., 7-, 8-, 9- or 10-membered) bridged cycloalkyl group. Based on the number of constituent rings, the bridged cycloalkyl group can be divided into bicyclic, tricyclic, tetracyclic, or polycyclic bridged cycloalkyl groups, preferably bicyclic, tricyclic, or tetracyclic, and more preferably bicyclic or tricyclic. Non-limiting examples of bridged cycloalkyl groups include:

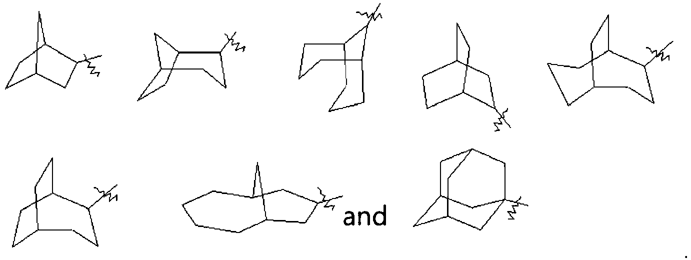

The cycloalkyl ring includes the cycloalkyl ring (comprising monocyclic, spiro, fused, and bridged rings) as defined herein fused to an aryl, heteroaryl, or heterocycloalkyl ring, wherein the ring attached to the parent structure is the cycloalkyl ring. Non-limiting examples include 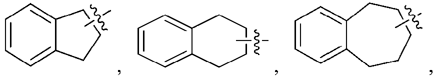 *etc.*; preferably 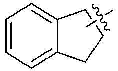 and 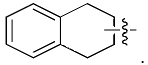 The cycloalkyl group may be substituted or unsubstituted.

The term "heterocyclyl" refers to a saturated or partially unsaturated monocyclic or polycyclic substituent containing 3 to 20 ring atoms, wherein one or more ring atoms are heteroatoms selected from the group consisting of nitrogen, oxygen, and sulfur (the sulfur may optionally be oxidized, *i.e.,* forming sulfoxide or sulfone), excluding ring portions containing -O-O-, -O-S-, or -S-S-, with the remaining ring atoms being carbon. Preferably, the heterocyclyl group contains 3 to 12 ring atoms (*e.g.,* 3, 4, 5, 6, 7, 8, 9, 10, 11, and 12), of which 1 to 4 (*e.g.,* 1, 2, 3, and 4) are heteroatoms; more preferably, the heterocyclyl group contains 3 to 8 ring atoms (*e.g.,* 3, 4, 5, 6, 7, and 8), of which 1 to 3 (*e.g.,* 1, 2, and 3) are heteroatoms; even more preferably, the heterocyclyl group contains 3 to 6 ring atoms, of which 1 to 3 are heteroatoms; and most preferably, the heterocyclyl group contains 5 or 6 ring atoms, of which 1 to 3 are heteroatoms. Non-limiting examples of monocyclic heterocyclyl groups include pyrrolidinyl, tetrahydropyranyl, 1,2,3,6-tetrahydropyridinyl, piperidinyl, piperazinyl, morpholinyl, thiomorpholinyl, homopiperazinyl, *etc.* Polycyclic heterocyclyl groups include spiro, fused, and bridged heterocyclyl groups.

The term "spiroheterocyclyl" refers to a 5- to 20-membered polycyclic heterocyclyl group wherein each monocycle in the system shares one atom (referred to as the spiro atom), with one or more ring atoms being heteroatoms selected from the group consisting of nitrogen, oxygen, and sulfur (wherein the sulfur may optionally be oxidized to form sulfoxide or sulfone), and the remaining ring atoms being carbon. The spiroheterocyclyl group may contain one or more double bonds. Preferably a 6- to 14-membered spiroheterocyclyl group, more preferably a 7- to 10-membered (*e.g*., 7-, 8-, 9- or 10-membered) spiroheterocyclyl group. Spiroheterocyclyl groups are divided into monocyclic, bicyclic, or polycyclic spiroheterocyclyl groups based on the number of shared spiro atoms between rings, with monocyclic and bicyclic spiroheterocyclyl groups being preferred. More preferably, the spiroheterocyclyl group is a 3-membered/5-membered, 3-membered/6-membered, 4-membered/4-membered, 4-membered/5-membered, 4-membered/6-membered, 5-membered/5-membered, or 5-membered/6-membered monocyclic spiroheterocyclyl group. Non-limiting examples of spiroheterocyclyl groups include:

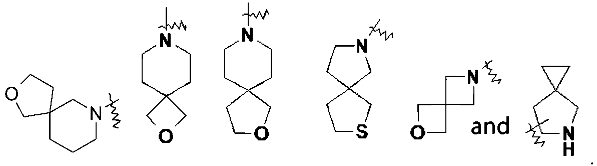

The term "fused heterocyclyl" refers to a 5- to 20-membered polycyclic heterocyclyl group wherein each ring in the system shares an adjacent pair of atoms with other rings in the system. One or more rings may contain one or more double bonds, with one or more ring atoms being heteroatoms selected from the group consisting of nitrogen, oxygen, and sulfur (where the sulfur may optionally be oxidized, *i.e.,* forming sulfoxide or sulfone), and the remaining ring atoms being carbon. Preferably a 6- to 14-membered fused heterocyclyl group, more preferably a 7- to 10-membered (*e.g*., 7-, 8-, 9- or 10-membered) fused heterocyclyl group. Based on the number of constituent rings, the fused heterocyclyl group can be divided into bicyclic, tricyclic, tetracyclic, or polycyclic fused heterocyclyl groups, preferably bicyclic or tricyclic, and more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered, and 6-membered/6-membered bicyclic fused heterocyclyl groups. Non-limiting examples of fused heterocyclyl groups include:

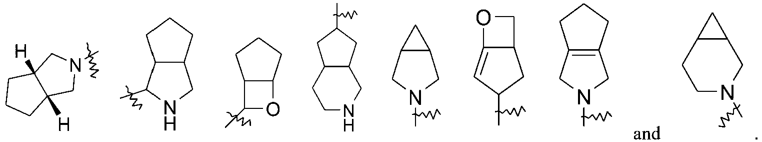

The term "bridged heterocyclyl" refers to a 5- to 14-membered polycyclic heterocyclyl group wherein any two rings share two non-directly connected atoms. The bridged heterocyclyl group may contain one or more double bonds, with one or more ring atoms being heteroatoms selected from the group consisting of nitrogen, oxygen, and sulfur (where the sulfur may optionally be oxidized, *i.e.,* forming sulfoxide or sulfone), and the remaining ring atoms being carbon. Preferably a 6- to 14-membered bridged heterocyclyl group, more preferably a 7- to 10-membered (*e.g*., 7-, 8-, 9- or 10-membered) bridged heterocyclyl group. Based on the number of constituent rings, the bridged heterocyclyl groups can be divided into bicyclic, tricyclic, tetracyclic, or polycyclic bridged heterocyclyl groups, preferably bicyclic, tricyclic, or tetracyclic, and more preferably bicyclic or tricyclic. Non-limiting examples of bridged heterocyclyl groups include:

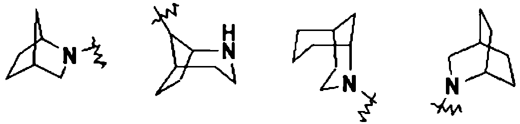

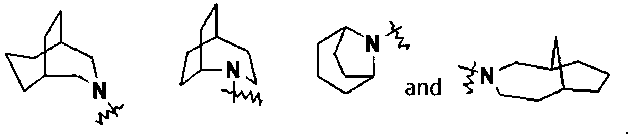

The heterocyclyl ring includes the heterocyclyl ring as described herein (comprising monocyclic, spiro, fused, and bridged heterocycles) fused to an aryl, heteroaryl, or cycloalkyl ring, wherein the ring attached to the parent structure is the heterocyclyl ring. Non-limiting examples include: 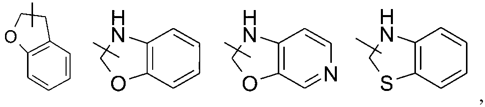 *etc.* The heterocyclyl group may be substituted or unsubstituted.

The term "aryl" refers to an all-carbon 6- to 14-membered monocyclic or fused polycyclic group (fused polycyclic group refers to rings sharing adjacent pairs of carbon atoms) with a conjugated π-electron system, preferably 6- to 10-membered, such as phenyl and naphthyl. The aryl ring includes the aryl ring as described herein fused to a heteroaryl, heterocyclyl, or cycloalkyl ring, with the ring attached to the parent structure being the aryl ring. Non-limiting examples include: 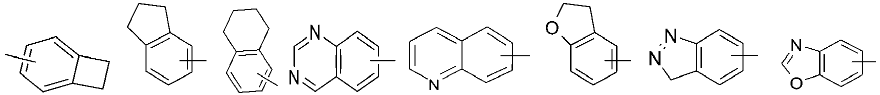 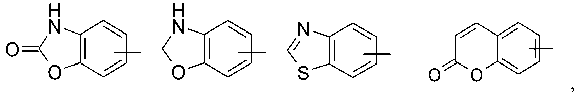 *etc.* The aryl group may be substituted or unsubstituted.

The term "heteroaryl" refers to a heteroaromatic system containing 1 to 4 (*e.g.,* 1, 2, 3, and 4) heteroatoms and 5 to 14 ring atoms, wherein the heteroatoms are selected from oxygen, sulfur, and nitrogen. The heteroaryl group is preferably a 5- to 10-membered (*e.g.,* 5-, 6-, 7-, 8-, 9-, or 10-membered) heteroaryl group, more preferably a 5-membered or 6-membered heteroaryl group, such as furanyl, thienyl, pyridinyl, pyrrolyl, *N*-alkylpyrrolyl, pyrimidinyl, pyrazinyl, pyridazinyl, imidazolyl, pyrazolyl, triazolyl, tetrazolyl, *etc.* The heteroaryl ring includes the heteroaryl ring as described herein fused to an aryl, heterocyclyl, or cycloalkyl ring, wherein the ring attached to the parent structure is the heteroaryl ring. Non-limiting examples include: 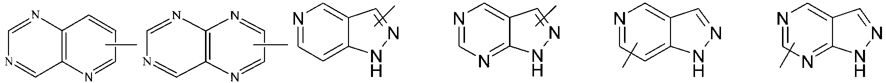 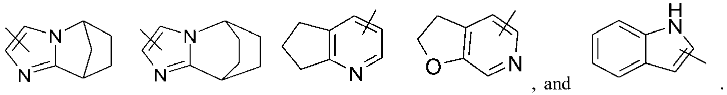 The heteroaryl group may be substituted or unsubstituted.

The terms "alkyl", "alkoxy", "cycloalkyl", "heterocyclyl", "aryl", "heteroaryl", and the like herein may be substituted or unsubstituted. When substituted, they may be substituted at any available connecting point, and the substituents are preferably independently and optionally selected from the group consisting of one or more identical or different substituents, including but not limited to halogen, alkyl, alkoxy, haloalkyl, haloalkoxy, hydroxyl, hydroxyalkyl, cyano, amino, nitro, cycloalkyl, heterocyclyl, aryl, and heteroaryl.

The cycloalkyl, heterocyclyl, aryl, and heteroaryl groups include residues derived by removing one hydrogen atom from a parent ring atom, or residues derived by removing two hydrogen atoms from the same or two different ring atoms of the parent ring atom, namely "divalent cycloalkyl", "divalent heterocyclyl", "arylene", and "heteroarylene".

The term "cycloalkyloxy" refers to cycloalkyl-O-, wherein the cycloalkyl is as defined herein.

The term "heterocyclyloxy" refers to heterocyclyl-O-, wherein the heterocyclyl is as defined herein.

The term "haloalkyl" refers to an alkyl group substituted by one or more halogens, wherein the alkyl is as defined herein.

The term "haloalkoxy" refers to an alkoxy group substituted by one or more halogens, wherein the alkoxy is as defined herein.

The term "hydroxyalkyl" refers to an alkyl group substituted by one or more hydroxyl groups, wherein the alkyl is as defined herein.

The term "halogen" refers to F, Cl, Br, or I.

The term "hydroxyl" refers to -OH.

The term "amino" refers to -NH₂.

The term "cyano" refers to -CN.

The term "nitro" refers to -NO₂.

The term "oxo group" or "oxo" refers to "=O".

The term "carbonyl" refers to C=O.

The term "carboxyl" refers to -C(O)OH.

The term "carboxylate ester group" refers to -C(O)O(alkyl), -C(O)O(cycloalkyl), (alkyl)C(O)O-, or (cycloalkyl)C(O)O-, wherein the alkyl and cycloalkyl are as defined herein.

"Optional" or "optionally" means that the subsequently described event or circumstance may, but need not, occur, and that the description includes instances where the event or circumstance occurs and instances where it does not. For example, "a heterocycloalkyl group optionally substituted by an alkyl group" means that the alkyl group may but does not have to be present, and this description includes both cases where the heterocycloalkyl group is substituted by an alkyl group and where the heterocycloalkyl group is not substituted by an alkyl group.

"Substituted" refers to one or more hydrogen atoms in a group, preferably up to 5, and more preferably 1 to 3 hydrogen atoms, being independently replaced by a corresponding number of substituents. It goes without saying that substituents are only present at their possible chemical positions, and those skilled in the art can determine (through experimentation or theory) possible or impossible substitutions without undue effort. For example, amino or hydroxyl groups with free hydrogen may be unstable when combined with carbon atoms containing unsaturated (*e.g.*, olefinic) bonds.

### DETAILED DESCRIPTION OF THE PREFERRED EMBODIMENT

The technical solutions of the present disclosure will be further illustrated in detail with reference to the following specific examples. It should be understood that the following examples are merely exemplary illustrations and explanations of the present disclosure, and should not be construed as limiting the scope of the present disclosure. All techniques implemented based on the contents of the present disclosure are encompassed within the intended scope of the present disclosure.

Unless otherwise specified, the raw materials and reagents used in the following examples are commercially available or can be prepared by known methods.

The structure of compounds is determined by nuclear magnetic resonance (NMR) or/and mass spectrometry (MS). NMR chemical shifts (*δ*) are given in units of 10⁻⁶ (ppm). The NMR measurements are performed using a Bruker ASCEND^{™}-400 nuclear magnetic resonance spectrometer, using deuterated dimethyl sulfoxide (DMSO-*d₆*), deuterated chloroform (CDCl₃), and deuterated methanol (CD₃OD) as solvents, with tetramethylsilane (TMS) as the internal standard.

The MS measurement is performed using Agilent 6110, Agilent 1100, Agilent 6120, and Agilent G6125B liquid chromatography-mass spectrometers.

The HPLC measurement is performed using a Shimadzu HPLC-2010C high-performance liquid chromatograph (XBRIDGE 2.1 × 50 mm, 3.5 µm chromatographic column).

Chiral HPLC analysis is performed using THARSFC X5.

Thin-layer chromatography (TLC) silica gel plates use Yantai Qingdao GF254 silica gel plates. The specification for TLC is 0.15 mm to 0.2 mm, while the specification for thin-layer chromatographic purification of products is 0.4 mm to 0.5 mm.

Column chromatography is generally performed using Qingdao Haiyang Silica Gel 200 to 300 mesh silica gel as the carrier.

The preparative high-performance liquid chromatography is performed using Waters 2767, Waters 2545, and Chuangxin Tongheng LC3000 preparative chromatograph.

Chiral preparative column chromatography is performed using Shimadzu LC-20AP and THARSFC PREP 80.

The CombiFlash rapid preparative instrument uses Combiflash Rf200 (TELEDYNE ISCO).

The pressurized hydrogenation reaction is performed using a Beijing Jiawei Kechuang Technology GCD-500G hydrogen generator.

The microwave reaction is performed using a Biotage initiator+ microwave reactor.

Unless otherwise specified in the examples, all reactions are conducted under an argon or nitrogen atmosphere.

An argon or nitrogen atmosphere refers to connecting the reaction flask to an argon or nitrogen balloon with a volume of approximately 1 liter.

A hydrogen atmosphere refers to connecting the reaction flask to a hydrogen balloon with a volume of approximately 1 liter.

Unless otherwise specified in the examples, the reaction temperature is room temperature, ranging from 20°C to 30°C.

A person skilled in the art should understand that the resolved chiral compounds can be distinguished by their retention times on a chiral chromatographic column. Therefore, the chiral compounds resolved based on the sequence of retention times are correspondingly distinguished by the numbered suffixes P1 and P2. The suffix P1 corresponds to the first resolved chiral structure, and the suffix P2 corresponds to the subsequently resolved chiral structure. If the absolute configuration of a compound is listed in the reaction formula, it does not imply a one-to-one correspondence with the compounds numbered with suffixes P1 and P2, but merely illustrates the two possible forms of the absolute configuration. The absolute configuration of compounds with the suffix P1 or P2 is determined based on the absolute configuration corresponding to the specific retention time.

### Synthesis of Intermediate Compound A1

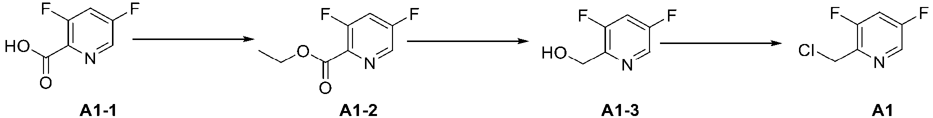

### Step 1: Synthesis of Compound A1-2

Thionyl chloride (22.43 g, 0.19 mol) was slowly added dropwise to a solution of compound A1-1 (20 g, 0.13 mol) in ethanol (60 mL). The reaction mixture was stirred at 60°C for 3 hours. After the reaction was completed, the reaction mixture was concentrated under reduced pressure to obtain compound A1-2 (20 g, crude product). The product was directly used in the next step without purification.

MS m/z (ESI): 187.9 [M+1]⁺.

Step 2: Synthesis of Compound A1-3

At 0°C, sodium borohydride (6.48 g, 0.17 mol) was added in batches to a solution of compound A1-2 (16 g, 0.086 mol) in ethanol (60 mL). The reaction mixture was slowly warmed to room temperature and stirred at room temperature for 3 hours. After the reaction was completed, the reaction mixture was concentrated under reduced pressure. The resulting residue was purified by silica gel column chromatography (petroleum ether/ethyl acetate = 1/0 to 1/5) to obtain compound A1-3 (16 g, yield: 73.5%).

MS m/z (ESI): 146.1 [M+1]⁺.

Step 3: Synthesis of Compound A1

5 drops of *N,N*-dimethylformamide were added to a solution of compound A1-3 (1.80 g, 12.4 mmol) in dichloromethane (50 mL), and the reaction mixture was stirred for 10 minutes. Thionyl chloride (1.77 g, 14.88 mmol) was slowly added to the above solution at room temperature. The resulting mixture was stirred at room temperature for 1 hour. After the reaction was completed, 4 M ammonium chloride solution was added to the reaction mixture until the pH = 7, followed by extraction with dichloromethane (60 mL × 3). The combined organic phases were dried over anhydrous sodium sulfate and filtered. The filtrate was concentrated under reduced pressure, and the resulting residue was purified by silica gel column chromatography (petroleum ether/ethyl acetate = 1/0 to 5/1) to obtain compound A1 (1.80 g, yield: 84.7%).

¹HNMR (400 MHz, CDCl₃) δ 8.35 (d, *J* = 2.4 Hz, 1H), 7.26 (ddd, *J=* 9.1, 8.0, 2.6 Hz, 1H), 4.72 (d, *J=* 2.1 Hz, 2H).

### Synthesis of Intermediate Compound A2

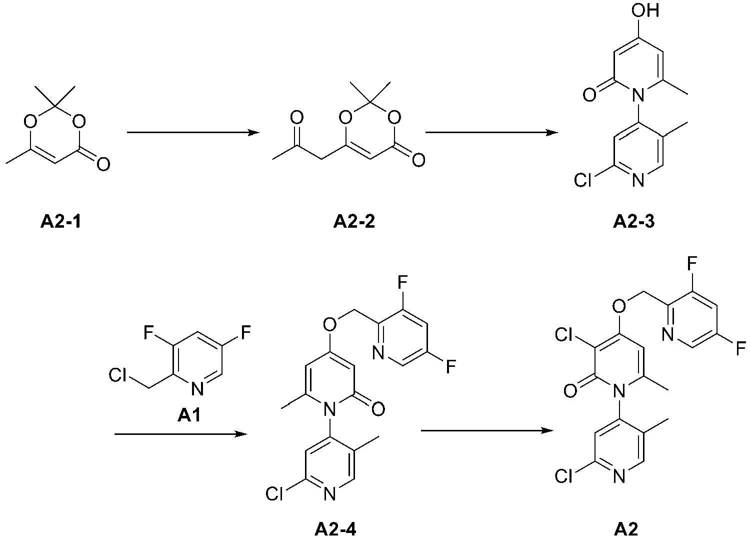

### Step 1: Synthesis of Compound A2-2

At -78°C, a solution of lithium bis(trimethylsilyl)amide in tetrahydrofuran (141 mL, 141 mmol) was slowly added to a solution of compound A2-1 (20 g, 141 mmol) in tetrahydrofuran (500 mL). The reaction mixture was stirred at -78°C for 1 hour, followed by the slow dropwise addition of acetyl chloride (6.60 g, 844 mmol). The resulting mixture was stirred at -78°C for 1 hour. After the reaction was completed, the reaction mixture was poured into saturated ammonium chloride solution (500 mL) and extracted with ethyl acetate (300 mL × 3). The combined organic phases were washed with saturated brine (300 mL × 2), dried over anhydrous sodium sulfate, and filtered. The filtrate was concentrated under reduced pressure, and the resulting residue was purified by column chromatography (petroleum ether/ethyl acetate = 1/0 to 2/1) to obtain compound A2-2 (6.60 g, yield: 25.5%).

MS m/z (ESI): 185.1 [M+1]⁺.

### Step 2: Synthesis of Compound A2-3

Compound A2-2 (8.98 g, 48.7 mmol) was added to a solution of 2-chloro-4-amino-5-methylpyridine (4.63 g, 32.5 mmol, CAS: 79055-62-2) in 1,4-dioxane (150 mL). The reaction mixture was heated to 90°C and stirred at this temperature for 3.5 hours. The reaction mixture was cooled to room temperature, and methanesulfonic acid (3.12 g, 32.5 mmol) was added. The reaction was then heated to 50°C and stirred at this temperature for 3 hours. After the reaction was completed, the reaction mixture was cooled to room temperature and filtered. The filter cake was collected and dried to obtain compound A2-3 (5.60 g, yield: 68.7%).

MS m/z (ESI): 251.0 [M+1]⁺.

### Step 3: Synthesis of Compound A2-4

Compound A1 (4.01 g, 24.6 mmol) was added to a solution of compound A2-3 (5.60 g, 22.3 mmol), potassium carbonate (7.69 g, 55.7 mmol), and 18-crown-6 (1.18 g, 4.4 mmol) in N,N-dimethylformamide (80 mL). The reaction mixture was stirred at room temperature for 16 hours. After the reaction was completed, the reaction mixture was diluted with water (100 mL) and extracted with ethyl acetate (80 mL × 3). The combined organic phases were washed with brine (20 mL × 3), dried over anhydrous sodium sulfate, and filtered, and the filtrate was concentrated under reduced pressure to obtain compound A2-4 (8.40 g, yield: 99.5%). The product was directly used in the next step without purification.

MS m/z (ESI): 378.0 [M+1]⁺.

Step 4: Synthesis of Compound A2

N-Chlorosuccinimide (265 mg, 1.98 mmol) was added to a solution of compound A2-4 (500 mg, 1.32 mmol) in isopropanol (20 mL). The reaction mixture was heated to 90°C under a nitrogen atmosphere and stirred at this temperature for 12 hours. After the reaction was completed, the reaction mixture was cooled to room temperature, diluted with water (100 mL), and extracted with ethyl acetate (50 mL × 3). The combined organic phases were washed with saturated brine (50 mL × 1), dried over anhydrous sodium sulfate, and filtered. The filtrate was concentrated under reduced pressure. The resulting residue was purified by silica gel column chromatography (dichloromethane/methanol = 1/0 to 10/1) to obtain compound A2 (500 mg, yield: 91.7%).

MS m/z (ESI): 412.6 [M+1]⁺.

### Synthesis of Intermediate Compound A3

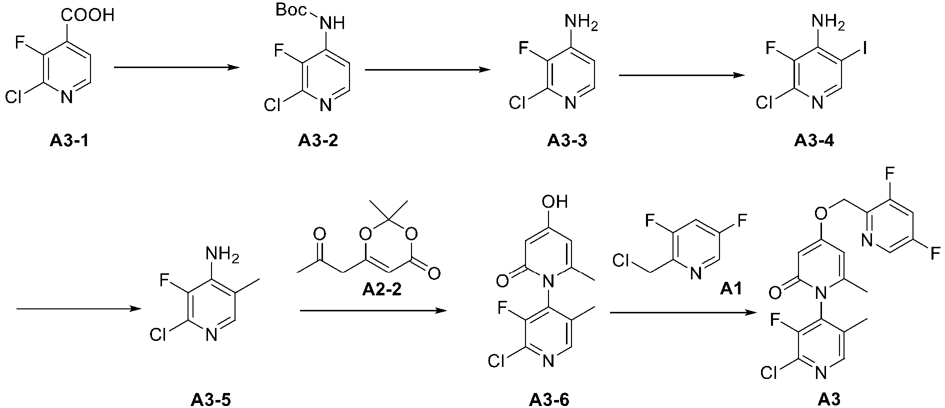

### Step 1: Synthesis of Compound A3-2

Compound A3-1 (10 g, 0.057 mol) was added to a solution of diphenylphosphoryl azide (23.5 g, 0.085 mol) and triethylamine (17.3 g, 0.17 mol) in *tert*-butanol and toluene (50 mL/50 mL). The reaction was heated to 110°C and stirred at this temperature for 16 hours. After the reaction was completed, the reaction mixture was poured into water and extracted with dichloromethane (200 mL × 3). The combined organic phases were washed with saturated brine (200 mL × 1), dried over anhydrous sodium sulfate, and filtered. The filtrate was concentrated under reduced pressure, and the resulting residue was purified by silica gel column chromatography (petroleum ether/ethyl acetate = 1/0 to 10/1) to obtain compound A3-2 (3.6 g, yield: 25%).

MS m/z (ESI): 247.0 [M+1]⁺.

### Step 2: Synthesis of Compound A3-3

Compound A3-2 (3.6 g, 14.50 mol) was added to a mixed solvent of trifluoroacetic acid and dichloromethane (15 mL/30 mL), and the reaction mixture was stirred at room temperature for 16 hours. After the reaction was completed, the reaction mixture was concentrated under reduced pressure, and the resulting residue was purified by silica gel column chromatography (dichloromethane/methanol = 1/0 to 20/1) to obtain compound A3-3 (3.1 g, crude product).

MS m/z (ESI): 147.0 [M+1]⁺.

### Step 3: Synthesis of Compound A3-4

Compound A3-3 (3.10 g, 0.021 mol) was added to a solution of silver sulfate (6.61 g, 0.021 mol) and iodine (5.38 g, 0.021 mol) in ethanol (50 mL). The reaction mixture was heated to 50°C and stirred at this temperature for 16 hours. After the reaction was completed, the reaction mixture was concentrated under reduced pressure. The resulting residue was purified by silica gel column chromatography (dichloromethane/methanol = 1/0 to 10/1) to obtain compound A3-4 (4.90 g, yield: 65%).

MS m/z (ESI): 272.7 [M+1]⁺.

### Step 4: Synthesis of Compound A3-5

Under a nitrogen atmosphere, 1,1'-bis(diphenylphosphino)ferrocene palladium(II)dichloride (450 mg, 0.55 mmol) was added to a solution of methylboronic acid (530 mg, 8.80 mmol), cesium carbonate (8.96 g, 27.5 mmol), and compound A3-4 (1.50 g, 5.50 mmol) in 1,4-dioxane (30 mL). The reaction mixture was heated to 100°C and stirred at this temperature for 1.5 hours. After the reaction was completed, the reaction mixture was cooled to room temperature, and saturated sodium bicarbonate solution (50 mL) was added. The mixture was extracted with ethyl acetate (50 mL × 3). The combined organic phases were washed with saturated brine (50 mL × 1), dried over anhydrous sodium sulfate, and filtered. The filtrate was concentrated under reduced pressure, and the resulting residue was purified by silica gel column chromatography (petroleum ether/ethyl acetate = 1/0 to 3/2) to obtain compound A3-5 (0.43 g, yield: 48%).

MS m/z (ESI): 161.0 [M+1]⁺.

### Step 5: Synthesis of Compound A3-6

Compound A3-5 (850 mg, 5.29 mmol) was added to a solution of compound A2-2 (1.2 g, 6.32 mmol) in 1,4-dioxane (8 mL). The reaction was heated to 110°C and stirred at this temperature for 1 hour. The reaction mixture was cooled to 50°C, and methanesulfonic acid (285 mg, 2.96 mmol) was added. The reaction mixture was stirred at 50°C for another 1 hour. After the reaction was completed, the reaction mixture was cooled to room temperature, diluted with water (50 mL), and extracted with ethyl acetate (100 mL × 3). The combined organic phases were washed with saturated brine (100 mL × 2), dried over anhydrous sodium sulfate, and filtered. The filtrate was concentrated under reduced pressure. The resulting residue was purified by silica gel column chromatography (dichloromethane/methanol = 1/0 to 100/1) to recover part of the raw material A3-5 (310 mg) and obtain compound A3-6 (530 mg, yield: 59%).

MS m/z (ESI): 269.0 [M+1]⁺.

### Step 6: Synthesis of Compound A3

Potassium carbonate (1.14 g, 8.28 mmol), 18-crown-6 (175 mg, 0.66 mmol), and compound A1 (702 mg, 4.30 mmol) were sequentially added to a solution of compound A3-6 (890 mg, 3.31 mmol) in N,Ndimethylformamide (15 mL). The reaction mixture was heated to 40°C and stirred at this temperature for 16 hours. After the reaction was completed, the reaction mixture was cooled to room temperature, diluted with water (50 mL), and extracted with ethyl acetate (50 mL × 3). The combined organic phases were washed with saturated brine (50 mL × 1), dried over anhydrous sodium sulfate, and filtered, and the filtrate was concentrated under reduced pressure to obtain compound A3 (1.5 g, crude product). The product was directly used in the next step without purification.

MS m/z (ESI): 395.8 [M+1]⁺.

### Example 1 Synthesis of Compound 1

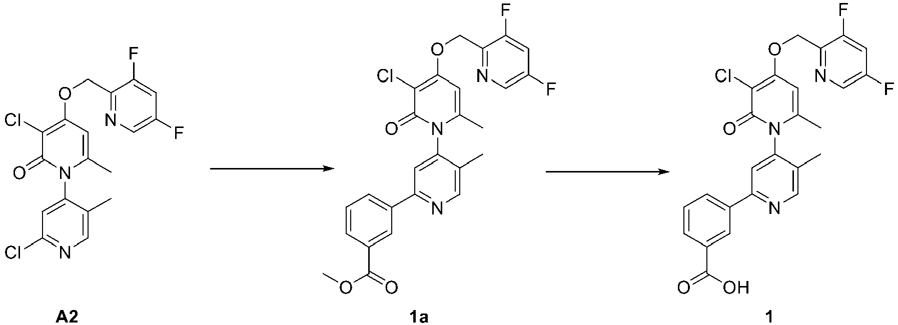

### Step 1: Synthesis of Compound 1a

1,1'-Bis(diphenylphosphino)ferrocene palladium(II)dichloride (118 mg, 0.14 mmol) was added to a mixed solution of compound A2 (330 mg, 0.72 mmol), 3-methoxycarbonylphenylboronic acid (196 mg, 1.09 mmol), and potassium carbonate (201 mg, 1.45 mmol) in 1,4-dioxane and water (10 mL/1 mL). The reaction mixture was heated to 90°C under a nitrogen atmosphere and stirred at this temperature overnight. After the reaction was completed, the reaction mixture was diluted with water (20 mL) and extracted with ethyl acetate (20 mL × 3). The combined organic phases were washed with saturated brine (30 mL × 2), dried over anhydrous sodium sulfate, and filtered. The filtrate was concentrated under reduced pressure. The resulting residue was purified by column chromatography (dichloromethane/methanol = 1/0 to 10/1) to obtain compound 1a (300 mg, yield: 72.46%).

MS m/z (ESI): 511.7 [M+1]⁺.

### Step 2: Synthesis of Compound 1

Compound 1a (300 mg, 0.58 mmol) and lithium hydroxide (14.06 mg, 0.58 mmol) were sequentially added to a mixed solvent of tetrahydrofuran and water (3 mL/3 mL), and the reaction mixture was stirred at room temperature for 5 hours. After the reaction was completed, the reaction mixture was adjusted to pH = 5-6 with 1 M hydrochloric acid, diluted with water (50 mL), and extracted with ethyl acetate (50 mL × 3). The combined organic phases were washed with saturated brine (50 mL × 1), dried over anhydrous sodium sulfate, and filtered. The filtrate was concentrated under reduced pressure, and the resulting residue was purified by column chromatography (dichloromethane/methanol = 1/0 to 10/1) to obtain compound 1 (280 mg, crude product). The crude product (70 mg) was purified by preparative high-performance liquid chromatography (HPLC) (chromatographic column: Gemini-C18, 150 × 21.2 mm, 5 µm; mobile phase: acetonitrile-water (0.1% formic acid); gradient: 40-70%; column temperature: 25°C; flow rate: 20 mL/min; wavelength: 214 nm; column pressure: 80 bar) to obtain compound 1 (10.6 mg, yield: 6.4%).

MS m/z (ESI): 498.6 [M+1]⁺.

¹H NMR (400 MHz, CD₃OD) *δ* 8.78 (s, 1H), 8.72 (t, *J =* 1.6 Hz, 1H), 8.48 (d, *J* = 2.4 Hz, 1H), 8.29 - 8.24 (m, 1H), 8.13 (dd, *J=* 7.8, 1.3 Hz, 1H), 7.91 (s, 1H), 7.79 - 7.72 (m, 1H), 7.63 (t, *J=* 7.8 Hz, 1H), 6.85 (s, 1H), 5.53 (d, *J=* 1.9 Hz, 2H), 2.16 (s, 3H), 2.11 (s, 3H).

### Example 2 Synthesis of Compound 2

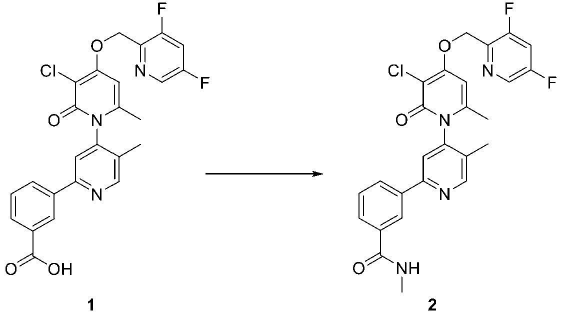

Methylamine hydrochloride (20.3 mg, 0.30 mmol), 1-hydroxybenzotriazole (32 mg, 0.24 mmol), 1-(3-dimethylaminopropyl)-3-ethylcarbodiimide hydrochloride (46 mg, 0.24 mmol), and triethylamine (81 mg, 0.80 mmol) were sequentially added to a solution of compound 1 (100 mg, 0.20 mmol) in dichloroethane (5 mL). The reaction mixture was stirred at room temperature for 16 hours. After the reaction was completed, the reaction mixture was diluted with water (50 mL) and extracted with ethyl acetate (50 mL × 3). The combined organic phases were washed with saturated brine (50 mL × 2), dried over anhydrous sodium sulfate, and filtered. The filtrate was concentrated under reduced pressure, and the resulting residue was purified by silica gel column chromatography (dichloromethane/methanol = 1/0 to 10/1) to obtain a crude product of compound 2. The crude product was purified by preparative HPLC (chromatographic column: Gemini-C18, 150 × 21.2 mm, 5 µm; mobile phase: acetonitrile-water (0.1% formic acid); gradient: 35-55%; column temperature: 25°C; flow rate: 20 mL/min; wavelength: 214 nm; column pressure: 80 bar) to obtain compound 2 (19.5 mg, yield: 19%).

MS m/z (ESI): 511.7 [M+1]⁺.

¹H NMR (400 MHz, CD₃OD) *δ* 8.77 (s, 1H), 8.49 (dd, *J=* 4.0, 2.1 Hz, 2H), 8.25 - 8.20 (m, 1H), 7.94 - 7.87 (m, 2H), 7.80 - 7.72 (m, 1H), 7.61 (t, *J* = 7.8 Hz, 1H), 6.85 (s, 1H), 5.53 (d, *J* = 1.9 Hz, 2H), 2.97 (s, 3H), 2.14 (d, *J =* 18.1 Hz, 6H).

### Example 3 Synthesis of Compound 3

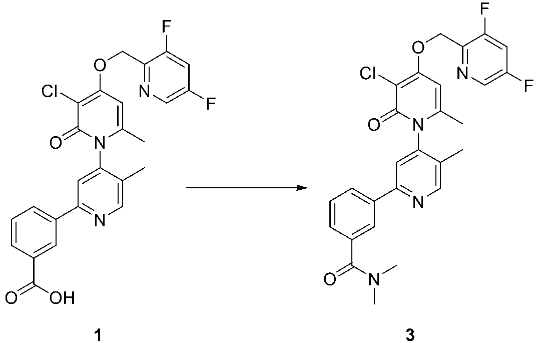

Dimethylamine hydrochloride (24 mg, 0.30 mmol), 1-hydroxybenzotriazole (32 mg, 0.24 mmol), 1-(3-dimethylaminopropyl)-3-ethylcarbodiimide hydrochloride (46 mg, 0.24 mmol), and triethylamine (81 mg, 0.80 mmol) were sequentially added to a solution of compound 1 (100 mg, 0.20 mmol) in dichloroethane (5 mL). The reaction mixture was stirred at room temperature for 16 hours. After the reaction was completed, the reaction mixture was diluted with water (50 mL) and extracted with ethyl acetate (50 mL × 3). The combined organic phases were washed with saturated brine (50 mL × 1), dried over anhydrous sodium sulfate, and filtered. The filtrate was concentrated under reduced pressure, and the resulting residue was purified by column chromatography (dichloromethane/methanol = 1/0 to 10/1) to obtain a crude product of compound 3. The crude product of compound 3 was purified by preparative HPLC (chromatographic column: Gemini-C18, 150 × 21.2 mm, 5 µm; mobile phase: acetonitrile-water (0.1% formic acid); gradient: 40-45%; column temperature: 25°C; flow rate: 20 mL/min; wavelength: 214 nm; column pressure: 80 bar) to obtain compound 3 (21.1 mg, yield: 20%).

MS m/z (ESI): 525.7 [M+1]⁺.

¹H NMR (400 MHz, CDCl₃) *δ* 8.72 (s, 1H), 8.41 (d, *J* = 2.3 Hz, 1H), 8.04 (d, *J=* 7.4 Hz, 1H), 7.99 (s, 1H), 7.53 - 7.43 (m, 3H), 7.36 - 7.30 (m, 1H), 6.40 (s, 1H), 5.42 (d, *J=* 1.7 Hz, 2H), 3.13 (s, 3H), 3.01 (s, 3H), 2.14 (s, 3H), 1.99 (s, 3H).

### Example 4 Synthesis of Compound 4

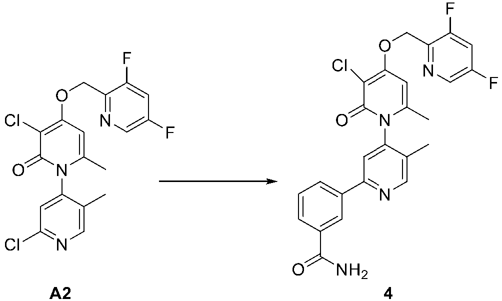

1,1'-Bis(diphenylphosphino)ferrocene palladium(II)dichloride (326.5 mg, 0.4 mmol) was added to a mixed solution of compound A2 (330 mg, 2 mmol), 3-carbamoylphenylboronic acid (824.6 mg, 4 mmol), and potassium carbonate (552 mg, 4 mmol) in 1,4-dioxane and water (10 mL/1 mL). The reaction mixture was heated to 90°C under a nitrogen atmosphere and stirred at this temperature overnight. After the reaction was completed, the reaction mixture was diluted with water (20 mL) and extracted with ethyl acetate (20 mL × 3). The combined organic phases were washed with saturated brine (30 mL × 2), dried over anhydrous sodium sulfate, and filtered. The filtrate was concentrated under reduced pressure, and the resulting residue was purified by silica gel column chromatography (dichloromethane/methanol = 1/0 to 10/1) to obtain a crude product of compound 4. The crude product of compound 4 was purified by preparative HPLC (chromatographic column: Gemini-C18, 150 × 21.2 mm, 5 µm; mobile phase: acetonitrile-water (0.1% formic acid); gradient: 20-50%; column temperature: 25°C; flow rate: 20 mL/min; wavelength: 214 nm; column pressure: 80 bar) to obtain compound 4 (57.20 mg, yield: 6.4%).

MS m/z (ESI): 497.6 [M+1]⁺.

¹H NMR (400 MHz, CD₃OD ) *δ* 8.77 (s, 1H), 8.54 (t, *J=* 1.6 Hz, 1H), 8.48 (d, *J=* 2.4 Hz, 1H), 8.27 - 8.21 (m, 1H), 8.00 - 7.95 (m, 1H), 7.90 (s, 1H), 7.80 - 7.71 (m, 1H), 7.62 (t, *J=* 7.8 Hz, 1H), 6.85 (s, 1H), 5.53 (d, *J* = 1.9 Hz, 2H), 2.14 (d, *J =* 17.8 Hz, 6H).

### Example 5 Synthesis of Compound 5

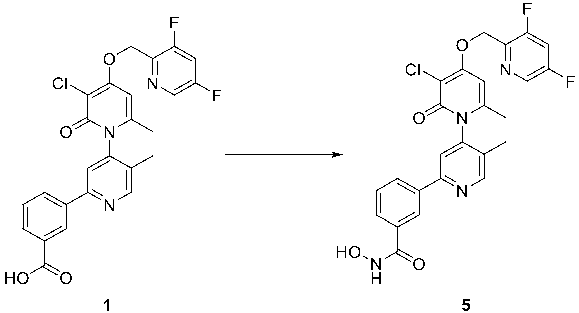

Hydroxylamine hydrochloride (31 mg, 0.45 mmol), 1-hydroxybenzotriazole (20 mg, 0.15 mmol), 1-(3-dimethylaminopropyl)-3-ethylcarbodiimide hydrochloride (28.87 mg, 0.15 mmol), and triethylamine (110 mg, 1.09 mmol) were sequentially added to a solution of compound 1 (50 mg, 0.10 mmol) in dichloroethane (5 mL). The reaction mixture was stirred at room temperature for 16 hours. After the reaction was completed, the reaction mixture was diluted with water (50 mL) and extracted with ethyl acetate (50 mL × 3). The combined organic phases were washed with saturated brine (50 mL × 1), dried over anhydrous sodium sulfate, and filtered. The filtrate was concentrated under reduced pressure, and the resulting residue was purified by silica gel column chromatography (dichloromethane/methanol = 1/0 to 10/1) to obtain a crude product of compound 5. The crude product of compound 5 was purified by preparative HPLC (chromatographic column: Gemini-C18, 150 × 21.2 mm, 5 µm; mobile phase: acetonitrile-water (0.1% formic acid); gradient: 20-60%; column temperature: 25°C; flow rate: 20 mL/min; wavelength: 214 nm; column pressure: 80 bar) to obtain compound 5 (4.1 mg, yield: 7.87%).

MS m/z (ESI): 513.1 [M+1]⁺.

¹H NMR (400 MHz, CD₃OD) δ 8.78 (s, 1H), 8.48 (d, *J* = 2.3 Hz, 1H), 8.44 (s, 1H), 8.23 (d, *J* = 8.0 Hz, 1H), 7.88 (s, 1H), 7.85 (d, *J* = 7.2 Hz, 1H), 7.81 - 7.71 (m, 1H), 7.62 (t, *J =* 7.8 Hz, 1H), 6.85 (s, 1H), 5.53 (s, 2H), 2.16 (s, 3H), 2.11 (s, 3H).

### Example 6 Synthesis of Compound 6

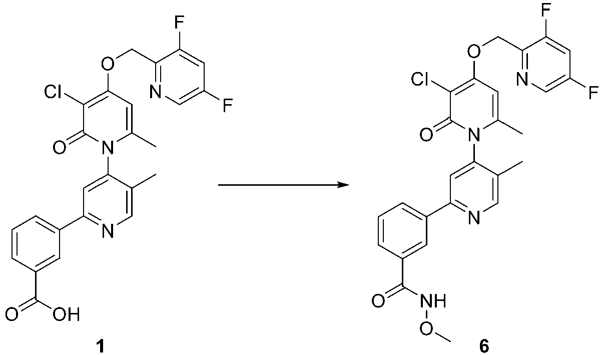

Methoxyamine hydrochloride (35 mg, 0.42 mmol), 1-hydroxybenzotriazole (20 mg, 0.15 mmol), 1-(3-dimethylaminopropyl)-3-ethylcarbodiimide hydrochloride (28.87 mg, 0.15 mmol), and triethylamine (110 mg, 0.80 mmol) were sequentially added to a solution of compound 1 (70 mg, 0.14 mmol) in dichloroethane (5 mL). The reaction mixture was stirred at room temperature for 16 hours. After the reaction was completed, the reaction mixture was diluted with water (50 mL) and extracted with ethyl acetate (50 mL × 3). The combined organic phases were washed with saturated brine (50 mL × 1), dried over anhydrous sodium sulfate, and filtered. The filtrate was concentrated under reduced pressure, and the resulting residue was purified by silica gel column chromatography (dichloromethane/methanol = 1/0 to 10/1) to obtain a crude product of compound 6. The crude product of compound 6 was purified by preparative HPLC (chromatographic column: Gemini-C18, 150 × 21.2 mm, 5 µm; column temperature: 25°C; flow rate: 20 mL/min; wavelength: 214 nm; column pressure: 80 bar; mobile phase: acetonitrile-water (0.1% formic acid); gradient: 30-70%) to obtain compound 6 (30.2 mg, yield: 42.32%).

MS m/z (ESI): 527.1 [M+1]⁺.

¹H NMR (400 MHz, CD₃OD) δ 8.77 (s, 1H), 8.48 (d, *J* = 2.3 Hz, 1H), 8.44 (s, 1H), 8.24 (d, *J* = 7.6 Hz, 1H), 7.89 (s, 1H), 7.86 (d, *J* = 7.8 Hz, 1H), 7.79 - 7.71 (m, 1H), 7.62 (t, *J* = 7.8 Hz, 1H), 6.85 (s, 1H), 5.53 (d, *J* = 1.8 Hz, 2H), 3.85 (s, 3H), 2.16 (s, 3H), 2.11 (s, 3H).

### Example 7 Synthesis of Compound 7

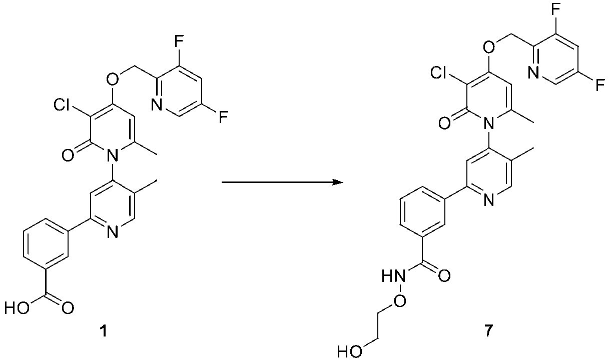

2-(Aminooxy)ethanol (19 mg, 0.24 mmol), 2-(7-azabenzotriazol-1-yl)-*N,N,N'N'*-tetramethyluronium hexafluorophosphate (92 mg, 0.24 mmol), and triethylamine (37 mg, 0.36 mmol) were sequentially added to a solution of compound 1 (60 mg, 0.12 mmol) in *N,N*-dimethylformamide (5 mL). The reaction mixture was stirred at room temperature for 16 hours. After the reaction was completed, the reaction mixture was diluted with water (50 mL) and extracted with ethyl acetate (50 mL × 3). The combined organic phases were washed with saturated brine (50 mL × 1), dried over anhydrous sodium sulfate, and filtered. The filtrate was concentrated under reduced pressure, and the resulting residue was purified by preparative HPLC (chromatographic column: Gemini-C18; 150 × 21.2 mm, 5 µm; mobile phase: acetonitrile-water (0.1% formic acid); gradient: 30-40%; column temperature: 25°C; flow rate: 20 mL/min; wavelength: 214 nm; column pressure: 80 bar) to obtain compound 7 (17.1 mg, yield: 24.5%).

MS m/z (ESI): 556.7 [M+1]⁺.

¹H NMR (400 MHz, CD₃OD) δ 8.64 (s, 1H), 8.36 (d, *J* = 2.4 Hz, 1H), 8.32 (t, *J=* 1.6 Hz, 1H), 8.11 (d, *J* = 7.9 Hz, 1H), 7.78 (s, 1H), 7.77 - 7.73 (m, 1H), 7.63 (ddd, *J* = 9.6, 8.6, 2.4 Hz, 1H), 7.49 (t, *J* = 7.8 Hz, 1H), 6.73 (s, 1H), 5.41 (d, *J =* 1.8 Hz, 2H), 4.02 - 3.92 (m, 2H), 3.69 (dd, *J* = 5.9, 3.4 Hz, 2H), 2.03 (s, 3H), 1.99 (s, 3H).

### Example 8 Synthesis of Compound 9

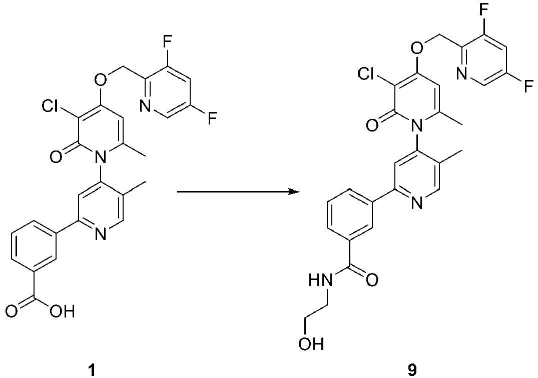

2-Aminoethanol (36 mg, 0.6 mmol), 1-hydroxybenzotriazole (54 mg, 0.4 mmol), 1-(3-dimethylaminopropyl)-3-ethylcarbodiimide hydrochloride (76.8 mg, 0.4 mmol), and triethylamine (110 mg, 1.09 mmol) were sequentially added to a solution of compound 1 (100 mg, 0.2 mmol) in dichloroethane (5 mL). The reaction mixture was stirred at room temperature for 16 hours. After the reaction was completed, the reaction mixture was diluted with water (50 mL) and extracted with ethyl acetate (50 mL × 3). The combined organic phases were washed with saturated brine (50 mL × 1), dried over anhydrous sodium sulfate, and filtered. The filtrate was concentrated under reduced pressure, and the resulting residue was purified by silica gel column chromatography (dichloromethane/methanol = 1/0 to 10/1) to obtain a crude product of compound 9. The crude product of compound 9 was further purified by preparative HPLC (chromatographic column: Gemini-C18, 150 × 21.2 mm, 5 µm; column temperature: 25°C; flow rate: 20 mL/min; wavelength: 214 nm; column pressure: 80 bar; mobile phase: acetonitrile-water (0.1% formic acid); gradient: 30-50%) to obtain compound 9 (30.9 mg, yield: 43.26%).

MS m/z (ESI): 540.7 [M+1]⁺.

¹H NMR (400 MHz, CD₃OD) δ 8.72 (s, 1H), 8.47 (t, *J* = 1.7 Hz, 1H), 8.43 (d, *J=* 2.4 Hz, 1H), 8.21 - 8.16 (m, 1H), 7.91 - 7.88 (m, 1H), 7.86 (s, 1H), 7.71 (ddd, *J* = 9.7, 8.6, 2.4 Hz, 1H), 7.57 (t, *J=* 7.8 Hz, 1H), 6.80 (d, *J* = 0.7 Hz, 1H), 5.48 (d, *J* = 2.0 Hz, 2H), 3.71 (t, *J* = 5.8 Hz, 2H), 3.50 (dd, *J* = 8.7, 3.2 Hz, 2H), 2.11 (s, 3H), 2.07 (d, *J* = 0.5 Hz, 3H).

### Example 9 Synthesis of Compound 10

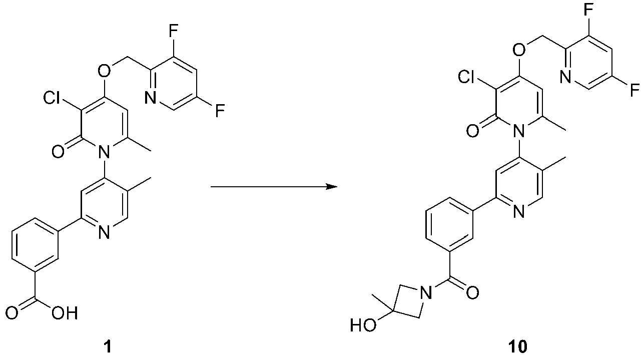

3-Methylazetidin-3-ol hydrochloride (35 mg, 0.28 mmol, CAS: 124668-46-8), 1-hydroxybenzotriazole (84 mg, 0.70 mmol), 1-(3-dimethylaminopropyl)-3-ethylcarbodiimide hydrochloride (138 mg, 0.70 mmol), and triethylamine (85 mg, 0.84 mmol) were sequentially added to a solution of compound 1 (70 mg, 0.14 mmol) in dichloroethane (5 mL). The reaction mixture was stirred at room temperature for 16 hours. After the reaction was completed, the reaction mixture was diluted with water (50 mL) and extracted with ethyl acetate (50 mL × 3). The combined organic phases were dried over anhydrous sodium sulfate and filtered. The filtrate was concentrated under reduced pressure, and the resulting residue was purified by silica gel column chromatography (dichloromethane/methanol = 1/0 to 10/1) to obtain a crude product of compound 10. The crude product was further purified by preparative HPLC (chromatographic column: Gemini-C18; 150 × 21.2 mm, 5 µm; mobile phase: acetonitrile-water (0.1% formic acid); gradient: 30-40%; column temperature: 25°C; flow rate: 20 mL/min; wavelength: 214 nm; column pressure: 80 bar) to obtain compound 10 (38.8 mg, yield: 49.0%).

MS m/z (ESI): 566.8 [M+1]⁺.

¹H NMR (400 MHz, CD₃OD) δ 8.73 (s, 1H), 8.43 (d, *J* = 2.4 Hz, 1H), 8.29 (t, *J=* 1.6 Hz, 1H), 8.15 (dd, *J =* 7.9, 1.2 Hz, 1H), 7.85 (s, 1H), 7.71 (dd, *J* = 2.2, 1.2 Hz, 1H), 7.69 (d, *J* = 1.7 Hz, 1H), 7.57 (t, *J* = 7.8 Hz, 1H), 6.80 (d, *J* = 0.5 Hz, 1H), 5.48 (d, *J* = 2.0 Hz, 2H), 4.24 (d, *J =* 5.3 Hz, 2H), 4.05 (d, *J =* 8.0 Hz, 2H), 2.11 (s, 3H), 2.06 (s, 3H), 1.48 (s, 3H).

### Example 10 Synthesis of Compound 11

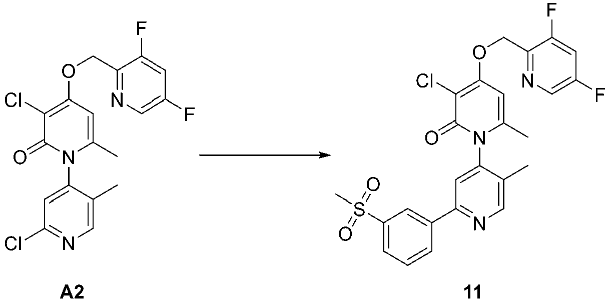

1,1'-Bis(diphenylphosphino)ferrocene palladium(II)dichloride (17 mg, 0.024 mmol) was added to a mixed solution of compound A2 (100 mg, 0.24 mmol), 3-(methylsulfonyl)phenylboronic acid (58 mg, 0.29 mmol), and potassium carbonate (67 mg, 0.48 mmol) in 1,4-dioxane and water (10 mL/1 mL). The reaction was heated to 90°C under a nitrogen atmosphere and stirred at this temperature overnight. After the reaction was completed, the reaction mixture was concentrated under reduced pressure, and the resulting residue was purified by preparative HPLC (chromatographic column: Gemini-C18, 150 × 21.2 mm, 5 µm; mobile phase: acetonitrile-water (0.1% formic acid); gradient: 35-60%; column temperature: 25°C; flow rate: 14 mL/min; wavelength: 214 nm; column pressure: 80 bar) to obtain compound 11 (74 mg, yield: 56%).

MS m/z (ESI): 531.7 [M+1]⁺.

¹H NMR (400 MHz, CDCl₃) δ 8.74 (s, 1H), 8.50 (s, 1H), 8.40 (d, *J =* 2.2 Hz, 1H), 8.33 (d, *J* = 6.8 Hz, 1H), 7.99 (d, *J* = 7.5 Hz, 1H), 7.67 (s, 1H), 7.56 (s, 1H), 7.32 (dd, *J =* 8.0, 6.8 Hz, 1H), 6.42 (s, 1H), 5.42 (s, 2H), 3.11 (s, 3H), 2.17 (s, 3H), 2.01 (s, 3H).

### Example 11 Synthesis of Compound 17

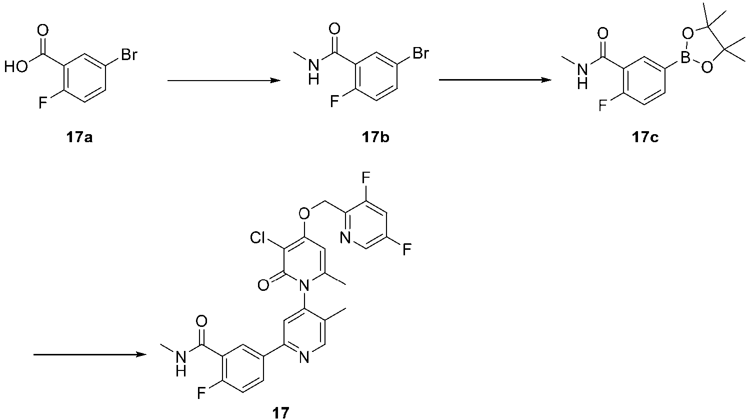

### Step 1: Synthesis of Compound 17b

Methylamine hydrochloride (616 mg, 9.13 mmol), 1-hydroxybenzotriazole (1.85 g, 13.70 mmol), 1-(3-dimethylaminopropyl)-3-ethylcarbodiimide hydrochloride (2.63 g, 13.70 mmol), and triethylamine (1.38 g, 13.7 mmol) were sequentially added to a solution of compound 17a (500 mg, 2.28 mmol) in dichloromethane (15 mL). The reaction mixture was stirred at room temperature for 16 hours. After the reaction was completed, the reaction mixture was diluted with water (20 mL) and extracted with ethyl acetate (20 mL ×3). The combined organic phases were dried over anhydrous sodium sulfate and filtered. The filtrate was concentrated under reduced pressure, and the resulting residue was purified by silica gel column chromatography (petroleum ether/ethyl acetate = 1/0 to 3/1) to obtain compound 17b (260 mg, yield: 49.2%). MS m/z (ESI): 231.9 [M+1]⁺.

### Step 2: Synthesis of Compound 17c

Bis(pinacolato)diboron (241 mg, 0.95 mmol), potassium acetate (254 mg, 2.59 mmol), and 1,1'-bis(diphenylphosphino)ferrocene-palladium(II)dichloride dichloromethane complex (70 mg, 0.09 mmol) were sequentially added to a mixed solution of compound 17b (200 mg, 0.86 mmol) in 1,4-dioxane and water (5 mL/1 mL). The reaction was heated to 50°C and stirred at this temperature for 6 hours. After the reaction was completed, the reaction mixture was diluted with water (20 mL) and extracted with ethyl acetate (20 mL × 3). The combined organic phases were dried over anhydrous sodium sulfate and filtered. The filtrate was concentrated under reduced pressure, and the resulting residue was purified by silica gel column chromatography (petroleum ether/ethyl acetate = 1/0 to 2/1) to obtain compound 17c (190 mg, yield: 78.2%). MS m/z (ESI): 280.1 [M+H]⁺.

### Step 3: Synthesis of Compound 17

1,1'-Bis(diphenylphosphino)ferrocene-palladium(II)dichloride dichloromethane complex (51 mg, 0.06 mmol) was added to a mixed solution of compound 17c (132 mg, 0.47 mmol), compound A2 (130 mg, 0.32 mmol), and potassium carbonate (87 mg, 0.63 mmol) in 1,4-dioxane and water (5 mL/1 mL). The reaction was heated to 90°C and stirred at this temperature for 16 hours. After the reaction was completed, the reaction mixture was diluted with water (20 mL) and extracted with ethyl acetate (20 mL × 3). The combined organic phases were dried over anhydrous sodium sulfate and filtered. The filtrate was concentrated under reduced pressure, and the resulting residue was purified by preparative HPLC (chromatographic column: Gemini-C18; 150 × 21.2 mm, 5 µm; mobile phase: acetonitrile-water (0.1% formic acid); gradient: 40-50%; column temperature: 25°C; flow rate: 20 mL/min; wavelength: 214 nm; column pressure: 80 bar) to obtain compound 17 (46.6 mg, yield: 28.4%).

MS m/z (ESI): 528.8 [M+1]⁺.

¹H NMR (400 MHz, CD₃OD) δ 8.75 (s, 1H), 8.48 (d, *J* = 2.4 Hz, 1H), 8.43 (dd, *J* = 7.0, 2.4 Hz, 1H), 8.22 (ddd, *J* = 8.7, 4.8, 2.5 Hz, 1H), 7.87 (s, 1H), 7.79 - 7.72 (m, 1H), 7.35 (dd, *J =* 10.4, 8.7 Hz, 1H), 6.85 (s, 1H), 5.53 (d, *J =* 1.9 Hz, 2H), 2.97 (s, 3H), 2.15 (s, 3H), 2.10 (s, 3H).

### Example 12 Synthesis of Compounds 20, 20-P1, and 20-P2

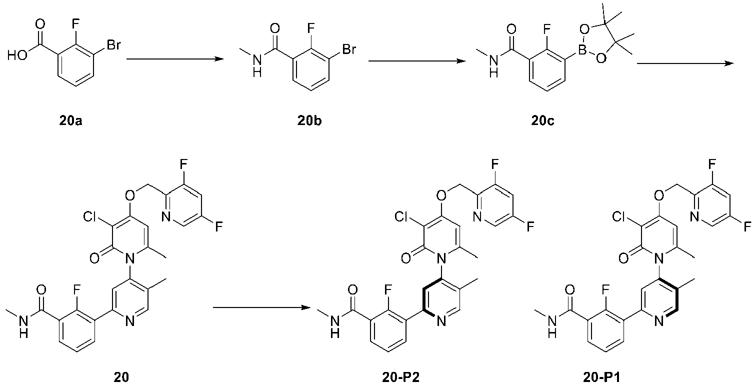

### Step 1: Synthesis of Compound 20b

Methylamine (283 mg, 9.13 mmol), 1-(3-dimethylaminopropyl)-3-ethylcarbodiimide hydrochloride (2.63 g, 13.70 mmol), 1-hydroxybenzotriazole (1.85 g, 13.70 mmol), and triethylamine (1.38 mg, 13.7 mmol) were sequentially added to a solution of compound 20a (1.00 g, 4.56 mmol) in dichloromethane (50 mL). The reaction mixture was stirred at room temperature for 12 hours. After the reaction was completed, the reaction mixture was diluted with water (30 mL) and extracted with dichloromethane (30 mL × 3). The combined organic phases were dried over anhydrous sodium sulfate and filtered. The filtrate was concentrated under reduced pressure, and the resulting residue was purified by silica gel column chromatography (petroleum ether/ethyl acetate = 1/0 to 5/2) to obtain compound 20b (500 mg, yield: 42.5%).

MS m/z (ESI): 231.9, 233.9 [M+1]⁺.

### Step 2: Synthesis of Compound 20c

Bis(pinacolato)diboron (558 mg, 2.2 mmol), potassium carbonate (588 mg, 6.0 mmol), and 1,1'-bis(diphenylphosphino)ferrocene palladium(II)dichloride (146 mg, 0.20 mmol) were sequentially added to a mixed solution of compound 20b (464 mg, 2.0 mmol) in 1,4-dioxane and water (30 mL/3 mL). The reaction mixture was heated to 90°C and stirred at this temperature for 12 hours. After the reaction was completed, the reaction mixture was filtered, and the filtrate was concentrated under reduced pressure. The resulting residue was purified by silica gel column chromatography (petroleum ether/ethyl acetate = 1/0 to 3/1) to obtain compound 20c (300 mg, yield: 53.8%).

MS m/z (ESI): 280.0 [M+1]⁺.

### Step 3: Synthesis of Compound 20

1,1'-Bis(diphenylphosphino)ferrocene-palladium(II)dichloride dichloromethane complex (44 mg, 0.053 mmol) was added to a mixed solution of compound 20c (150 mg, 0.53 mmol), compound A2 (221 mg, 0.54 mmol), and potassium carbonate (148 mg, 1.07 mmol) in 1,4-dioxane and water (10 mL/1 mL). The reaction mixture was heated to 90°C and stirred at this temperature overnight. After the reaction was completed, the reaction mixture was concentrated under reduced pressure, and the resulting residue was purified by silica gel column chromatography (dichloromethane/methanol = 1/0 to 4/1) to obtain a crude product of compound 20 (120 mg). The crude product was further purified by preparative HPLC (chromatographic column: Gemini-C18, 150 × 21.2 mm, 5 µm; mobile phase: acetonitrile-water (0.1% formic acid); gradient: 40-50%; column temperature: 25°C; flow rate: 20 mL/min; wavelength: 214 nm; column pressure: 80 bar) to obtain compound 20 (21.3 mg, yield: 7.6%).

MS m/z (ESI): 528.7 [M+1]⁺.

¹H NMR (400 MHz, CD₃OD) δ 8.82 (s, 1H), 8.48 (d, *J* = 2.4 Hz, 1H), 8.05 (td, *J =* 7.6, 1.8 Hz, 1H), 7.83 - 7.71 (m, 3H), 7.44 (t, *J* = 7.7 Hz, 1H), 6.85 (s, 1H), 5.52 (d, *J =* 1.9 Hz, 2H), 2.96 (s, 3H), 2.18 (s, 3H), 2.11 (s, 3H).

Compound 20 (100 mg, scale-up batch) was resolved by supercritical fluid chiral chromatography (equipment: SFC Thar prep 80, column: CHIRALPAK AD-H 250 mm × 20 mm, 5 µm, mobile phase: 40% ethanol (ethanol/carbon dioxide, 0.2% ammonia water), total flow rate: 12.5 g/min) to obtain compounds 20-P1 (35.4 mg, yield: 35.4%) and 20-P2 (32.1 mg, yield: 32.1%).

### Compound 20-P1:

MS m/z (ESI): 528.7 [M+1]⁺. Supercritical fluid chromatography (SFC): retention time = 2.20 min, UV = 214 nm. ¹H NMR (400 MHz, CD₃OD) δ 8.77 (s, 1H), 8.45 (d, J = 2.4 Hz, 1H), 8.02 (td, J = 7.6, 1.8 Hz, 1H), 7.81 - 7.66 (m, 3H), 7.40 (t, J = 7.7 Hz, 1H), 6.81 (s, 1H), 5.49 (d, J = 1.9 Hz, 2H), 2.93 (s, 3H), 2.14 (s, 3H), 2.08 (s, 3H).

### Compound 20-P2:

MS m/z (ESI): 528.7 [M+1]⁺. Supercritical fluid chromatography (SFC): retention time = 3.20 min, UV = 214 nm. ¹H NMR (400 MHz, CD₃OD) δ 8.77 (s, 1H), 8.44 (d, J = 2.4 Hz, 1H), 8.01 (td, J = 7.6, 1.8 Hz, 1H), 7.83 - 7.64 (m, 3H), 7.40 (t, J = 7.7 Hz, 1H), 6.81 (s, 1H), 5.49 (d, J = 1.9 Hz, 2H), 2.93 (d, J = 3.6 Hz, 3H), 2.14 (s, 3H), 2.07 (s, 3H).

### Example 13 Synthesis of Compounds 21, 21-P1, and 21-P2

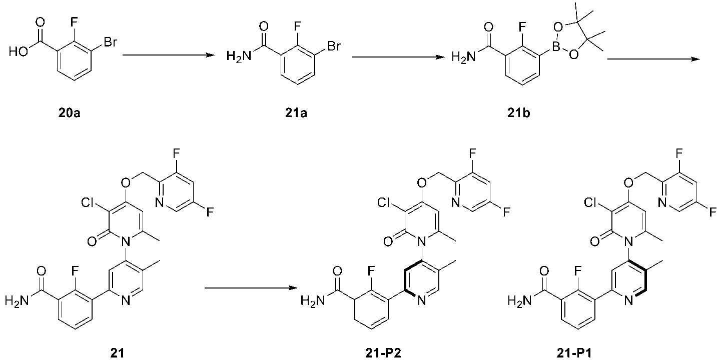

### Step 1: Synthesis of Compound 21a

At 0°C, *N,N*-dimethylformamide (two drops) and oxalyl chloride (1285.14 mg, 10.05 mmol) were sequentially added to a solution of compound 20a (2 g, 9.13 mmol) in dichloromethane (20 mL). The reaction mixture was stirred for 15 minutes, and the resulting residue was concentrated under reduced pressure. Ammonia water (10 mL) and N,N-diisopropylethylamine (2.35 g, 18.26 mmol) were added to the residue, and the resulting mixture was stirred at 0°C for 15 minutes. After the reaction was completed, the reaction mixture was concentrated under reduced pressure. The residue was purified by silica gel column chromatography (dichloromethane/methanol = 1/0 to 10/1) to obtain compound 21a (1.4 g, yield: 63.3%). MS m/z (ESI): 217.9 [M+1]⁺.

### Step 2: Synthesis of Compound 21b

Bis(pinacolato)diboron (1665 mg, 6.56 mmol), potassium acetate (1170 mg, 11.93 mmol), and 1,1'-bis(diphenylphosphino)ferrocene palladium(II)dichloride (493 mg, 0.60 mmol) were sequentially added to a solution of compound 21a (1.3 g, 5.96 mmol) in 1,4-dioxane (20 mL). The reaction mixture was heated to 90°C and stirred at this temperature for 12 hours. After the reaction was completed, the reaction mixture was filtered, and the filtrate was concentrated under reduced pressure. The residue was purified by silica gel column chromatography (petroleum ether/ethyl acetate = 1/0 to 3/1) to obtain compound 21b (1.0 g, yield: 56.9%). MS m/z (ESI): 266.0 [M+1]⁺.

### Step 3: Synthesis of Compounds 21-P1 and 21-P2

Compound 21b (200 mg, 0.75 mmol), potassium carbonate (208.56 mg, 1.51 mmol), and 1,1'-bis(diphenylphosphino)ferrocene palladium(II)dichloride (62.38 mg, 0.08 mmol) were sequentially added to a mixed solution of compound A2 (342.12 mg, 0.83 mmol) in 1,4-dioxane and water (10 mL/1 mL). The reaction mixture was heated to 90°C under a nitrogen atmosphere and stirred at this temperature for 12 hours. After the reaction was completed, the reaction mixture was concentrated under reduced pressure, and the residue was purified by silica gel column chromatography (dichloromethane/methanol = 1/0 to 10/1) to obtain a crude product of compound 21. The crude product of compound 21 was further resolved by supercritical fluid chiral chromatography (equipment: SFC Thar prep 80, column: CHIRALPAK AD-H 250 mm × 20 mm, 5 µm, mobile phase: 40% ethanol (ethanol/carbon dioxide, 0.2% ammonia water), total flow rate: 40 g/min) to obtain compounds 21-P1 (33.3 mg, yield: 8.5%) and 21-P2 (32.6 mg, yield: 8.3%).

### Compound 21-P1:

MS m/z (ESI): 514.8 [M+1]⁺. Supercritical fluid chromatography (SFC): retention time = 2.56 min, UV = 214 nm. ¹H NMR (400 MHz, CD₃OD) δ 8.77 (s, 1H), 8.44 (d, *J =* 2.4 Hz, 1H), 8.04 (td, *J =* 7.6, 1.8 Hz, 1H), 7.88 - 7.80 (m, 1H), 7.78 - 7.65 (m, 2H), 7.41 (t, *J* = 7.7 Hz, 1H), 6.81 (s, 1H), 5.49 (d, *J =* 1.9 Hz, 2H), 2.14 (s, 3H), 2.08 (s, 3H).

### Compound 21-P2:

MS m/z (ESI): 514.7 [M+1]⁺. Supercritical fluid chromatography (SFC): retention time = 3.68 min, UV = 214 nm. 1H NMR (400 MHz, CD₃OD) δ 8.77 (s, 1H), 8.44 (d, J = 2.4 Hz, 1H), 8.04 (td, J = 7.6, 1.8 Hz, 1H), 7.90 - 7.80 (m, 1H), 7.72 (ddd, J = 12.0, 7.9, 3.2 Hz, 2H), 7.41 (t, J = 7.7 Hz, 1H), 6.81 (s, 1H), 5.49 (d, J = 1.9 Hz, 2H), 2.14 (s, 3H), 2.08 (s, 3H).

### Example 14 Synthesis of Compound 23

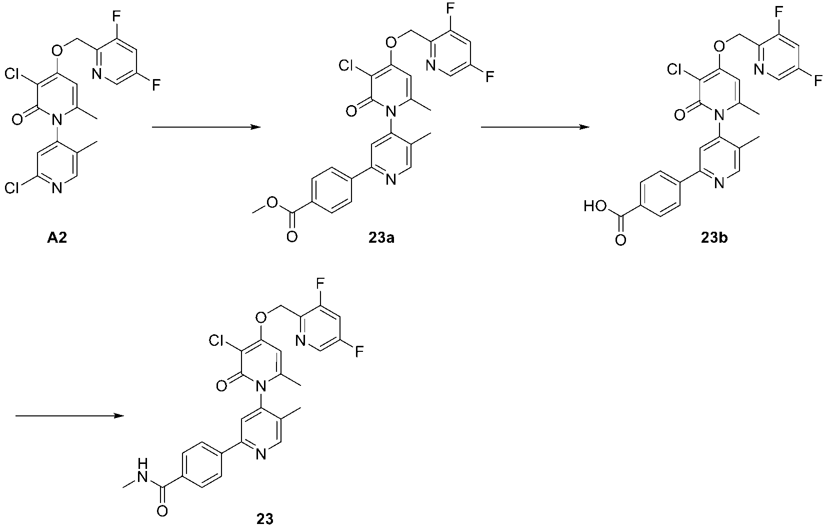

### Step 1: Synthesis of Compound 23a

1,1'-Bis(diphenylphosphino)ferrocene palladium(II)dichloride (30 mg, 0.036 mmol) was added to a mixed solution of compound A2 (150 mg, 0.36 mmol), 4-methoxycarbonylphenylboronic acid (78 mg, 0.43 mmol), and potassium carbonate (100 mg, 0.72 mmol) in 1,4-dioxane and water (10 mL/1 mL). Under a nitrogen atmosphere, the reaction mixture was heated to 90°C and stirred at this temperature for 12 hours. After the reaction was completed, the reaction mixture was concentrated under reduced pressure. The resulting residue was purified by silica gel column chromatography (petroleum ether/ethyl acetate = 1/0 to 5/1) to obtain compound 23a (150 mg, yield: 72.46%). MS m/z (ESI): 511.8 [M+1]⁺.

### Step 2: Synthesis of Compound 23b

Lithium hydroxide (24 mg, 0.58 mmol) was added to a mixed solution of compound 23a (150 mg, 0.24 mmol) in tetrahydrofuran and water (5 mL/5 mL). The reaction mixture was stirred at room temperature for 12 hours. After the reaction was completed, the reaction mixture was concentrated under reduced pressure to obtain compound 23b (150 mg, crude product), which was directly used in the next step without purification. MS m/z (ESI): 498.8 [M+1]⁺.

### Step 3: Synthesis of Compound 23

Methylamine (46 mg, 1.5 mmol), 1-(3-dimethylaminopropyl)-3-ethylcarbodiimide hydrochloride (231 mg, 1.2 mmol), 1-hydroxybenzotriazole (162 mg, 1.2 mmol), and triethylamine (182 mg, 1.8 mmol) were sequentially added to a solution of compound 23b (150 mg, 0.3 mmol) in dichloromethane (10 mL). The reaction mixture was stirred at room temperature for 12 hours. After the reaction was completed, the reaction mixture was filtered, and the filtrate was concentrated under reduced pressure. The resulting residue was purified by preparative HPLC (chromatographic column: Gemini-C18, 150 × 21.2 mm, 5 µm; mobile phase: acetonitrile-water (0.1% formic acid), gradient: 20-60%, temperature: 45°C; pressure: 80 bar; wavelength: 214/254 nm; flow rate: 20 mL/min) to obtain compound 23 (43.2 mg, yield: 27.8%). MS m/z (ESI): 510.8 [M+1]⁺. ¹H NMR (400 MHz, DMSO-*d₆*) δ 8.78 (s, 1H), 8.61 (d, *J* = 2.3 Hz, 1H), 8.54 (d, *J* = 4.6 Hz, 1H), 8.23 (s, 1H), 8.21 (s, 1H), 8.15 - 8.09 (m, 1H), 8.08 (d, *J* = 2.6 Hz, 1H), 7.96 (s, 1H), 7.94 (s, 1H), 6.82 (s, 1H), 5.50 (d, *J* = 1.4 Hz, 2H), 2.81 (d, *J* = 4.5 Hz, 3H), 2.05 (s, 3H), 2.00 (s, 3H).

### Example 15 Synthesis of Compounds 29, 29-P1, and 29-P2

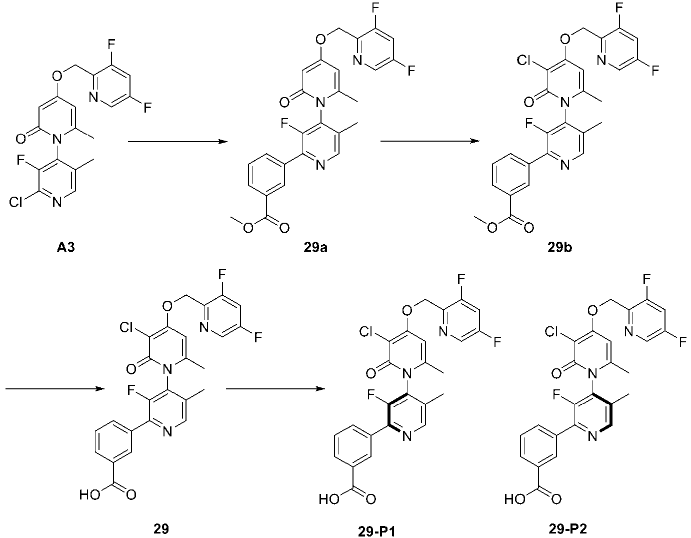

### Step 1: Synthesis of Compound 29a

1,1'-Bis(diphenylphosphino)ferrocene palladium(II)dichloride (133 mg, 0.18 mmol) was added to a mixed solution of compound A3 (360 mg, 0.91 mmol), 3-methoxycarbonylphenylboronic acid (246 mg, 1.37 mmol), and potassium carbonate (251 mg, 1.82 mmol) in 1,4-dioxane and water (10 mL/2 mL). Under an argon atmosphere, the reaction mixture was heated to 90°C and stirred at this temperature for 16 hours. After the reaction was completed, the reaction mixture was concentrated under reduced pressure. The resulting residue was purified by silica gel column chromatography (petroleum ether/ethyl acetate = 1/0 to 1/1) to obtain compound 29a (400 mg, yield: 88.7%). MS m/z (ESI): 496.1 [M+1]⁺.

### Step 2: Synthesis of Compound 29b

*N*-Chlorosuccinimide (123 mg, 0.92 mmol) was added to a solution of compound 29a (380 mg, 0.77 mmol) in isopropanol (50 mL). The reaction mixture was heated to 80°C and stirred at this temperature for 3 hours. After the reaction was completed, the reaction mixture was concentrated under reduced pressure. The resulting residue was purified by silica gel column chromatography (petroleum ether/ethyl acetate = 1/0 to 1/1) to obtain compound 29b (400 mg, yield: 98%). MS m/z (ESI): 530.0 [M+1]⁺.

### Step 3: Synthesis of Compound 29

Lithium hydroxide (95 mg, 2.26 mmol) was added to a mixed solution of compound 29b (400 mg, 0.75 mmol) in tetrahydrofuran and water (20 mL/5 mL). The reaction mixture was stirred at room temperature for 16 hours. After the reaction was completed, the reaction mixture was concentrated under reduced pressure, diluted with water (20 mL), and adjusted to pH = 5-6 by dropwise addition of 1 M hydrochloric acid. The mixture was then extracted with dichloromethane (20 mL × 3). The combined organic phases were washed with saturated brine (30 mL × 1), dried over anhydrous sodium sulfate, and filtered, and the filtrate was concentrated under reduced pressure to obtain a crude product of compound 29 (360 mg, crude product). The crude product of compound 29 (100 mg) was resolved by preparative supercritical fluid chiral chromatography (equipment: SFC Thar prep 80, column: CHIRALPAK AD-H 250 mm × 20 mm, 5 µm, mobile phase: 40% ethanol (ethanol/carbon dioxide, 0.2% ammonia water), flow rate: 15 g/min) to obtain compounds 29-P1 (30 mg, yield: 30%) and 29-P2 (25 mg, yield: 25%).

### Compound 29-P1:

MS m/z (ESI): 515.8.0 [M+1]⁺. Supercritical fluid chromatography (SFC): retention time = 3.32 min, UV = 220 nm. ¹H NMR (400 MHz, DMSO-*d₆*) δ 13.27 (s, 1H), 8.73 (s, 1H), 8.61 (d, *J =* 2.3 Hz, 1H), 8.54 (s, 1H), 8.19 (d, *J* = 7.0 Hz, 1H), 8.15 - 8.03 (m, 2H), 7.68 (t, *J* = 7.8 Hz, 1H), 6.90 (s, 1H), 5.52 (s, 2H), 2.13 (s, 3H), 2.08 (s, 3H).

### Compound 29-P2:

MS m/z (ESI): 515.8 [M+1]⁺. Supercritical fluid chromatography (SFC): retention time = 10.38 min, UV = 220 nm. ¹H NMR (400 MHz, DMSO-*d₆*) δ 13.29 (s, 1H), 8.73 (s, 1H), 8.61 (d, *J =* 2.4 Hz, 1H), 8.54 (s, 1H), 8.19 (d, *J* = 7.1 Hz, 1H), 8.15 - 8.03 (m, 2H), 7.68 (t, *J* = 7.8 Hz, 1H), 6.90 (s, 1H), 5.52 (s, 2H), 2.13 (s, 3H), 2.08 (s, 3H).

### Example 16 Synthesis of Compounds 30, 30-P1, and 30-P2

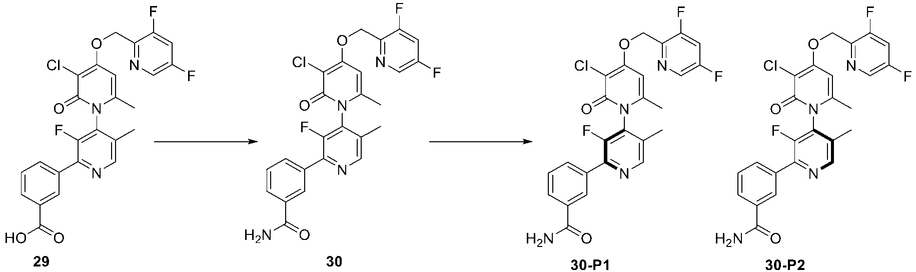

### Step 1: Synthesis of Compound 30

At room temperature, a solution of ammonia in tetrahydrofuran (1.5 mL, 0.4 M), 1-(3-dimethylaminopropyl)-3-ethylcarbodiimide hydrochloride (74 mg, 0.38 mmol), 1-hydroxybenzotriazole (52 mg, 0.38 mmol), and *N,N-*diisopropylethylamine (100 mg, 0.78 mmol) were sequentially added to a solution of compound 29 (100 mg, 0.19 mmol) in dichloromethane (10 mL). The reaction mixture was stirred at room temperature for 12 hours. After the reaction was completed, the reaction mixture was diluted with water (20 mL) and extracted with dichloromethane (20 mL × 3). The combined organic phases were dried over anhydrous sodium sulfate and filtered. The filtrate was concentrated under reduced pressure, and the resulting residue was purified by silica gel column chromatography (dichloromethane/methanol = 1/0 to 10/1) to obtain compound 30 (100 mg). The compound 30 was resolved by preparative supercritical fluid chiral chromatography (equipment: SFC Thar prep 80, column: CHIRALPAK AD-H 250 mm × 20 mm, 5 µm, mobile phase: 40% methanol (methanol/carbon dioxide, 0.2% ammonia water), flow rate: 12.5 g/min) to obtain compounds 30-P1 (30 mg, yield: 30%) and 30-P2 (30 mg, yield: 30%).

### Compound 30-P1:

MS m/z (ESI): 514.7 [M+1]⁺. Supercritical fluid chromatography (SFC): retention time = 4.25 min, UV = 254 nm. ¹H NMR (400 MHz, CDCl₃) δ 8.54 (d, J = 0.5 Hz, 1H), 8.40 (d, *J* = 2.3 Hz, 1H), 8.37 (d, *J =* 1.0 Hz, 1H), 8.08 (dd, *J* = 7.9, 1.2 Hz, 1H), 7.95 - 7.89 (m, 1H), 7.54 (t, *J* = 7.8 Hz, 1H), 7.35-7.33 (m, 1H), 6.56 (s, 1H), 6.45 (s, 1H), 5.81 (s, 1H), 5.42 (d, *J =* 1.8 Hz, 2H), 2.17 (s, 3H), 2.03 (s, 3H).

### Compound 30-P2:

MS m/z (ESI): 514.7 [M+1]⁺. Supercritical fluid chromatography (SFC): retention time = 9.30 min, UV = 254 nm. ¹H NMR (400 MHz, CDCl₃) δ 8.55 (d, *J =* 0.6 Hz, 1H), 8.40 (d, *J* = 2.3 Hz, 1H), 8.37 (d, *J =* 1.1 Hz, 1H), 8.10 (dd, *J* = 7.9, 1.2 Hz, 1H), 8.01 - 7.90 (m, 1H), 7.56 (t, *J* = 7.8 Hz, 1H), 7.54-7.33 (m, 1H), 6.45 (s, 2H), 5.73 (s, 1H), 5.42 (d, *J =* 1.8 Hz, 2H), 2.18 (s, 3H), 2.04 (s, 3H).

### Example 17 Synthesis of Compounds 31, 31-P1, and 31-P2

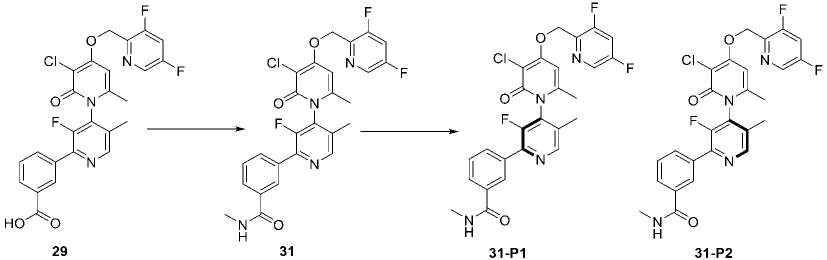

### Step 1: Synthesis of Compound 31

Methylamine hydrochloride (26 mg, 0.38 mmol), 1-(3-dimethylaminopropyl)-3-ethylcarbodiimide hydrochloride (74 mg, 0.38 mmol), 1-hydroxybenzotriazole (52 mg, 0.38 mmol), and *N,N*-diisopropylethylamine (100 mg, 0.78 mmol) were sequentially added to a solution of compound 29 (100 mg, 0.19 mmol) in dichloromethane (10 mL) at room temperature. The reaction mixture was stirred at room temperature for 12 hours. After the reaction was completed, the reaction mixture was diluted with water (20 mL) and extracted with dichloromethane (20 mL × 3). The combined organic phases were dried over anhydrous sodium sulfate and filtered. The filtrate was concentrated under reduced pressure, and the resulting residue was purified by silica gel column chromatography (dichloromethane/methanol = 1/0 to 10/1) to obtain compound 31 (100 mg, yield: 97.5%). The compound 31 was resolved by preparative supercritical fluid chiral chromatography (SFC Thar prep 80, column: CHIRALPAK AD-H 250 mm × 20 mm, 5 µm, mobile phase: 40% ethanol (ethanol/carbon dioxide, 0.2% ammonia water), flow rate: 12.5 g/min) to obtain compounds 31-P1 (40 mg, yield: 40%) and 31-P2 (40 mg, yield: 40%).

### Compound 31-P1:

MS m/z (ESI): 528.8 [M+1]⁺. Supercritical fluid chromatography (SFC): retention time = 3.51 min, UV = 214 nm. ¹HNMR: (400 MHz, DMSO-*d*₆) δ 8.73 (s, 1H), 8.67 - 8.57 (m, 2H), 8.40 (s, 1H), 8.18 - 8.05 (m, 2H), 7.96 (dd, *J =* 7.8, 1.1 Hz, 1H), 7.63 (t, *J* = 7.8 Hz, 1H), 6.90 (s, 1H), 5.53 (s, 2H), 2.81 (d, *J* = 4.5 Hz, 3H), 2.14 (s, 3H), 2.07 (s, 3H).

### Compound 31-P2:

MS m/z (ESI): 528.8 [M+1]⁺. Supercritical fluid chromatography (SFC): retention time = 6.84 min, UV = 214 nm. ¹HNMR (400 MHz, DMSO-*d*₆) δ 8.73 (s, 1H), 8.67 - 8.57 (m, 2H), 8.40 (s, 1H), 8.18 - 8.05 (m, 2H), 7.96 (dd, *J* = 7.8, 1.1 Hz, 1H), 7.63 (t, *J* = 7.8 Hz, 1H), 6.90 (s, 1H), 5.53 (s, 2H), 2.81 (d, *J* = 4.5 Hz, 3H), 2.14 (s, 3H), 2.07 (s, 3H).

### Example 18 Synthesis of Compounds 32, 32-P1, and 32-P2

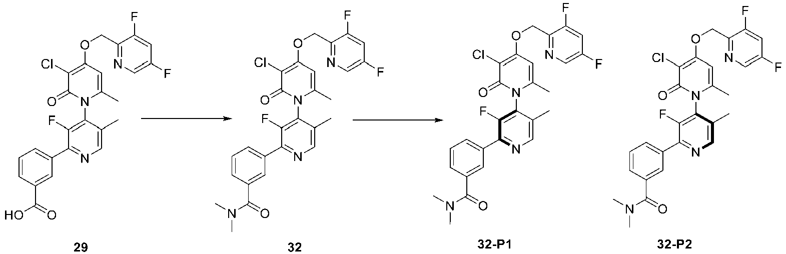

### Step 1: Synthesis of Compound 32

Dimethylamine (14 mg, 0.31 mmol), 1-hydroxybenzotriazole (63 mg, 0.47 mmol), 1-(3-dimethylaminopropyl)-3-ethylcarbodiimide hydrochloride (90 mg, 0.47 mmol), and triethylamine (63 mg, 0.63 mmol) were sequentially added to a solution of compound 29 (81 mg, 0.16 mmol) in dichloroethane (8 mL). The reaction mixture was stirred at room temperature for 12 hours. After the reaction was completed, the reaction mixture was concentrated under reduced pressure, diluted with water (50 mL), and extracted with ethyl acetate (50 mL × 3). The combined organic phases were washed with saturated brine (50 mL × 2), dried over anhydrous sodium sulfate, and filtered. The filtrate was concentrated under reduced pressure, and the resulting residue was purified by silica gel column chromatography (ethyl acetate/petroleum ether = 1/0 to 1/1) to obtain compound 32. The compound 32 was purified by preparative supercritical fluid chiral chromatography (equipment: SFC Thar prep 80; column: CHIRALPAK AD-H, 250 mm × 20 mm; 5 µm, mobile phase: 40% ethanol (ethanol/carbon dioxide; 0.2% ammonia water), flow rate: 15 g/min) to obtain compounds 32-P1 (21.0 mg, yield: 24.7%) and 32-P2 (22.3 mg, yield: 26.2%).

### Compound 32-P1:

MS m/z (ESI): 543.1 [M+1]⁺. Supercritical fluid chromatography (SFC): retention time = 4.08 min, UV = 254 nm. ¹H NMR (400 MHz, CD₃OD) δ 8.66 (s, 1H), 8.48 (d, *J* = 2.3 Hz, 1H), 8.07 (dd, *J* = 7.8, 1.3 Hz, 1H), 8.03 (d, *J =* 1.3 Hz, 1H), 7.80 - 7.72 (m, 1H), 7.64 (t, *J* = 7.7 Hz, 1H), 7.59 - 7.55 (m, 1H), 6.90 (s, 1H), 5.54 (d, *J =* 1.8 Hz, 2H), 3.15 (s, 3H), 3.07 (s, 3H), 2.21 (s, 3H), 2.15 (s, 3H).

### Compound 32-P2:

MS m/z (ESI): 543.1 [M+1]⁺. Supercritical fluid chromatography (SFC): retention time = 9.62 min, UV = 254 nm. ¹H NMR (400 MHz, CD₃OD) δ 8.66 (d, *J* = 0.6 Hz, 1H), 8.48 (d, *J=* 2.3 Hz, 1H), 8.07 (dd, *J* = 7.8, 1.3 Hz, 1H), 8.03 (d, *J =* 1.2 Hz, 1H), 7.79 - 7.71 (m, 1H), 7.64 (t, *J =* 7.7 Hz, 1H), 7.60 - 7.53 (m, 1H), 6.90 (s, 1H), 5.54 (d, *J =* 1.8 Hz, 2H), 3.15 (s, 3H), 3.07 (s, 3H), 2.21 (s, 3H), 2.15 (s, 3H).

### Example 19 Synthesis of Compounds 33, 33-P1, and 33-P2

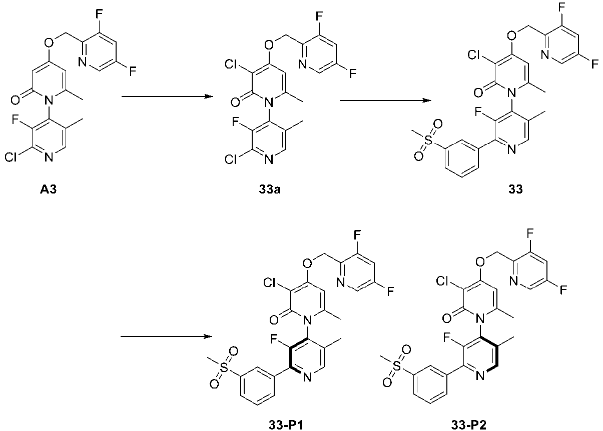

### Step 1: Synthesis of Compound 33a

*N*-Chlorosuccinimide (173 mg, 1.3 mmol) was added to a solution of compound A3 (396 mg, 1.00 mmol) in isopropanol (10 mL). The reaction mixture was heated to 90°C and stirred at this temperature for 3 hours. After the reaction was completed, the reaction mixture was filtered, and the filtrate was concentrated under reduced pressure. The resulting residue was purified by silica gel column chromatography (dichloromethane/methanol = 1/0 to 20/1) to obtain compound 33a (200 mg, yield: 41.8%). MS m/z (ESI): 429.7 [M+1]⁺.

### Step 2: Synthesis of Compound 33

1,1'-Bis(diphenylphosphino)ferrocene palladium(II)dichloride (38 mg, 0.05 mmol) was added to a mixed solution of compound 33a (200 mg, 0.47 mmol), 3-(methylsulfonyl)phenylboronic acid (120 mg, 0.60 mmol), and potassium carbonate (128 mg, 0.93 mmol) in 1,4-dioxane and water (10 mL/1 mL). Under a nitrogen atmosphere, the reaction mixture was heated to 90°C and stirred at this temperature overnight. After the reaction was completed, the reaction mixture was concentrated under reduced pressure, and the resulting residue was purified by silica gel column chromatography (dichloromethane/methanol = 1/0 to 16/1) to obtain a crude product of compound 33 (120 mg). The crude product was purified by preparative supercritical fluid chiral chromatography (equipment: SFC Thar prep 80; column: CHIRALPAK AD-H, 250 mm × 20 mm; 5 µm, mobile phase: 40% ethanol (ethanol/carbon dioxide; 0.2% ammonia water), flow rate: 12.5 g/min) to obtain compounds 33-P1 (59.6 mg, yield: 23.9%) and 33-P2 (53.4 mg, yield: 21.5%).

### Compound 33-P1:

MS m/z (ESI): 549.7 [M+1]⁺. Supercritical fluid chromatography (SFC): retention time = 3.84 min, UV = 220 nm. ¹H NMR (400 MHz, DMSO-*d*₆) δ 8.76 (s, 1H), 8.61 (d, *J* = 2.3 Hz, 1H), 8.49 (s, 1H), 8.30 (d, *J* = 7.1 Hz, 1H), 8.15 - 8.01 (m, 2H), 7.84 (t, *J* = 7.9 Hz, 1H), 6.90 (s, 1H), 5.53 (s, 2H), 3.29 (s, 3H), 2.15 (s, 3H), 2.08 (d, *J =* 3.3 Hz, 3H).

### Compound 33-P2:

MS m/z (ESI): 549.7 [M+1]⁺. Supercritical fluid chromatography (SFC): retention time = 5.29 min, UV = 220 nm. ¹H NMR (400 MHz, DMSO-*d*₆) δ 8.76 (d, *J* = 0.5 Hz, 1H), 8.61 (d, *J* = 2.4 Hz, 1H), 8.48 (s, 1H), 8.30 (d, *J =* 7.0 Hz, 1H), 8.15 - 8.01 (m, 2H), 7.84 (t, *J* = 7.9 Hz, 1H), 6.90 (s, 1H), 5.52 (s, 2H), 3.29 (s, 3H), 2.15 (s, 3H), 2.08 (d, J = 3.2 Hz, 3H).

### Example 20 Synthesis of Compound 34

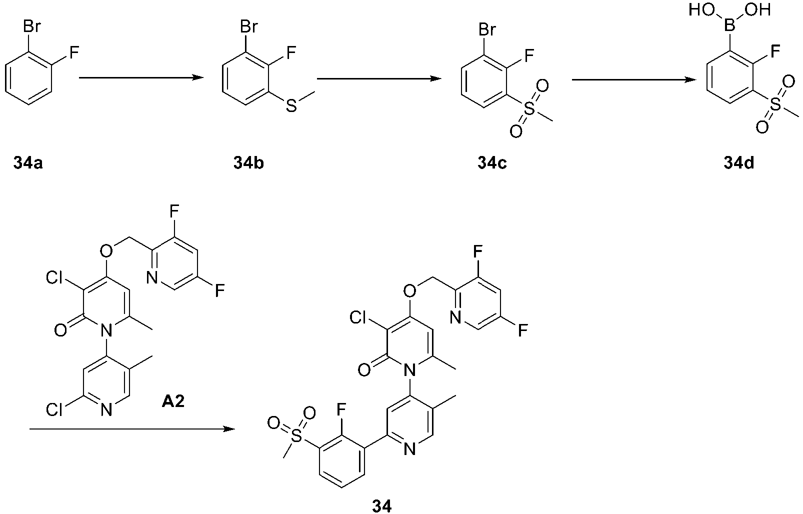

### Step 1: Synthesis of Compound 34b

At -78°C, lithium diisopropylamide (8 mL, 16 mmol) was added to a solution of compound 34a (2.0 g, 11.4 mmol) in tetrahydrofuran (50 mL). Under a nitrogen atmosphere, the reaction mixture was stirred for five minutes, followed by the addition of dimethyl disulfide (1.5 g, 16 mmol). The reaction mixture was stirred at -78°C for another 1 hour. After the reaction was completed, the reaction was quenched with 10% sulfuric acid (50 mL) and extracted with ethyl acetate (100 mL × 3). The combined organic phases were dried over anhydrous sodium sulfate and filtered. The filtrate was concentrated under reduced pressure, and the residue was purified by silica gel column chromatography (petroleum ether/ethyl acetate = 1/0 to 1/1) to obtain compound 34b (1.00 g, yield: 36%). ¹H NMR (400 MHz, DMSO-*d*₆) δ 7.36 - 7.32 (m, 1H), 7.26 - 7.18 (m, 1H), 7.17 - 7.11 (m, 1H), 2.46 (s, 3H).

### Step 2: Synthesis of Compound 34c

*m*-Chloroperoxybenzoic acid (2.33 g, 13.5 mmol) was added to a solution of compound 34b (1 g, 4.5 mmol) in dichloromethane (30 mL), and the reaction mixture was stirred at room temperature for 16 hours. After the reaction was completed, the reaction mixture was filtered, and the filtrate was concentrated under reduced pressure. The residue was purified by silica gel column chromatography (petroleum ether/ethyl acetate = 1/0 to 2/1) to obtain compound 34c (0.7 g, yield: 62.2%). MS m/z (ESI): 252.6 [M+1]⁺.

### Step 3: Synthesis of Compound 34d

1,1'-Bis(diphenylphosphino)ferrocene-palladium(II)dichloride dichloromethane complex (96.73 mg, 0.12 mmol) was added to a solution of compound 34c (300 mg, 1.19 mmol), bis(pinacolato)diboron (451.53 mg, 1.78 mmol), and potassium acetate (232.67 mg, 2.37 mmol) in 1,4-dioxane (10 mL). The reaction mixture was heated to 90°C and stirred at this temperature for 16 hours under a nitrogen atmosphere. After the reaction was completed, the reaction mixture was filtered, and the filtrate was concentrated under reduced pressure to obtain compound 34d (500 mg, crude product). MS m/z (ESI): 218.9 [M+1]⁺.

### Step 4: Synthesis of Compound 34

1,1'-Bis(diphenylphosphino)ferrocene-palladium(II)dichloride dichloromethane complex (135.93 mg, 0.17 mmol) was added to a mixed solution of compound 34d (500 mg, 1.67 mmol), compound A2 (343.34 mg, 0.83 mmol), and potassium carbonate (230.23 mg, 1.67 mmol) in 1,4-dioxane and water (10 mL/1 mL). The reaction mixture was heated to 90°C and stirred at this temperature for 16 hours under a nitrogen atmosphere. After the reaction was completed, the reaction mixture was concentrated under reduced pressure. The residue was diluted with water (50 mL) and extracted with dichloromethane (20 mL × 3). The combined organic phases were dried over anhydrous sodium sulfate and filtered. The filtrate was concentrated under reduced pressure, and the residue was purified by silica gel column chromatography (dichloromethane/methanol = 1/0 to 10/1) to obtain a crude product of compound 34. The crude product was further purified by preparative HPLC (chromatographic column: Gemini-C18; 150 × 21.2 mm, 5 µm; mobile phase: acetonitrile-water (0.1% formic acid); gradient: 40-60%; column temperature: 25°C; flow rate: 20 mL/min; wavelength: 214 nm; column pressure: 80 bar) to obtain compound 34 (11 mg, yield: 7.7%). MS m/z (ESI): 549.8 [M+1]⁺. ¹H NMR (400 MHz, DMSO-*d*₆) δ 8.86 (s, 1H), 8.60 (d, *J =* 2.2 Hz, 1H), 8.32 (t, *J* = 6.9 Hz, 1H), 8.13 - 8.06 (m, 1H), 7.96 (t, *J =* 6.4 Hz, 1H), 7.89 (s, 1H), 7.61 (t, *J =* 7.8 Hz, 1H), 6.82 (s, 1H), 5.49 (s, 2H), 3.39 (s, 3H), 2.09 (s, 3H), 2.00 (s, 3H).

### Example 21 Synthesis of Compounds 35-1, 35-2, 35-P1, 35-P2, 35-P3, and 35-P4

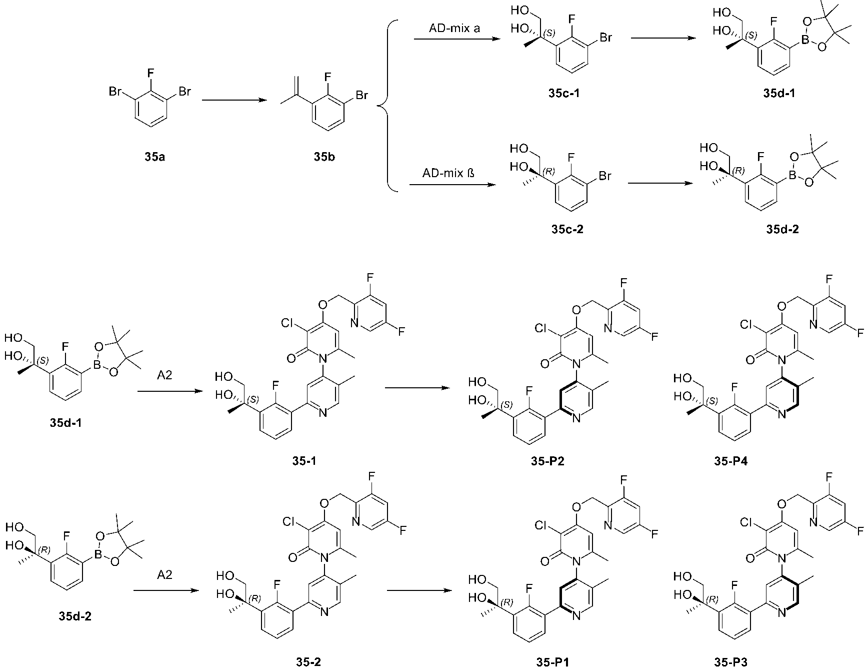

### Step 1: Synthesis of Compound 35b

1,1'-Bis(diphenylphosphino)ferrocene-palladium(II)dichloride dichloromethane complex (488 mg, 0.59 mmol) was added to a mixed solution of compound 35a (3 g, 11.8 mmol), isopropenylboronic acid pinacol ester (1.98 g, 11.8 mmol), and potassium carbonate (3.2 g, 23.6 mmol) in 1,4-dioxane and water (50 mL /10 mL). The reaction mixture was stirred at 80°C for 16 hours. After the reaction was completed, the reaction mixture was diluted with water (200 mL) and extracted with dichloromethane (40 mL × 3). The combined organic phases were dried over anhydrous sodium sulfate and filtered. The filtrate was concentrated under reduced pressure, and the residue was purified by silica gel column chromatography (petroleum ether/ethyl acetate = 1/0 to 20/1) to obtain compound 35b (2 g, yield: 77.92%). ¹HNMR (400 MHz, CDCl₃) δ 7.52 - 7.42 (m, 1H), 7.25 - 7.15 (m, 1H), 7.06 - 6.89 (m, 1H), 5.22 (t, *J* = 14.9 Hz, 2H), 2.14 (d, *J* = 5.4 Hz, 3H).

### Step 2: Synthesis of Compound 35c-1

AD-mix-α (10 g, CAS: 153130-59-7) was added to a mixed solution of compound 35b (1 g, 4.65 mmol) in tert-butanol and water (20 mL/20 mL). The reaction mixture was stirred at room temperature for 16 hours. After the reaction was completed, the reaction mixture was quenched by adding 40 mL of sodium thiosulfate solution, followed by extraction with dichloromethane (10 mL × 5). The combined organic phases were washed with saturated brine (20 mL × 1), dried over anhydrous sodium sulfate, and filtered. The filtrate was concentrated under reduced pressure, and the residue was purified by silica gel column chromatography (dichloromethane/methanol = 1/0 to 20/1) to obtain compound 35c-1 (600 mg, yield: 46.63%). ¹HNMR (400 MHz, CDCl₃) δ 7.63-7.59 (m, 1H), 7.51-7.48 (m, 1H), 7.06 (t, *J* = 7.9Hz, 1H), 4.01 (d, *J* = 12.0 Hz, 1H), 3.75 (d, *J* = 12.3 Hz, 1H), 1.58 (s, 3H).

### Step 3: Synthesis of Compound 35d-1

1,1'-Bis(diphenylphosphino)ferrocene-palladium(II)dichloride dichloromethane complex (98 mg, 0.12 mmol) was added to a solution of compound 35c-1 (600 mg, 2.4 mmol), bis(pinacolato)diboron (917 mg, 3.6 mmol), and potassium acetate (472 mg, 4.8 mmol) in 1,4-dioxane (10 mL). The reaction mixture was stirred at 80°C for 16 hours. After the reaction was completed, the reaction mixture was concentrated under reduced pressure, and the residue was purified by silica gel column chromatography (dichloromethane/methanol = 1/0 to 20/1) to obtain compound 35d-1 (400 mg, yield: 56.07%). MS m/z (ESI):297.1 [M+1]⁺.

### Step 4: Synthesis of Compounds 35-P2 and 35-P4

Tetrakis(triphenylphosphine)palladium (39 mg, 0.05 mmol) was added to a mixed solution of compound A2 (267 mg, 0.67 mmol), compound 35d-1 (200 mg, 0.67 mmol), and potassium carbonate (186 mg, 1.35 mmol) in 1,4-dioxane and water (10 mL/2 mL). The reaction mixture was stirred at 80°C for 16 hours. After the reaction was completed, the reaction mixture was concentrated under reduced pressure, and the residue was purified by silica gel column chromatography (dichloromethane/methanol = 1/0 to 10/1) to obtain compound 35-1 (100 mg). Compound 35-1 (100 mg) was resolved by supercritical fluid chiral chromatography (equipment: SFC Thar prep 80, column: CHIRALPAK AD-H 250 mm × 20 mm, 5 µm, mobile phase: 40% EtOH/CO₂ (NH₄OH 0.2%), total flow rate: 40 g/min) to obtain compounds 35-P2 (4.2 mg, yield: 1.14%) and 35-P4 (5.7 mg, yield: 1.54%).

### Compound 35-P2:

MS m/z (ESI): 546.0 [M+1]⁺. Supercritical fluid chromatography (SFC): retention time = 3.14 min, UV = 250 nm. ¹HNMR (400 MHz, CDCl₃) δ 8.74 (s, 1H), 8.40 (d, J = 2.3Hz, 1H), 7.86 (t, J = 8.3Hz, 1H), 7.73 (t, J = 7.5 Hz, 1H), 7.52 (d, J = 1.4 Hz, 1H), 7.37 - 7.28 (m, 2H), 6.41 (s, 1H), 5.42 (s, 2H), 4.06 (d, J = 11.3 Hz, 1H), 3.74 (d, J = 11.0 Hz, 1H), 2.16 (s, 3H), 2.02 (s, 3H), 1.58 (s, 3H).

### Compound 35-P4:

MS m/z (ESI): 546.0 [M+1]⁺. Supercritical fluid chromatography (SFC): retention time = 6.41 min, UV = 250 nm. ¹H NMR (400 MHz, CDCl₃) δ 8.72 (s, 1H), 8.40 (d, J = 2.3 Hz, 1H), 7.84 (t, J = 10.6 Hz, 1H), 7.71 (t, J = 8.4 Hz, 1H), 7.50 (s, 1H), 7.37 - 7.27 (m, 2H), 6.40 (s, 1H), 5.41 (s, 2H), 4.00 (d, J = 11.2Hz, 1H), 3.73 (d, J = 11.0Hz, 1H), 2.15 (s, 3H), 2.02 (s, 3H), 1.59 (s, 3H).

### Step 5: Synthesis of Compound 35c-2

AD-mix-β (10 g, CAS: 148618-32-0) was added to a mixed solution of compound 35b (1 g, 4.65 mmol) in tert-butanol and water (20 mL/20 mL). The reaction mixture was stirred at room temperature for 16 hours. After the reaction was completed, the reaction mixture was quenched by adding sodium thiosulfate solution (40 mL), followed by extraction with dichloromethane (10 mL × 5). The combined organic phases were washed with saturated brine (20 mL × 1), concentrated under reduced pressure, and the residue was purified by silica gel column chromatography (dichloromethane/methanol = 1/0 to 20/1) to obtain compound 35c-2 (400 mg, yield: 31.08%). ¹HNMR(400 MHz,CDCl₃ ) δ 7.59 (d, *J* = 1.2 Hz, 1H), 7.46 (t, *J* = 1.5Hz, 1H), 7.03 (s, 1H), 5.24 - 5.19 (m, 1H), 5.16 - 5.11 (m, 1H), 1.57 (s, 3H).

### Step 6: Synthesis of Compound 35d-2

1,1'-Bis(diphenylphosphino)ferrocene-palladium(II)dichloride dichloromethane complex (41 mg, 0.05 mmol) was added to a solution of compound 35c-2 (250 mg, 1.0 mmol), bis(pinacolato)diboron (500 mg, 2.0 mmol), and potassium acetate (197 mg, 2.0 mmol) in 1,4-dioxane (8 mL). The reaction mixture was stirred at 80°C for 16 hours. After the reaction was completed, the reaction mixture was concentrated under reduced pressure. The residue was purified by silica gel column chromatography (dichloromethane/methanol = 1/0 to 20/1) to obtain compound 35d-2 (150 mg, yield: 45.42%). MS m/z (ESI):297.1 [M+1]⁺.

### Step 7: Synthesis of Compound 35-P1 and 35-P3

Tetrakis(triphenylphosphine)palladium (28 mg, 0.025 mmol) was added to a mixed solution of compound A2 (200 mg, 0.49 mmol), compound 35d-2 (140 mg, 0.49 mmol), and potassium carbonate (134 mg, 0.97 mmol) in 1,4-dioxane and water (8 mL/2 mL). The reaction mixture was stirred at 80°C for 16 hours. After the reaction was completed, the reaction mixture was concentrated under reduced pressure, and the residue was purified by silica gel column chromatography (dichloromethane/methanol = 1/0 to 10/1) to obtain compound 35-2 (100 mg). Compound 35-2 (100 mg) was resolved by supercritical fluid chiral chromatography (equipment: SFC Thar prep 80, column: CHIRALPAK AD-H 250 mm × 20 mm, 5 µm, mobile phase: 40% EtOH/CO₂ (NH₄OH 0.2%), total flow rate: 40 g/min) to obtain compounds 35-P1 (18.7 mg, yield: 7.07%) and 35-P3 (20.4 mg, yield: 7.71%).

### Compound 35-P1:

MS m/z (ESI): 546.1 [M+1]⁺. Supercritical fluid chromatography (SFC): retention time = 2.66 min, UV = 250 nm. ¹HNMR (400 MHz, CD₃OD) δ 8.72 (s, 1H), 8.43 (d, *J* = 2.4Hz, 1H), 7.80 - 7.74 (m, 2H), 7.70 (dd, *J =* 10.8, 7.3Hz, 1H), 7.65 (s, 1H), 7.28 (t, *J* = 7.8Hz, 1H), 6.79 (s, 1H), 5.48 (d, *J =* 1.9Hz, 2H), 3.82 (d, *J* = 12.2Hz, 1H), 3.72 (d, *J* = 12.4Hz, 1H), 2.12 (s, 3H), 2.07 (d, *J* = 4.2 Hz, 3H), 1.54 (d, *J* = 1.1Hz, 3H).

### Compound 35-P3:

MS m/z (ESI): 546.1 [M+1]⁺. Supercritical fluid chromatography (SFC): retention time = 4.05 min, UV = 250 nm. ¹HNMR (400 MHz,CD₃OD ) δ 8.73 (s, 1H), 8.43 (d, *J =* 2.4 Hz, 1H), 7.80 - 7.74 (m, 2H), 7.73 - 7.67 (m, 1H), 7.64 (d, *J* =2.0 Hz, 1H), 7.28 (t, *J* = 7.8 Hz, 1H), 6.79 (s, 1H), 5.48 (d, *J =* 1.9 Hz, 2H), 3.84 (d, *J* = 12.1Hz, 1H), 3.74 - 3.69 (m, 1H), 2.12 (s, 3H), 2.07 (s, 3H), 1.53 (d, *J* = 1.0 Hz, 3H).

### Example 22 Synthesis of Compound 37-1

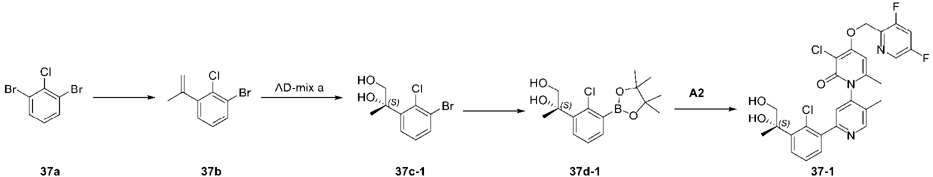

### Step 1: Synthesis of Compound 37b

1,1'-Bis(diphenylphosphino)ferrocene-palladium(II)dichloride dichloromethane complex (301 mg, 0.37 mmol) was added to a mixed solution of compound 37a (2.0 g, 7.40 mmol), isopropenylboronic acid pinacol ester (1.2 g, 7.40 mmol), and potassium carbonate (2.0 g, 14.80 mmol) in 1,4-dioxane and water (30 mL/6 mL). The reaction mixture was stirred at 80°C for 16 hours. After the reaction was completed, the reaction mixture was diluted with water (100 mL) and extracted with dichloromethane (100 mL × 3). The combined organic phases were dried over anhydrous sodium sulfate and filtered. The filtrate was concentrated under reduced pressure, and the residue was purified by silica gel column chromatography (petroleum ether/ethyl acetate = 1/0 to 20:1) to obtain compound 37b (1.2 g, yield: 63.06%). ¹HNMR (400 MHz, CDCl₃) δ 7.44 (dd, *J* = 7.8, 1.5 Hz, 1H), 7.04 (dd, *J* = 7.6, 1.5 Hz, 1H), 6.98 (dd, *J* = 13.4, 5.7 Hz, 1H), 5.18 (dt, *J* = 3.3, 1.6 Hz, 1H), 4.84 - 4.82 (m, 1H), 2.00 (s, 3H).

### Step 2: Synthesis of Compound 37c-1

AD-mix-α (6 g, CAS: 153130-59-7) was added to a mixed solution of compound 37b (600 mg, 2.59 mmol) in *tert-*butanol and water (10 mL/10 mL). The reaction mixture was stirred at room temperature for 16 hours. After the reaction was completed, the reaction mixture was quenched with sodium thiosulfate solution (40 mL), followed by extraction with dichloromethane (40 mL × 3). The combined organic phases were washed with saturated brine (20 mL × 1), concentrated under reduced pressure, and the residue was purified by silica gel column chromatography (dichloromethane/methanol = 1/0 to 20/1) to obtain compound 37c-1 (200 mg, yield: 26.16%). ¹HNMR (400 MHz, CDCl₃) δ 7.78 (dd, *J* = 8.0, 1.6 Hz, 1H), 7.59 (dd, *J* = 7.9, 1.6 Hz, 1H), 7.17 - 7.10 (m, 1H), 4.31 (dd, *J* = 11.2, 4.6 Hz, 1H), 3.82 (dd, *J* = 11.2, 3.6 Hz, 1H), 1.67 (s, 3H).

### Step 3: Synthesis of Compound 37d-1

1,1'-Bis(diphenylphosphino)ferrocene-palladium(II)dichloride dichloromethane complex (61 mg, 0.08 mmol) was added to a solution of compound 37c-1 (200 mg, 0.75 mmol), bis(pinacolato)diboron (230 mg, 0.90 mmol), and potassium acetate (147 mg, 1.50 mmol) in 1,4-dioxane (20 mL). The reaction mixture was stirred at 80°C for 16 hours. After the reaction was completed, the reaction mixture was filtered, and the filtrate was concentrated under reduced pressure to obtain compound 37d-1 (200 mg, crude product). MS m/z (ESI):312.9 [M+1]⁺.

### Step 4: Synthesis of Compound 37-1

Tetrakis(triphenylphosphine)palladium (36 mg, 0.03 mmol) was added to a mixed solution of compound 37d-1 (200 mg, 0.63 mmol), compound A2 (130 mg, 0.32 mmol), and sodium carbonate (67 mg, 0.63 mmol) in 1,4-dioxane and water (10 mL/2 mL). The reaction mixture was stirred at 80°C for 16 hours. After the reaction was completed, the reaction mixture was concentrated under reduced pressure. The residue was purified by silica gel column chromatography (dichloromethane/methanol = 1/0 to 30:1) to obtain a crude product (100 mg). The crude product (100 mg) was further purified by preparative HPLC (chromatographic column: AQ-C18; 150 × 21.2 mm, 5 µm; mobile phase: acetonitrile-water (0.1% formic acid); gradient: 28-45%; column temperature: 25°C; flow rate: 20 mL/min; wavelength: 214 nm; column pressure: 80 bar) to obtain compound 37-1 (25.4 mg, yield: 6.71%). MS m/z (ESI): 562.1 [M+1]⁺. ¹H NMR (400 MHz, CD₃OD) δ 8.73 (s, 1H), 8.47 (d, *J* = 2.3 Hz, 1H), 8.07 - 7.99 (m, 1H), 7.78 - 7.71 (m, 1H), 7.54 (s, 1H), 7.49 - 7.43 (m, 2H), 6.84 (s, 1H), 5.51 (s, 2H), 4.14 (dd, *J* = 21.3, 11.3 Hz, 1H), 3.91 (dd, *J =* 11.3, 4.8 Hz, 1H), 2.18 (s, 3H), 2.13 (d, *J* = 2.9 Hz ,3H), 1.69 (d, *J* = 7.1 Hz, 3H).

### Example 23 Synthesis of Compounds 50-1, 50-2, 50-P1, 50-P2, 50-P3, and 50-P4

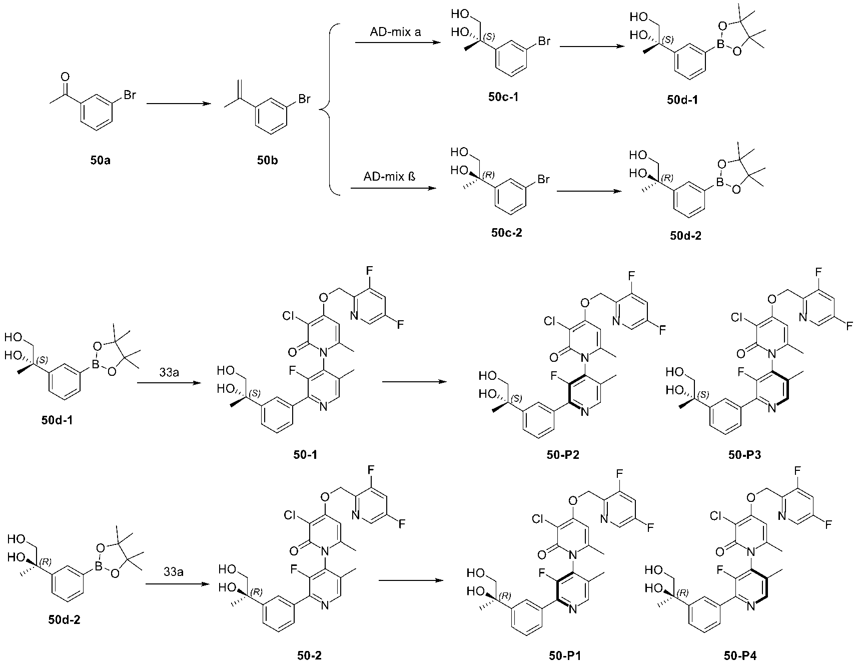

### Step 1: Synthesis of Compound 50b

Methylphosphonium ylide (17.2 g, 48.2 mmol) was added to a solution of potassium tert-butoxide (5.41 g, 48.2 mmol) in tetrahydrofuran (100 mL). The mixture was stirred at room temperature for 0.5 hours, then cooled to 0°C. Compound 50a (8 g, 40.2 mmol) was added, and the reaction mixture was stirred at room temperature for 3 hours. After the reaction was completed, the reaction mixture was quenched with water (100 mL) and extracted with dichloromethane (50 mL × 3). The combined organic phases were washed with saturated brine (20 mL × 1) and concentrated under reduced pressure. The residue was purified by silica gel column chromatography (petroleum ether) to obtain compound 50b (1.5 g, yield: 17.91%). ¹H NMR (400 MHz, CDCl₃) δ 7.59 (t, *J* = 1.8 Hz, 1H), 7.45 - 7.33 (m, 2H), 7.18 (t, *J* = 7.9 Hz, 1H), 5.36 (s, 1H), 5.17 - 5.05 (m, 1H), 2.14 - 2.09 (m, 3H).

### Step 2: Synthesis of Compound 50c-1

AD-mix-α (7.5 g, CAS: 153130-59-7) was added to a mixed solution of compound 50b (0.75 g, 3.8 mmol) in *tert-*butanol and water (20 mL/20 mL). The reaction mixture was stirred at room temperature for 16 hours. After the reaction was completed, the reaction mixture was quenched with sodium thiosulfate solution (40 mL) and extracted with dichloromethane (40 mL × 3). The combined organic phases were washed with saturated brine (80 mL × 1) and concentrated under reduced pressure. The residue was purified by silica gel column chromatography (petroleum ether/ethyl acetate = 1/0 to 1/1) to obtain compound 50c-1 (500 mg, yield: 51.17%). ¹H NMR (400 MHz, CD₃OD) δ 7.72 (t, *J* = 1.8 Hz, 1H), 7.50 - 7.33 (m, 2H), 7.24 (t, *J* = 7.9 Hz, 1H), 3.61 (q, *J* = 11.2 Hz, 2H), 1.51 (s, 3H).

### Step 3: Synthesis of Compound 50d-1

1,1'-Bis(diphenylphosphino)ferrocene-palladium(II)dichloride dichloromethane complex (158 mg, 0.21 mmol) was added to a solution of compound 50c-1 (500 mg, 2 mmol), bis(pinacolato)diboron (604 mg, 2.38 mmol), and potassium acetate (424 mg, 4.32 mmol) in 1,4-dioxane (15 mL). The reaction mixture was stirred at 90°C for 16 hours. After the reaction was completed, the reaction mixture was filtered, and the filtrate was concentrated under reduced pressure to obtain compound 50d-1 (500 mg, crude product).

### Step 4: Synthesis of Compounds 50-P2 and 50-P3

Tetrakis(triphenylphosphine)palladium (103 mg, 0.09 mmol) was added to a mixed solution of compound 50d-1 (500 mg, crude product), compound 33a (143 mg, 0.33 mmol), and sodium carbonate (190 mg, 1.79 mmol) in 1,4-dioxane and water (15 mL/1.5 mL). The reaction mixture was stirred at 90°C for 16 hours. After the reaction was completed, the reaction mixture was concentrated under reduced pressure. The residue was subjected to silica gel column chromatography (dichloromethane/methanol = 1/0 to 10/1) to obtain a crude product (100 mg). The crude product (100 mg) was purified by preparative HPLC (chromatographic column: Gemini-C18; 150 × 21.2 mm, 5 µm; mobile phase: acetonitrile-water (0.1% formic acid); gradient: 30-40%; column temperature: 25°C; flow rate: 20 mL/min; wavelength: 214 nm; column pressure: 80 bar) to obtain compound 50-1 (60 mg). Compound 50-1 (60 mg) was resolved by supercritical fluid chiral chromatography (instrument: SFC Thar prep 80; column: CHIRALPAK AD-H 250 mm × 20 mm, 5 µm; mobile phase: 40% EtOH/CO₂ (NH₄OH 0.2%); total flow rate: 40 g/min) to obtain compounds 50-P2 (27.5 mg, yield: 1.4%) and 50-P3 (25.8 mg, yield: 1.32%).

### Compound 50-P2:

MS m/z (ESI): 546.0 [M+1]⁺. Supercritical fluid chromatography (SFC): retention time = 2.52 min, UV = 214 nm. ¹H NMR (400 MHz, CD₃OD) δ 8.63 (s, 1H), 8.48 (d, *J* = 2.1 Hz, 1H), 8.08 (d, *J =* 1.2 Hz, 1H), 7.84 - 7.71 (m, 2H), 7.65 (d, *J =* 7.9 Hz, 1H), 7.50 (t, *J =* 7.8 Hz, 1H), 6.90 (s, 1H), 5.54 (d, *J =* 1.7 Hz, 2H), 3.67 (q, *J =* 11.2 Hz, 2H), 2.17 (d, *J =* 20.6 Hz, 6H), 1.58 (s, 3H).

### Compound 50-P3:

MS m/z (ESI): 546.1 [M+1]⁺. Supercritical fluid chromatography (SFC): retention time = 4.78 min, UV = 214 nm. ¹H NMR (400 MHz, CD₃OD) δ 8.51 (s, 1H), 8.36 (d, *J =* 2.2 Hz, 1H), 7.96 (d, *J* = 1.3 Hz, 1H), 7.84 - 7.71 (m, 2H), 7.53 (d, *J* = 7.9 Hz, 1H), 7.38 (t, *J =* 7.8 Hz, 1H), 6.77 (s, 1H), 5.41 (d, *J =* 1.8 Hz, 2H), 3.55 (q, *J* = 11.2 Hz, 2H), 2.05 (d, *J* = 20.5 Hz, 6H), 1.46 (s, 3H).

### Step 5: Synthesis of Compound 50c-2

AD-mix-β (7.5 g, CAS: 148618-32-0) was added to a mixed solution of compound 50b (0.75 g, 3.48 mmol) in *tert-*butanol and water (20 mL/20 mL). The reaction mixture was stirred at room temperature for 16 hours. After the reaction was completed, the reaction mixture was quenched with sodium thiosulfate solution (40 mL) and extracted with dichloromethane (40 mL × 3). The combined organic phases were washed with saturated brine (50 mL × 1) and concentrated under reduced pressure. The residue was purified by silica gel column chromatography (petroleum ether/ethyl acetate = 1/0 to 1/1) to obtain compound 50c-2 (700 mg, yield: 71.64%). ¹H NMR (400 MHz,CDCl₃) δ 7.63 (t, *J* = 1.9 Hz, 1H), 7.48 - 7.30 (m, 2H), 7.30 - 7.22 (m, 1H), 3.75 (d, *J* = 11.1 Hz, 1H), 3.61 (d, *J* = 11.2 Hz, 1H), 1.50 (s, 3H).

### Step 6: Synthesis of Compound 50d-2

1,1'-Bis(diphenylphosphino)ferrocene-palladium(II)dichloride dichloromethane complex (221 mg, 0.3 mmol) was added to a solution of compound 50c-2 (700 mg, 3.03 mmol), bis(pinacolato)diboron (846 mg, 3.33 mmol), and potassium acetate (594 mg, 6.05 mmol) in 1,4-dioxane (15 mL). The reaction mixture was stirred at 90°C for 16 hours. After the reaction was completed, the reaction mixture was filtered, and the filtrate was concentrated under reduced pressure to obtain compound 50d-2 (700 mg, crude product).

### Step 7: Synthesis of compounds 50-P1 and 50-P4

Tetrakis(triphenylphosphine)palladium (145 mg, 0.125 mmol) was added to a mixed solution of compound 50d-2 (700 mg), compound 33a (162 mg, 0.37 mmol), and sodium carbonate (213 mg, 2.01 mmol) in 1,4-dioxane and water (15 mL/1.5 mL). The reaction mixture was stirred at 90°C for 16 hours. After the reaction was completed, the reaction mixture was concentrated under reduced pressure. The residue was purified by silica gel column chromatography (dichloromethane/methanol = 1/0 to 10/1) to obtain a crude product (80 mg). The crude product (80 mg) was further purified by preparative HPLC (chromatographic column: Gemini-C18; 150 × 21.2 mm, 5 µm; mobile phase: acetonitrile-water (0.1% formic acid); gradient: 30-40%; column temperature: 25°C; flow rate: 20 mL/min; wavelength: 214 nm; column pressure: 80 bar) to obtain compound 50-2 (40 mg). Compound 50-1 (40 mg) was resolved by supercritical fluid chiral chromatography (instrument: SFC Thar prep 80; column: CHIRALPAK AD-H 250 mm × 20 mm, 5 µm; mobile phase: 40% EtOH/CO₂ (NH₄OH 0.2%); total flow rate: 40 g/min) to obtain compounds 50-P1 (13.4 mg, yield: 0.97%) and 50-P4 (17.9 mg, yield: 1.42%).

### Compound 50-P1:

MS m/z (ESI): 546.0 [M+1]⁺. Supercritical fluid chromatography (SFC): retention time = 2.49 min, UV = 214 nm. ¹H NMR (400 MHz, CD₃OD) δ 8.63 (s, 1H), 8.48 (d, *J =* 2.3 Hz, 1H), 8.08 (d, *J =* 1.4 Hz, 1H), 7.89 - 7.71 (m, 2H), 7.65 (d, *J =* 7.9 Hz, 1H), 7.50 (t, *J =* 7.8 Hz, 1H), 6.90 (s, 1H), 5.54 (d, *J =* 1.7 Hz, 2H), 3.67 (q, *J =* 11.2 Hz, 2H), 2.18 (d, *J =* 20.1 Hz, 6H), 1.58 (s, 3H).

### Compound 50-P4:

MS m/z (ESI): 546.0 [M+1]⁺. Supercritical fluid chromatography (SFC): retention time = 4.92 min, UV = 214 nm. ¹H NMR (400 MHz, CD₃OD) δ 8.51 (s, 1H), 8.36 (d, *J =* 2.3 Hz, 1H), 7.96 (d, *J =* 1.3 Hz, 1H), 7.74 - 7.58 (m, 2H), 7.58 - 7.50 (m, 1H), 7.38 (t, *J* = 7.8 Hz, 1H), 6.78 (s, 1H), 5.42 (d, *J =* 1.7 Hz, 2H), 3.55 (q, *J=* 11.2 Hz, 2H), 2.05 (d, *J =* 20.5 Hz, 6H), 1.46 (s, 3H).

### Example 24 Synthesis of Compounds 51-1, 51-2, 51-P1, 51-P2, 51-P3, and 51-P4

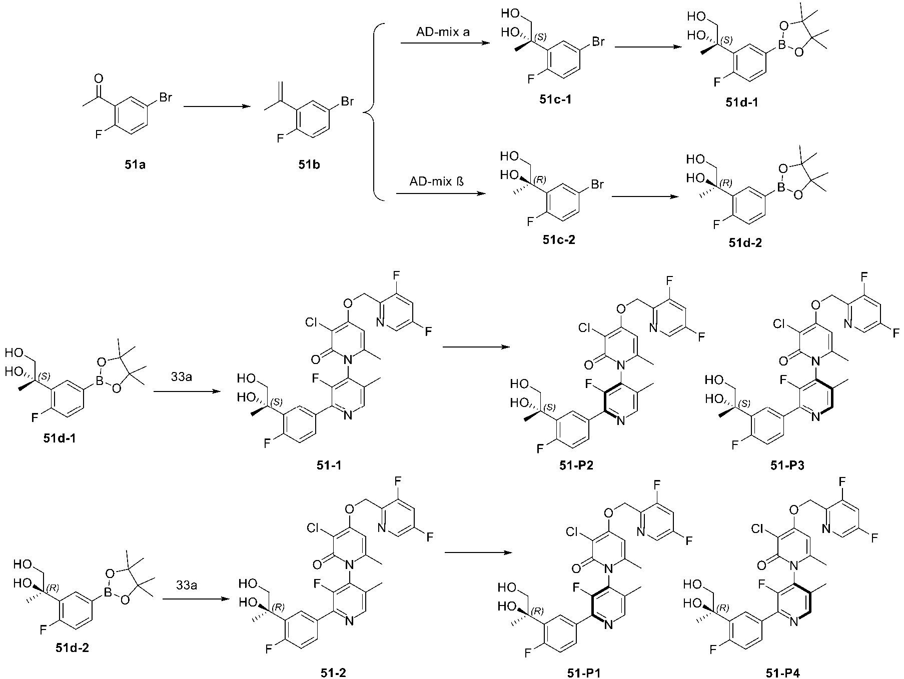

### Step 1: Synthesis of Compound 51b

Methylphosphonium ylide (11.8 g, 33.1 mmol) was added to a solution of potassium tert-butoxide (3.72 g, 33.1 mmol) in tetrahydrofuran (100 mL). The mixture was stirred at room temperature for 0.5 hours, then cooled to 0°C. Compound 51a (6 g, 27.6 mmol) was added, and the reaction mixture was slowly warmed to room temperature and stirred for 3 hours. After the reaction was completed, the reaction mixture was quenched with water (100 mL) and extracted with dichloromethane (50 mL × 3). The combined organic phases were washed with saturated brine (20 mL × 1) and concentrated under reduced pressure. The residue was purified by silica gel column chromatography (petroleum ether) to obtain compound 51b (1.5 g, yield: 23.91%). ¹H NMR (400 MHz, CDCl₃) δ 7.41 (dd, *J* = 6.8, 2.5 Hz, 1H), 7.32 (ddd, *J* = 8.6, 4.3, 2.6 Hz, 1H), 6.91 (dd, *J=* 10.5, 8.7 Hz, 1H), 5.28 - 5.21 (m, 2H), 2.11 (d, *J* = 0.8 Hz, 3H).

### Step 2: Synthesis of Compound 51c-1

AD-mix-α (7.5 g, CAS: 153130-59-7) was added to a mixed solution of compound 51b (0.75 g, 3.48 mmol) in *tert-*butanol and water (20 mL/20 mL). The reaction mixture was stirred at room temperature for 16 hours. After the reaction was completed, the reaction mixture was quenched with sodium thiosulfate solution (40 mL) and extracted with dichloromethane (40 mL × 3). The combined organic phases were then washed with saturated brine (20 mL × 1) and concentrated under reduced pressure. The residue was purified by silica gel column chromatography (petroleum ether/ethyl acetate = 1/0 to 1/1) to obtain compound 51c-1 (500 mg, yield: 51.81%). ¹H NMR (400 MHz, CD₃OD) δ 7.84 (dd, *J* = 7.1, 2.6 Hz, 1H), 7.42 (ddd, *J* = 8.6, 4.2, 2.7 Hz, 1H), 7.00 (dd, *J =* 11.5, 8.6 Hz, 1H), 3.73 (ddd, *J* = 29.2, 11.2, 1.1 Hz, 2H), 1.52 (d, *J* = 1.4 Hz, 3H).

### Step 3: Synthesis of Compound 51d-1

1,1'-Bis(diphenylphosphino)ferrocene-palladium(II)dichloride dichloromethane complex (146 mg, 0.2 mmol) was added to a solution of compound 51c-1 (500 mg, 2 mmol), bis(pinacolato)diboron (560 mg, 2.2 mmol), and potassium acetate (394 mg, 4 mmol) in 1,4-dioxane (15 mL). The reaction mixture was stirred at 90°C for 16 hours. After the reaction was completed, the reaction mixture was filtered, and the filtrate was concentrated under reduced pressure to obtain compound 51d-1 (500 mg, crude product).

### Step 4: Synthesis of Compounds 51-P2 and 51-P3

Tetrakis(triphenylphosphine)palladium (97 mg, 0.08 mmol) was added to a mixed solution of compound 51d-1 (500 mg, crude product), compound 33a (145 mg, 0.33 mmol), and sodium carbonate (178 mg, 1.68 mmol) in 1,4-dioxane and water (15 mL/1.5 mL). The reaction mixture was stirred at 90°C for 16 hours. After the reaction was completed, the reaction mixture was concentrated under reduced pressure. The residue was purified by silica gel column chromatography (dichloromethane/methanol = 1/0 to 10/1) to obtain a crude product (100 mg). The crude product (100 mg) was further purified by preparative HPLC (chromatographic column: Gemini-C18; 150 × 21.2 mm, 5 µm; mobile phase: acetonitrile-water (0.1% formic acid); gradient: 30-40%; column temperature: 25°C; flow rate: 20 mL/min; wavelength: 214 nm; column pressure: 80 bar) to obtain compound 51-1 (50 mg). Compound 51-1 (50 mg) was then resolved by supercritical fluid chiral chromatography (instrument: SFC Thar prep 80; column: CHIRALPAK AD-H 250 mm × 20 mm, 5 µm; mobile phase: 40% EtOH/CO₂ (NH₄OH 0.2%); total flow rate: 40 g/min) to obtain compounds 51-P2 (retention time: 3.55 min, 17.8 mg, yield: 3.68%) and 51-P3 (retention time: 5.36 min, 27.4 mg, yield: 7.77%).

### Compound 51-P2:

MS m/z (ESI): 564.1 [M+1]⁺. Supercritical fluid chromatography (SFC): retention time = 3.55 min, UV = 220 nm. ¹H NMR (400 MHz, CD₃OD) δ 8.62 (s, 1H), 8.48 (d, *J* = 2.1 Hz, 1H), 8.34 (d, *J =* 7.7 Hz, 1H), 7.89 (dd, *J* = 7.6, 3.2 Hz, 1H), 7.80 - 7.71 (m, 1H), 7.21 (dd, *J* = 11.6, 8.6 Hz, 1H), 6.89 (s, 1H), 5.54 (s, 2H), 3.80 (q, *J* = 11.2 Hz, 2H), 2.17 (d, *J* = 17.8 Hz, 6H), 1.60 (d, *J* = 0.9 Hz, 3H).

### Compound 51-P3:

MS m/z (ESI): 564.1 [M+1]⁺. Supercritical fluid chromatography (SFC): retention time = 5.36 min, UV = 220 nm. ¹H NMR (400 MHz, CD₃OD) δ 8.62 (s, 1H), 8.48 (d, *J* = 2.3 Hz, 1H), 8.34 (d, *J =* 7.7 Hz, 1H), 7.93 - 7.85 (m, 1H), 7.79 - 7.70 (m, 1H), 7.21 (dd, *J* = 11.6, 8.6 Hz, 1H), 6.89 (s, 1H), 5.54 (d, *J* = 1.7 Hz, 2H), 3.79 (dt, *J* = 18.4, 9.4 Hz, 2H), 2.17 (d, *J=* 17.6 Hz, 6H), 1.59 (s, 3H).

### Step 5: Synthesis of Compound 51c-2

AD-mix-β (7.5 g, CAS: 148618-32-0) was added to a mixed solution of compound 51b (0.75 g, 3.48 mmol) in *tert-*butanol and water (20 mL/20 mL). The reaction mixture was stirred at room temperature for 16 hours. After the reaction was completed, the reaction mixture was quenched with sodium thiosulfate solution (40 mL) and extracted with dichloromethane (40 mL × 3). The combined organic phases were washed with saturated brine (20 mL × 1) and concentrated under reduced pressure. The residue was purified by silica gel column chromatography (petroleum ether/ethyl acetate = 1/0 to 1/1) to obtain compound 51c-2 (500 mg, yield: 51.81%). ¹H NMR (400 MHz, CDCl₃) δ 7.80 (dd, *J* = 7.1, 2.6 Hz, 1H), 7.38 (ddd, *J* = 8.6, 4.3, 2.6 Hz, 1H), 6.92 (dd, *J* = 11.3, 8.6 Hz, 1H), 3.97 (d, *J* = 11.1 Hz, 1H), 3.73 (dd, *J* = 11.1, 1.1 Hz, 1H), 1.56 (d, *J* = 1.1 Hz, 3H).

### Step 6: Synthesis of Compound 51d-2

1,1'-Bis(diphenylphosphino)ferrocene-palladium(II)dichloride dichloromethane complex (146 mg, 0.2 mmol) was added to a solution of compound 51c-2 (500 mg, 2 mmol), bis(pinacolato)diboron (560 mg, 2.2 mmol), and potassium acetate (394 mg, 4 mmol) in 1,4-dioxane (15 mL). The reaction mixture was stirred at 90°C for 16 hours. After the reaction was completed, the reaction mixture was filtered, and the filtrate was concentrated under reduced pressure to obtain compound 51d-2 (500 mg, crude product).

### Step 7: Synthesis of compounds 51-P1 and 51-P4

Tetrakis(triphenylphosphine)palladium (97 mg, 0.08 mmol) was added to a mixed solution of compound 51d-2 (500 mg, crude product), compound 33a (145 mg, 0.33 mmol), and sodium carbonate (178 mg, 1.68 mmol) in 1,4-dioxane and water (15 mL/1.5 mL). The reaction mixture was stirred at 90°C for 16 hours. After the reaction was completed, the reaction mixture was concentrated under reduced pressure. The residue was subjected to silica gel column chromatography (dichloromethane/methanol = 1/0 to 10:1) to obtain a crude product (100 mg). The crude product (100 mg) was purified by preparative HPLC (chromatographic column: Gemini-C18; 150 × 21.2 mm, 5 µm; mobile phase: acetonitrile-water (0.1% formic acid); gradient: 30-40%; column temperature: 25°C; flow rate: 20 mL/min; wavelength: 214 nm; column pressure: 80 bar) to obtain compound 51-2 (50 mg). Compound 51-2 (50 mg) was then resolved by supercritical fluid chiral chromatography (instrument: SFC Thar prep 80; column: CHIRALPAK AD-H 250 mm × 20 mm, 5 µm; mobile phase: 40% EtOH/CO₂ (NH₄OH 0.2%); total flow rate: 40 g/min) to obtain compounds 51-P1 (retention time: 3.38 min, 17.9 mg, yield: 3.76%) and 51-P4 (retention time: 7.06 min, 29 mg, yield: 6.06%).

### Compound 51-P1:

MS m/z (ESI): 564.1 [M+1]⁺. Supercritical fluid chromatography (SFC): retention time = 3.38 min, UV = 220 nm. ¹H NMR (400 MHz, CD₃OD) δ 8.62 (s, 1H), 8.48 (d, *J =* 2.3 Hz, 1H), 8.34 (d, *J =* 7.0 Hz, 1H), 7.98 - 7.83 (m, 1H), 7.82 - 7.69 (m, 1H), 7.21 (dd, *J* = 11.6, 8.6 Hz, 1H), 6.89 (s, 1H), 5.54 (d, J = 1.8 Hz, 2H), 3.88 - 3.70 (m, 2H), 2.17 (d, *J* = 20.6 Hz, 6H), 1.60 (d, *J* = 1.1 Hz, 3H).

### Compound 51-P4:

MS m/z (ESI): 564.1 [M+1]⁺. Supercritical fluid chromatography (SFC): retention time = 7.06 min, UV = 220 nm. ¹H NMR (400 MHz, CD₃OD) δ 8.50 (s, 1H), 8.36 (d, *J =* 2.3 Hz, 1H), 8.22 (d, *J =* 7.8 Hz, 1H), 7.80 - 7.72 (m, 1H), 7.68 - 7.58 (m, 1H), 7.09 (dd, *J =* 11.7, 8.6 Hz, 1H), 6.77 (s, 1H), 5.42 (d, *J =* 1.8 Hz, 2H), 3.68 (q, *J* = 11.4 Hz, 2H), 2.05 (d, *J =* 17.6 Hz, 6 H), 1.48 (d, *J* = 1.1 Hz, 3H).

### Example 25 Synthesis of Compounds 65-1 and 65-2

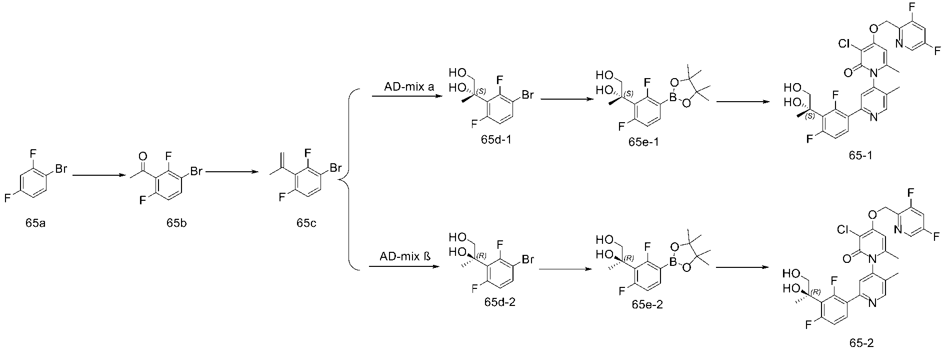

### Step 1: Synthesis of Compound 65b

At -15°C, lithium diisopropylamide (33.7 mL, 2 M) was added to a solution of compound 65a (10 g, 51.8 mmol) in tetrahydrofuran (50 mL). The mixture was stirred at -15°C for 1 hour, followed by the dropwise addition of N-methoxy-N-methylacetamide (8.01 g, 77.7 mmol). The reaction was stirred at -15°C for 1 hour. After the reaction was completed, the reaction mixture was quenched with saturated aqueous ammonium chloride solution (100 mL) and extracted with ethyl acetate (100 mL × 3). The combined organic phases were dried over anhydrous sodium sulfate and filtered. The filtrate was concentrated under reduced pressure. The residue was purified by silica gel column chromatography (petroleum ether/ethyl acetate = 1/0 to 3/1) to obtain compound 65b (4 g, yield: 29.5%). ¹H NMR (400 MHz, CDCl₃) δ 7.64-7.59 (m, 1H), 6.93-6.88 (m, 1H), 2.61 (t, *J* = 1.7 Hz, 3H).

### Step 2: Synthesis of Compound 65c

At 0°C, methyltriphenylphosphonium bromide (7.3 g, 20.4 mmol) was added to a solution of compound 65b (4 g, 17.0 mmol) and potassium *tert*-butoxide (2.3 g, 20.4 mmol) in tetrahydrofuran (50 mL). The reaction mixture was stirred at 0°C for 5 hours. After the reaction was completed, the reaction mixture was quenched with water (100 mL) and extracted with ethyl acetate (100 mL × 3). The combined organic phases were dried over anhydrous sodium sulfate, filtered, and concentrated under reduced pressure. The residue was purified by silica gel column chromatography (petroleum ether/ethyl acetate = 1/0 to 10/1) to obtain compound 65c (2.4 g, yield: 54.7%). ¹H NMR (400 MHz, CDCl₃) δ 7.43 - 7.38 (m, 1H), 6.81 (td, *J =* 8.9, 1.7 Hz, 1H), 5.49 - 5.38 (m, 1H), 5.11 (s, 1H), 2.08 (s, 3H).

### Step 3: Synthesis of Compound 65d-1

AD-mix-α (7.5 g, CAS: 153130-59-7) was added to a mixed solution of compound 65c (600 mg, 2.6 mmol) in *tert-*butanol and water (20 mL/20 mL). The reaction mixture was stirred at room temperature for 16 hours. After the reaction was completed, the reaction mixture was quenched with sodium thiosulfate solution (20 mL), followed by extraction with ethyl acetate (50 mL × 3). The combined organic phases were washed with saturated brine (20 mL × 1), concentrated under reduced pressure, and the residue was subjected to silica gel column chromatography (dichloromethane/methanol = 1/0 to 20/1) to obtain compound 65d-1 (150 mg, yield: 19.63%). ¹H NMR (400 MHz, CDCl₃) δ 7.48 - 7.30 (m, 1H), 6.83 - 6.67 (m, 1H), 3.93 (d, *J =* 11.3 Hz, 1H), 3.70 - 3.38 (m, 3H), 1.55 (d, *J* = 1.3 Hz, 3H).

### Step 4: Synthesis of Compound 65e-1

1,1'-Bis(diphenylphosphino)ferrocene-palladium(II)dichloride dichloromethane complex (33 mg, 0.04 mmol) was added to a solution of compound 65d-1 (120 mg, 0.45 mmol), bis(pinacolato)diboron (171 mg, 0.67 mmol), and potassium acetate (88 mg, 0.89 mmol) in 1,4-dioxane (10 mL). The reaction mixture was stirred at 90°C for 16 hours. After the reaction was completed, the reaction mixture was filtered, and the filtrate was concentrated under reduced pressure to obtain compound 65e-1 (300 mg, crude product).

### Step 5: Synthesis of Compound 65-1

Tetrakis(triphenylphosphine)palladium (88 mg, 0.07 mmol) was added to a mixed solution of compound 65e-1 (240 mg, crude product), compound A2 (157 mg, 0.38 mmol), and sodium carbonate (162 mg, 1.5 mmol) in 1,4-dioxane and water (10 mL/1 mL). The reaction mixture was stirred at 90°C overnight. After the reaction was completed, the reaction mixture was concentrated under reduced pressure. The residue was purified by silica gel column chromatography (dichloromethane/methanol = 1/0 to 4/1) to obtain a crude product. The crude product was further purified by preparative HPLC (chromatographic column: Gemini-C18; 150 × 21.2 mm, 5 µm; mobile phase: acetonitrile-water (0.1% formic acid); gradient: 30-40%; column temperature: 25°C; flow rate: 20 mL/min; wavelength: 214 nm; column pressure: 80 bar) to obtain compound 65-1 (50 mg, yield: 11.5%). MS m/z (ESI): 564.2 [M+1]⁺. ¹H NMR (400 MHz, CDCl₃) δ 8.72 (s, 1H), 8.41 (d, *J =* 2.3 Hz, 1H), 7.88 (dd, *J =* 8.5, 2.2 Hz, 1H), 7.49 (d, *J* = 1.4 Hz, 1H), 7.33 (ddd, *J* = 9.0, 8.2, 2.4 Hz, 1H), 7.02 (dd, *J =* 11.6, 8.9 Hz, 1H), 6.40 (s, 1H), 5.42 (d, *J* = 1.7 Hz, 2H), 4.11 (dd, *J* = 11.2, 5.2 Hz, 1H), 3.74 (d, *J* = 11.3 Hz, 1H), 3.50 (s, 1H), 2.30 (dd, *J =* 25.2, 20.1 Hz, 1H), 2.15 (s, 3H), 2.01 (s, 3H), 1.65 (s, 3H).

### Step 6: Synthesis of Compound 65d-2

AD-mix-β (6.0 g, CAS: 148618-32-0) was added to a mixed solution of compound 65c (600 mg, 2.6 mmol) in *tert-*butanol and water (20 mL/20 mL). The reaction mixture was stirred at room temperature for 16 hours. After the reaction was completed, the reaction mixture was quenched with sodium thiosulfate solution (20 mL), followed by extraction with ethyl acetate (30 mL × 3). The combined organic phases were washed with saturated brine (40 mL × 1), concentrated under reduced pressure, and the residue was purified by silica gel column chromatography (dichloromethane/methanol = 20/1) to obtain compound 65d-2 (150 mg, yield: 19.6%). ¹HNMR (400 MHz, CDCl₃) δ 7.46 - 7.40 (m, 1H), 6.89 - 6.69 (m, 1H), 3.98 (d, *J* = 11.2 Hz, 1H), 3.66 (d, *J* = 11.3 Hz, 1H), 3.55 (s, 1H), 1.59 (s, 3H).

### Step 7: Synthesis of Compound 65e-2

1,1'-Bis(diphenylphosphino)ferrocene-palladium(II)dichloride dichloromethane complex (33 mg, 0.04 mmol) was added to a solution of compound 65d-2 (120 mg, 0.45 mmol), bis(pinacolato)diboron (171 mg, 0.67 mmol), and potassium acetate (88 mg, 0.89 mmol) in 1,4-dioxane (10 mL). The reaction mixture was stirred at 90°C for 16 hours. After the reaction was completed, the reaction mixture was filtered, and the filtrate was concentrated under reduced pressure to obtain compound 65e-2 (120 mg, crude product).

### Step 8: Synthesis of compound 65-2

Tetrakis(triphenylphosphine)palladium (44 mg, 0.04 mmol) was added to a mixed solution of compound 65e-2 (120 mg, crude product), compound A2 (79 mg, 0.38 mmol), and sodium carbonate (162 mg, 1.5 mmol) in 1,4-dioxane and water (10 mL/1 mL). The reaction mixture was stirred at 90°C overnight. After the reaction was completed, the reaction mixture was concentrated under reduced pressure. The resulting residue was purified by silica gel column chromatography (dichloromethane/methanol = 1/0 to 4/1) to obtain a crude product. The crude product was further purified by preparative HPLC (chromatographic column: Gemini-C18; 150 × 21.2 mm, 5 µm; mobile phase: acetonitrile-water (0.1% formic acid); gradient: 30-40%; column temperature: 25°C; flow rate: 20 mL/min; wavelength: 214 nm; column pressure: 80 bar) to obtain compound 65-2 (50 mg, yield: 22.9%).

MS m/z (ESI): 564.1 [M+1]⁺. ¹H NMR (400 MHz, CDCl₃) δ 8.91 (s, 1H), 8.41 (d, *J =* 1.8 Hz, 1H), 7.62 (d, *J =* 19.3 Hz, 2H), 7.36 (dd, *J =* 12.1, 4.8 Hz, 1H), 7.08 - 7.01 (m,1H), 6.53 (s, 1H), 5.45 (s, 2H), 4.13 (d, *J* = 9.7 Hz, 1H), 3.73 (d, *J* = 11.1 Hz, 1H), 2.24 (s, 3H), 2.05 (s, 3H), 1.64 (d, *J =* 5.8 Hz, 3H).

### Example 26 Synthesis of Compound 97

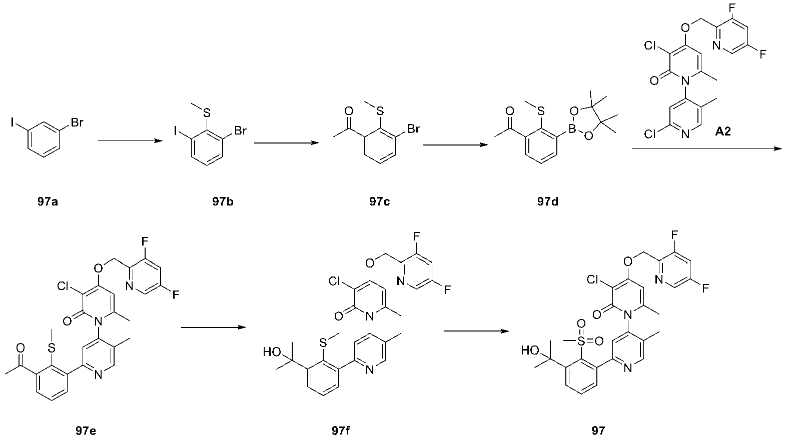

### Step 1: Synthesis of Compound 97b

At -90°C, lithium diisopropylamide (7.9 mL, 15.9 mmol) was added dropwise to a solution of compound 97a (3 g, 10.6 mmol) in tetrahydrofuran (60 mL). The mixture was stirred at -78°C for 1 hour, followed by the dropwise addition of dimethyl sulfide (1.6 g, 17.0 mmol). The reaction mixture was stirred at -78°C for 2 hours. After the reaction was completed, the reaction mixture was quenched with ammonium chloride solution (60 mL) and extracted with ethyl acetate (60 mL × 3). The combined organic phases were dried over anhydrous sodium sulfate, filtered, and concentrated under reduced pressure. The resulting residue was purified by silica gel column chromatography (petroleum ether/ethyl acetate = 1/0 to 10/1) to obtain compound 97b (2.8 g, yield: 74.2%). ¹H NMR (400 MHz, CDCl₃) δ 7.88 (dd, *J* = 7.9, 1.2 Hz, 1H), 7.64 (dd, *J* = 8.0, 1.2 Hz, 1H), 6.78 (t, *J* = 7.9 Hz, 1H), 2.43 (s, 3H).

### Step 2: Synthesis of Compound 97c

Bis(triphenylphosphine)palladium(II) chloride (0.43 g, 0.06 mmol) was added to a solution of compound 97b (2 g, 6.1 mmol) and tributyl(1-ethoxyvinyl)stannane (2.2 g, 6.1 mmol) in 1,4-dioxane (40 mL). The reaction mixture was stirred at 120°C for 2 hours. After the reaction was completed, the reaction mixture was diluted with water (100 mL) and extracted with ethyl acetate (100 mL × 3). The combined organic phases were dried over anhydrous sodium sulfate and filtered. The filtrate was concentrated under reduced pressure, and the residue was purified by silica gel column chromatography (ethyl acetate/petroleum ether = 1/0 to 1/5) to obtain compound 97c (1 g, yield: 60.66%). ¹H NMR (400 MHz, CDCl₃) δ 7.70 (dd, *J* = 7.1, 2.2 Hz, 1H), 7.26 - 7.18 (m, 2H), 2.62 (s, 3H), 2.41 (s, 3H).

### Step 3: Synthesis of Compound 97d

1,1'-Bis(diphenylphosphino)ferrocene palladium(II)dichloride (0.17 g, 0.24 mmol) was added to a solution of compound 97c (0.6 g, 2.4 mmol), bis(pinacolato)diboron (1.2 g, 4.9 mmol), and potassium acetate (0.48 g, 4.9 mmol) in 1,4-dioxane. The reaction mixture was stirred at 90°C for 16 hours. After the reaction was completed, the reaction mixture was filtered, and the filtrate was concentrated under reduced pressure to obtain compound 97d (0.8 g, crude product).

### Step 4: Synthesis of Compound 97e

Tetrakis(triphenylphosphine)palladium (11 mg, 0.01 mmol) was added to a mixed solution of compound 97d (90 mg, 0.3 mmol), compound **A2** (42 mg, 0.1 mmol), and sodium carbonate (21 mg, 0.2 mmol) in 1,4-dioxane and water (5 mL/1 mL). The reaction mixture was stirred at 90°C overnight. After the reaction was completed, the reaction mixture was concentrated under reduced pressure, and the resulting residue was purified by a thin-layer chromatography silica gel plate (dichloromethane/methanol = 10/1) to obtain compound 97e (30 mg, yield: 51%). MS m/z (ESI): 542.1 [M+1]⁺.

### Step 5: Synthesis of Compound 97f

At 0°C, methylmagnesium bromide (0.07 mL, 3 M, 0.21 mmol) was added to a solution of compound 97e (40 mg, 0.07 mmol) in tetrahydrofuran (50 mL). The reaction mixture was stirred at 0°C for 3 hours. After the reaction was completed, the reaction mixture was quenched with ammonium chloride solution (60 mL) and extracted with ethyl acetate (60 mL × 3). The combined organic phases were dried over anhydrous sodium sulfate and filtered. The filtrate was concentrated under reduced pressure, and the resulting residue was purified by a thin-layer chromatography silica gel plate (dichloromethane/methanol = 10/1) to obtain compound 97f (20 mg, yield: 43%). MS m/z (ESI): 558.1 [M+1]⁺.

### Step 6: Synthesis of Compound 97

Potassium monopersulfate (62 mg, 0.18 mmol, CAS: 37222-66-5) was added to a solution of compound 97f (20 mg, 0.03 mmol) in methanol (5 mL). The reaction mixture was stirred at 35°C for 12 hours. After the reaction was completed, the reaction mixture was concentrated under reduced pressure. The resulting residue was purified by a thin-layer chromatography silica gel plate (dichloromethane/methanol = 10/1) to obtain a crude product. The crude product was further purified by preparative HPLC (chromatographic column: Gemini-C18; 150 × 21.2 mm, 5 µm; mobile phase: acetonitrile-water (0.1% TFA); gradient: 45-65%; column temperature: 25°C; flow rate: 20 mL/min; wavelength: 214 nm; column pressure: 80 bar) to obtain compound 97 (4 mg, yield: 18.72%).

MS m/z (ESI): 590.2 [M+1]⁺. ¹H NMR (400 MHz, CDCl₃) δ 8.57 (s, 1H), 8.39 (d, *J* = 2.3 Hz, 1H), 7.53 (d, *J* = 6.4 Hz, 2H), 7.33 (ddd, *J =* 17.1, 8.2, 2.4 Hz, 2H), 7.20 (s, 1H), 6.36 (s, 1H), 5.40 (d, *J* = 1.6 Hz, 2H), 5.24 (s, 1H), 3.71 (s, 3H), 2.17 (s, 3H), 2.02 (s, 3H), 1.82 (s, 3H), 1.71 (s, 3H).

### Example 27 Synthesis of Compound 101

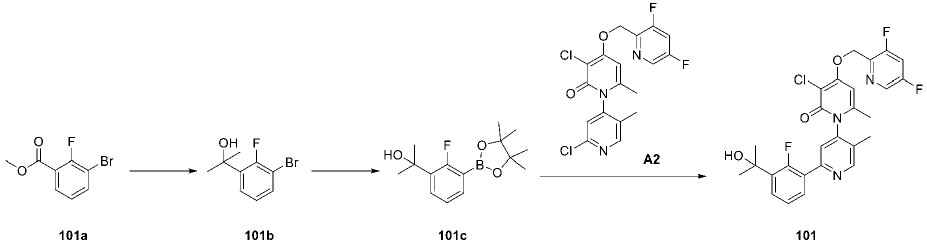

### Step 1: Synthesis of Compound 101b

At 0°C, methylmagnesium bromide (4.29 mL, 3 M, 12.87 mmol) was added to a solution of compound 101a (1.00 g, 4.29 mmol) in tetrahydrofuran (20 mL), and the reaction mixture was stirred at room temperature for 1.5 hours. After the reaction was completed, the reaction mixture was quenched with ammonium chloride solution (50 mL) and extracted with ethyl acetate (50 mL × 3). The combined organic phases were dried over anhydrous sodium sulfate, filtered, and concentrated under reduced pressure. The residue was purified by silica gel column chromatography (petroleum ether/ethyl acetate = 1/0 to 5/1) to obtain compound 101b (0.50 g, yield: 50.0%). ¹H NMR (400 MHz, CDCl₃) δ 7.57 - 7.39 (m, 2H), 7.01 - 6.97 (m, 1H), 1.62 (d, *J* = 1.3 Hz, 6H).

### Step 2: Synthesis of Compound 101c

1,1'-Bis(diphenylphosphino)ferrocene palladium(II)dichloride (0.13 g, 0.18 mmol) was added to a solution of compound 101b (0.40 g, 1.71 mmol), bis(pinacolato)diboron (0.87 g, 3.43 mmol), and potassium acetate (0.33 g, 3.43 mmol) in 1,4-dioxane (10 mL). The reaction mixture was stirred at 90°C for 16 hours. After the reaction was completed, the reaction mixture was filtered, and the filtrate was concentrated under reduced pressure. The residue was purified by silica gel column chromatography (petroleum ether/ethyl acetate = 1/0 to 10/1) to obtain compound 101c (0.21 g, yield: 41.8%). ¹H NMR (400 MHz, CDCl₃) δ 7.76 - 7.55 (m, 2H), 7.13 (t, *J* = 7.5 Hz, 1H), 1.65 (d, *J* = 0.8 Hz, 6H), 1.37 (s, 12H).

### Step 3: Synthesis of Compound 101

Tetrakis(triphenylphosphine)palladium (0.05 g, 0.04 mmol) was added to a mixed solution of compound 101c (0.10 g, 0.35 mmol), compound A2 (0.14 g, 0.35 mmol), and sodium carbonate (74.20 mg, 0.70 mmol) in 1,4-dioxane and water (5 mL/1 mL). The reaction mixture was stirred at 90°C overnight. After the reaction was completed, the reaction mixture was concentrated under reduced pressure. The residue was purified by a thin-layer chromatography silica gel plate (dichloromethane/methanol = 10/1) to obtain a crude product of compound 101. The crude product was further purified by preparative HPLC (chromatographic column: Gemini-C18; 150 × 21.2 mm, 5 µm; mobile phase: acetonitrile-water (0.1% TFA); gradient: 45-45%; column temperature: 25°C; flow rate: 20 mL/min; wavelength: 214 nm; column pressure: 80 bar) to obtain compound 101 (30 mg, yield: 16.17%).

MS m/z (ESI): 530.2 [M+1]⁺. ¹H NMR (400 MHz, DMSO-*d*₆) δ 8.79 (s, 1H), 8.61 (d, *J =* 2.3 Hz, 1H), 8.10 (td, *J* = 9.9, 2.4 Hz, 1H), 7.81 - 7.66 (m, 3H), 7.29 (t, *J* = 7.7 Hz, 1H), 6.80 (s, 1H), 5.48 (s, 2H), 2.05 (s, 3H), 1.98 (s, 3H), 1.53 (s, 6H).

### Example 28 Synthesis of Compound 102

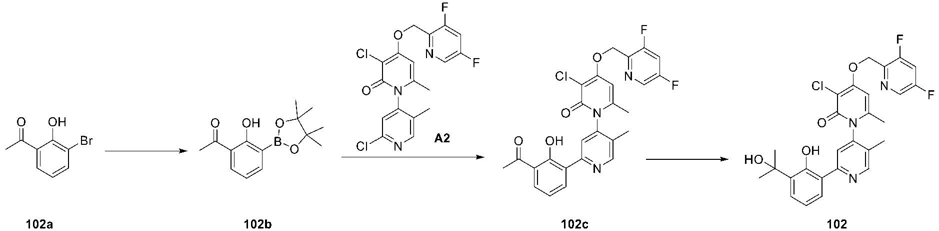

### Step 1: Synthesis of Compound 102b

1,1'-Bis(diphenylphosphino)ferrocene palladium(II)dichloride (190 mg, 0.23 mmol) was added to a solution of compound 102a (0.5 g, 2.3 mmol), bis(pinacolato)diboron (1.18 g, 4.65 mmol), and potassium acetate (685 mg, 7 mmol) in 1,4-dioxane (30 mL). The reaction mixture was stirred at 110°C for 12 hours. After the reaction was completed, the reaction mixture was filtered to remove the filter residue, and the filtrate was concentrated under reduced pressure. The resulting residue was purified by silica gel column chromatography (petroleum ether/ethyl acetate = 1/0 to 10/1) to obtain compound 102b (0.25 g, 50%, yield: 35.4%). MS m/z (ESI): 263.1 [M+1]⁺.

Step 2: Synthesis of Compound 102c 1,1'-Bis(diphenylphosphino)ferrocene-palladium(II)dichloride dichloromethane complex (110 mg, 0.095 mmol) was added to a mixed solution of compound 102b (250 mg, 0.95 mmol), compound A2 (393 mg, 0.95 mmol), and sodium carbonate (202 mg, 1.9 mmol) in 1,4-dioxane and water (10 mL/1 mL). The reaction mixture was stirred at 90°C overnight. After the reaction was completed, the reaction mixture was concentrated under reduced pressure, and the residue was purified by silica gel column chromatography (dichloromethane/methanol = 1/0 to 4/1) to obtain compound 102c (0.25 g, 40%, yield: 46%). MS m/z (ESI): 512.2 [M+1]⁺.

### Step 3: Synthesis of Compound 102

At 0°C, methylmagnesium bromide (0.5 mL, 3 M, 1.46 mmol) was added to a solution of compound 102c (250 mg, 0.48 mmol) in tetrahydrofuran (10 mL). The reaction mixture was stirred at 0°C for 1.5 hours. After the reaction was completed, the reaction mixture was quenched with ammonium chloride solution (20 mL) and extracted with ethyl acetate (20 mL × 3). The combined organic phases were concentrated under reduced pressure, and the resulting residue was purified by silica gel column chromatography (dichloromethane/methanol = 1/0 to 4/1) to obtain a crude product of compound 102 (50 mg). The crude product was purified by preparative HPLC (chromatographic column: Gemini-C18, 150 × 21.2 mm, 5 µm; mobile phase: acetonitrile-water (0.1% formic acid); gradient: 30-40%; column temperature: 25°C; flow rate: 20 mL/min; wavelength: 214 nm; column pressure: 80 bar) to obtain compound **102** (14.4 mg, yield: 5.5%). MS m/z (ESI): 528.2 [M+1]⁺. ¹H NMR (400 MHz, CD₃OD) δ 8.67 (s, 1H), 8.48 (d, *J* = 2.3 Hz, 1H), 8.06 (s, 1H), 7.82 - 7.72 (m, 2H), 7.52 (dd, *J* = 7.7, 1.4 Hz, 1H), 6.91 (t, *J* = 7.8 Hz, 1H), 6.84 (s, 1H), 5.52 (d, *J* = 1.8 Hz, 2H), 2.13 (d, *J* = 17.4 Hz, 6H), 1.69 (d, *J =* 3.2 Hz, 6H).

### Example 29 Synthesis of Compound 36-1

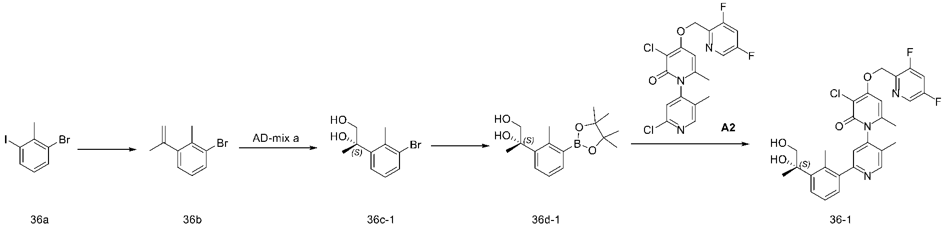

### Step 1: Synthesis of Compound 36b

1,1'-Bis(diphenylphosphino)ferrocene-palladium(II)dichloride dichloromethane complex (275 mg, 0.34 mmol) was added to a mixed solution of compound 36a (2.0 g, 6.74 mmol), isopropenylboronic acid pinacol ester (1.13 g, 6.74 mmol), and potassium carbonate (1.86 g, 13.47 mmol) in 1,4-dioxane and water (30 mL /6 mL). The reaction mixture was stirred at 80°C for 16 hours under a nitrogen atmosphere. After the reaction was completed, the reaction mixture was diluted with water (100 mL) and extracted with dichloromethane (50 mL × 3). The combined organic phases were dried over anhydrous sodium sulfate and filtered. The filtrate was concentrated under reduced pressure, and the residue was purified by silica gel column chromatography (petroleum ether/ethyl acetate = 1/0 to 20/1) to obtain compound 36b (1.0 g, yield: 63.30%). ¹HNMR (400 MHz, CDCl₃) δ 7.59 - 7.50 (m, 1H), 7.14- 7.10 (m, 1H), 7.09 - 7.04 (m, 1H), 5.23 - 5.17 (m, 1H), 4.96 - 4.91 (m, 1H), 2.01 (s, 3H), 1.61 (s, 3H).

### Step 2: Synthesis of Compound 36c-1

AD-mix-α (4 g, CAS: 153130-59-7) was added to a mixed solution of compound 36b (400 mg, 1.89 mmol) in *tert-*butanol and water (10 mL/10 mL). The reaction mixture was stirred at room temperature for 16 hours. After the reaction was completed, the reaction mixture was quenched with saturated sodium thiosulfate solution (40 mL) and extracted with dichloromethane (20 mL × 5). The combined organic phases were concentrated under reduced pressure, and the residue was purified by silica gel column chromatography (dichloromethane/methanol = 1/0 to 20/1) to obtain compound 36c-1 (130 mg, yield: 23.25%). ¹H NMR (400 MHz, CDCl₃) δ 7.78 (dd, *J* = 8.2, 4.2 Hz, 1H), 7.41 (t, *J* = 8.1 Hz, 1H), 7.03 (t, *J* = 7.9 Hz, 1H), 4.05 (d, *J* = 11.2 Hz, 1H), 3.70 (d, *J* = 11.2 Hz, 1H), 2.62 (s, 3H), 1.60 (s, 3H).

### Step 3: Synthesis of Compound 36d-1

1,1'-Bis(diphenylphosphino)ferrocene-palladium(II)dichloride dichloromethane complex (40 mg, 0.05 mmol) was added to a solution of compound 36c-1 (120 mg, 0.49 mmol), bis(pinacolato)diboron (150 mg, 0.59 mmol), and potassium acetate (96.1 mg, 0.98 mmol) in 1,4-dioxane (5 mL). The reaction mixture was stirred at 80°C under a nitrogen atmosphere for 16 hours. After the reaction was completed, the reaction mixture was concentrated under reduced pressure to obtain compound 36d-1 (120 mg, crude product). MS m/z (ESI):292.9 [M+1]⁺.

### Step 4: Synthesis of Compound 36-1

Tetrakis(triphenylphosphine)palladium (23.8 mg, 0.02 mmol) was added to a mixed solution of compound 36d-1 (120 mg, 0.41 mmol), compound A2 (85 mg, 0.21 mmol), and sodium carbonate (43.7 mg, 0.41 mmol) in 1,4-dioxane and water (10 mL/2 mL). The reaction mixture was stirred at 80°C under a nitrogen atmosphere for 16 hours. After the reaction was completed, the reaction mixture was concentrated under reduced pressure. The residue was subjected to silica gel column chromatography (dichloromethane/methanol = 1/0 to 30/1) to obtain crude product 36-1 (50 mg). The crude product was further purified by preparative HPLC (chromatographic column: Gemini-C18; 150 × 21.2 mm, 5 µm; mobile phase: acetonitrile-water (0.1% formic acid); gradient: 28-35%; column temperature: 25°C; flow rate: 20 mL/min; wavelength: 214 nm; column pressure: 80 bar) to obtain compound 36-1 (8.0 mg, yield: 7.10%). MS m/z (ESI): 542.2 [M+1]⁺. ¹HNMR (400 MHz, CD₃OD) δ 8.67 (s, 1H), 8.42 (d, *J* = 2.4 Hz, 1H), 7.70 (ddd, *J* = 9.6, 8.6, 2.4 Hz, 1H), 7.59 (dd, *J* = 7.3, 2.2 Hz, 1H), 7.35 (s, 1H), 7.24 - 7.19 (m, 2H), 6.78 (d, *J* = 0.7 Hz, 1H), 5.46 (d, *J* = 1.9 Hz, 2H), 3.83 (d, *J* = 11.3 Hz, 1H), 3.73 (dd, *J* = 11.3, 1.6 Hz, 1H), 2.41 (d, *J* = 0.8 Hz, 3H), 2.13 (s, 3H), 2.08 (s, 3H), 1.60 (s, 3H).

### Example 30 Synthesis of Compounds 37-2, 37-P1, and 37-P2

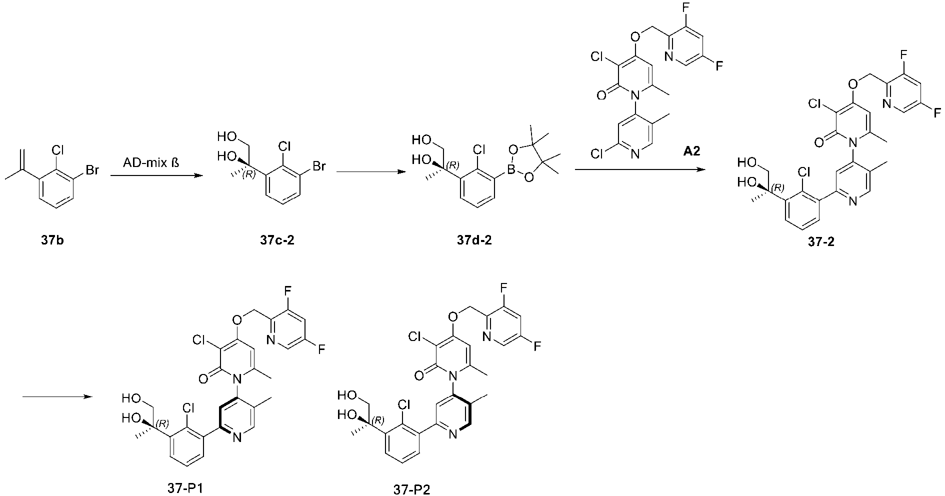

### Step 1: Synthesis of Compound 37c-2

AD-mix-β (6.0 g, CAS: 148618-32-0) was added to a mixed solution of compound 37b (600 mg, 2.59 mmol) in *tert-*butanol and water (10 mL/10 mL). The reaction mixture was stirred at room temperature for 16 hours. After the reaction was completed, the reaction mixture was quenched with saturated sodium thiosulfate solution (40 mL), extracted with dichloromethane (20 mL × 5), and the combined organic phases were concentrated under reduced pressure (20 mL × 1). The residue was purified by silica gel column chromatography (dichloromethane/methanol = 1/0 to 20/1) to obtain compound 37c-2 (250 mg, yield: 32.70%). ¹HNMR (400 MHz, CDCl₃) δ 7.78 (dd, *J* = 8.0, 1.4 Hz, 1H), 7.60 (dd, *J* = 7.9, 1.4 Hz, 1H), 7.16 (t, *J* = 8.0 Hz, 1H), 4.31 (d, *J* = 11.2 Hz, 1H), 3.81 (d, *J =* 11.2 Hz, 1H), 1.67 (s, 3H).

### Step 2: Synthesis of Compound 37d-2

1,1'-Bis(diphenylphosphino)ferrocene-palladium(II)dichloride dichloromethane complex (38.4 mg, 0.05 mmol) was added to a solution of compound 37c-2 (250 mg, 0.94 mmol), bis(pinacolato)diboron (286.9 mg, 1.13 mmol), and potassium acetate (184.8 mg, 1.89 mmol) in 1,4-dioxane (8 mL). The reaction mixture was stirred at 80°C for 16 hours. After the reaction was completed, the reaction mixture was concentrated under reduced pressure to obtain compound 37d-2 (250 mg, yield: crude product). MS m/z (ESI):312.9 [M+1]⁺.

### Step 3: Synthesis of Compounds 37-P1 and 37-P2

Tetrakis(triphenylphosphine)palladium (77.6 mg, 0.03 mmol) was added to a mixed solution of compound 37d-2 (210 mg, 0.67 mmol), compound A2 (193.8 mg, 0.47 mmol), and sodium carbonate (142.4 mg, 1.34 mmol) in 1,4-dioxane and water (10 mL/2 mL). The reaction mixture was stirred at 80°C for 16 hours. After the reaction was completed, the reaction mixture was concentrated under reduced pressure, and the residue was subjected to silica gel column chromatography (dichloromethane/methanol = 1/0 to 30/1) to obtain compound 37-2 (100 mg). Compound 37-2 (100 mg) was then resolved by supercritical fluid chiral chromatography (equipment: SFC Thar prep 80, column: CHIRALPAK AD-H 250 mm × 20 mm, 5 µm, mobile phase: 40% MeOH/CO₂ (NH₄OH 0.2%), total flow rate: 40 g/min) to obtain compounds 37-P1 (53.0 mg, yield: 13.32%) and 37-P2 (45.5 mg, yield: 11.4%).

### Compound 37-P1:

MS m/z (ESI): 561.9 [M+1]⁺. Supercritical fluid chromatography (SFC): retention time = 3.22 min, UV = 254 nm. ¹H NMR (400 MHz, CD₃OD) δ 8.68 (s, 1H), 8.43 (d, *J =* 2.4 Hz, 1H), 7.98 (dd, *J =* 6.3, 3.4 Hz, 1H), 7.70 (ddd, *J =* 9.7, 8.6, 2.4 Hz, 1H), 7.49 (s, 1H), 7.41 (d, *J =* 3.0 Hz, 1H), 7.40 (s, 1H), 6.78 (s, 1H), 5.47 (d, *J* = 1.9 Hz, 2H), 4.07 (d, *J =* 11.4 Hz, 1H), 3.87 (d, *J* = 11.4 Hz, 1H), 2.14 (s, 3H), 2.08 (s, 3H), 1.66 (s, 3H).

### Compound 37-P2:

MS m/z (ESI): 562.0 [M+1]⁺. Supercritical fluid chromatography (SFC): retention time = 4.29 min, UV = 254 nm. ¹H NMR (400 MHz, CD₃OD) δ 8.68 (s, 1H), 8.43 (d, *J =* 2.4 Hz, 1H), 7.98 (dd, *J =* 6.4, 3.4 Hz, 1H), 7.70 (ddd, *J =* 9.6, 8.6, 2.4 Hz, 1H), 7.49 (s, 1H), 7.41 (d, *J* = 3.1 Hz, 1H), 7.40 (s, 1H), 6.79 (s, 1H), 5.47 (d, *J =* 1.9 Hz, 2H), 4.12 (d, *J* = 11.4 Hz, 1H), 3.86 (d, *J* = 11.4 Hz, 1H), 2.14 (s, 3H), 2.08 (s, 3H), 1.64 (s, 3H).

### Example 31 Synthesis of Compounds 42-2, 42-P1, and 42-P2

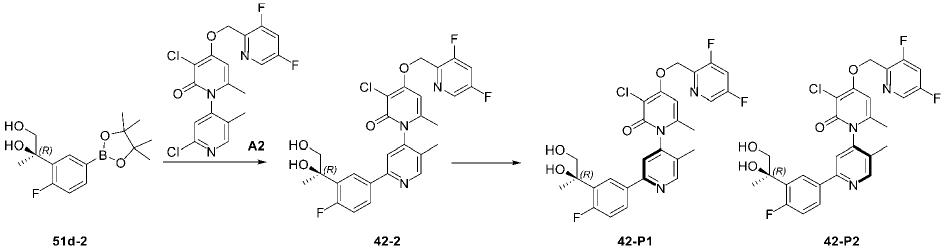

Tetrakis(triphenylphosphine)palladium (59 mg, 0.05 mmol) was added to a mixed solution of compound 51d-2 (300 mg, 1.01 mmol), compound A2 (209 mg, 0.51 mmol), and sodium acetate (215 mg, 2.03 mmol) in 1,4-dioxane and water (2.5 mL/0.5 mL). The reaction mixture was stirred at 90°C for 16 hours. After the reaction was completed, the reaction mixture was diluted with water (30 mL), extracted with ethyl acetate (50 mL × 3), and the combined organic phases were dried over anhydrous sodium sulfate and filtered. The filtrate was concentrated under reduced pressure. The residue was purified by silica gel column chromatography (dichloromethane/methanol = 1/0 to 10/1) to obtain compound 42-2. Compound 42-2 was further resolved by supercritical fluid chiral chromatography (equipment: SFC Thar prep 80, column: CHIRALPAK AD-H 250 mm × 20 mm, 5 µm, mobile phase: 40% MeOH/CO₂ (NH₄OH 0.2%), total flow rate: 40 g/min) to obtain compounds 42-P1 (34.9 mg, yield: 6.3%) and 42-P2 (20.6 mg, yield: 3.7%).

### Compound 42-P1:

MS m/z (ESI): 546.0 [M+1]⁺. Supercritical fluid chromatography (SFC): retention time = 3.28 min, UV = 254 nm. ¹H NMR (400 MHz, CD₃OD) δ 8.71 (s, 1H), 8.48 (d, *J* = 2.3 Hz, 1H), 8.33 (dd, *J* = 7.8*,* 2.4 Hz, 1H), 7.98 - 7.90 (m, 1H), 7.80 (s, 1H), 7.78 - 7.72 (m, 1H), 7.18 (dd, *J =* 11.6, 8.5 Hz, 1H), 6.84 (s, 1H), 5.52 (d, *J* = 1.8 Hz, 2H), 3.80 (dd, *J =* 31.9, 11.3 Hz, 2H), 2.14 (s, 3H), 2.10 (s, 3H), 1.59 (s, 3H).

### Compound 42-P2:

MS m/z (ESI): 546.1 [M+1]⁺. Supercritical fluid chromatography (SFC): retention time = 5.46 min, UV = 254 nm. ¹H NMR (400 MHz, CD₃OD) δ 8.70 (s, 1H), 8.46 (d, *J* = 2.3 Hz, 1H), 8.32 (dd, *J* = 7.8, 2.4 Hz, 1H), 7.98 - 7.90 (m, 1H), 7.80 (s, 1H), 7.78 - 7.69 (m, 1H), 7.16 (dd, *J =* 11.6, 8.5 Hz, 1H), 6.82 (s, 1H), 5.51 (d, *J =* 1.8 Hz, 2H), 3.79 (dd, *J =* 31.3, 11.2 Hz, 2H), 2.12 (s, 3H), 2.08 (s, 3H), 1.57 (s, 3H).

### Example 32 Synthesis of Compounds 52-2, 52-P1, and 52-P2

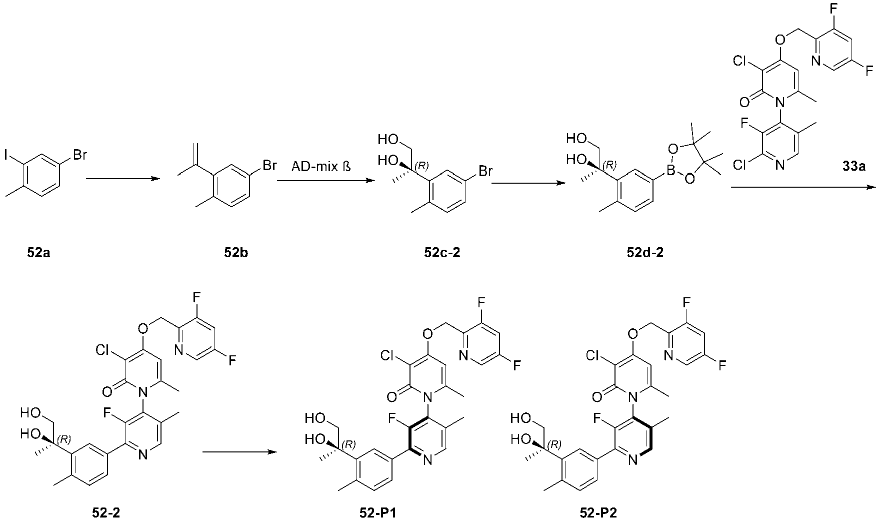

### Step 1: Synthesis of Compound 52b

1,1'-Bis(diphenylphosphino)ferrocene-palladium(II)dichloride dichloromethane complex (370 mg, 0.5 mmol) was added to a mixed solution of compound 52a (2.96 g, 10 mmol), isopropenylboronic acid pinacol ester (1.68 g, 10 mmol), and potassium carbonate (2.76 g, 20 mmol) in 1,4-dioxane and water (20 mL /4 mL). The reaction mixture was stirred at 80°C for 16 hours. After the reaction was completed, the reaction mixture was diluted with water (50 mL) and extracted with dichloromethane (50 mL × 3). The combined organic phases were dried over anhydrous sodium sulfate and filtered. The filtrate was concentrated under reduced pressure, and the residue was purified by silica gel column chromatography (petroleum ether/ethyl acetate = 1/0 to 20/1) to obtain compound 52b (1.5 g, yield: 43%). ¹HNMR (400 MHz, CDCl₃) δ 7.37 - 7.27 (m, 1H), 7.25 - 7.23 (m, 1H), 7.04 - 7.02 (m, 1H), 5.23 - 5.17 (m, 1H), 4.92 - 4.81 (m, 1H), 2.24 (s, 3H), 2.00 (d, *J* = 1.1 Hz, 3H).

### Step 2: Synthesis of Compound 52c-2

AD-mix-β (10 g, CAS: 148618-32-0) was added to a mixed solution of compound 52b (1 g, 4.7 mmol) in tert-butanol and water (20 mL/20 mL). The reaction mixture was stirred at room temperature for 16 hours. After the reaction was completed, the reaction mixture was quenched with sodium thiosulfate solution (40 mL) and extracted with dichloromethane (50 mL × 5). The combined organic phases were washed with saturated brine (20 mL × 1) and concentrated under reduced pressure. The residue was purified by silica gel column chromatography (petroleum ether/ethyl acetate = 1/0 to 1/1) to obtain compound 52c-2 (0.5 g, yield: 27.7%). ¹HNMR (400 MHz, CDCl₃) δ 7.54 (d, *J* = 2.2 Hz, 1H), 7.30 (dd, *J =* 8.1, 2.2 Hz, 1H), 7.01 (d, *J* = 8.1 Hz, 1H), 4.63 (d, *J =* 7.1 Hz, 1H), 4.11 (d, *J =* 7.1 Hz, 1H), 2.29 (s, 3H), 1.08 (d, *J =* 6.4 Hz, 3H).

### Step 3: Synthesis of Compound 52d-2

1,1'-Bis(diphenylphosphino)ferrocene-palladium(II)dichloride dichloromethane complex (0.15 g, 0.2 mmol) was added to a solution of compound 52c-2 (0.5 g, 2 mmol), bis(pinacolato)diboron (0.56 g, 2.24 mmol), and potassium acetate (0.4 g, 4.07 mmol) in 1,4-dioxane (20 mL). The reaction mixture was stirred at 90°C for 16 hours. After the reaction was completed, the reaction mixture was filtered, and the filtrate was concentrated under reduced pressure to obtain compound 52d-2 (0.7 g, crude product), which was directly used in the next step. MS m/z (ESI): 292.9 [M+1]⁺.

### Step 4: Synthesis of Compounds 52-P1 and 52-P2

Tetrakis(triphenylphosphine)palladium (158 mg, 0.13 mmol) was added to a mixed solution of compound 52d-2 (400 mg, 1.36 mmol), compound 33a (117 mg, 0.27 mmol), and sodium carbonate (290 mg, 2.73 mmol) in 1,4-dioxane and water (15 mL/1.5 mL). The reaction mixture was stirred at 90°C for 16 hours. After the reaction was completed, the reaction mixture was filtered, and the filtrate was concentrated under reduced pressure. The residue was purified by silica gel column chromatography (dichloromethane/methanol = 1/0 to 10/1) to obtain compound 52-2 (80 mg). Compound 52-2 was then resolved by supercritical fluid chiral chromatography (equipment: SFC Thar prep 80, column: SP-120-10-C18-BIO-C18 250 × 50 mm, 10 µm (pH 8-10), mobile phase: 40% EtOH/CO₂ (NH₄OH 0.2%), total flow rate: 40 g/min) to obtain compounds 52-P1 (30 mg, yield: 3.16%) and 52-P2 (30 mg, yield: 3.26%).

### Compound 52-P1:

MS m/z (ESI): 560.2 [M+1]⁺. Supercritical fluid chromatography (SFC): retention time = 5.30 min, UV = 254 nm. ¹HNMR (400 MHz, CD₃OD) δ 8.57 (s, 1H), 8.44 (d, *J* = 2.3 Hz, 1H), 8.00 (s, 1H), 7.70 (dd, *J =* 6.8, 5.1 Hz, 2H), 7.29 (d, *J =* 8.1 Hz, 1H), 6.85 (s, 1H), 5.50 (d, *J =* 1.8 Hz, 2H), 4.73 (d, *J* = 7.0 Hz, 1H), 3.93 - 3.87 (m, 1H), 2.43 (s, 3H), 2.15 (s, 3H), 2.11 (s, 3H), 1.02 (d, *J =* 6.4 Hz, 3H).

### Compound 52-P2:

MS m/z (ESI): 560.2 [M+1]⁺. Supercritical fluid chromatography (SFC): retention time = 7.17 min, UV = 254 nm. ¹HNMR (400 MHz, CD₃OD) δ 8.57 (s, 1H), 8.44 (d, *J =* 2.3 Hz, 1H), 7.98 (s, 1H), 7.70 (d, *J =* 9.1 Hz, 2H), 7.29 (d, *J =* 8.1 Hz, 1H), 6.85 (s, 1H), 5.50 (d, *J =* 1.7 Hz, 2H), 4.72 (d, *J =* 7.0 Hz, 1H), 3.94 - 3.89 (m, 1H), 2.43 (s, 3H), 2.15 (s, 3H), 2.10 (s, 3H), 1.03 (d, *J* = 6.4 Hz, 3H).

### Example 33 Synthesis of Compounds 53-1 and 53-2

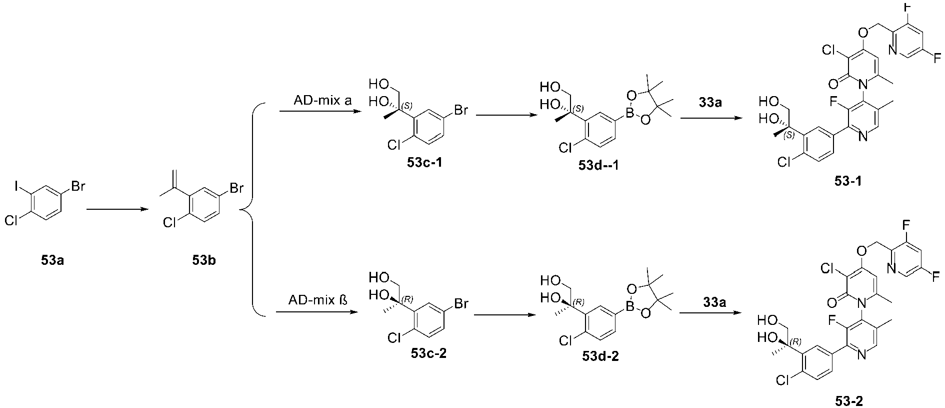

### Step 1: Synthesis of Compound 53b

1,1'-Bis(diphenylphosphino)ferrocene palladium(II)dichloride (138 mg, 0.19 mmol) was added to a mixed solution of compound 53a (1.2 g, 3.78 mmol), isopropenylboronic acid pinacol ester (635 mg, 3.78 mmol), and potassium carbonate (1.0 g, 7.56 mmol) in 1,4-dioxane and water (15 mL/3 mL). The reaction mixture was stirred at 80°C under a nitrogen atmosphere for 16 hours. After the reaction was completed, the reaction mixture was diluted with water (100 mL) and extracted with dichloromethane (50 mL × 3). The combined organic phases were dried over anhydrous sodium sulfate and filtered. The filtrate was concentrated under reduced pressure, and the residue was purified by silica gel column chromatography (petroleum ether/ethyl acetate = 1/0 to 20/1) to obtain compound 53b (900 mg, yield: 92.53%). ¹HNMR (400 MHz, CDCl₃) δ 7.35 - 7.30 (m, 1H), 7.25 - 7.19 (m, 1H), 5.24 (d, *J* = 1.4 Hz, 1H), 4.97 (d, *J* = 0.6 Hz, 1H), 2.07 (d, *J* = 0.8 Hz, 3H).

### Step 2: Synthesis of Compound 53c-1

AD-mix-α (4.5 g, CAS: 153130-59-7) was added to a mixed solution of compound 53b (450 mg, 2.16 mmol) in *tert-*butanol and water (80 mL/8 mL). The reaction mixture was stirred at room temperature for 16 hours. After the reaction was completed, the reaction mixture was quenched with sodium thiosulfate solution (40 mL) and extracted with dichloromethane (30 mL × 5). The combined organic phases were washed with saturated brine (20 mL × 1) and concentrated under reduced pressure. The residue was purified by silica gel column chromatography (dichloromethane/methanol = 1/0 to 20/1) to obtain compound 53c-1 (180 mg, yield: 31.39%). ¹HNMR (400 MHz, CDCl₃) δ 7.96 (d, *J=* 2.5 Hz, 1H), 7.36 - 7.31 (m, 1H), 7.21 (dd, *J* = 8.5, 2.5 Hz, 1H), 4.27 (d, *J* = 11.2 Hz, 1H), 3.81 (d, *J=* 11.2 Hz, 1H), 1.65 (s, 3H).

### Step 3: Synthesis of Compound 53d-1

1,1'-Bis(diphenylphosphino)ferrocene-palladium(II)dichloride dichloromethane complex (27 mg, 0.03 mmol) was added to a solution of compound 53c-1 (180 mg, 0.60 mmol), bis(pinacolato)diboron (206 mg, 0.81 mmol), and potassium acetate (133 mg, 1.36 mmol) in 1,4-dioxane (8 mL). The reaction mixture was stirred at 80°C for 16 hours. After the reaction was completed, the reaction mixture was filtered, and the filtrate was concentrated under reduced pressure to obtain compound 53d-1 (180 mg, crude product), which was directly used in the next step. MS m/z (ESI): 313.1 [M+1]⁺.

### Step 4: Synthesis of Compound 53-1

Tetrakis(triphenylphosphine)palladium (31 mg, 0.03 mmol) was added to a mixed solution of compound 53d-1 (170 mg, 0.54 mmol), compound 33a (163 mg, 0.38 mmol), and sodium carbonate (115 mg, 1.08 mmol) in 1,4-dioxane and water (3 mL/0.5 mL). The reaction mixture was stirred at 80°C under a nitrogen atmosphere for 16 hours. After the reaction was completed, the reaction mixture was concentrated under reduced pressure. The residue was purified by silica gel column chromatography (dichloromethane/methanol = 20/1) to obtain a crude product of compound 53-1 (100 mg). The crude product of compound 53-1 (100 mg) was further purified by a thin-layer chromatography silica gel plate (dichloromethane/methanol = 10/1) to obtain compound 53-1 (60.8 mg, yield: 16.57%).

MS m/z (ESI): 580.2 [M+1]⁺. ¹HNMR (400 MHz, CD₃OD) δ 8.60 (s, 1H), 8.49 (s, 1H), 8.44 (d, *J =* 2.3 Hz, 1H), 7.80 (ddd, *J* = 8.4, 6.9, 1.7 Hz, 1H), 7.71 (td, *J* = 9.5, 2.3 Hz, 1H), 7.49 (dd, *J* = 8.3, 4.1 Hz, 1H), 6.85 (s, 1H), 5.50 (d, *J* = 1.6 Hz, 2H), 4.05 (d, *J* = 11.3 Hz, 1H), 3.89 (d, *J* = 11.4 Hz, 1H), 2.16 (s, 3H), 2.10 (d, *J =* 2.2 Hz, 3H), 1.67 (s, 3H).

### Step 5: Synthesis of Compound 53c-2

AD-mix-β (10 g, CAS: 148618-32-0) was added to a mixed solution of compound 53b (450 mg, 2.16 mmol) in *tert-*butanol and water (8 mL/8 mL). The reaction mixture was stirred at room temperature for 16 hours. After the reaction was completed, the reaction mixture was quenched with sodium thiosulfate solution (40 mL) and extracted with dichloromethane (30 mL × 5). The combined organic phases were washed with saturated brine (20 mL × 1) and concentrated under reduced pressure. The residue was purified by silica gel column chromatography (dichloromethane/methanol = 1/0 to 20/1) to obtain compound 53c-2 (170 mg, yield: 29.6%). ¹HNMR (400 MHz, CDCl₃) δ 7.96 (d, *J =* 2.5 Hz, 1H), 7.35 - 7.32 (m, 1H), 7.21 (dd, *J* = 8.5, 2.5 Hz, 1H), 4.27 (d, *J* = 11.2 Hz, 1H), 3.81 (d, *J =* 11.2 Hz, 1H), 1.65 (s, 3H).

### Step 6: Synthesis of Compound 53d-2

1,1'-Bis(diphenylphosphino)ferrocene-palladium(II)dichloride dichloromethane complex (26 mg, 0.03 mmol) was added to a solution of compound 53c-2 (170 mg, 0.60 mmol), bis(pinacolato)diboron (195 mg, 0.77 mmol), and potassium acetate (125 mg, 1.28 mmol) in 1,4-dioxane (8 mL). The reaction mixture was stirred at 80°C for 16 hours. After the reaction was completed, the reaction mixture was filtered, and the filtrate was concentrated under reduced pressure to obtain compound 53d-2 (170 mg, crude product), which was directly used in the next step. MS m/z (ESI): 313.1 [M+1]⁺.

### Step 7: Synthesis of Compound 53-2

Tetrakis(triphenylphosphine)palladium (31 mg, 0.03 mmol) was added to a mixed solution of compound 53d-2 (170 mg, 0.54 mmol), compound 33a (163 mg, 0.38 mmol), and sodium carbonate (115 mg, 1.08 mmol) in 1,4-dioxane and water (3 mL/0.5 mL). The reaction mixture was stirred at 80°C under a nitrogen atmosphere for 16 hours. After the reaction was completed, the reaction mixture was concentrated under reduced pressure. The residue was purified by silica gel column chromatography (dichloromethane/methanol = 20/1) to obtain a crude product of compound 53-2 (100 mg). The crude product of compound 53-2 (100 mg) was further purified by a thin-layer chromatography silica gel plate (dichloromethane/methanol = 10/1) to obtain compound 53-2 (49.2 mg, yield: 13.72%). MS m/z (ESI): 580.2 [M+1]⁺. ¹H NMR (400 MHz, CD₃OD) δ 8.60 (s, 1H), 8.49 (s, 1H), 8.44 (d, J = 2.3 Hz, 1H), 7.84 - 7.76 (m, 1H), 7.71 (td, *J* = 9.3, 2.3 Hz, 1H), 7.49 (dd, *J* = 8.3, 4.1 Hz, 1H), 6.85 (s, 1H), 5.50 (d, *J* = 1.5 Hz, 2H), 4.05 (d, *J* = 11.3 Hz, 1H), 3.89 (d, *J* = 11.4 Hz, 1H), 2.16 (s, 3H), 2.10 (d, *J =* 2.1 Hz, 3H), 1.67 (s, 3H).

### Example 34 Synthesis of Compound 55-2

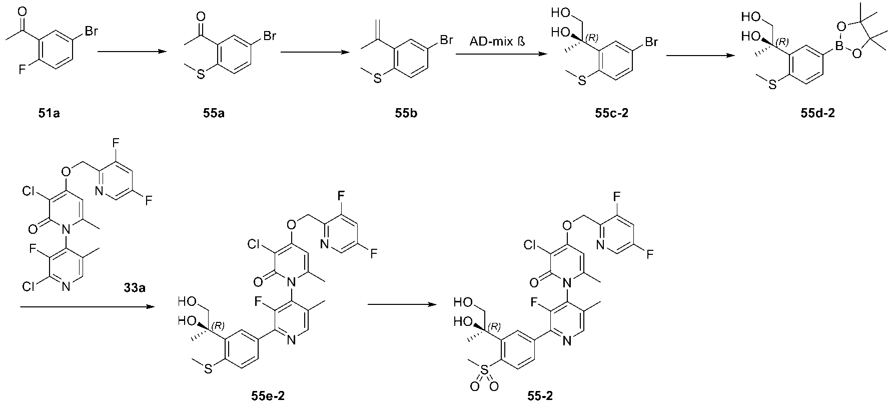

### Step 1: Synthesis of Compound 55a

Compound 51a (1 g, 4.61 mmol) and 20% aqueous sodium methanethiolate solution (2.4 g, 6.91 mmol) were dissolved in *N*,*N*-dimethylformamide (10 mL) under an ice bath. The reaction mixture was stirred at room temperature for 16 hours. After the reaction was completed, the reaction mixture was diluted with water (50 mL) and extracted with ethyl acetate (20 mL × 3). The combined organic phases were dried over anhydrous sodium sulfate, filtered, and concentrated under reduced pressure. The residue was purified by silica gel column chromatography (petroleum ether/ethyl acetate = 1/0 to 5/1) to obtain compound 55a (1.1 g, yield: 87.65%). ¹HNMR(400 MHz, CDCl₃) δ 7.91 (d, *J* = 2.0 Hz, 1H), 7.55 (dd, *J* = 8.7, 2.1 Hz, 1H), 7.17 (d, *J* = 8.6 Hz, 1H), 2.60 (s, 3H), 2.40 (s, 3H).

### Step 2: Synthesis of Compound 55b

At 0°C, methyltriphenylphosphonium bromide (3.2 g, 9.01 mmol) and potassium tert-butoxide (1.0 g, 9.01 mmol) were sequentially added to dry tetrahydrofuran (30 mL). The mixture was stirred for 1 hour, followed by the addition of compound 55a (2.0 g, 8.16 mmol). The reaction mixture was stirred at room temperature for 2 hours. After the reaction was completed, the reaction mixture was diluted with water (80 mL) and extracted with ethyl acetate (80 mL × 3). The combined organic phases were dried over anhydrous sodium sulfate and filtered. The filtrate was concentrated under reduced pressure, and the residue was purified by silica gel column chromatography (petroleum ether/ethyl acetate = 1/0 to 20/1) to obtain compound 55b (1.3 g, yield: 69.38%). ¹HNMR(400 MHz, CDCl₃) δ 7.34 (dd, *J* = 8.4, 2.3 Hz, 1H), 7.23 (d, *J=* 2.3 Hz, 1H), 7.03 (d, *J* = 8.5 Hz, 1H), 5.25 (p, *J* = 1.5 Hz, 1H), 4.96 (dd, *J* = 1.5, 0.8 Hz, 1H), 2.40 (s, 3H), 2.09 - 2.03 (m, 3H).

### Step 3: Synthesis of Compound 55c-2

AD-mix-β (1.0 g, CAS: 148618-32-0) was added to a mixed solution of compound 55b (100 mg, 0.41 mmol) in *tert-*butanol and water (5 mL/5 mL). The reaction mixture was stirred at room temperature for 16 hours. After the reaction was completed, the reaction mixture was quenched with sodium thiosulfate solution (10 mL) and extracted with dichloromethane (20 mL × 5). The combined organic phases were washed with saturated brine (20 mL × 1) and concentrated under reduced pressure. The residue was purified by silica gel column chromatography (dichloromethane/methanol = 1/0 to 20/1) to obtain compound 55c-2 (40 mg, yield: 31.59%). ¹HNMR (400 MHz, CDCl₃) δ 7.67 (d, *J =* 2.3 Hz, 1H), 7.36 (dd, *J =* 8.4, 2.3 Hz, 1H), 7.20 (d, *J* = 8.4 Hz, 1H), 4.16 (d, *J =* 11.3 Hz, 1H), 3.74 (d, *J =* 11.3 Hz, 1H), 2.47 (s, 3H), 1.63 (s, 3H).

### Step 4: Synthesis of Compound 55d-2

1,1'-bis(diphenylphosphino)ferrocene-palladium(II)dichloride dichloromethane complex (5 mg, 0.007 mmol) was added to a solution of compound 55c-2 (40 mg, 0.14 mmol), bis(pinacolato)diboron (44 mg, 0.17 mmol), and potassium acetate (28 mg, 1.49 mmol) in 1,4-dioxane (5 mL). The reaction mixture was stirred at 80°C for 16 hours. After the reaction was completed, the reaction mixture was filtered, and the filtrate was concentrated under reduced pressure to obtain compound 55d-2 (40 mg, crude product), which was directly used in the next step. MS m/z (ESI): 325.1 [M+1]⁺.

### Step 5: Synthesis of Compound 55e-2

Tetrakis(triphenylphosphine)palladium (31 mg, 0.03 mmol) was added to a mixed solution of compound 55d-2 (40 mg, crude product), compound 33a (37 mg, 0.09 mmol), and sodium carbonate (26 mg, 1.22 mmol) in 1,4-dioxane and water (3 mL/0.5 mL). The reaction mixture was stirred at 80°C under a nitrogen atmosphere for 16 hours. After the reaction was completed, the reaction mixture was concentrated under reduced pressure, and the residue was purified by a thin-layer chromatography silica gel plate (dichloromethane/methanol = 10/1) to obtain compound 55e-2 (50 mg, yield: 65.72%). MS m/z (ESI):592.2 [M+1]⁺.

### Step 6: Synthesis of compound 55-2

Potassium peroxymonosulfate (83 mg, 0.14 mmol) was added to a solution of compound 55e-2 (40 mg, 0.07 mmol) in methanol (5 mL). The reaction mixture was stirred at 25°C for 16 hours. After the reaction was completed, the reaction mixture was diluted with water (50 mL) and extracted with dichloromethane (50 mL × 3). The combined organic phases were dried over anhydrous sodium sulfate and filtered. The filtrate was concentrated under reduced pressure to obtain a crude product of compound 55-2, and the crude product was purified by a thin-layer chromatography silica gel plate (dichloromethane/methanol = 10/1) to obtain compound 55-2 (4.3 mg, yield: 9.76%). MS m/z (ESI): 624.0 [M+1]⁺. ¹HNMR (400 MHz, CD₃OD) δ 8.67 (s, 1H), 8.45 (d, *J* = 2.4 Hz, 1H), 8.34 (d, *J* = 8.5 Hz, 1H), 8.18 (d, *J* = 1.0 Hz, 1H), 8.06 - 7.99 (m, 1H), 7.72 (ddd, *J* = 9.6, 8.6, 2.4 Hz, 1H), 6.87 (s, 1H), 5.50 (d, *J* = 1.9 Hz, 2H), 3.97 (d, *J* = 11.3 Hz, 1H), 3.77 (d, *J* = 11.3 Hz, 1H), 3.46 (s, 3H), 2.19 (s, 3H), 2.12 (d, *J* = 4.1 Hz, 3H), 1.67 (d, *J =* 3.1 Hz, 3H).

### Example 35 Synthesis of Compound 61-1

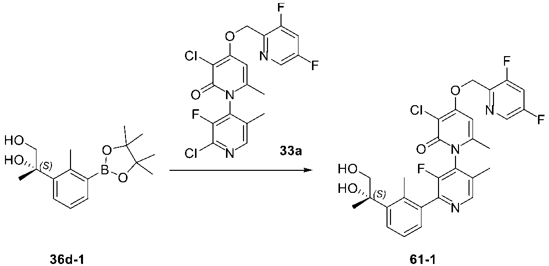

Tetrakis(triphenylphosphine)palladium (32 mg, 0.03 mmol) was added to a mixed solution of compound 36d-1 (80 mg, 0.27 mmol), compound 33a (58.9 mg, 0.14 mmol), and sodium carbonate (58 mg, 0.55 mmol) in 1,4-dioxane and water (5 mL/1 mL). The reaction mixture was stirred at 80°C for 16 hours. After the reaction was completed, the reaction mixture was concentrated under reduced pressure, and the residue was purified by silica gel column chromatography (dichloromethane/methanol = 10/1) to obtain a crude product of compound 61-1 (30 mg). The crude product was purified by preparative HPLC (chromatographic column: Gemini-C18, 150 × 21.2 mm, 5 µm; mobile phase: acetonitrile-water (0.1% formic acid); gradient: 30-50%; column temperature: 25°C; flow rate: 20 mL/min; wavelength: 214 nm; column pressure: 80 bar) to obtain compound 61-1 (8.0 mg, yield: 5.15%). MS m/z (ESI): 560.1 [M+1]⁺. ¹HNMR (400 MHz, CD₃OD) δ 8.56 (s, 1H), 8.43 (d, *J*=2.4, 1H), 7.70 (ddd, *J*=9.5, 8.7, 2.4, 1H), 7.62 (d, *J*=7.7, 1H), 7.26 (t, *J*=7.7, 1H), 7.20 (dd, *J*=7.5, 1.2, 1H), 6.84 (s, 1H), 5.48 (d, *J*=1.9, 2H), 3.83 (dd, *J*=11.1, 1.9, 1H), 3.73 (dd, *J*=11.3, 3.2, 1H), 2.37 (s, 3H), 2.18 (s, 3H), 2.11 (s, 3H), 1.61 (d, *J*=1.6, 3H).

### Example 36 Synthesis of Compounds 62-1, 62-2, 62-P1, 62-P2, 62-P3, and 62-P4

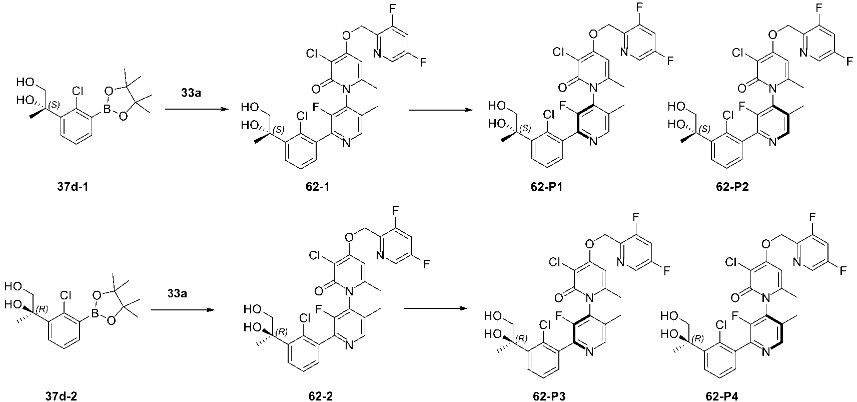

### Synthesis of Compounds 62-P1 and 62-P2

Tetrakis(triphenylphosphine)palladium (44 mg, 0.04 mmol) was added to a mixed solution of compound 37d-1 (120 mg, 0.38 mmol), compound 33a (83 mg, 0.19 mmol), and sodium carbonate (81 mg, 0.77 mmol) in 1,4-dioxane and water (4 mL/1 mL). The reaction mixture was stirred at 80°C for 16 hours. After the reaction was completed, the reaction mixture was concentrated under reduced pressure, and the residue was purified by silica gel column chromatography (dichloromethane/methanol = 30/1) to obtain a crude product of compound 62-1 (160 mg). The crude product was further resolved by supercritical fluid chiral chromatography (equipment: SFC Thar prep 80, column: CHIRALPAK AD-H 250 mm × 20 mm, 5 µm, mobile phase: 40% MeOH/CO₂ (NH₄OH 0.2%), total flow rate: 40 g/min) to obtain compounds 62-P1 (16.1 mg, yield: 6.95%) and 62-P2 (24.6 mg, yield: 10.71%).

### Compound 62-P1:

MS m/z (ESI): 580.0 [M+1]⁺. Supercritical fluid chromatography (SFC): retention time = 2.63 min, UV = 254 nm. ¹H NMR (400 MHz, CD₃OD) δ 8.57 (s, 1H), 8.43 (d, *J* = 2.4 Hz, 1H), 8.02 (dd, *J* = 7.9, 1.8 Hz, 1H), 7.74 - 7.66 (m, 1H), 7.45 (t, *J* = 7.7 Hz, 1H), 7.39 (dd, *J* = 7.5, 1.8 Hz, 1H), 6.85 (s, 1H), 5.49 (d, *J* = 1.7 Hz, 2H), 4.02 (d, *J* = 11.3 Hz, 1H), 3.90 (d, *J* = 11.3 Hz, 1H), 2.19 (s, 3H), 2.11 (s, 3H), 1.66 (d, *J =* 4.6 Hz, 3H).

### Compound 62-P2:

MS m/z (ESI): 580.0 [M+1]⁺. Supercritical fluid chromatography (SFC): retention time = 5.2 min, UV = 254 nm. ¹H NMR (400 MHz, CD₃OD) δ 8.57 (s, 1H), 8.43 (d, J = 2.4 Hz, 1H), 8.03 (dd, J = 7.9, 1.8 Hz, 1H), 7.70 (ddd, J = 9.4, 5.8, 2.4 Hz, 1H), 7.45 (t, J = 7.7 Hz, 1H), 7.39 (dd, J = 7.5, 1.8 Hz, 1H), 6.85 (s, 1H), 5.49 (d, J = 1.8 Hz, 2H), 4.02 (d, J = 11.3 Hz, 1H), 3.87 (d, J = 11.4 Hz, 1H), 2.19 (s, 3H), 2.12 (s, 3H), 1.67 (s, 3H).

### Synthesis of Compounds 62-P3 and 62-P4

Tetrakis(triphenylphosphine)palladium (33 mg, 0.03 mmol) was added to a mixed solution of compound 37d-2 (90 mg, 0.29 mmol), compound 33a (86 mg, 0.20 mmol), and sodium carbonate (61 mg, 0.58 mmol) in 1,4-dioxane and water (10 mL/2 mL). The reaction mixture was stirred at 80°C for 16 hours. After the reaction was completed, the reaction mixture was concentrated under reduced pressure, and the residue was purified by silica gel column chromatography (dichloromethane/methanol = 30/1) to obtain a crude product of compound 62-1 (50 mg). The crude product was further resolved by supercritical fluid chiral chromatography (equipment: SFC Thar prep 80, column: CHIRALPAK AD-H 250 mm × 20 mm, 5 µm, mobile phase: 40% MeOH/CO₂ (NH₄OH 0.2%), total flow rate: 40 g/min) to obtain compounds 62-P3 (15.3 mg, yield: 8.89%) and 62-P4 (22.9 mg, yield: 13.30%).

### Compound 62-P3:

MS m/z (ESI): 580.0 [M+1]⁺. Supercritical fluid chromatography (SFC): retention time = 5.51 min, UV = 254 nm. ¹HNMR (400 MHz, CD₃OD) δ 8.57 (s, 1H), 8.43 (d, *J* = 2.3 Hz, 1H), 8.02 (dd, *J* = 7.9, 1.8 Hz, 1H), 7.70 (td, *J* = 9.6, 2.3 Hz, 1H), 7.45 (t, *J =* 7.7 Hz, 1H), 7.39 (dd, *J* = 7.5, 1.7 Hz, 1H), 6.84 (s, 1H), 5.48 (d, *J =* 1.6 Hz, 2H), 4.02 (d, *J =* 11.3 Hz, 1H), 3.87 (d, *J* = 11.3 Hz, 1H), 2.19 (s, 3H), 2.11 (s, 3H), 1.66 (s, 3H).

### Compound 62-P4:

MS m/z (ESI): 580.0 [M+1]⁺. Supercritical fluid chromatography (SFC): retention time = 7.02 min, UV = 254 nm. ¹HNMR (400 MHz, CD₃OD) δ 8.56 (s, 1H), 8.43 (d, *J* = 2.3 Hz, 1H), 8.02 (dd, *J* = 7.9, 1.8 Hz, 1H), 7.73 - 7.66 (m, 1H), 7.45 (d, *J* = 15.5 Hz, 1H), 7.39 (dd, *J* = 7.5, 1.8 Hz, 1H), 6.84 (s, 1H), 5.48 (d, *J =* 1.8 Hz, 2H), 4.02 (d, *J* = 11.3 Hz, 1H), 3.90 (d, *J* = 11.3 Hz, 1H), 2.19 (s, 3H), 2.11 (s, 3H), 1.65 (s, 3H).

### Example 37 Synthesis of Compounds 65-P1, 65-P2, 65-P3, and 65-P4

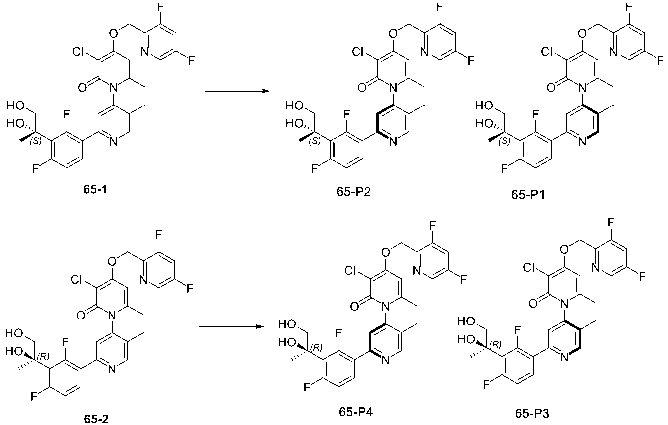

Compound 65-1 (100 mg) was resolved by supercritical fluid chiral chromatography (equipment: SFC Thar prep 80, column: CHIRALPAK OJ-H 250 mm × 20 mm, 5 µm, mobile phase: 40% IPA (NH₄OH 0.2%), total flow rate: 40 g/min) to obtain compounds 65-P1 (42 mg) and 65-P2 (43 mg).

### Compound 65-P1:

MS m/z (ESI): 564.1 [M+1]⁺. Supercritical fluid chromatography (SFC): retention time = 2.63 min, UV = 254 nm. ¹H NMR (400 MHz, CDCl₃) δ 8.73 (s, 1H), 8.40 (d, J = 2.1 Hz, 1H), 7.86 (d, J = 5.4 Hz, 1H), 7.48 (s, 1H), 7.38 - 7.29 (m, 1H), 7.00 (t, J = 10.0 Hz, 1H), 6.40 (s, 1H), 5.42 (s, 2H), 4.10 (d, J = 11.0 Hz, 1H), 3.73 (d, J = 11.0 Hz, 1H), 2.15 (s, 3H), 2.01 (s, 3H), 1.65 (s, 3H).

### Compound 65-P2:

MS m/z (ESI): 564.2 [M+1]⁺. Supercritical fluid chromatography (SFC): retention time = 4.64 min, UV = 254 nm. ¹H NMR (400 MHz, CDCl₃) δ 8.70 (s, 1H), 8.40 (d, J = 2.3 Hz, 1H), 7.88 (dd, J = 14.9, 8.7 Hz, 1H), 7.49 (d, J = 1.9 Hz, 1H), 7.37 - 7.29 (m, 1H), 7.02 (dd, J = 11.2, 9.4 Hz, 1H), 6.40 (s, 1H), 5.42 (d, J = 1.7 Hz, 2H), 4.11 (d, J = 11.2 Hz, 1H), 3.75 (d, J = 11.2 Hz, 1H), 2.15 (s, 3H), 2.01 (s, 3H), 1.66 (s, 3H).

Compound 65-2 (100 mg) was resolved by supercritical fluid chiral chromatography (equipment: SFC Thar prep 80, column: CHIRALPAK OJ-H 250 mm × 20 mm, 5 µm, mobile phase: 40% IPA (NH₄OH 0.2%), total flow rate: 40 g/min) to obtain compounds 65-P3 (42 mg) and 65-P4 (33 mg).

### Compound 65-P3:

MS m/z (ESI): 564.1 [M+1]⁺. Supercritical fluid chromatography (SFC): retention time = 2.1 min, UV = 254 nm. ¹H NMR (400 MHz, CDCl₃) δ 8.71 (s, 1H), 8.40 (d, *J =* 2.1 Hz, 1H), 7.86 (d, J = 5.4 Hz, 1H), 7.48 (s, 1H), 7.38 - 7.29 (m, 1H), 7.00 (t, J = 10.0 Hz, 1H), 6.40 (s, 1H), 5.42 (s, 2H), 4.10 (d, J = 11.0 Hz, 1H), 3.73 (d, J = 10.9 Hz, 1H), 2.15 (s, 3H), 2.01 (s, 3H), 1.65 (s, 3H).

### Compound 65-P4:

MS m/z (ESI): 564.1 [M+1]⁺. Supercritical fluid chromatography (SFC): retention time = 3.63 min, UV = 254 nm. ¹H NMR (400 MHz, CDCl₃) δ 8.70 (s, 1H), 8.39 (d, J = 2.3 Hz, 1H), 7.86 (dd, J = 15.0, 8.6 Hz, 1H), 7.47 (s, 1H), 7.37 - 7.27 (m, 1H), 6.99 (dd, J = 11.5, 9.1 Hz, 1H), 6.38 (s, 1H), 5.40 (d, J = 1.6 Hz, 2H), 4.07 (d, J = 11.2 Hz, 1H), 3.71 (d, J = 11.0 Hz, 1H), 2.13 (s, 3H), 1.99 (s, 3H), 1.63 (s, 3H).

### Example 38 Synthesis of Compounds 70, 70-P1, and 70-P2

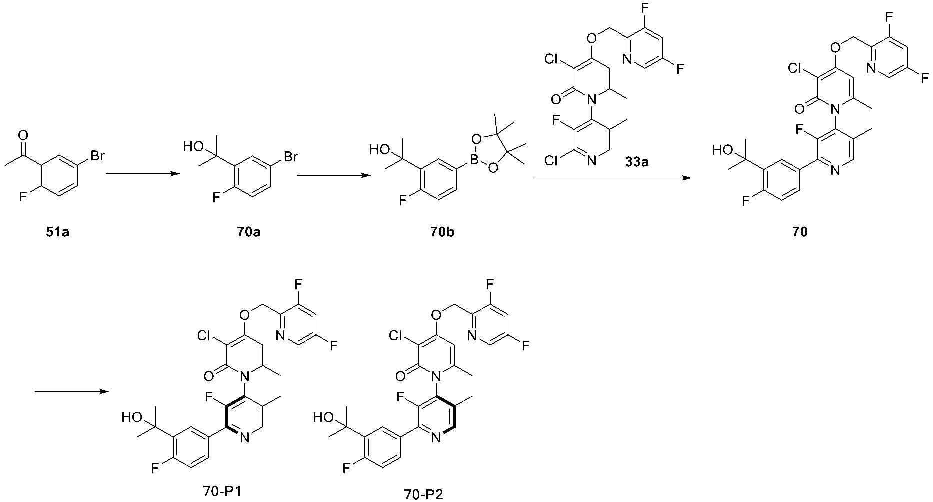

### Step 1: Synthesis of Compound 70a

At 0°C, methylmagnesium bromide (1.5 mL, 4.61 mmol) was added to a solution of compound 51a (500 mg, 2.31 mmol) in tetrahydrofuran (10 mL), and the reaction mixture was stirred at 0°C for 3 hours. After the reaction was completed, the reaction mixture was quenched with saturated ammonium chloride solution (20 mL), diluted with water (20 mL), and extracted with ethyl acetate (50 mL × 3). The combined organic phases were dried over anhydrous sodium sulfate and filtered. The filtrate was concentrated under reduced pressure, and the residue was purified by silica gel column chromatography (petroleum ether/ethyl acetate = 1/0 to 5/1) to obtain compound 70a (200 mg, yield: 37.3%). ¹HNMR (400 MHz, CDCl₃) δ 7.95 - 7.76 (m, 1H), 7.74 - 7.55 (m, 1H), 7.32 - 7.24 (m, 1H), 6.94 - 6.76 (m, 1H), 1.61 (s, 6H).

### Step 2: Synthesis of Compound 70b

1,1'-Bis(diphenylphosphino)ferrocene palladium(II)dichloride (35 mg, 0.04 mmol) was added to a solution of compound 70a (200 mg, 0.86 mmol), bis(pinacolato)diboron (327 mg, 1.29 mmol), and potassium acetate (168 mg, 1.71 mmol) in 1,4-dioxane (5 mL). The reaction mixture was stirred at 80°C under a nitrogen atmosphere for 16 hours. After the reaction was completed, the reaction mixture was concentrated under reduced pressure to obtain compound 70b (200 mg, crude product). MS m/z (ESI): 281.1 [M+1]⁺.

### Step 3: Synthesis of Compounds 70-P1 and 70-P2

Tetrakis(triphenylphosphine)palladium (42 mg, 0.04 mmol) was added to a mixed solution of compound 70b (200 mg, 0.72 mmol), compound 33a (153 mg, 0.36 mmol), and sodium acetate (151 mg, 1.43 mmol) in 1,4-dioxane and water (2.5 mL/0.5 mL). The reaction mixture was stirred at 90°C for 16 hours. After the reaction was completed, the reaction mixture was diluted with water (30 mL) and extracted with ethyl acetate (50 mL × 3). The combined organic phases were dried over anhydrous sodium sulfate and filtered. The filtrate was concentrated under reduced pressure, and the residue was purified by silica gel column chromatography (dichloromethane/methanol = 1/0 to 10/1) to obtain a crude product of compound 70. The crude product was then resolved by supercritical fluid chiral chromatography (equipment: SFC Thar prep 80, column: CHIRALPAK IC 250 mm × 20 mm, 5 µm, mobile phase: 40% MeOH/CO₂ (NH₄OH 0.2%), total flow rate: 40 g/min) to obtain compounds 70-P1 (8.4 mg, yield: 2.1%) and 70-P2 (8.7 mg, yield: 2.2%).

### Compound 70-P1:

MS m/z (ESI): 548.0 [M+1]⁺. Supercritical fluid chromatography (SFC): retention time = 3.69 min, UV = 254 nm. ¹H NMR (400 MHz, CD₃OD) δ 8.60 (d, *J =* 0.7 Hz, 1H), 8.46 (d, *J=* 2.4 Hz, 1H), 8.27-8.25 m, 1H), 7.84-7.79 (m, 1H), 7.73-7.68 (m, 1H), 7.19 (dd, *J=* 11.7, 8.5 Hz, 1H), 6.87 (d, *J* = 0.5 Hz, 1H), 5.52 (d, *J* = 1.9 Hz, 2H), 2.17 (s, 3H), 2.13 (s, 3H), 1.62 (d, *J* = 1.0 Hz, 6H).

### Compound 70-P2:

MS m/z (ESI): 548.1 [M+1]⁺. Supercritical fluid chromatography (SFC): retention time = 14.55 min, UV = 254 nm. ¹H NMR (400 MHz, CD₃OD) δ 8.60 (s, 1H), 8.46 (d, *J* = 2.4 Hz, 1H), 8.27 (dd, *J* = 7.9*,* 1.4 Hz, 1H), 7.84-7.79 (m, 1H), 7.78 - 7.65 (m, 1H), 7.19 (dd, *J =* 11.6, 8.5 Hz, 1H), 6.87 (s, 1H), 5.52 (d, *J* = 1.9 Hz, 2H), 2.17 (s, 3H), 2.13 (s, 3H), 1.62 (s, 6H).

### Example 39 Synthesis of Compound 86

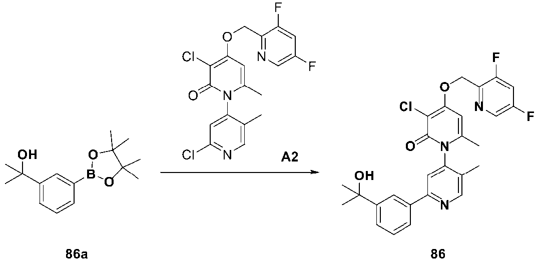

Tetrakis(triphenylphosphine)palladium (56.1 mg, 0.048 mmol) was added to a mixed solution of compound 86a (200 mg, 0.485 mmol), compound A2 (191 mg, 0.728 mmol), and sodium carbonate (102.8 mg, 0.97 mmol) in 1,4-dioxane and water (3 mL/0.3 mL). The reaction mixture was stirred at 90°C overnight. After the reaction was completed, the reaction mixture was concentrated under reduced pressure, and the residue was purified by silica gel column chromatography (dichloromethane/methanol = 1/0 to 20/1) to obtain a crude product of compound 86. The crude product was further purified by preparative HPLC (chromatographic column: Gemini-C18; 150 × 21.2 mm, 5 µm; YMC Triart C18; 12 nm 10u; 50 × 250 mm; mobile phase: acetonitrile-water (0.1% ammonia water); column temperature: 25°C; flow rate: 40 mL/min) to obtain compound 86 (115.1 mg, yield: 46.3%). MS m/z (ESI): 512.1 [M+1]⁺. ¹H NMR (400 MHz, DMSO-*d₆*) δ 8.74 (s, 1H), 8.61 (s, 1H), 8.21 (s, 1H), 8.10 (t, *J* = 9.2 Hz, 1H), 7.99 (s, 1H), 7.95 (d, *J =* 7.6 Hz, 1H), 7.54 (d, *J* = 7.6 Hz, 1H), 7.41 (t, *J =* 7.6 Hz, 1H), 6.81 (s, 1H), 5.49 (s, 2H), 2.03 (s, 3H), 1.99 (s, 3H), 1.48 (s, 6H).

### Example 40 Synthesis of Compounds 87-2, 87-P1, and 87-P2

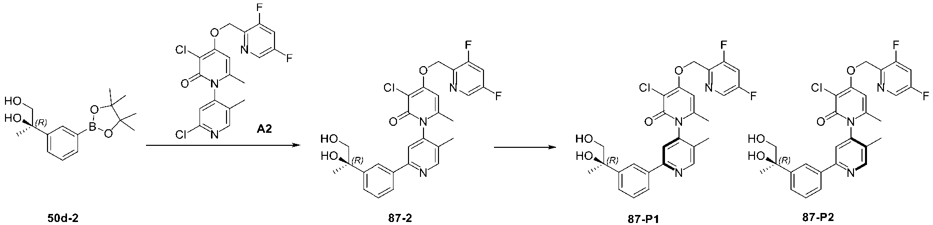

Tetrakis(triphenylphosphine)palladium (103 mg, 0.09 mmol) was added to a mixed solution of compound 50d-2 (250 mg, crude product), compound A2 (111 mg, 0.27 mmol), and sodium carbonate (190 mg, 1.8 mmol) in 1,4-dioxane and water (15 mL, 1.5 mL). The reaction mixture was stirred at 90°C for 16 hours. After the reaction was completed, the reaction mixture was concentrated under reduced pressure, and the residue was purified by silica gel column chromatography (dichloromethane/methanol = 10/1) to obtain compound 87-2 (100 mg). Compound 87-2 (100 mg) was then resolved by supercritical fluid chiral chromatography (equipment: SFC Thar prep 80, column: CHIRALPAK AD-H 250 mm × 20 mm, 5 µm, mobile phase: 40% MeOH (NH₄OH 0.2%): 60% CO₂, total flow rate: 40 g/min) to obtain compounds 87-P1 (30.5 mg, yield: 6.36%) and 87-P2 (31.4 mg, yield: 6.55%).

### Compound 87-P1:

MS m/z (ESI): 528.2 [M+1]⁺. Supercritical fluid chromatography (SFC): retention time = 3.01 min, UV = 220 nm. ¹H NMR (400 MHz, CD₃OD) δ 8.70 (s, 1H), 8.45 (d, *J* = 2.3 Hz, 1H), 8.11 (s, 1H), 7.86 (d, *J =* 7.8 Hz, 1H), 7.80 (s, 1H), 7.77 - 7.68 (m, 1H), 7.59 (d, *J =* 8.0 Hz, 1H), 7.45 (t, *J= 7.8* Hz, 1H), 6.82 (s, 1H), 5.50 (d, *J=* 1.4 Hz, 2H), 3.66 (q, *J=* 11.2 Hz, 2H), 2.10 (d, *J =* 17.2 Hz, 6H), 1.55 (s, 3H).

### Compound 87-P2:

MS m/z (ESI): 528.2 [M+1]⁺. Supercritical fluid chromatography (SFC): retention time = 6.08 min, UV = 220 nm. ¹H NMR (400 MHz, CD₃OD) δ 8.71 (s, 1H), 8.46 (d, *J=* 2.2 Hz, 1H), 8.12 (s, 1H), 7.86 (d, *J=* 7.7 Hz, 1H), 7.80 (s, 1H), 7.77 - 7.66 (m, 1H), 7.59 (d, *J* = 7.8 Hz, 1H), 7.45 (t, *J* = 7.8 Hz, 1H), 6.82 (s, 1H), 5.50 (d, *J =* 1.2 Hz, 2H), 3.66 (q, *J =* 11.2 Hz, 2H), 2.10 (d, *J* = 16.7 Hz, 6H), 1.56 (s, 3H).

### Example 41 Synthesis of Compounds 88, 88-P1, and 88-P2

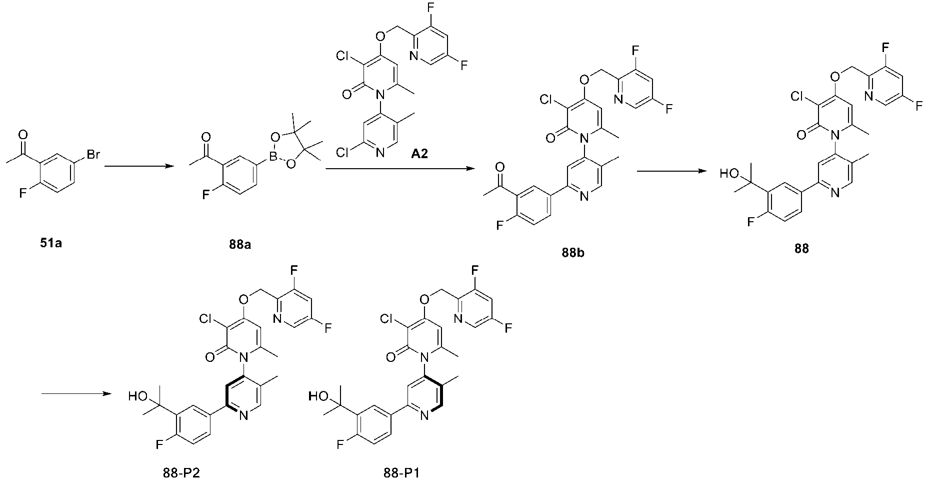

### Step 1: Synthesis of Compound 88a

Compound 51a (500 mg, 2.3 mmol), bis(pinacolato)diboron (877 mg, 3.4 mmol), potassium acetate (452 mg, 4.6 mmol), and 1,1'-bis(diphenylphosphino)ferrocene-palladium(II)dichloride dichloromethane complex (168 mg, 0.2 mmol) were sequentially added to 1,4-dioxane (10 mL). The reaction mixture was stirred at 90°C for 16 hours. After the reaction was completed, the reaction mixture was filtered, and the filtrate was concentrated under reduced pressure to obtain compound 88a (600 mg, crude product), which was directly used in the next step. MS m/z (ESI): 265.1 [M+1]⁺.

### Step 2: Synthesis of Compound 88b

Tetrakis(triphenylphosphine)palladium (131 mg, 0.1 mmol) was added to a mixed solution of compound 88a (300 mg, 1.1 mmol), compound A2 (421 mg, 1.0 mmol), and sodium carbonate (240 mg, 2.2 mmol) in 1,4-dioxane and water (1 mL/l mL). The reaction mixture was stirred at 80°C overnight. After the reaction was completed, the reaction mixture was concentrated under reduced pressure, and the residue was purified by silica gel column chromatography (dichloromethane/methanol = 1/0 to 10/1) to obtain compound 88b (300 mg, yield: 46.3%). MS m/z (ESI): 514.1 [M+1]⁺.

### Step 3: Synthesis of Compounds 88-P1 and 88-P2

Methylmagnesium bromide (0.4 mL, 3 M) was added to a solution of compound 88b (200 mg, 0.4 mmol) in tetrahydrofuran (10 mL). The reaction mixture was slowly warmed to room temperature and stirred for 16 hours. After the reaction was completed, the reaction mixture was quenched with saturated ammonium chloride solution (30 mL) and extracted with ethyl acetate (50 mL × 3). The combined organic phases were washed with saturated brine (100 mL × 1), concentrated under reduced pressure, and the residue was purified by silica gel column chromatography (dichloromethane/methanol = 1/0 to 10/1) to obtain a crude product of compound 88 (100 mg). The crude product was further resolved by supercritical fluid chiral chromatography (equipment: SFC Thar prep 80, column: CHIRALPAK AD-H 250 mm × 20 mm, 5 µm, mobile phase: 40% MeOH/CO₂ (NH₄OH 0.2%), total flow rate: 40 g/min) to obtain compounds 88-P1 (retention time: 6.68 minutes, 25.4 mg, yield: 24.6%) and 88-P2 (retention time: 12.19 minutes, 30.2 mg, yield: 29.3%).

### Compound 88-P1:

MS m/z (ESI): 530.2 [M+1]⁺. Supercritical fluid chromatography (SFC): retention time = 6.68 min, UV = 214 nm. ¹H NMR (400 MHz, CD₃OD) δ 8.68 (s, 1H), 8.44 (d, *J= 2.3* Hz, 1H), 8.27 (dd, *J* = 7.9*,* 2.4 Hz, 1H), 7.88 (ddd, *J=* 8.5, 4.5, 2.5 Hz, 1H), 7.74 - 7.68 (m, 2H), 7.13 (dd, *J=* 11.6, 8.5 Hz, 1H), 6.80 (s, 1H), 5.48 (d, *J* = 1.8 Hz, 2H), 2.10 (s, 3H), 2.06 (s, 3H), 1.60 (s, 6H).

### Compound 88-P2:

MS m/z (ESI): 530.2 [M+1]⁺. Supercritical fluid chromatography (SFC): retention time = 12.19 min, UV = 214 nm. ¹H NMR (400 MHz, CD₃OD) δ 8.68 (s, 1H), 8.43 (d, *J* = 2.3 Hz, 1H), 8.26 (dd, *J=* 7.9, 2.4 Hz, 1H), 7.88 (ddd, *J =* 8.4, 4.5, 2.4 Hz, 1H), 7.71 (dt, *J* = 9.6, 2.4 Hz, 2H), 7.13 (dd, *J =* 11.6, 8.5 Hz, 1H), 6.80 (s, 1H), 5.48 (d, *J* = 1.8 Hz, 2H), 2.09 (s, 3H), 2.06 (s, 3H), 1.59 (s, 6H).

### Example 42 Synthesis of Compounds 106-P1 and 106-P2

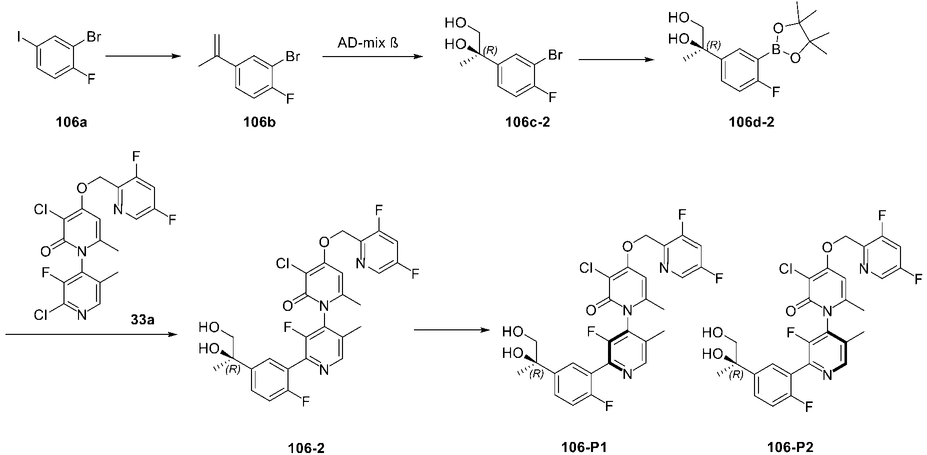

### Step 1: Synthesis of Compound 106b

Bis(triphenylphosphine)palladium(II) chloride (230 mg, 0.3 mmol) was added to a mixed solution of compound 106a (1.0 g, 3.3 mmol), isopropenylboronic acid pinacol ester (550.0 mg, 3.3 mmol), and potassium carbonate (910.0 mg, 6.6 mmol) in 1,4-dioxane and water (20 mL/4 mL). The reaction mixture was stirred at 80°C for 16 hours. After the reaction was completed, the reaction mixture was diluted with water (50 mL) and extracted with dichloromethane (50 mL × 3). The combined organic phases were dried over anhydrous sodium sulfate and filtered. The filtrate was concentrated under reduced pressure, and the residue was purified by silica gel column chromatography (petroleum ether/ethyl acetate = 1/0 to 8/1) to obtain compound 106b (400 mg, yield: 51.5%). ¹HNMR (400 MHz, CDCl₃) δ 7.61 (dd, *J=* 6.6, 2.3 Hz, 1H), 7.33-7.29 (m, 1H), 7.03 (t, *J* = 8.5 Hz, 1H), 5.29 (s, 1H), 5.08 (s, 1H), 2.07 (t, *J* = 11.6 Hz, 3H).

### Step 2: Synthesis of Compound 106c-2

AD-mix-β (4.0 g) was added to a mixed solution of compound 106b (400.0 mg, 1.8 mmol) in tert-butanol and water (10 mL/10 mL). The reaction mixture was stirred at room temperature for 16 hours. After the reaction was completed, the reaction mixture was quenched with saturated sodium thiosulfate solution (40 mL) and extracted with ethyl acetate (50 mL × 5). The combined organic phases were washed with saturated brine (50 mL × 1), concentrated under reduced pressure, and the residue was purified by silica gel column chromatography (dichloromethane/methanol = 20/1) to obtain compound 106c-2 (200.0 mg, yield: 38.86%). ¹HNMR (400 MHz, CDCl₃) δ 7.67 (dd, *J =* 6.6, 2.3 Hz, 1H), 7.35 (ddd, *J =* 8.6, 4.6, 2.3 Hz, 1H), 7.10 (t, *J =* 8.4 Hz, 1H), 3.74 (d, *J* = 11.1 Hz, 1H), 3.62 (d, *J =* 11.1 Hz, 1H), 2.24 (s, 2H), 1.51 (s, 3H).

### Step 3: Synthesis of Compound 106d-2

1,1'-Bis(diphenylphosphino)ferrocene-palladium(II)dichloride dichloromethane complex (44 mg, 0.06 mmol) was added to a solution of compound 106c-2 (150 mg, 0.6 mmol), bis(pinacolato)diboron (229.3 mg, 0.9 mmol), and potassium acetate (118 mg, 1.2 mmol) in 1,4-dioxane (10 mL). The reaction mixture was stirred at 90°C for 16 hours. After the reaction was completed, the reaction mixture was filtered, and the filtrate was concentrated under reduced pressure to obtain compound 106d-2 (200 mg, crude product), which was directly used in the next step. MS m/z (ESI): 297.2 [M+1]⁺.

### Step 4: Synthesis of Compounds 106-P1 and 106-P2

Tetrakis(triphenylphosphine)palladium (58 mg, 0.05 mmol) was added to a mixed solution of compound 106d-2 (150 mg, 0.5 mmol), compound 33a (152 mg, 0.3 mmol), and sodium carbonate (107 mg, 1.0 mmol) in 1,4-dioxane and water (10 mL/l mL). The reaction mixture was stirred at 90°C overnight. After the reaction was completed, the resulting residue from the concentration of the reaction mixture under reduced pressure was purified by silica gel column chromatography (dichloromethane/methanol = 1/0 to 4/1) to obtain a crude product of compound 106-2. The crude product was then resolved by supercritical fluid chiral chromatography (equipment: SFC Thar prep 80, column: CHIRALPAK IC 250 mm × 20 mm, 5 µm, mobile phase: 40% MeOH/CO₂ (NH₄OH 0.2%), total flow rate: 40 g/min) to obtain compounds 106-P1 (42 mg, yield: 16.62%) and 106-P2 (42 mg, yield: 16.62%).

### Compound 106-P1:

MS m/z (ESI): 564.2 [M+1]⁺. Supercritical fluid chromatography (SFC): retention time = 3.26 min, UV = 214 nm. ¹H NMR (400 MHz, CDCl₃) δ 8.57 (s, 1H), 8.40 (d, *J =* 2.3 Hz, 1H), 7.69 (d, *J =* 4.6 Hz, 1H), 7.62 - 7.55 (m, 1H), 7.32 (ddd, *J =* 9.1, 8.1, 2.4 Hz, 1H), 7.19 - 7.11 (m, 1H), 6.44 (s, 1H), 5.41 (d, *J=* 1.8 Hz, 2H), 3.81 (d, *J* = 11.2 Hz, 1H), 3.65 (d, *J= 11.1* Hz, 1H), 2.21 (s, 3H), 2.07 (s, 3H), 1.53 (d, *J* = 8.3 Hz, 3H).

### Compound 106-P2:

MS m/z (ESI): 564.2 [M+1]⁺. Supercritical fluid chromatography (SFC): retention time = 11.93 min, UV = 214 nm. ¹H NMR (400 MHz, CDCl₃) δ 8.54 (s, 1H), 8.40 (d, *J* = 2.3 Hz, 1H), 7.67 (d, *J =* 4.7 Hz, 1H), 7.62 - 7.52 (m, 1H), 7.38 - 7.28 (m, 1H), 7.13 (t, *J* = 9.1 Hz, 1H), 6.44 (s, 1H), 5.41 (d, *J* = 1.2 Hz, 2H), 3.81 (d, *J* = 11.1 Hz, 1H), 3.64 (d, *J* = 11.2 Hz, 1H), 2.20 (s, 3H), 2.07 (s, 3H), 1.53 (s, 3H).

### Example 43 Synthesis of Compounds 107-P1 and 107-P2

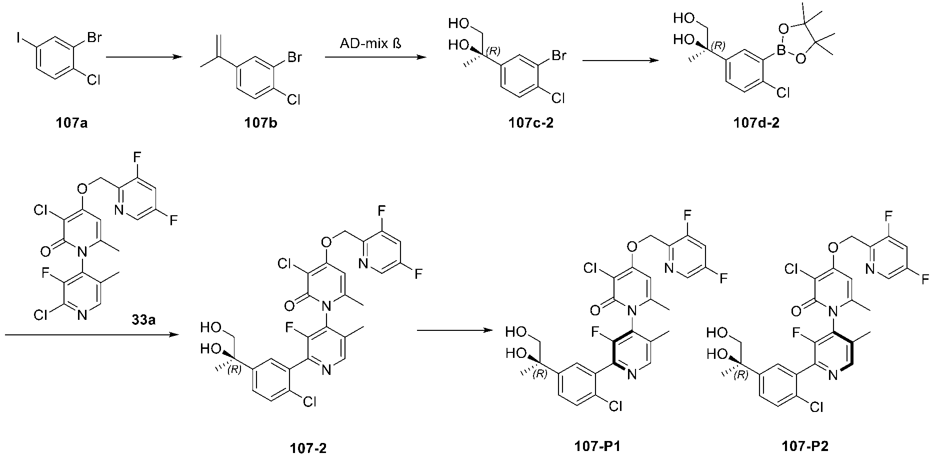

### Step 1: Synthesis of Compound 107b

1,1'-Bis(diphenylphosphino)ferrocene-palladium(II)dichloride dichloromethane complex (115 mg, 0.16 mmol) was added to a mixed solution of compound 107a (1.0 g, 3.15 mmol), isopropenylboronic acid pinacol ester (529 mg, 3.15 mmol), and potassium carbonate (871 mg, 6.30 mmol) in 1,4-dioxane and water (15 mL /3 mL). The reaction mixture was stirred at 80°C for 16 hours. After the reaction was completed, the reaction mixture was diluted with water (50 mL) and extracted with dichloromethane (50 mL × 3). The combined organic phases were dried over anhydrous sodium sulfate and filtered. The filtrate was concentrated under reduced pressure, and the residue was purified by silica gel column chromatography (petroleum ether/ethyl acetate = 1/0 to 20/1) to obtain compound 107b (360 mg, yield: 44.41%). ¹HNMR (400 MHz, CDCl₃) δ 7.69 (d, *J* =2.1 Hz, 1H), 7.41 -7.36 (m, 1H), 7.35 - 7.30 (m, 1H), 5.36 (s, 1H), 5.14 - 5.11 (m, 1H), 2.11 (s, 3H).

### Step 2: Synthesis of Compound 107c-2

AD-mix-β (3.6 g) was added to a mixed solution of compound 107b (360 mg, 1.55 mmol) in tert-butanol and water (10 mL/10 mL). The reaction mixture was stirred at room temperature for 16 hours. After the reaction was completed, the reaction mixture was quenched with saturated sodium thiosulfate solution (40 mL) and extracted with ethyl acetate (50 mL × 5). The combined organic phases were washed with saturated brine (50 mL × 1), concentrated under reduced pressure, and the residue was purified by silica gel column chromatography (dichloromethane/methanol = 20/1) to obtain compound 107c-2 (260.0 mg, yield: 56.7%). ¹HNMR (400 MHz, CDCl₃) δ 7.74 (d, *J* =2.1 Hz, 1H), 7.43 (d, *J* =8.4 Hz, 1H), 7.31 (dd, J=8.4, 2.2 Hz, 1H), 3.74 (d, *J* =11.1 Hz, 1H), 3.62 (d, J=11.1 Hz, 1H), 1.50 (s, 3H).

### Step 3: Synthesis of Compound 107d-2

1,1'-Bis(diphenylphosphino)ferrocene-palladium(II)dichloride dichloromethane complex (40 mg, 0.05 mmol) was added to a solution of compound 107c-2 (260 mg, 0.98 mmol), bis(pinacolato)diboron (298 mg, 1.17 mmol), and potassium acetate (192 mg, 1.96 mmol) in 1,4-dioxane (6 mL). The reaction mixture was stirred at 80°C for 16 hours. After the reaction was completed, the reaction mixture was filtered, and the filtrate was concentrated under reduced pressure to obtain compound 107d-2 (260 mg, crude product), which was directly used in the next step. MS m/z (ESI): 312.9 [M+1]⁺.

### Step 4: Synthesis of Compounds 107-P1 and 107-P2

Tetrakis(triphenylphosphine)palladium (37 mg, 0.03 mmol) was added to a mixed solution of compound 107d-2 (150 mg, 0.5 mmol), compound 33a (193 mg, 0.45 mmol), and sodium carbonate (136 mg, 1.28 mmol) in 1,4-dioxane and water (8 mL/2 mL). The reaction mixture was stirred at 80°C overnight. After the reaction was completed, the resulting residue from the concentration of the reaction mixture under reduced pressure was purified by silica gel column chromatography (dichloromethane/methanol = 1/0 to 30/1) to obtain a crude product of compound 107-2. The crude product was then resolved by supercritical fluid chiral chromatography (equipment: SFC Thar prep 80, column: CHIRALPAK IC 250 mm × 20 mm, 5 µm, mobile phase: 40% MeOH/CO₂ (NH₄OH 0.2%), total flow rate: 40 g/min) to obtain compounds 107-P1 (53.1 mg, yield: 14.21%) and 107-P2 (42.1 mg, yield: 10.96%).

### Compound 107-P1:

MS m/z (ESI): 580.2 [M+1]⁺. Supercritical fluid chromatography (SFC): retention time = 3.38 min, UV = 214 nm. ¹HNMR (400 MHz, CD₃OD) δ 8.60 (s, 1H), 8.44 (d, *J=* 2.3 Hz, 1H), 7.71 (ddd, J=9.5, 8.7, 2.3 Hz, 1H), 7.66 (d, *J=* 2.2 Hz, 1H), 7.62 (dd, *J* = 8.4, 2.3 Hz, 1H), 7.48 *(d, J=* 8.5 Hz, 1H), 6.85 (s, 1H), 5.49 *(d, J=* 1.8 Hz, 2H), 3.62 (d, *J* = 11.1 Hz, 1H), 3.57 (d, *J* = 11.2 Hz, 1H), 2.20 (s, 3H), 2.13 (s, 3H), 1.51 (s, 3H).

### Compound 107-P2:

MS m/z (ESI): 580.2 [M+1]⁺. Supercritical fluid chromatography (SFC): retention time = 12.16 min, UV = 214 nm. ¹HNMR (400 MHz, CD₃OD) δ 8.60 (s, 1H), 8.44 (d, *J =* 2.3 Hz, 1H), 7.74 - 7.68 (m, 1H), 7.66 (d, *J =* 2.2 Hz, 1H), 7.62 (dd, *J =* 8.4, 2.3 Hz, 1H), 7.49 (d, *J =* 8.4 Hz, 1H), 6.86 (s, 1H), 5.49 (d, *J =* 1.8 Hz, 2H), 3.63 (d, *J =* 11.2 Hz, 1H), 3.58 (d, *J =* 11.2 Hz, 1H), 2.20 (s, 3H), 2.13 (s, 3H), 1.51 (s, 3H).

### Example 44 Synthesis of Compounds 108-2, 108-P1, and 108-P2

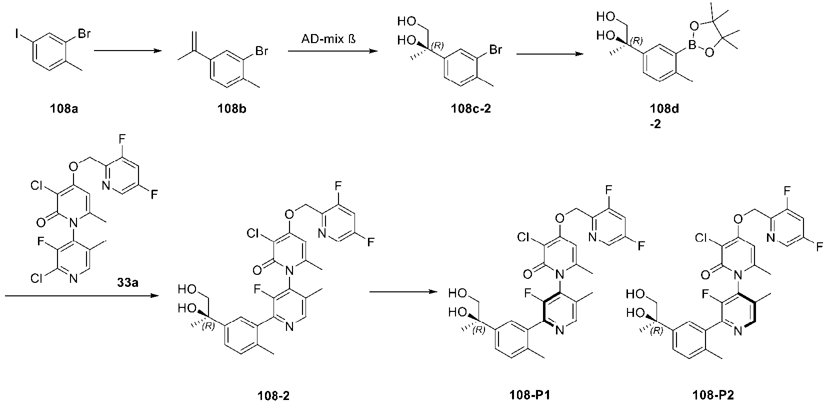

### Step 1: Synthesis of Compound 108b

1,1'-Bis(diphenylphosphino)ferrocene-palladium(II)dichloride dichloromethane complex (410 mg, 0.5 mmol) was added to a mixed solution of compound 108a (2.96 g, 10 mmol), isopropenylboronic acid pinacol ester (1.68 g, 10 mmol), and potassium carbonate (2.76 g, 20 mmol) in 1,4-dioxane and water (20 mL /4 mL). The reaction mixture was stirred at 80°C for 16 hours. After the reaction was completed, the reaction mixture was diluted with water (100 mL) and extracted with dichloromethane (60 mL × 3). The combined organic phases were dried over anhydrous sodium sulfate and filtered. The filtrate was concentrated under reduced pressure, and the residue was purified by silica gel column chromatography (petroleum ether/ethyl acetate = 1/0 to 20/1) to obtain compound 108b (1.4 g, yield: 53%). ¹H NMR (400 MHz, CDCl₃) δ 7.66 (d, *J* = 8.4 Hz, 1H), 7.31 (d, *J* = 2.4 Hz, 1H), 7.07 (dd, *J* = 8.4*,* 2.4 Hz, 1H), 5.23 (s, 1H), 4.90 (s, 1H), 2.04 (d, *J=* 1.0 Hz, 3H).

### Step 2: Synthesis of Compound 108c-2

AD-mix-β (5 g) was added to a mixed solution of compound 108b (0.5 g, 2.36 mmol) in tert-butanol and water (20 mL/20 mL). The reaction mixture was stirred at room temperature for 16 hours. After the reaction was completed, the reaction mixture was quenched with saturated sodium thiosulfate solution (40 mL) and extracted with ethyl acetate (50 mL × 5). The combined organic phases were washed with saturated brine (50 mL × 1), concentrated under reduced pressure, and the residue was purified by silica gel column chromatography (dichloromethane/methanol = 20/1) to obtain compound 108c-2 (0.5 g, yield: 86.2%). MS m/z (ESI): 245.1 [M+1]⁺.

### Step 3: Synthesis of Compound 108d-2

1,1'-Bis(diphenylphosphino)ferrocene-palladium(II)dichloride dichloromethane complex (0.16 g, 0.22 mmol) was added to a solution of compound 108c-2 (0.5 g, 2.04 mmol), bis(pinacolato)diboron (0.6 g, 2.46 mmol), and potassium acetate (0.44 g, 4.48 mmol) in 1,4-dioxane (6 mL). The reaction mixture was stirred at 90°C for 16 hours. After the reaction was completed, the reaction mixture was filtered, and the filtrate was concentrated under reduced pressure to obtain compound 108d-2 (500 mg, crude product), which was directly used in the next step. MS m/z (ESI): 292.9 [M+1]⁺.

### Step 4: Synthesis of Compounds 108-P1 and 108-P2

Tetrakis(triphenylphosphine)palladium (158 mg, 0.13 mmol) was added to a mixed solution of compound 108d-2 (400 mg, 1.36 mmol), compound 33a (117 mg, 0.27 mmol), and sodium carbonate (290 mg, 2.73 mmol) in 1,4-dioxane and water (15 mL/1.5 mL). The reaction mixture was stirred at 90°C overnight. After the reaction was completed, the resulting residue from the concentration of the reaction mixture under reduced pressure was purified by silica gel column chromatography (dichloromethane/methanol = 1/0 to 10/1) to obtain a crude product of compound 108-2 (100 mg). The crude product was then resolved by supercritical fluid chiral chromatography (equipment: SFC Thar prep 80, column: CHIRALPAK IC 250 mm × 20 mm, 5 µm, mobile phase: 40% MeOH/CO₂ (NH₄OH 0.2%), total flow rate: 40 g/min) to obtain compounds 108-P1 (41.5 mg, yield: 5.38%) and 108-P2 (32 mg, yield: 4.02%).

### Compound 108-P1:

MS m/z (ESI): 560.2 [M+1]⁺. Supercritical fluid chromatography (SFC): retention time = 3.5 min, UV = 220 nm. ¹H NMR (400 MHz) δ 8.59 (*d, J =* 0.7 Hz, 1H), 8.44 *(d, J=* 2.4 Hz, 1H), 7.71 (ddd, *J* = 9.6, 8.6, 2.4 Hz, 1H), 7.54 - 7.46 (m, 2H), 7.30 *(d, J=* 9.0 Hz, 1H), 6.86 *(d, J=* 0.4 Hz, 1H), 5.50 *(d, J=* 1.9 Hz, 2H), 3.60 (d, *J=* 6.5 Hz, 2H), 2.20 *(d, J=* 3.4 Hz, 6H), 2.13 (s, 3H), 1.51 (s, 3H).

### Compound 108-P2:

MS m/z (ESI): 560.2 [M+1]⁺. Supercritical fluid chromatography (SFC): retention time = 5.84 min, UV = 220 nm. ¹HNMR (400 MHz) δ 8.59 (d, *J =* 0.5 Hz, 1H), 8.45 (d, *J =* 2.4 Hz, 1H), 7.71 (dd, *J=* 2.3, 1.0 Hz, 1H), 7.53 - 7.49 (m, 2H), 7.30 *(d, J=* 7.7 Hz, 1H), 6.86 (d, *J=* 0.4 Hz, 1H), 5.50 *(d, J=* 1.9 Hz, 2H), 3.60 (q, *J=* 11.1 Hz, 2H), 2.20 *(d, J=* 3.3 Hz, 6H), 2.13 (s, 3H), 1.51 (s, 3H).

### Example 45 Synthesis of Compounds 109-2, 109-P1, and 109-P2

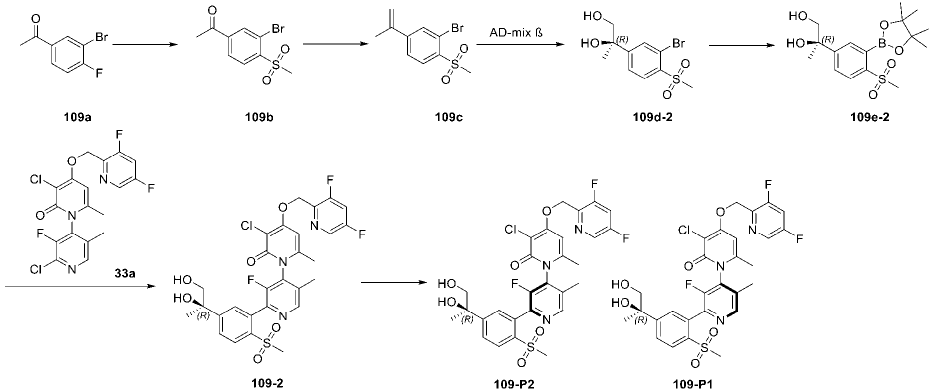

### Step 1: Synthesis of Compound 109b

Sodium methanesulfinate (2.82 g, 27.60 mmol) was added to a solution of compound 109a (3 g, 13.80 mmol) in dimethyl sulfoxide (50 mL), and the reaction mixture was stirred at 120°C for 16 hours. After the reaction was completed, the reaction mixture was diluted with water (100 mL) and extracted with ethyl acetate (100 mL × 3). The combined organic phases were dried over anhydrous sodium sulfate and filtered. The filtrate was concentrated under reduced pressure, and the residue was purified by silica gel column chromatography (petroleum ether/ethyl acetate = 1/0 to 20/1) to obtain compound 109b (1.3 g, yield: 30.43%). ¹HNMR (400 MHz, CDCl₃) δ 8.29 (dd, *J =* 4.9, 3.2 Hz, 2H), 8.03 (dd, *J =* 8.2, 1.6 Hz, 1H), 3.30 (s, 3H), 2.65 (s, 3H).

### Step 2: Synthesis of Compound 109c

Methyltriphenylphosphonium bromide (2 g, 5.62 mmol) was added to a solution of potassium tert-butoxide (0.63 g, 5.63 mmol) in tetrahydrofuran (30 mL), and the mixture was stirred at room temperature for 1 hour. Compound 109b (1.3 g, 4.69 mmol) was added, and the reaction mixture was stirred at room temperature for 16 hours. After the reaction was completed, the reaction mixture was diluted with water (100 mL) and extracted with ethyl acetate (60 mL × 3). The combined organic phases were dried over anhydrous sodium sulfate, filtered, and concentrated under reduced pressure. The residue was purified by silica gel column chromatography (petroleum ether/ethyl acetate = 1/0 to 20/1) to obtain compound 109c (1.2 g, yield: 83.67%). ¹HNMR (400 MHz, CDCl₃) δ 8.11 (*d, J =* 8.3 Hz, 1H), 7.80 (d, *J =* 1.8 Hz, 1H), 7.54 (dd, *J* = 8.3, 1.8 Hz, 1H), 5.49 (s, 1H), 5.31 - 5.20 (m, 1H), 3.26 (s, 3H), 2.14 (d, *J=* 1.2 Hz, 3H).

### Step 3: Synthesis of Compound 109d-2

AD-mix-β (6 g) was added to a mixed solution of compound 109c (600 mg, 2.18 mmol) in tert-butanol and water (10 mL/10 mL). The reaction mixture was stirred at room temperature for 16 hours. After the reaction was completed, the reaction mixture was quenched with saturated sodium thiosulfate solution (40 mL) and extracted with ethyl acetate (50 mL × 5). The combined organic phases were washed with saturated brine (50 mL × 1), concentrated under reduced pressure, and the residue was purified by silica gel column chromatography (dichloromethane/methanol = 20/1) to obtain compound 109d-2 (260 mg, yield: 34.71%). ¹HNMR (400 MHz, CDCl₃) δ 8.13 (d, *J =* 8.3 Hz, 1H), 7.90 (d, *J=* 1.8 Hz, 1H), 7.56 (dd, *J=* 8.3, 1.8 Hz, 1H), 3.79 (d, *J* = 11.0 Hz, 1H), 3.68 (d, *J* = 11.0 Hz, 1H), 3.27 (s, 3H), 1.52 (s, 3H).

### Step 4: Synthesis of Compound 109e-2

1,1'-Bis(diphenylphosphino)ferrocene-palladium(II)dichloride dichloromethane complex (30 mg, 0.04 mmol) was added to a solution of compound 109d-2 (230 mg, 0.74 mmol), bis(pinacolato)diboron (227 mg, 0.89 mmol), and potassium acetate (146 mg, 1.49 mmol) in 1,4-dioxane (6 mL). The reaction mixture was stirred at 80°C for 16 hours. After the reaction was completed, the reaction mixture was filtered, and the filtrate was concentrated under reduced pressure to obtain compound 109e-2 (200 mg, crude product), which was directly used in the next step. MS m/z (ESI): 356.9 [M+1]⁺.

### Step 5: Synthesis of Compounds 109-P1 and 109-P2

Tetrakis(triphenylphosphine)palladium (32 mg, 0.03 mmol) was added to a mixed solution of compound 109e-2 (200 mg, crude product), compound 33a (120 mg, 0.28 mmol), and sodium carbonate (119 mg, 1.22 mmol) in 1,4-dioxane and water (8 mL/2 mL). The reaction mixture was stirred at 80°C for 16 hours. After the reaction was completed, the reaction mixture was concentrated under reduced pressure, and the residue was subjected to silica gel column chromatography (dichloromethane/methanol = 1/0 to 20/1) to obtain compound 109-2 (50 mg). Compound 109-2 (50 mg) was then resolved by supercritical fluid chiral chromatography (equipment: SFC Thar prep 80, column: CHIRALPAK AD-H 250 mm × 20 mm, 5 µm, mobile phase: 40% MeOH/CO₂ (NH₄OH 0.2%), total flow rate: 40 g/min) to obtain compounds 109-P1 (retention time: 2.24 minutes, 15.1 mg, yield: 4.10%) and 109-P2 (retention time: 3.49 minutes, 17.5 mg, yield: 4.74%).

### Compound 109-P1:

MS m/z (ESI): 624.0 [M+1]⁺. Supercritical fluid chromatography (SFC): retention time = 2.24 min, UV = 214 nm. ¹HNMR (400 MHz, CD₃OD) δ 8.59 (s, 1H), 8.47 (d, *J =* 2.3 Hz, 1H), 8.14 (d, *J =* 8.4 Hz, 1H), 7.91 (dd, *J* = 8.4, 1.8 Hz, 1H), 7.79 - 7.71 (m, 2H), 6.87 (s, 1H), 5.52 (d, *J =* 1.5 Hz, 2H), 3.69 (d, *J =* 11.1 Hz, 1H), 3.64 (d, *J =* 11.2 Hz, 1H), 3.17 (s, 3H), 2.24 (s, 3H), 2.16 (s, 3H), 1.57 (s, 3H).

### Compound 109-P2:

MS m/z (ESI): 624.0 [M+1]⁺. Supercritical fluid chromatography (SFC): retention time = 3.49 min, UV = 214 nm. ¹HNMR (400 MHz, CD₃OD) δ 8.59 (s, 1H), 8.47 (d, *J =* 2.3 Hz, 1H), 8.15 (d, *J=* 8.4 Hz, 1H), 7.93 (dd, *J* = 8.4, 1.8 Hz, 1H), 7.78 - 7.71 (m, 2H), 6.87 (s, 1H), 5.52 (d, *J=* 1.6 Hz, 2H), 3.70 (d, *J=* 11.1 Hz, 1H), 3.64 (d, *J=* 11.2 Hz, 1H), 3.18 (s, 3H), 2.24 (s, 3H), 2.16 (s, 3H), 1.56 (s, 3H).

### Example 46 Synthesis of Compounds 110-1, 110-2, 110-P1, and 110-P2

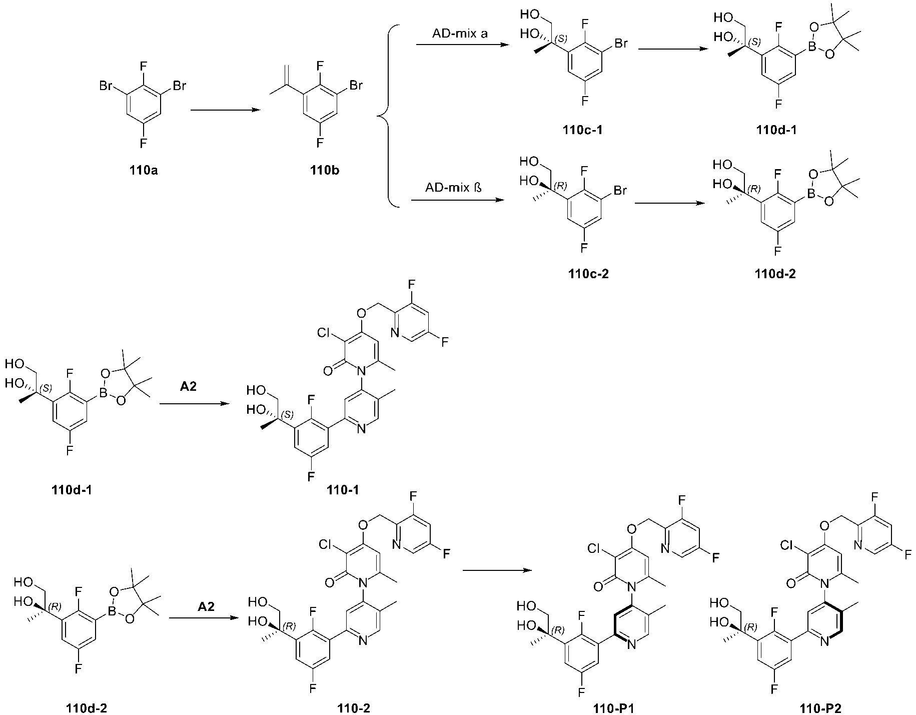

### Step 1: Synthesis of Compound 110b

1,1'-Bis(diphenylphosphino)ferrocene palladium(II)dichloride (148 mg, 0.20 mmol) was added to a mixed solution of compound 110a (1.1 g, 4.05 mmol), isopropenylboronic acid pinacol ester (680 mg, 4.05 mmol), and potassium acetate (1.1 g, 8.10 mmol) in 1,4-dioxane (10 mL). The reaction mixture was stirred at 80°C for 16 hours. After the reaction was completed, the reaction mixture was diluted with water (50 mL) and extracted with ethyl acetate (50 mL × 3). The combined organic phases were dried over anhydrous sodium sulfate, filtered, and concentrated under reduced pressure. The residue was purified by silica gel column chromatography (petroleum ether/ethyl acetate = 1/0 to 5/1) to obtain compound 110b (600 mg, yield: 63.5%). ¹HNMR (400 MHz, CDCl₃) δ 7.29 - 7.15 (m, 1H), 6.98 - 6.84 (m, 1H), 5.24 (t, *J* = 16.3 Hz, 2H), 2.11 (dd, *J* = 2.8, 1.2 Hz, 3H).

### Step 2: Synthesis of Compound 110c-1

AD-mix-α (7.5 g) was added to a mixed solution of compound 110b (600 mg, 2.57 mmol) in tert-butanol and water (15 mL/15 mL), and the reaction mixture was stirred at 40°C for 48 hours. After the reaction was completed, the reaction mixture was diluted with water (30 mL) and extracted with dichloromethane (30 mL × 5). The combined organic phases were dried over anhydrous sodium sulfate and filtered. The filtrate was concentrated under reduced pressure, and the residue was purified by silica gel column chromatography (petroleum ether/ethyl acetate = 1/0 to 1/2) to obtain compound 110c-1 (400 mg, yield: 58.5%). ¹H NMR (400 MHz, CDCl₃) δ 7.41-7.36 (m,1H), 7.26-7.20 (m,1H),, 3.99 (d, *J=* 11.0 Hz, 1H), 3.75 (d, *J =* 10.9 Hz, 1H), 1.57 (d, *J= 1.3* Hz, 3H).

### Step 3: Synthesis of Compound 110d-1

1,1'-Bis(diphenylphosphino)ferrocene palladium(II)dichloride (55 mg, 0.07 mmol) was added to a solution of compound 11c-1 (400 mg, 1.50 mmol), bis(pinacolato)diboron (570 mg, 2.25 mmol), and potassium acetate (294 mg, 3.00 mmol) in 1,4-dioxane (5 mL). The reaction mixture was stirred at 80°C for 16 hours. After the reaction was completed, the reaction mixture was filtered, and the filtrate was concentrated under reduced pressure to obtain compound 110d-1 (300 mg, crude product). MS m/z (ESI): 314.1 [M+1]⁺.

### Step 4: Synthesis of Compound 110-1

Tetrakis(triphenylphosphine)palladium (55 mg, 0.05 mmol) was added to a mixed solution of compound 110d-1 (300 mg, crude product), compound A2 (197 mg, 0.48 mmol), and sodium acetate (202 mg, 1.91 mmol) in 1,4-dioxane and water (2.5 mL/0.5 mL). The reaction mixture was stirred at 90°C for 16 hours. After the reaction was completed, the reaction mixture was diluted with water (30 mL) and extracted with ethyl acetate (50 mL × 3). The combined organic phases were dried over anhydrous sodium sulfate and filtered. The filtrate was concentrated under reduced pressure, and the residue was purified by a thin-layer chromatography silica gel plate (dichloromethane/methanol = 10/1) to obtain compound 110-1 (77.5 mg, yield: 14.3%). MS m/z (ESI): 564.0 [M+1]⁺. ¹H NMR (400 MHz, CD₃OD) δ 8.79 (s, 1H), 8.48 (d, *J* = 2.3 Hz, 1H), 7.75 (dt, *J* = 9.3, 2.7 Hz, 2H), 7.60 - 7.46 (m, 2H), 6.84 (s, 1H), 5.52 (d, *J* = 1.7 Hz, 2H), 3.88 (dd, *J* = 11.4, 6.3 Hz, 1H), 3.73 (d, *J* = 11.2 Hz, 1H), 2.17 (s, 3H), 2.10 (d, *J* = 3.8 Hz, 3H), 1.56 (d, *J=* 1.6 Hz, 3H).

### Step 5: Synthesis of Compound 110c-2

AD-mix-β (3.74 g) was added to a mixed solution of compound 110b (230 mg, 0.99 mmol) in tert-butanol and water (10 mL/10 mL). The reaction mixture was stirred at 40°C for 48 hours. After the reaction was completed, the reaction mixture was diluted with water (30 mL) and extracted with dichloromethane (30 mL × 5). The combined organic phases were dried over anhydrous sodium sulfate and filtered. The filtrate was concentrated under reduced pressure, and the residue was purified by silica gel column chromatography (petroleum ether/ethyl acetate = 1/0 to 1/2) to obtain compound 110c-2 (200 mg, yield: 75.9%). ¹H NMR (400 MHz, CDCl₃) δ 7.41-7.36 (m,1H), 7.26-7.20 (m,1H), 3.99 (d, *J* = 11.0 Hz, 1H), 3.75 (d, *J=* 10.9 Hz, 1H), 1.57 (d, *J= 1.3* Hz, 3H).

### Step 6: Synthesis of Compound 110d-2

1,1'-Bis(diphenylphosphino)ferrocene palladium(II)dichloride (27 mg, 0.03 mmol) was added to a solution of compound 11c-2 (200 mg, 0.75 mmol), bis(pinacolato)diboron (285 mg, 1.12 mmol), and potassium acetate (147 mg, 1.50 mmol) in 1,4-dioxane (5 mL). The reaction mixture was stirred at 80°C for 16 hours. After the reaction was completed, the reaction mixture was filtered, and the filtrate was concentrated under reduced pressure to obtain compound 110d-2 (200 mg, crude product). MS m/z (ESI): 314.1 [M+1]⁺.

### Step 7: Synthesis of compounds 110-P1 and 110-P2

Tetrakis(triphenylphosphine)palladium (37 mg, 0.03 mmol) was added to a mixed solution of compound 110d-2 (200 mg, crude product), compound A2 (131 mg, 0.32 mmol), and sodium acetate (135 mg, 1.27 mmol) in 1,4-dioxane and water (2.5 mL/0.5 mL). The reaction mixture was stirred at 90°C for 16 hours. After the reaction was completed, the reaction mixture was diluted with water (30 mL) and extracted with ethyl acetate (50 mL × 3). The combined organic phases were dried over anhydrous sodium sulfate and filtered. The filtrate was concentrated under reduced pressure, and the residue was purified by silica gel column chromatography (dichloromethane/methanol = 1/0 to 10/1) to obtain compound 110-2 (50 mg). Compound 110-2 (50 mg) was then resolved by supercritical fluid chiral chromatography (equipment: SFC Thar prep 80, column: CHIRALPAK IC 250 mm × 20 mm, 5 µm, mobile phase: 40% MeOH/CO₂ (NH₄OH 0.2%), total flow rate: 40 g/min) to obtain compounds 110-P1 (18.7 mg, yield: 5.2%) and 110-P2 (24.8 mg, yield: 6.9%).

### Compound 110-P1:

MS m/z (ESI): 564.0 [M+1]⁺. Supercritical fluid chromatography (SFC): retention time = 5.3 min, UV = 214 nm. ¹H NMR (400 MHz, CD₃OD) δ 8.77 (s, 1H), 8.46 (d, *J =* 2.4 Hz, 1H), 7.78 - 7.68 (m, 2H), 7.62 - 7.48 (m, 2H), 6.82 (s, 1H), 5.50 (d, *J* = 1.9 Hz, 2H), 3.86 (dd, *J=* 11.3, 1.3 Hz, 1H), 3.72 (dd, *J=* 11.3, 1.2 Hz, 1H), 2.15 (s, 3H), 2.05 (s, 3H), 1.55 (d, *J* = 1.3 Hz, 3H).

### Compound 110-P2:

MS m/z (ESI): 564.0 [M+1]⁺. Supercritical fluid chromatography (SFC): retention time = 10.8 min, UV = 214 nm. ¹H NMR (400 MHz, CD₃OD) δ 8.77 (s, 1H), 8.46 (d, *J =* 2.3 Hz, 1H), 7.82 - 7.65 (m, 2H), 7.59 - 7.43 (m, 2H), 6.82 (s, 1H), 5.50 (d, *J* = 1.8 Hz, 2H), 3.87 (dd, *J=* 11.4, 1.2 Hz, 1H), 3.71 (dd, *J=* 11.4, 1.0 Hz, 1H), 2.15 (s, 3H), 2.09 (s, 3H), 1.54 (d, *J* = 1.1 Hz, 3H).

### Example 47 Synthesis of Compounds 111, 111-P1, and 111-P2

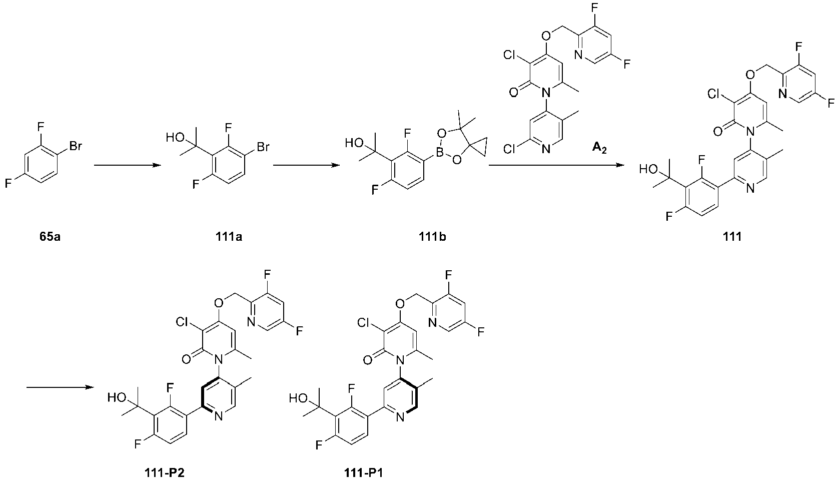

### Step 1: Synthesis of Compound 111a

At -78°C, lithium diisopropylamide (10 mL, 20 mmol) was added to a solution of compound 65a (2 g, 10 mmol) in tetrahydrofuran (50 mL), and the mixture was stirred at -78°C for 1 hour. Acetone (5 g, 86 mmol) was added dropwise, and the reaction mixture was stirred at -78°C for 30 minutes, then slowly warmed to -20°C and stirred at -20°C for 2 hours. After the reaction was completed, the reaction mixture was quenched with saturated ammonium chloride solution (50 mL) and extracted with ethyl acetate (50 mL × 3). The combined organic phases were dried over anhydrous sodium sulfate and filtered. The filtrate was concentrated under reduced pressure, and the residue was purified by silica gel column chromatography (petroleum ether/ethyl acetate = 1/0 to 3/1) to obtain compound 111a (1.3 g, yield: 50%). ¹H NMR (400 MHz, CDCl₃) δ 7.43-7.39 (m, 1H), 6.89 - 6.64 (m, 1H), 3.59 - 2.50 (m, 1H), 1.73 - 1.67 (m, 6H).

### Step 2: Synthesis of Compound 111b

1,1'-Bis(diphenylphosphino)ferrocene palladium(II)dichloride (190 mg, 0.27 mmol) was added to a solution of compound 11a (1 g, 4 mmol), bis(pinacolato)diboron (1.12 g, 4.4 mmol), and potassium acetate (532 mg, 5.4 mmol) in 1,4-dioxane (30 mL). The reaction mixture was stirred at 90°C for 12 hours. After the reaction was completed, the reaction mixture was filtered, and the filtrate was concentrated under reduced pressure to obtain compound 111b (500 mg, crude product), which was directly used in the next step.

### Step 3: Synthesis of compounds 111-P1 and 111-P2

Tetrakis(triphenylphosphine)palladium (97 mg, 0.08 mmol) was added to a mixed solution of compound 111b (500 mg), compound A2 (346 mg, 0.84 mmol), and sodium carbonate (178 mg, 1.68 mmol) in 1,4-dioxane and water (15 mL/1.5 mL). The reaction mixture was stirred at 90°C for 16 hours. After the reaction was completed, the reaction mixture was concentrated under reduced pressure, and the residue was subjected to silica gel column chromatography (dichloromethane/methanol = 1/0 to 10/1) to obtain solid compound 111 (100 mg). Compound 111 (100 mg) was resolved by supercritical fluid chiral chromatography (equipment: SFC Thar prep 80, column: chiralpak-OJ, mobile phase: CO₂-IPA(DEA), total flow rate: 40 g/min) to obtain compounds 111-P1 (12.5 mg, yield: 2.27%) and 111-P2 (20.4 mg, yield: 3.71%).

### Compound 111-P1:

MS m/z (ESI): 548.2 [M+1]⁺. Supercritical fluid chromatography (SFC): retention time = 2.16 min, UV = 214 nm. ¹HNMR (400 MHz,CD₃OD ) δ 8.73 (s, 1H), 8.44 (d, *J=* 2.2 Hz, 1H), 7.78 - 7.69 (m, 2H), 7.63 (d, *J =* 2.0 Hz, 1H), 7.08 - 7.03 (m, 1H), 6.80 (s, 1H), 5.48 (d, *J=* 1.5 Hz, 2H), 2.12 (s, 3H), 2.07 (s, 3H), 1.70 (s, 6H).

### Compound 111-P2:

MS m/z (ESI): 548.2 [M+1]⁺. Supercritical fluid chromatography (SFC): retention time = 4.36 min, UV = 214 nm. ¹HNMR (400 MHz, CD₃OD) δ 8.74 (s, 1H), 8.45 (d, *J=* 2.4 Hz, 1H), 7.79 - 7.69 (m, 2H), 7.63 (d, *J= 2.3* Hz, 1H), 7.06 (ddd, *J =* 11.8, 8.7, 1.5 Hz, 1H), 6.81 (s, 1H), 5.49 (d, *J* = 2.0 Hz, 2H), 2.13 (s, 3H), 2.08 (s, 3H), 1.71 (s, 6H).

### Example 48 Synthesis of Compounds 112-2, 112-P1, and 112-P2

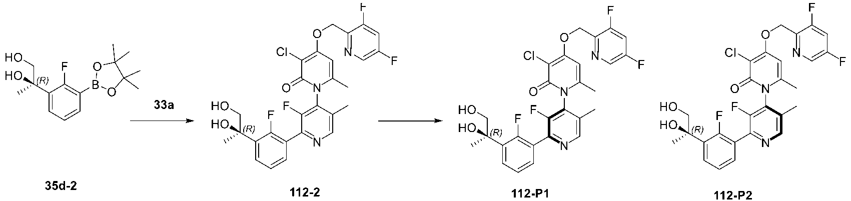

Tetrakis(triphenylphosphine)palladium (0.2 g, 0.17 mmol) was added to a mixed solution of compound 35d-2 (0.5 g, 1.68 mmol), compound 33a (0.3 g, 0.84 mmol), and sodium carbonate (0.36 g, 3.38 mmol) in 1,4-dioxane and water (15 mL/1.5 mL). The reaction mixture was stirred at 90°C for 16 hours. After the reaction was completed, the reaction mixture was filtered, and the filtrate was concentrated under reduced pressure. The residue was purified by silica gel column chromatography (dichloromethane/methanol = 1/0 to 10/1) to obtain compound 112-2 (100 mg). Compound 112-2 (100 mg) was then resolved by supercritical fluid chiral chromatography (equipment: SFC Thar prep 80, column: CHIRALPAK IC 250mm × 20 mm, 5 µm mobile phase: 40% MeOH (NH₄OH 0.2%): 60% CO₂, total flow rate: 40 g/min) to obtain compounds 112-P1 (41 mg, yield: 4.31%) and 112-P2 (44.4 mg, yield: 4.67%).

### Compound 112-P1:

MS m/z (ESI): 564.1 [M+1]⁺. Supercritical fluid chromatography (SFC): retention time = 4.6 min, UV = 214 nm. ¹HNMR (400 MHz, CD₃OD) δ 8.62 (s, 1H), 8.45 (d, *J* = 2.3 Hz, 1H), 7.85 (td, *J* = 7.8, 1.7 Hz, 1H), 7.72 (td, *J* = 9.6, 2.3 Hz, 1H), 7.55 - 7.49 (m, 1H), 7.34 (t, *J* = 7.7 Hz, 1H), 6.87 (s, 1H), 5.51 (d, *J* = 1.7 Hz, 2H), 3.75 (d, *J=* 10.6 Hz, 2H), 2.20 (s, 3H), 2.13 (s, 3H), 1.56 (s, 3H).

### Compound 112-P2:

MS m/z (ESI): 564.1 [M+1]⁺. Supercritical fluid chromatography (SFC): retention time = 7.24 min, UV = 214 nm. ¹HNMR (400 MHz, CD₃OD) δ 8.62 (s, 1H), 8.45 (d, *J* = 2.3 Hz, 1H), 7.85 (td, *J* = 7.8, 1.7 Hz, 1H), 7.72 (td, *J* = 9.6, 2.3 Hz, 1H), 7.58 - 7.45 (m, 1H), 7.34 (t, *J* = 7.7 Hz, 1H), 6.87 (s, 1H), 5.51 (d, *J* = 1.7 Hz, 2H), 3.76 (q, *J =* 11.3 Hz, 2H), 2.20 (s, 3H), 2.13 (s, 3H), 1.55 (s, 3H).

### Example 49 Synthesis of Compounds 113-P1 and 113-P2

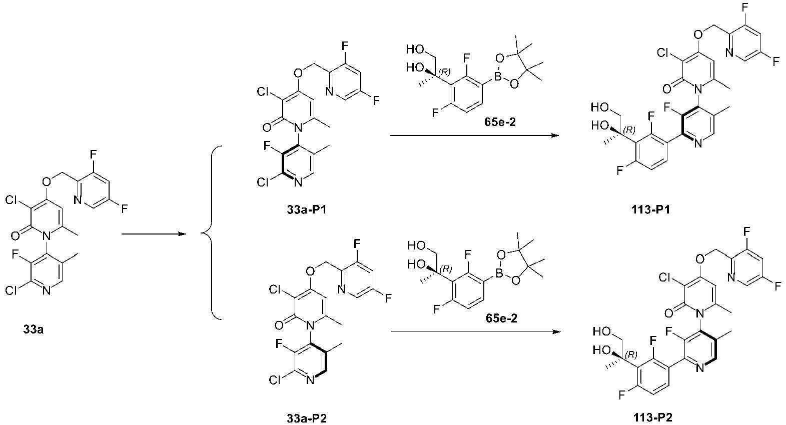

Compound 33a (300 mg) was resolved by supercritical fluid chiral chromatography (equipment: SFC Thar prep 80, column: CHIRALPAK IC 250 mm × 20 mm, 5 µm; mobile phase: 40% MeOH (NH₄OH 0.2%): 60% CO₂; total flow rate: 40 g/min) to obtain compounds 33a-P1 (120 mg) and 33a-P2 (130 mg).

Tetrakis(triphenylphosphine)palladium (36 mg, 0.03 mmol) was added to a mixed solution of compound 65e-2 (100 mg, 0.32 mmol), compound 33a-P1 (109 mg, 0.25 mmol), and sodium carbonate (67 mg, 0.64 mmol) in 1,4-dioxane and water (10 mL/l mL). The reaction mixture was stirred at 90°C overnight. After the reaction was completed, the reaction mixture was concentrated under reduced pressure. The residue was purified by silica gel column chromatography (dichloromethane/methanol = 1/0 to 4/1) to obtain a crude product. The crude product was further purified by preparative HPLC (chromatographic column: Gemini-C18; 150 × 21.2 mm, 5 µm; mobile phase: acetonitrile-water (0.1% formic acid); gradient: 30-40%; column temperature: 25°C; flow rate: 20 mL/min; wavelength: 214 nm; column pressure: 80 bar) to obtain compound 113-P1 (35 mg, yield: 17.9%).

MS m/z (ESI): 582.2 [M+1]⁺. Supercritical fluid chromatography (SFC): retention time = 6.21 min, UV = 214 nm. ¹H NMR (400 MHz, CD₃OD) δ 8.60 (s, 1H), 8.43 (d, *J =* 2.3 Hz, 1H), 7.76 - 7.65 (m, 1H), 7.54 (d, *J =* 6.0 Hz, 1H), 7.09 (dd, *J =* 11.3, 9.2 Hz, 1H), 6.84 (s, 1H), 5.49 (d, *J =* 1.4 Hz, 2H), 3.83 (d, *J= 11.1* Hz, 1H), 3.74 (d, *J=* 11.1 Hz, 1H), 2.18 (s, 3H), 2.10 (s, 3H), 1.65 (s, 3H).

Tetrakis(triphenylphosphine)palladium (36 mg, 0.03 mmol) was added to a mixed solution of compound 65e-2 (102 mg, 0.32 mmol), compound 33a-P2 (110 mg, 0.25 mmol), and sodium carbonate (67 mg, 0.64 mmol) in 1,4-dioxane and water (10 mL/l mL). The reaction mixture was stirred at 90°C overnight. After the reaction was completed, the reaction mixture was concentrated under reduced pressure. The residue was purified by silica gel column chromatography (dichloromethane/methanol = 1/0 to 4/1) to obtain a crude product. The crude product was further purified by preparative HPLC (chromatographic column: Gemini-C18; 150 × 21.2 mm, 5 µm; mobile phase: acetonitrile-water (0.1% formic acid); gradient: 30-40%; column temperature: 25°C; flow rate: 20 mL/min; wavelength: 214 nm; column pressure: 80 bar) to obtain compound 113-P2 (33 mg, yield: 17.8%).

MS m/z (ESI): 582.2 [M+1]⁺. Supercritical fluid chromatography (SFC): retention time = 7.21 min, UV = 214 nm. ¹H NMR (400 MHz, CD₃OD) δ 8.60 (s, 1H), 8.43 (d, *J =* 2.3 Hz, 1H), 7.76 - 7.65 (m, 1H), 7.54 (d, *J =* 6.0 Hz, 1H), 7.09 (dd, *J =* 11.3, 9.2 Hz, 1H), 6.85 (s, 1H), 5.49 (d, *J =* 1.4 Hz, 2H), 3.83 (d, *J= 11.1* Hz, 1H), 3.74 (d, *J= 11.1* Hz, 1H), 2.18 (s, 3H), 2.10 (s, 3H), 1.65 (s, 3H).

### Example 50 Synthesis of Compound 114

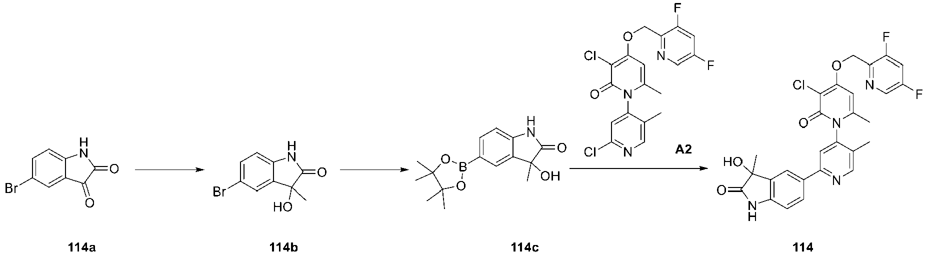

### Step 1: Synthesis of Compound 114b

Trimethylaluminum (4.4 mL, 8.84 mmol) was added to a solution of compound 11a (1 g, 4.42 mmol) in tetrahydrofuran (10 mL), and the reaction mixture was stirred at 70°C for 3 hours. After the reaction was completed, the reaction mixture was quenched with saturated ammonium chloride solution (20 mL), diluted with water (20 mL), and extracted with ethyl acetate (50 mL × 3). The combined organic phases were dried over anhydrous sodium sulfate and filtered. The filtrate was concentrated under reduced pressure, and the residue was purified by silica gel column chromatography (petroleum ether/ethyl acetate = 1/0 to 1/2) to obtain compound 114b (600 mg, yield: 56.1%). MS m/z (ESI): 223.9 [M-17]⁺.

### Step 2: Synthesis of Compound 114c

1,1'-Bis(diphenylphosphino)ferrocene palladium(II)dichloride (101 mg, 0.12 mmol) was added to a solution of compound 114b (600 mg, 2.48 mmol), bis(pinacolato)diboron (944 mg, 3.72 mmol), and potassium acetate (486 mg, 4.96 mmol) in 1,4-dioxane (5 mL). The reaction mixture was stirred at 90°C for 16 hours. After the reaction was completed, the reaction mixture was diluted with water (20 mL) and extracted with ethyl acetate (50 mL × 3). The combined organic phases were dried over anhydrous sodium sulfate and filtered. The filtrate was concentrated under reduced pressure, and the residue was purified by silica gel column chromatography (petroleum ether/ethyl acetate = 1/0 to 1/2) to obtain compound 114c (300 mg, yield: 41.8%). MS m/z (ESI): 272.1 [M-17]⁺.

### Step 3: Synthesis of Compound 114

Tetrakis(triphenylphosphine)palladium (60 mg, 0.05 mmol) was added to a mixed solution of compound 114c (300 mg, 1.04 mmol), compound A2 (214 mg, 0.52 mmol), and sodium acetate (220 mg, 2.08 mmol) in 1,4-dioxane and water (3 mL/0.5 mL). The reaction mixture was stirred at 90°C for 16 hours. After the reaction was completed, the reaction mixture was diluted with water (30 mL) and extracted with ethyl acetate (50 mL × 3). The combined organic phases were dried over anhydrous sodium sulfate and filtered. The filtrate was concentrated under reduced pressure, and the residue was purified by silica gel column chromatography (dichloromethane/methanol = 1/0 to 10/1) to obtain compound 114 (140 mg, yield: 25.2%). MS m/z (ESI): 539.1 [M+1]⁺. ¹H NMR (400 MHz, DMSO-d6) δ 10.44 (s, 1H), 8.69 (s, 1H), 8.61 (d, *J =* 2.3 Hz, 1H), 8.15 - 8.09 (m, 2H), 8.04 (d, *J* = 8.2 Hz, 1H), 7.96 (s, 1H), 6.90 (d, *J=* 8.2 Hz, 1H), 6.81 (s, 1H), 5.96 (d, *J =* 1.5 Hz, 1H), 5.49 (s, 2H), 2.02 (s, 3H), 1.99 (s, 3H), 1.42 (s, 3H).

### Example 51 Synthesis of Compounds 115, 115-P1, 115-P2, 115-P3, and 115-P4

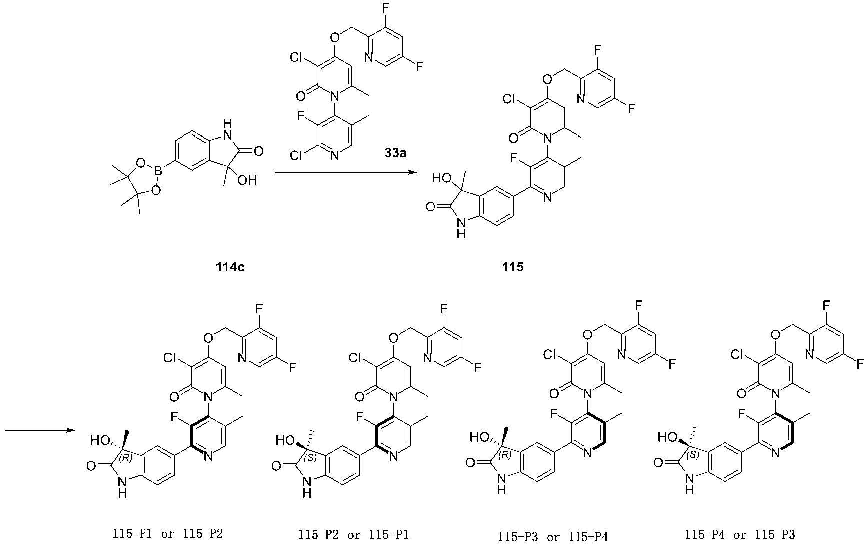

Tetrakis(triphenylphosphine)palladium (40 mg, 0.04 mmol) was added to a mixed solution of compound 114c (200 mg, 0.69 mmol), compound 33a (238 mg, 0.55 mmol), and sodium acetate (147 mg, 1.38 mmol) in 1,4-dioxane and water (2.5 mL/0.5 mL). The reaction mixture was stirred at 90°C for 16 hours. After the reaction was completed, the reaction mixture was diluted with water (30 mL) and extracted with ethyl acetate (50 mL × 3). The combined organic phases were dried over anhydrous sodium sulfate and filtered. The filtrate was concentrated under reduced pressure, and the residue was purified by silica gel column chromatography (dichloromethane/methanol = 1/0 to 10/1) to obtain compound 115 (120 mg, yield: 31.2%). Compound 115 (120 mg) was then resolved by supercritical fluid chiral chromatography (equipment: SFC Thar prep 80, column: CHIRALPAK IC 250 mm × 20 mm, 5 µm, mobile phase: 40% MeOH (NH₄OH 0.2%): 60% CO₂, total flow rate: 40 g/min) to obtain compound 115-P1 (8.3 mg, yield: 2.2%), 115-P2 (9.3 mg, yield: 2.4%), 115-P3 (9.0 mg, yield: 2.3%), and 115-P4 (14.2 mg, yield: 3.7%).

### Compound 115-P1:

MS m/z (ESI): 557.2 [M+1]⁺. Supercritical fluid chromatography (SFC): retention time = 8.41 min, UV = 214 nm. ¹H NMR (400 MHz, CD₃OD) δ 8.57 (s, 1H), 8.46 (d, *J =* 2.3 Hz, 1H), 7.95 (s, 1H), 7.86 (d, *J =* 8.2 Hz, 1H), 7.79 - 7.63 (m, 1H), 7.02 (d, *J* = 8.2 Hz, 1H), 6.86 (s, 1H), 5.51 (d, *J* = 1.6 Hz, 2H), 2.16 (s, 3H), 2.12 (s, 3H), 1.55 (s, 3H.

### Compound 115-P2:

MS m/z (ESI): 557.0 [M+1]⁺. Supercritical fluid chromatography (SFC): retention time = 12.85 min, UV = 214 nm. ¹H NMR (400 MHz, CD₃OD) δ 8.57 (s, 1H), 8.45 (d, *J =* 2.3 Hz, 1H), 7.95 (s, 1H), 7.86 (d, *J =* 8.3 Hz, 1H), 7.77 - 7.64 (m, 1H), 7.01 (d, *J* = 8.2 Hz, 1H), 6.86 (s, 1H), 5.51 (d, *J* = 1.7 Hz, 2H), 2.16 (s, 3H), 2.12 (s, 3H), 1.54 (s, 3H).

### Compound 115-P3:

MS m/z (ESI): 557.0 [M+1]⁺. Supercritical fluid chromatography (SFC): retention time = 20.88 min, UV = 214 nm. ¹H NMR (400 MHz, CD₃OD) δ 8.57 (s, 1H), 8.45 (d, *J* = 2.3 Hz, 1H), 7.95 (s, 1H), 7.86 (d, *J* = 8.2 Hz, 1H), 7.72 (d, *J* = 1.4 Hz, 1H), 7.01 (d, *J* = 8.2 Hz, 1H), 6.86 (s, 1H), 5.51 (d, *J* = 1.6 Hz, 2H), 2.15 (s, 3H), 2.12 (s, 3H), 1.55 (s, 3H).

### Compound 115-P4:

MS m/z (ESI): 557.0 [M+1]⁺. Supercritical fluid chromatography (SFC): retention time = 34.49 min, UV = 214 nm. ¹H NMR (400 MHz, CD₃OD)δ 8.57 (s, 1H), 8.45 (d, *J* = 2.3 Hz, 1H), 7.95 (s, 1H), 7.86 (d, *J* = 8.0 Hz, 1H), 7.71 (d, *J* = 9.3 Hz, 1H), 7.01 (d, *J* = 8.2 Hz, 1H), 6.86 (s, 1H), 5.50 (s, 2H), 2.15 (s, 3H), 2.12 (s, 3H), 1.54 (s, 3H).

### Example 52 Synthesis of Compounds 116, 116-P1, 116-P2, 116-P3, and 116-P4

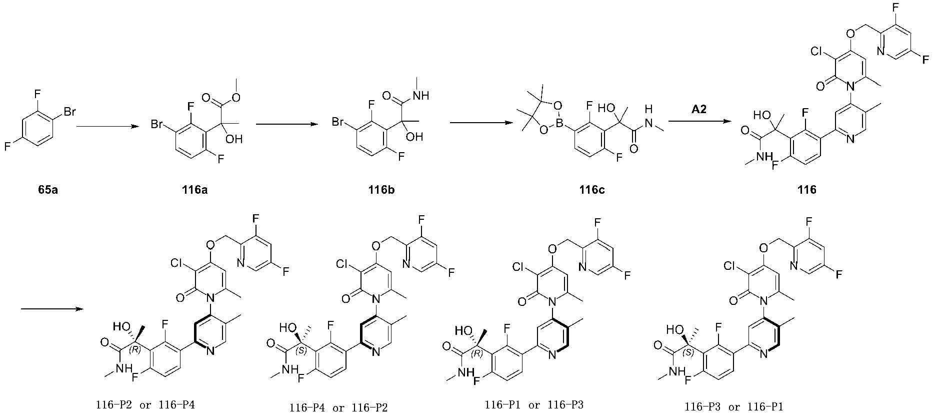

### Step 1: Synthesis of Compound 116a

At -50°C, lithium diisopropylamide (8.5 mL, 17 mmol) was added dropwise to a solution of compound 65a (3 g, 15.5 mmol) in tetrahydrofuran (50 mL), and the mixture was stirred at -50°C for 1 hour. Methyl pyruvate (1.74 g, 17 mmol) was added dropwise, and the reaction mixture was stirred at room temperature for 2 hours. After the reaction was completed, the reaction mixture was quenched with saturated ammonium chloride solution (80 mL) and extracted with ethyl acetate (100 mL × 3). The combined organic phases were dried over anhydrous sodium sulfate and filtered. The filtrate was concentrated under reduced pressure. The residue was purified by silica gel column chromatography (petroleum ether/ethyl acetate = 1/0 to 3/1) to obtain compound 116a (1.9 g, yield: 37.2%). ¹HNMR (400 MHz, CDCl₃) δ 7.49 (ddd, *J* = 8.9, 7.6, 5.6 Hz, 1H), 6.82 (ddd, *J* = 10.8, 9.0, 1.8 Hz, 1H), 3.82 (s, 3H), 1.92 (s, 3H).

### Step 2: Synthesis of Compound 116b

Compound 116a (1.9 g, 6.4 mmol) was added to a solution of methylamine in ethanol (20 mL), and the reaction mixture was stirred at 80°C for 16 hours. After the reaction was completed, the reaction mixture was concentrated under reduced pressure. The residue was purified by silica gel column chromatography (petroleum ether/ethyl acetate = 1/0 to 10/1) to obtain compound 116b (800 mg, yield: 33.8%). MS m/z (ESI): 296.0 [M+1]⁺.

### Step 3: Synthesis of Compound 116c

1,1'-Bis(diphenylphosphino)ferrocene-palladium(II)dichloride dichloromethane complex (200 mg, 0.27 mmol) was added to a solution of compound 116b (800 mg, 0.27 mmol), bis(pinacolato)diboron (1.07 g, 4.05 mmol), and potassium acetate (530 mg, 5.4 mmol) in 1,4-dioxane (15 mL). The reaction mixture was stirred at 90°C for 16 hours. After the reaction was completed, the reaction mixture was filtered, and the filtrate was concentrated under reduced pressure to obtain compound 116c (500 mg, 44.4%). MS m/z (ESI): 342.0 [M+1]⁺.

### Step 4: Synthesis of compounds 116-P1, 116-P2, 116-P3, and 116-P4

Tetrakis(triphenylphosphine)palladium (170 mg, 0.14 mmol) was added to a mixed solution of compound 116c (500 mg, 1.46 mmol), compound A2 (120 mg, 0.3 mmol), and sodium carbonate (310 mg, 2.9 mmol) in 1,4-dioxane and water (10 mL/l mL). The reaction mixture was stirred at 90°C overnight. After the reaction was completed, the reaction mixture was concentrated under reduced pressure to remove the solvent, and the residue was purified by silica gel column chromatography (dichloromethane/methanol = 1/0 to 20/1) to obtain compound 116 (110 mg, yield: 11.31%). Compound 116 (100 mg) was then resolved by supercritical fluid chiral chromatography (equipment: SFC Thar prep 80, column: CHIRALPAK AD-H 250 mm × 20 mm, 5 µm, mobile phase: 40% MeOH (NH₄OH 0.2%): 60% CO₂, total flow rate: 40 g/min) to obtain compounds 116-P1 (14.2 mg, yield: 1.7%), 116-P2 (22.9 mg, yield: 2.6%), 116-P3 (19.4 mg, yield: 2.2%), and 116-P4 (20.1 mg, yield: 2.27%).

### Compound 116-P1:

MS m/z (ESI): 591.2 [M+1]⁺. Supercritical fluid chromatography (SFC): retention time = 8.14 min, UV = 214 nm. ¹H NMR (400 MHz, CD₃OD) δ 8.66 (s, 1H), 8.36 (d, *J* = 2.1 Hz, 1H), 7.78 (dd, *J =* 14.7, 8.5 Hz, 1H), 7.67 - 7.61 (m, 1H), 7.56 (s, 1H), 7.06 - 6.95 (m, 1H), 6.72 (s, 1H), 5.40 (s, 2H), 2.71 (s, 3H), 2.04 (s, 3H), 1.97 (s, 3H), 1.82 (s, 3H).

### Compound 116-P2:

MS m/z (ESI): 591.2 [M+1]⁺. Supercritical fluid chromatography (SFC): retention time = 8.36 min, UV = 214 nm. ¹H NMR (400 MHz, CD₃OD) δ 8.67 (s, 1H), 8.38 (d, *J=* 2.3 Hz, 1H), 7.89 - 7.75 (m, 1H), 7.66 (dd, *J=* 12.4, 5.7 Hz, 1H), 7.57 (s, 1H), 7.02 (t, *J=* 10.0 Hz, 1H), 6.74 (s, 1H), 5.43 (s, 2H), 2.73 (s, 3H), 2.06 (s, 3H), 1.99 (s, 3H), 1.84 (s, 3H).

### Compound 116-P3:

MS m/z (ESI): 591.2 [M+1]⁺. Supercritical fluid chromatography (SFC): retention time = 8.73 min, UV = 214 nm. ¹H NMR (400 MHz, CD₃OD) δ 8.65 (s, 1H), 8.36 (d, *J =* 2.3 Hz, 1H), 7.78 (td, *J =* 8.6, 6.1 Hz, 1H), 7.64 (td, *J* = 9.6, 2.3 Hz, 1H), 7.55 (d, *J* = 2.0 Hz, 1H), 7.07 - 6.95 (m, 1H), 6.72 (s, 1H), 5.41 (d, *J=* 1.7 Hz, 2H), 2.71 (s, 3H), 2.04 (s, 3H), 1.97 (s, 3H), 1.81 (dd, *J* = 3.8, 2.9 Hz, 3H).

### Compound 116-P4:

MS m/z (ESI): 591.2 [M+1]⁺. Supercritical fluid chromatography (SFC): retention time = 9.22 min, UV = 214 nm. ¹H NMR (400 MHz, CD₃OD) δ 8.66 (s, 1H), 8.36 (d, *J =* 2.3 Hz, 1H), 7.78 (td, *J =* 8.6, 6.1 Hz, 1H), 7.64 (td, *J* = 9.6, 2.3 Hz, 1H), 7.57 (d, *J* = 1.9 Hz, 1H), 7.06 - 6.96 (m, 1H), 6.72 (s, 1H), 5.41 (d, *J=* 1.7 Hz, 2H), 2.71 (s, 3H), 2.05 (s, 3H), 1.98 (s, 3H), 1.82 *(t, J= 3.4* Hz, 3H).

### Example 53 Synthesis of Compounds 117, 117-P1, and 117-P2

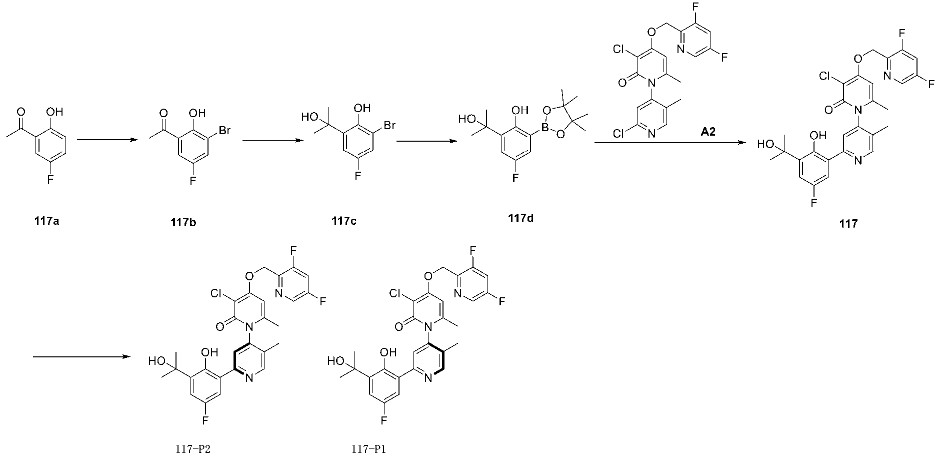

### Step 1: Synthesis of Compound 117b

N-Bromosuccinimide (2 g, 13 mmol) was added to a solution of compound 117a (2.31 g, 13 mmol) in *N,N-*dimethylformamide (20 mL), and the reaction mixture was stirred at room temperature for 16 hours. After the reaction was completed, the reaction mixture was diluted with water (100 mL) and extracted with ethyl acetate (50 mL × 3). The combined organic phases were dried over anhydrous sodium sulfate and filtered. The filtrate was concentrated under reduced pressure, and the residue was purified by silica gel column chromatography (petroleum ether/ethyl acetate = 1/0 to 5/1) to obtain compound 117b (1 g, yield: 33.1%). ¹HNMR (400 MHz, CDCl₃) δ 12.68 (s, 1H), 7.55 (dd, *J* = 7.5, 3.0 Hz, 1H), 7.43 (dd, J = 8.3, 3.0 Hz, 1H), 2.64 (d, J = 12.3 Hz, 3H).

### Step 2: Synthesis of Compound 117c

Methylmagnesium bromide (2 mL, 6.14 mmol) was added to a solution of compound 117b (1 g, 4.3 mmol) in tetrahydrofuran (30 mL), and the reaction mixture was stirred at 0°C for 3 hours. After the reaction was completed, the reaction mixture was quenched with saturated ammonium chloride solution (20 mL), diluted with water (20 mL), and extracted with ethyl acetate (50 mL × 3). The combined organic phases were dried over anhydrous sodium sulfate and filtered. The filtrate was concentrated under reduced pressure and then subjected to silica gel column chromatography (petroleum ether/ethyl acetate = 1/0 to 5/1) to obtain compound 117c (500 mg, yield: 46.70%). ¹HNMR (400 MHz, CD₃OD) δ 7.15 (dd, J = 7.7, 3.0 Hz, 1H), 6.95 (dd, J = 9.8, 3.0 Hz, 1H), 1.57 (s, 6H).

### Step 3: Synthesis of Compound 117d

1,1'-Bis(diphenylphosphino)ferrocene palladium(II)dichloride (87 mg, 0.12 mmol) was added to a solution of compound 117c (300 mg, 1.21 mmol), bis(pinacolato)diboron (336 mg, 1.32 mmol), and potassium acetate (236 mg, 2.41 mmol) in 1,4-dioxane (15 mL). The reaction mixture was stirred at 80°C for 16 hours. After the reaction was completed, the reaction mixture was filtered, and the filtrate was concentrated under reduced pressure to obtain compound 117d (500 mg, crude product). MS m/z (ESI): 297.1 [M+1]⁺.

### Step 4: Synthesis of Compounds 117-P1 and 117-P2

Compound 117d (500 mg, 1.69 mmol), compound A2 (348 mg, 0.84 mmol), sodium carbonate (358 mg, 3.38 mmol), and tetrakis(triphenylphosphine)palladium (98 mg, 0.08 mmol) were added to a mixed solution of 1,4-dioxane and water (30 mL/6 mL). The reaction mixture was stirred at 90°C for 16 hours. After the reaction was completed, the reaction mixture was filtered, and the filtrate was concentrated under reduced pressure. The residue was purified by silica gel column chromatography (dichloromethane/methanol = 1/0 to 10/1) to obtain compound 117. Compound 117 was resolved by supercritical fluid chiral chromatography (equipment: SFC Thar prep 80, column: CHIRALPAK AD-H 250 mm × 20 mm, 5 µm, mobile phase: 40% IPA (NH₄OH 0.2%), total flow rate: 40 g/min) to obtain compounds 117-P1 (10.9 mg, yield: 1.2%) and 117-P2 (13.2 mg, yield: 1.4%).

### Compound 117-P1:

MS m/z (ESI): 546.1 [M+1]⁺. Supercritical fluid chromatography (SFC): retention time = 3.03 min, UV = 254 nm. ¹H NMR (400 MHz, CD₃OD) δ 8.65 (s, 1H), 8.45 (d, J = 2.3 Hz, 1H), 8.04 (s, 1H), 7.76 - 7.68 (m, 1H), 7.53 (dd, J = 9.6, 3.1 Hz, 1H), 7.30 (dd, J = 9.9, 3.1 Hz, 1H), 6.82 (s, 1H), 5.50 (d, J = 1.7 Hz, 2H), 2.10 (s, 3H), 1.66 (s, 3H), 1.27 (s, 6H).

### Compound 117-P2:

MS m/z (ESI): 546.1 [M+1]⁺. Supercritical fluid chromatography (SFC): retention time = 3.41 min, UV = 254 nm. ¹H NMR (400 MHz, CD₃OD) δ 8.65 (s, 1H), 8.45 (d, J = 2.3 Hz, 1H), 8.04 (s, 1H), 7.75 - 7.69 (m, 1H), 7.52 (dd, J = 9.6, 3.1 Hz, 1H), 7.30 (dd, J = 9.9, 3.0 Hz, 1H), 6.82 (s, 1H), 5.50 (d, J *=* 1.6 Hz, 2H), 2.13 (s, 3H), 1.67 (s, 3H), 1.28 (s, 6H).

### Example 54 Synthesis of Compounds 119-2, 119-P1, and 119-P2

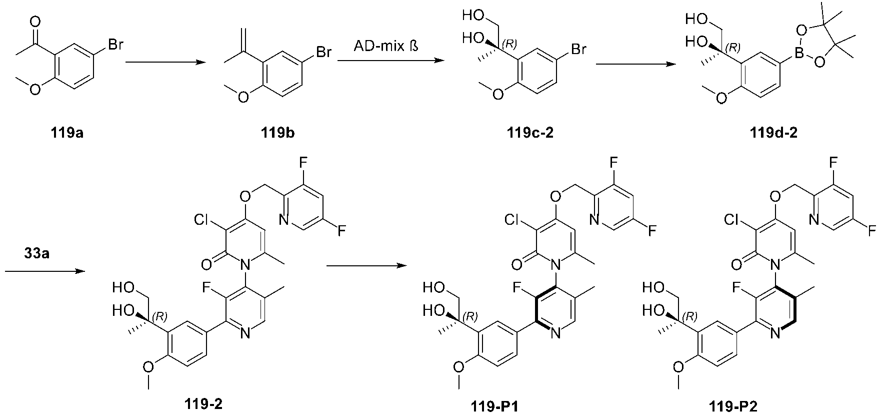

### Step 1: Synthesis of Compound 119b

Methylenetriphenylphosphine (3.74 g, 10.5 mmol) was added to a solution of potassium tert-butoxide (1.12 g, 10.5 mmol) in tetrahydrofuran (50 mL). The mixture was stirred at room temperature for 0.5 hours, followed by the addition of compound 119a (2 g, 8.7 mmol). The reaction mixture was reacted at room temperature for 3 hours. After the reaction was completed, the reaction mixture was quenched with water (50 mL) and extracted with dichloromethane (50 mL × 3). The combined organic phases were washed with saturated brine (20 mL × 1) and concentrated under reduced pressure. The residue was purified by silica gel column chromatography (petroleum ether) to obtain compound 119b (1.5 g, yield: 76%). ¹HNMR (400 MHz,CDCl₃) δ 7.31 (dd, J = 10.9, 5.6 Hz, 2H), 6.74 (d, J = 8.6 Hz, 1H), 5.21 - 5.11 (m, 1H), 5.06 (d, J = 0.5 Hz, 1H), 3.80 (s, 3H), 2.08 (s, 3H).

### Step 2: Synthesis of Compound 119c-2

AD-mix-β (15 g) was added to a mixed solution of compound 119b (1.5 g, 5.7 mmol) in tert-butanol and water (20 mL/20 mL). The reaction mixture was stirred at room temperature for 48 hours. After the reaction was completed, the reaction mixture was quenched with saturated sodium thiosulfate solution (40 mL), extracted with dichloromethane (50 mL × 5), and the combined organic phases were washed with saturated brine (20 mL × 1) and concentrated under reduced pressure. The residue was subjected to silica gel column chromatography (petroleum ether/ethyl acetate = 1/0 to 1/1) to obtain compound 119c-2 (160 mg, yield: 10.67%). ¹HNMR (400 MHz, CDCl₃) δ 7.59 (d, J = 2.5 Hz, 1H), 7.37 (dd, J = 8.7, 2.5 Hz, 1H), 6.79 (d, J = 8.7 Hz, 1H), 3.98 (d, J = 11.0 Hz, 1H), 3.86 (s, 3H), 3.66 (d, J = 11.0 Hz, 1H), 1.54 (s, 3H).

### Step 3: Synthesis of Compound 119d-2

1,1'-Bis(diphenylphosphino)ferrocene-palladium(II)dichloride dichloromethane complex (47 mg, 0.065 mmol) was added to a solution of compound 119c-2 (160 mg, 0.61 mmol), bis(pinacolato)diboron (182 mg, 0.72 mmol), and potassium acetate (128 mg, 1.3 mmol) in 1,4-dioxane (15 mL). The reaction mixture was stirred at 90°C for 16 hours. After the reaction was completed, the reaction mixture was filtered, and the filtrate was concentrated under reduced pressure to obtain compound 119d-2 (300 mg, crude product), which was directly used in the next step. MS m/z (ESI): 309.1 [M+1]⁺.

### Step 4: Synthesis of Compounds 119-P1 and 119-P2

Compound 119d-2 (300 mg, crude product), compound 33a (237 mg, 0.55 mmol), sodium carbonate (233 mg, 2.21 mmol), and tetrakis(triphenylphosphine)palladium (127 mg, 0.11 mmol) were added to a mixed solution of 1,4-dioxane and water (15 mL/1.5 mL). The reaction mixture was stirred at 90°C for 16 hours. After the reaction was completed, the reaction mixture was concentrated under reduced pressure, and the residue was subjected to silica gel column chromatography (dichloromethane/methanol = 10/1) to obtain compound 119-2 (120 mg). Compound 119-2 (120 mg) was then resolved by supercritical fluid chiral chromatography (equipment: SFC Thar prep 80, column: CHIRALPAK AD-H 250 mm × 20 mm, 5 µm, mobile phase: 40% MeOH (NH₄OH 0.2%): 60% CO₂, total flow rate: 40 g/min) to obtain compounds 119-P1 (22.4 mg, yield: 3.40%) and 119-P2 (35.2 mg, yield: 6.28%).

### Compound 119-P1:

MS m/z (ESI): 576.2 [M+1]⁺. Supercritical fluid chromatography (SFC): retention time = 2.93 min, UV = 220 nm. ¹HNMR (400 MHz,CD₃OD) δ 8.54 (s, 1H), 8.45 (d, J = 2.3 Hz, 1H), 8.23 (d, J = 1.3 Hz, 1H), 7.84 - 7.81 (m, 1H), 7.72 (td, J = 9.6, 2.3 Hz, 1H), 7.10 (d, J *=* 8.7 Hz, 1H), 6.85 (s, 1H), 5.50 (d, J = 1.7 Hz, 2H), 3.90 (s, 3H), 3.87 (d, J = 11.1 Hz, 1H), 3.80 (d, J = 11.1 Hz, 1H), 2.14 (s, 3H), 2.11 (s, 3H), 1.55 (s, 3H).

### Compound 119-P2:

MS m/z (ESI): 576.2 [M+1]⁺. Supercritical fluid chromatography (SFC): retention time = 8.28 min, UV = 220 nm. ¹HNMR (400 MHz, CD₃OD) δ 8.54 (d, J = 0.6 Hz, 1H), 8.45 (d, J *=* 2.3 Hz, 1H), 8.23 (dd, J = 2.1, 1.5 Hz, 1H), 7.83 (dt, J = 8.6, 2.1 Hz, 1H), 7.72 (ddd, J = 9.6, 8.6, 2.4 Hz, 1H), 7.10 (d, J = 8.6 Hz, 1H), 6.85 (d, J = 0.4 Hz, 1H), 5.50 (d, J = 1.9 Hz, 2H), 3.90 (s, 3H), 3.87 (d, J = 11.1 Hz, 1H), 3.79 (d, J = 11.1 Hz, 1H), 2.14 (s, 3H), 2.11 (s, 3H), 1.55 (s, 3H).

### Example 55 Synthesis of Compounds 120-2, 120-P1, and 120-P2

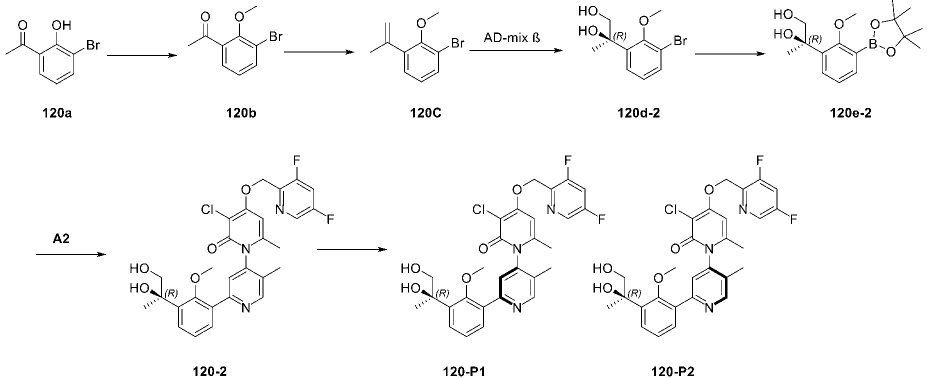

### Step 1: Synthesis of Compound 120b

Iodomethane (13.2 g, 390 mmol) was added to a solution of compound 120a (5 g, 23.25 mmol) and potassium carbonate (5.14 g, 37.2 mmol) in DMF (30 mL). The reaction mixture was stirred at room temperature for 16 hours. After the reaction was completed, the reaction mixture was quenched with water (100 mL) and extracted with ethyl acetate (50 mL × 3). The combined organic phases were washed with saturated brine (20 mL × 1) and concentrated under reduced pressure. The residue was subjected to silica gel column chromatography (petroleum ether/ethyl acetate = 1/0 to 20/1) to obtain compound 120b (4 g, yield: 63.44%). ¹HNMR. NMR (400 MHz, CDCl₃) δ 7.70 (dd, *J =* 7.9, 1.6 Hz, 1H), 7.56 (dd, *J=* 7.8, 1.6 Hz, 1H), 7.07 (t, *J =* 7.8 Hz, 1H), 3.88 (s, 3H), 2.70 - 2.61 (m, 3H).

### Step 2: Synthesis of Compound 120c

Methyltriphenylphosphonium (7.5 g, 21 mmol) was added to a solution of potassium tert-butoxide (2.36 g, 21 mmol) in tetrahydrofuran (100 mL). The mixture was stirred at room temperature for 0.5 hours, followed by the addition of compound 120b (4 g, 17.5 mmol). The reaction mixture was reacted at room temperature for 3 hours. After the reaction was completed, the reaction mixture was quenched with water (100 mL) and extracted with dichloromethane (100 mL × 3). The combined organic phases were washed with saturated brine (100 mL × 1) and concentrated under reduced pressure. The residue was purified by silica gel column chromatography (petroleum ether) to obtain compound 120c (3 g, yield: 68%). ¹HNMR (400 MHz, CDCl₃) δ 7.46 (dd, *J* = 8.0*,* 1.5 Hz, 1H), 7.14 (dd, *J* = 7.6, 1.5 Hz, 1H), 6.93 (t, *J= 7.8* Hz, 1H), 5.21 - 5.17 (m, 1H), 5.12 (d, *J=* 0.8 Hz, 1H), 3.77 (s, 3H), 2.13 (s, 3H).

### Step 3: Synthesis of Compound 120d-2

AD-mix-β (30 g) was added to a mixed solution of compound 120c (3 g, 13.2 mmol) in tert-butanol and water (20 mL/20 mL). The reaction mixture was stirred at room temperature for 16 hours. After the reaction was completed, the reaction mixture was quenched with saturated sodium thiosulfate solution (40 mL), extracted with dichloromethane (50 mL × 5), and the combined organic phases were washed with saturated brine (50 mL × 1) and concentrated under reduced pressure. The residue was subjected to silica gel column chromatography (petroleum ether/ethyl acetate = 1/0 to 1/1) to obtain compound 120d-2 (200 mg, yield: 4.93%). ¹HNMR (400 MHz, CDCl₃) δ 7.46 (ddd, *J* = 25.1, 7.9, 1.4 Hz, 2H), 7.00 (t, *J* = 7.9 Hz, 1H), 3.98 (s, 3H), 3.93 (d, *J* = 11.1 Hz, 1H), 3.69 (d, *J* = 11.1 Hz, 1H), 1.55 (s, 3H).

### Step 4: Synthesis of Compound 120e-2

1,1'-bis(diphenylphosphino)ferrocene-palladium(II)dichloride dichloromethane complex (55 mg, 0.076 mmol) was added to a solution of compound 120d-2 (200 mg, 0.76 mmol), bis(pinacolato)diboron (213 mg, 0.84 mmol), and potassium acetate (150 mg, 1.53 mmol) in 1,4-dioxane (15 mL). The reaction mixture was stirred at 90°C for 16 hours. After the reaction was completed, the reaction mixture was filtered, and the filtrate was concentrated under reduced pressure to obtain compound 120e-2 (200 mg, crude product), which was directly used in the next step. MS m/z (ESI): 309.1 [M+1]⁺.

### Step 4: Synthesis of Compounds 120-P1 and 120-P2

Compound 120e-2 (200 mg, crude product), compound 33a (267 mg, 0.64 mmol), sodium carbonate (343 mg, 3.24 mmol), and tetrakis(triphenylphosphine)palladium (187 mg, 0.16 mmol) were added to a mixed solution of 1,4-dioxane and water (15 mL/1.5 mL). The reaction mixture was stirred at 90°C for 16 hours. After the reaction was completed, the reaction mixture was concentrated under reduced pressure, and the residue was subjected to silica gel column chromatography (dichloromethane/methanol = 10/1) to obtain compound 120-2 (120 mg). Compound 120-2 (120 mg) was then resolved by supercritical fluid chiral chromatography (equipment: SFC Thar prep 80, column: CHIRALPAK AD-H 250 mm × 20 mm, 5 µm, mobile phase: 40% MeOH (NH₄OH 0.2%): 60% CO₂, total flow rate: 40 g/min) to obtain compounds 120-P1 (55.4 mg, yield: 5.96%) and 120-P2 (59.6 mg, yield: 6.52%).

### Compound 120-P1:

MS m/z (ESI): 558.2 [M+1]⁺. Supercritical fluid chromatography (SFC): retention time = 5.46 min, UV = 254 nm. ¹HNMR (400 MHz, CD₃OD) δ 8.75 (s, 1H), 8.45 (s, 1H), 7.82 - 7.69 (m, 3H), 7.53 (d, *J=* 7.1 Hz, 1H), 7.23 (t, *J=* 7.7 Hz, 1H), 6.80 (s, 1H), 5.50 (s, 2H), 3.91 (d, *J* = 11.0 Hz, 1H), 3.79 (d, *J =* 11.1 Hz, 1H), 3.42 (s, 3H), 2.15 (s, 3H), 2.09 (s, 3H), 1.57 (s, 3H).

### Compound 120-P2:

MS m/z (ESI): 558.2 [M+1]⁺. Supercritical fluid chromatography (SFC): retention time = 7.14 min, UV = 254 nm. ¹HNMR (400 MHz, CD₃OD) δ 8.75 (s, 1H), 8.46 (d, *J =* 2.2 Hz, 1H), 7.78 - 7.70 (m, 2H), 7.68 (s, 1H), 7.52 (dd, *J* = 7.5*,* 1.5 Hz, 1H), 7.23 (t, *J* = 7.7 Hz, 1H), 6.81 (s, 1H), 5.50 (s, 2H), 3.91 (d, *J* = 11.1 Hz, 1H), 3.78 (d, *J= 11.1* Hz, 1H), 3.41 (d, *J* = 3.4 Hz, 3H), 2.15 (s, 3H), 2.11 (s, 3H), 1.57 (s, 3H).

### Example 56 Synthetis of Compounds 121, 121-P1, 121-P2, 121-P3, and 121-P4

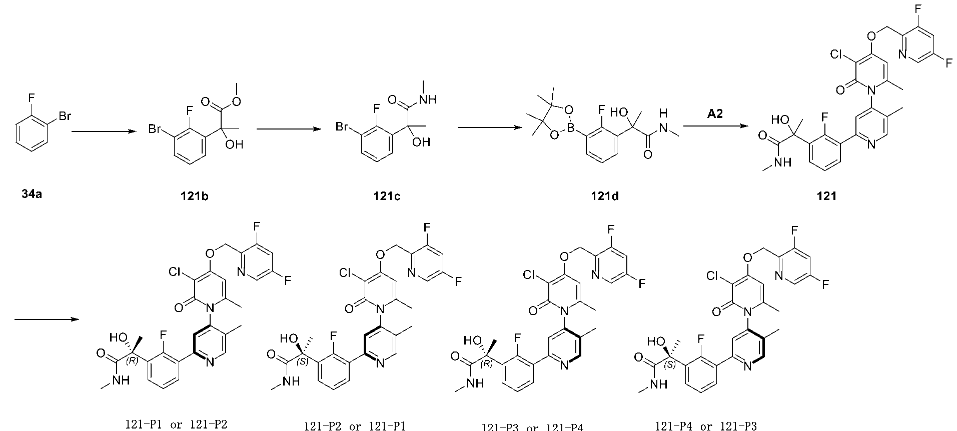

### Step 1: Synthesis of Compound 121b

At -50°C, lithium diisopropylamide (15.7 mL, 31.5 mmol) was added dropwise to a solution of compound 34a (5 g, 28.6 mmol) in tetrahydrofuran (100 mL), and the mixture was stirred at -50°C for 1 hour. Methyl pyruvate (3.21 g, 31.5 mmol) was added dropwise, and the reaction mixture was stirred at room temperature for 2 hours. After the reaction was completed, the reaction mixture was quenched with saturated ammonium chloride solution (100 mL), extracted with ethyl acetate (100 mL × 3), and the combined organic phases were dried over anhydrous sodium sulfate and filtered. The filtrate was concentrated under reduced pressure, and the residue was purified by silica gel column chromatography (petroleum ether/ethyl acetate = 1/0 to 1/1) to obtain compound 121b (1.9 g, yield: 37.2%). ¹HNMR (400 MHz, CDCl₃) δ 7.49 (dt, *J=* 15.0, 7.4 Hz, 2H), 7.03 (t, *J=* 7.9 Hz, 1H), 3.78 (s, 3H), 1.79 (s, 3H).

### Step 2: Synthesis of Compound 121c

Compound 121b (600 mg, 2.1 mmol) was added to a solution of methylamine in ethanol (50 mL), and the reaction mixture was stirred at 80°C for 16 hours. After the reaction was completed, the reaction mixture was concentrated under reduced pressure. The residue was purified by silica gel column chromatography (petroleum ether/ethyl acetate = 1/0 to 15/1) to obtain compound 121c (500 mg, yield: 75.3%). MS m/z (ESI): 276.0 [M+1]⁺.

### Step 3: Synthesis of Compound 121d

1,1'-Bis(diphenylphosphino)ferrocene-palladium(II)dichloride dichloromethane complex (53 mg, 0.07 mmol) was added to a solution of compound 121c (200 mg, 0.72 mmol), bis(pinacolato)diboron (276 mg, 1.1 mmol), and potassium acetate (142 mg, 1.4 mmol) in 1,4-dioxane (10 mL). The reaction mixture was stirred at 90°C for 16 hours. After the reaction was completed, the reaction mixture was filtered, and the filtrate was concentrated under reduced pressure to obtain compound 121d (300 mg, crude product). MS m/z (ESI): 324.0 [M+1]⁺.

### Step 4: Synthesis of compounds 121-P1, 121-P2, 121-P3, and 121-P4

Tetrakis(triphenylphosphine)palladium (44 mg, 0.04 mmol) was added to a mixed solution of compound 121d (300 mg, crude product), compound A2 (127 mg, 0.3 mmol), and sodium carbonate (65 mg, 0.6 mmol) in 1,4-dioxane and water (10 mL/l mL). The reaction mixture was stirred at 80°C overnight. After the reaction was completed, the reaction mixture was concentrated under reduced pressure to remove the solvent. The residue was purified by silica gel column chromatography (dichloromethane/methanol = 1/0 to 20/1) to obtain compound 121 (100 mg). Compound 121 (100 mg) was then resolved by supercritical fluid chiral chromatography (equipment: SFC Thar prep 80, column: CHIRALPAK AD-H 250 mm × 20 mm, 5 µm, mobile phase: 40% MeOH (NH₄OH 0.2%): 60% CO₂, total flow rate: 40 g/min) to obtain compounds 121-P1 (30 mg, yield: 15%), 121-P4 (30 mg, yield: 15%), and a mixture of 121-P2 and 121-P3. The mixture of P2 and P3 was further resolved by supercritical fluid chiral chromatography (equipment: SFC Thar prep 80, column: CHIRALPAK AS-H 250 mm × 20 mm, 5 µm, mobile phase: 40% IPA (NH₄OH 0.2%), total flow rate: 40 g/min) to obtain compound 121-P2 (30 mg, yield: 15%) and 121-P3 (30 mg, yield: 15%).

### Compound 121-P1:

MS m/z (ESI): 573.2 [M+1]⁺. Supercritical fluid chromatography (SFC): retention time = 2.91 min, UV = 254 nm. ¹H NMR (400 MHz, CD₃OD) δ 8.65 (s, 1H), 8.36 (d, *J= 2.3* Hz, 1H), 7.76 (t, *J =* 6.6 Hz, 1H), 7.62 (ddd, J= 14.9, 11.1, 5.6 Hz, 3H), 7.22 (t, *J* = 7.8 Hz, 1H), 6.72 (s, 1H), 5.40 (d, *J =* 1.7 Hz, 2H), 2.68 (s, 1H), 2.04 (s, 3H), 1.98 (s, 3H), 1.72 (s, 3H).

### Compound 121-P2:

MS m/z (ESI): 573.2 [M+1]⁺. Supercritical fluid chromatography (SFC): retention time = 1.85 min, UV = 254 nm. ¹H NMR (400 MHz, CD₃OD) δ 8.77 (s, 1H), 8.48 (d, *J =* 2.2 Hz, 1H), 7.86 (t, *J=* 7.4 Hz, 1H), 7.79 - 7.67 (m, 3H), 7.34 (t, *J* = 7.8 Hz, 1H), 6.83 (s, 1H), 5.52 (s, 2H), 2.80 (s, 3H), 2.16 (s, 3H), 2.09 (s, 3H), 1.84 (s, 3H).

### Compound 121-P3:

MS m/z (ESI): 573.2 [M+1]⁺. Supercritical fluid chromatography (SFC): retention time = 2.23 min, UV = 254 nm. ¹H NMR (400 MHz, CD₃OD) δ 8.65 (s, 1H), 8.36 (d, *J =* 2.3 Hz, 1H), 7.77 - 7.70 (m, 1H), 7.67 - 7.55 (m, 3H), 7.22 (t, *J =* 7.8 Hz, 1H), 6.71 (s, 1H), 5.40 (d, *J=* 1.7 Hz, 2H), 2.68 (s, 3H), 2.04 (s, 3H), 1.97 (s, 3H), 1.72 (s, 3H).

### Compound 121-P4:

MS m/z (ESI): 573.2 [M+1]⁺. Supercritical fluid chromatography (SFC): retention time = 4.36 min, UV = 254 nm. ¹H NMR (400 MHz, CD₃OD) δ 8.65 (s, 1H), 8.36 (d, *J =* 2.3 Hz, 1H), 7.76 (s, 1H), 7.68 - 7.53 (m, 3H), 7.22 (t, *J =* 7.8 Hz, 1H), 6.71 (s, 1H), 5.40 (d, *J =* 1.6 Hz, 2H), 2.68 (s, 3H), 2.04 (s, 3H), 1.97 (s, 3H), 1.72 (s, 3H).

### Example 57 Synthesis of Compounds 122, 122-P1, 122-P2, 122-P3, and 122-P4

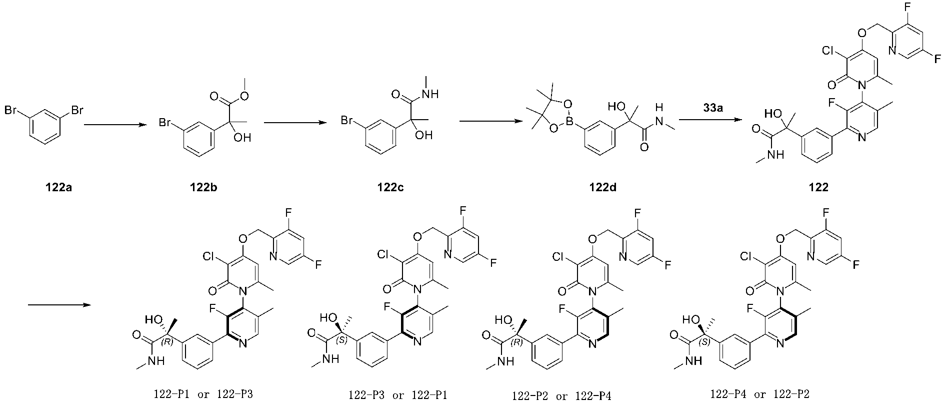

### Step 1: Synthesis of Compound 122b

At -50°C, a solution of n-butyllithium in tetrahydrofuran (5.3 mL, 12.72 mmol) was added dropwise to a solution of compound 122a (3.0 g, 12.72 mmol) in tetrahydrofuran (40 mL), and the mixture was stirred at -50°C for 1 hour. Methyl pyruvate (3.9 g, 38.15 mmol) was added dropwise, and the reaction mixture was stirred at room temperature for 2 hours. After the reaction was completed, the reaction mixture was quenched with saturated ammonium chloride solution (100 mL), extracted with ethyl acetate (100 mL × 3), and the combined organic phases were dried over anhydrous sodium sulfate and filtered. The filtrate was concentrated under reduced pressure, and the residue was purified by silica gel column chromatography (petroleum ether/ethyl acetate = 1/0 to 8/1) to obtain compound 122b (1.4 g, yield: 38.24%). MS m/z (ESI): 259.2 [M+1]⁺.

### Step 2: Synthesis of Compound 122c

Compound 122b (2.5 g, 9 mmol) was added to a solution of methylamine in ethanol (20 mL), and the reaction mixture was stirred at 80°C for 16 hours. After the reaction was completed, the reaction mixture was concentrated under reduced pressure. The residue was purified by silica gel column chromatography (petroleum ether/ethyl acetate = 1/0 to 4/1) to obtain compound 122c (400 mg, yield: 30.11%). MS m/z (ESI): 258.1 [M+1]⁺.

### Step 3: Synthesis of Compound 122d

1,1'-Bis(diphenylphosphino)ferrocene-palladium(II)dichloride dichloromethane complex (58 mg, 0.08 mmol) was added to a solution of compound 122c (400 mg, 1.55 mmol), bis(pinacolato)diboron (512 mg, 2.01 mmol), and potassium acetate (304 mg, 3.10 mmol) in 1,4-dioxane (8 mL). The reaction mixture was stirred at 80°C for 16 hours. After the reaction was completed, the reaction mixture was filtered, and the filtrate was concentrated under reduced pressure to obtain compound 122 d (400 mg, crude product). MS m/z (ESI): 306.1 [M+1]⁺.

### Step 4: Synthesis of compounds 122-P1, 122-P2, 122-P3, and 122-P4

Tetrakis(triphenylphosphine)palladium (38 mg, 0.03 mmol) was added to a mixed solution of compound 122d (400 mg, crude product), compound 33a (197 mg, 0.46 mmol), and sodium carbonate (139 mg, 1.31 mmol) in 1,4-dioxane and water (10 mL/2 mL). The reaction mixture was stirred at 80°C overnight. After the reaction was completed, the reaction mixture was concentrated under reduced pressure to remove the solvent. The residue was purified by silica gel column chromatography (dichloromethane/methanol = 1/0 to 20/1) to obtain compound 122. Compound 122 was resolved by supercritical fluid chiral chromatography (equipment: SFC Thar prep 80, column: CHIRALPAK WHELK-01 250 mm × 21.1 mm, 5 µm, mobile phase: 40% MeOH (NH₄OH 0.2%): 60% CO₂, total flow rate: 50 g/min) to obtain compounds 122-P1 (9.1 mg, yield: 2.43%), 122-P2 (16.2 mg, yield: 4.21%), 122-P3 (18.6 mg, yield: 4.95%), and 122-P4 (13.3 mg, yield: 3.48%).

### Compound 122-P1:

MS m/z (ESI): 573.2 [M+1]⁺. Supercritical fluid chromatography (SFC): retention time = 5.33 min, UV = 254 nm. ¹HNMR (400 MHz,CD₃OD) δ 8.63 (s, 1H), 8.48 (d, *J=* 2.3 Hz, 1H), 8.20 (d, *J= 1.3* Hz, 1H), 7.82 (d, *J=* 6.9 Hz, 1H), 7.78 - 7.70 (m, 2H), 7.48 (t, *J* = 7.8 Hz, 1H), 6.88 (s, 1H), 5.54 (d, *J=* 1.7 Hz, 2H), 2.74 (s, 3H), 2.20 (s, 3H), 2.14 (s, 3H), 1.80 (s, 3H).

### Compound 122-P2:

MS m/z (ESI): 573.2 [M+1]⁺. Supercritical fluid chromatography (SFC): retention time = 6.72 min, UV = 254 nm. ¹HNMR (400 MHz,CD₃OD ) δ 8.62 (s, 1H), 8.48 (d, *J=* 2.3 Hz, 1H), 8.21 (d, *J* = 1.4 Hz, 1H), 7.83 (d, *J=* 6.8 Hz, 1H), 7.78 - 7.70 (m, 2H), 7.49 (t, *J* = 7.8 Hz, 1H), 6.89 (s, 1H), 5.54 (d, *J=* 1.8 Hz, 2H), 2.74 (s, 3H), 2.20 (s, 3H), 2.15 (s, 3H), 1.80 (s, 3H).

### Compound 122-P3:

MS m/z (ESI): 573.1 [M+1]⁺. Supercritical fluid chromatography (SFC): retention time = 7.67 min, UV = 254 nm. ¹HNMR (400 MHz, CD₃OD) δ 8.60 (s, 1H), 8.46 *(d, J= 2.1* Hz, 1H), 8.19 (s, 1H), 7.81 (d, *J=* 7.3 Hz, 1H), 7.73 (dd, *J=* 14.1, 5.3 Hz, 2H), 7.47 (t, *J =* 7.8 Hz, 1H), 6.87 (s, 1H), 5.52 (s, 2H), 2.72 (s, 3H), 2.18 (s, 3H), 2.13 (s, 3H), 1.77 (s, 3H).

### Compound 122-P4:

MS m/z (ESI): 573.1 [M+1]⁺. Supercritical fluid chromatography (SFC): retention time = 8.98 min, UV = 254 nm. ¹HNMR (400 MHz,CD₃OD ) δ 8.63 (s, 1H), 8.48 (d, J= 2.3 Hz, 1H), 8.20 (d, *J=* 1.3 Hz, 1H), 7.82 (d, *J* = 8.0 Hz, 1H), 7.74 (td, *J=* 9.6, 2.1 Hz, 2H), 7.48 (t, *J=* 7.8 Hz, 1H), 6.88 (s, 1H), 5.54 (d, *J=* 1.7 Hz, 2H), 2.74 (s, 3H), 2.20 (s, 3H), 2.14 (s, 3H), 1.80 (s, 3H).

### Example 58 Synthesis of Compounds 123, 123-P1, and 123-P2

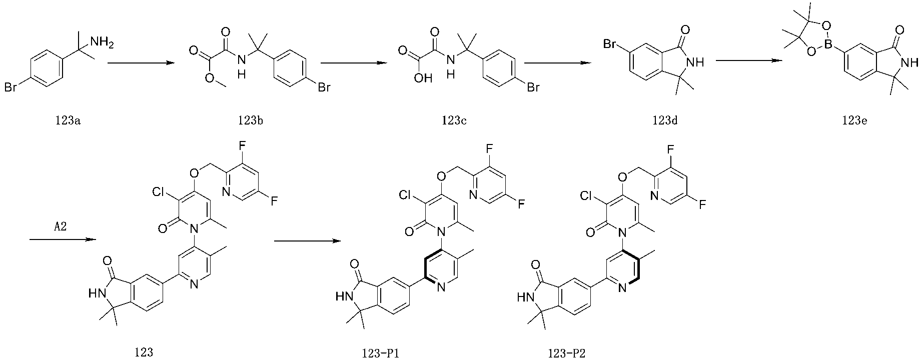

### Step 1: Synthesis of Compound 123b

At 0°C, triethylamine (950 mg, 9.4 mmol) was added dropwise to a solution of compound 123a (1 g, 4.7 mmol) and methyl oxalyl chloride (690 mg, 5.64 mmol) in dichloromethane (15 mL). The reaction mixture was stirred at room temperature for 1 hour. After the reaction was completed, the reaction mixture was quenched with 1 N hydrochloric acid solution (15 mL) and extracted with dichloromethane (30 mL × 3). The combined organic phases were dried over anhydrous sodium sulfate and filtered. The filtrate was concentrated under reduced pressure to obtain compound 123b (0.6 g, yield: 38.3%). MS m/z (ESI): 302.0 [M+1]⁺.

### Step 2: Synthesis of Compound 123c

Saturated sodium hydroxide solution (5 mL) was added to a solution of compound 123b (0.6 g, 6.4 mmol) in methanol, and the reaction mixture was stirred at room temperature for 0.5 hours. After the reaction was completed, the reaction mixture was concentrated under reduced pressure. The residue was adjusted to pH = 6-7 with 1 N hydrochloric acid solution and extracted with ethyl acetate (30 mL × 3). The combined organic phases were dried over anhydrous sodium sulfate and filtered. The filtrate was concentrated under reduced pressure to obtain compound 123c (0.42 g, yield: 66.09%). MS m/z (ESI): 284.0 [M-1]⁻.

### Step 3: Synthesis of Compound 123d

Ammonium persulfate (0.1 g, 2.93 mmol) was added to a mixed solution of compound 123d (0.42 g, 1.46 mmol) in dimethyl sulfoxide and water (11 mL/10 mL). The reaction mixture was stirred at 100°C for 4 hours. After the reaction was completed, the reaction mixture was quenched with water (30 mL) and extracted with ethyl acetate (30 mL × 3). The combined organic phases were dried over anhydrous sodium sulfate and filtered. The filtrate was concentrated under reduced pressure, and the residue was subjected to silica gel column chromatography (petroleum ether/ethyl acetate = 1/0 to 6/1) to obtain compound 123d (0.3 g, yield: 76.61%). MS m/z (ESI): 242.0 [M+1]⁺.

### Step 4: Synthesis of Compound 123e

1,1'-Bis(diphenylphosphino)ferrocene-palladium(II)dichloride dichloromethane complex (90 mg, 0.125 mmol) was added to a solution of compound 123d (300 mg, 1.25 mmol), bis(pinacolato)diboron (0.38 g, 1.5 mmol), and potassium acetate (245 mg, 2.5 mmol) in 1,4-dioxane (15 mL). The reaction mixture was stirred at 90°C for 16 hours. After the reaction was completed, the reaction mixture was filtered, and the filtrate was concentrated under reduced pressure to obtain compound 123 e (400 mg, crude product). MS m/z (ESI): 288.1 [M+1]⁺.

### Step 5: Synthesis of Compounds 123-P1 and 123-P2

Tris(diphenylphosphine)palladium (160 mg, 0.14 mmol) was added to a mixed solution of compound 123e (400 mg, 1.4 mmol), compound A2 (172 mg, 0.41 mmol), and sodium carbonate (295 mg, 2.78 mmol) in 1,4-dioxane and water (10 mL/1 mL). The reaction mixture was stirred at 90°C overnight. After the reaction was completed, the reaction mixture was concentrated under reduced pressure, and the residue was purified by silica gel column chromatography (dichloromethane/methanol = 1/0 to 10/1) to obtain compound 123 (80 mg). Compound 123 (80 mg) was then resolved by supercritical fluid chiral chromatography (equipment: chiralpak-AS, column: CHIRALPAK AD-H 250 mm × 20 mm, 5 µm, mobile phase: 40% MeOH (0.2% DEA)/60% CO₂, total flow rate: 40 g/min) to obtain compounds 123-P1 (retention time: 1.97 minutes, 25.6 mg, yield: 3.39%) and 123-P2 (retention time: 5.6 minutes, 23.8 mg, yield: 3.15%).

### Compound 123-P1:

MS m/z (ESI): 537.2 [M+1]⁺. Supercritical fluid chromatography (SFC): retention time = 1.97 min, UV = 254 nm. ¹H NMR (400 MHz,CDCl₃) δ 8.72 (s, 1H), 8.42 (d, *J* = 2.1 Hz, 1H), 8.32 (d, *J* = 8.0 Hz, 1H), 8.27 (s, 1H), 7.54 (s, 1H), 7.50 (d, J = 8.0 Hz, 1H), 7.36 - 7.31 (m, 1H), 6.37 (d, *J=* 24.4 Hz, 2H), 5.43 (s, 2H), 2.15 (s, 3H), 2.01 (s, 3H), 1.59 (s, 6H).

### Compound 123-P2:

MS m/z (ESI): 537.2 [M+1]⁺. Supercritical fluid chromatography (SFC): retention time = 5.6 min, UV = 254 nm. ¹H NMR (400 MHz, CDCl₃) δ 8.74 (s, 1H), 8.42 (s, 1H), 8.34 (d, *J =* 7.8 Hz, 1H), 8.27 (s, 1H), 7.56 (s, 1H), 7.51 (d, *J =* 8.0 Hz, 1H), 7.33 (t, *J =* 8.4 Hz, 1H), 6.40 (d, *J=* 10.5 Hz, 2H), 5.44 (s, 2H), 2.16 (s, 3H), 2.01 (s, 3H), 1.59 (s, 6H).

### Example 59 Synthesis of Compounds 124, 124-P1, 124-P2, 124-P3, and 124-P4

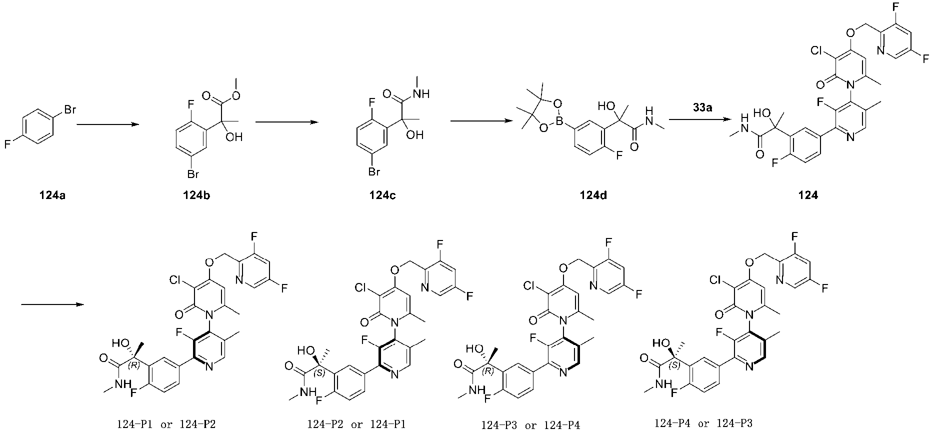

### Step 1: Synthesis of Compound 124b

At -50°C, a solution of lithium diisopropylamide in tetrahydrofuran (10 mL, 20 mmol) was added dropwise to a solution of compound 124a (3.5 g, 20 mmol) in tetrahydrofuran (50 mL), and the mixture was stirred at -50°C for 1 hour. Methyl pyruvate (2.45 g, 24 mmol) was added dropwise, and the reaction mixture was stirred at room temperature for 2 hours. After the reaction was completed, the reaction mixture was quenched with saturated ammonium chloride solution (100 mL), extracted with ethyl acetate (100 mL × 3), and the combined organic phases were dried over anhydrous sodium sulfate and filtered. The filtrate was concentrated under reduced pressure, and the residue was purified by silica gel column chromatography (petroleum ether/ethyl acetate = 1/0 to 8/1) to obtain compound 124b (2.5 g, yield: 45.13%). ¹HNMR (400 MHz, CDCl₃) δ 7.68 (dd, J = 6.9, 2.5 Hz, 1H), 7.42 (ddd, J = 8.6, 4.4, 2.5 Hz, 1H), 6.94 (dd, J = 10.8, 8.7 Hz, 1H), 3.79 (s, 3H), 1.80 (s, 3H).

### Step 2: Synthesis of Compound 124c

Compound 124b (2.5 g, 9 mmol) was added to a solution of methylamine in ethanol (20 mL), and the reaction mixture was stirred at 80°C for 16 hours. After the reaction was completed, the reaction mixture was concentrated under reduced pressure. The residue was purified by silica gel column chromatography (petroleum ether/ethyl acetate = 1/0 to 10/1) to obtain compound 124c (1 g, yield: 40.16%). MS m/z (ESI): 277.1 [M+1]⁺.

### Step 3: Synthesis of Compound 124d

1,1'-Bis(diphenylphosphino)ferrocene-palladium(II)dichloride dichloromethane complex (131 mg, 0.18 mmol) was added to a solution of compound 124c (500 mg, 1.81 mmol), bis(pinacolato)diboron (505.88 mg, 1.99 mmol), and potassium acetate (355.48 mg, 3.62 mmol) in 1,4-dioxane (30 mL). The reaction mixture was stirred at 90°C for 16 hours. After the reaction was completed, the reaction mixture was filtered, and the filtrate was concentrated under reduced pressure to obtain compound 124d (500 mg, crude product). MS m/z (ESI): 324.1 [M+1]⁺.

### Step 4: Synthesis of compounds 124-P1, 124-P2, 124-P3, and 124-P4

Tetrakis(triphenylphosphine)palladium (179 mg, 0.15 mmol) was added to a mixed solution of compound 124d (500 mg, crude product), compound 33a (318 mg, 0.77 mmol), and sodium carbonate (328 mg, 3.09 mmol) in 1,4-dioxane and water (30 mL/3 mL). The reaction mixture was stirred at 90°C overnight. After the reaction was completed, the reaction mixture was concentrated under reduced pressure to remove the solvent. The residue was purified by silica gel column chromatography (dichloromethane/methanol = 1/0 to 20/1) to obtain compound 124 (150 mg). Compound 124 (150 mg) was then resolved by supercritical fluid chiral chromatography (equipment: SFC Thar prep 80, column: CHIRALPAK AD-H 250 mm × 20 mm, 5 µm, mobile phase: 40% IPA (NH₄OH 0.2%)/60% CO₂, total flow rate: 50 g/min) to obtain compounds 124-P1 (15.7 mg, yield: 1.78%), 124-P2 (17.0 mg, yield: 1.92%), 124-P3 (17.1 mg, yield: 1.93%), and 124-P4 (13.1 mg, yield: 1.48%).

### Compound 124-P1:

MS m/z (ESI): 591.1 [M+1]⁺. Supercritical fluid chromatography (SFC): retention time = 2.22 min, UV = 254 nm. ¹HNMR (400 MHz,CD₃OD) δ 8.61 (s, 1H), 8.46 (d, J = 2.3 Hz, 1H), 8.23 (d, J = 7.7 Hz, 1H), 7.91 (s, 1H), 7.73 (dd, J = 9.7, 8.6 Hz, 1H), 7.20 (dd, J = 11.1, 8.7 Hz, 1H), 6.87 (s, 1H), 5.52 (s, 2H), 2.79 (s, 3H), 2.18 (s, 3H), 2.12 (s, 3H), 1.84 (s, 3H).

### Compound 124-P2:

MS m/z (ESI): 591.1 [M+1]⁺. Supercritical fluid chromatography (SFC): retention time = 2.55 min, UV = 254 nm. ¹HNMR (400 MHz, CD₃OD) δ 8.61 (s, 1H), 8.47 (d, J = 2.3 Hz, 1H), 8.22 (d, J = 6.4 Hz, 1H), 8.00 - 7.85 (m, 1H), 7.73 (td, J = 9.6, 2.3 Hz, 1H), 7.21 (dd, J = 11.1, 8.6 Hz, 1H), 6.88 (s, 1H), 5.52 (d, J = 1.7 Hz, 2H), 2.80 (s, 3H), 2.18 (s, 3H), 2.13 (s, 3H), 1.84 (s, 3H).

### Compound 124-P3:

MS m/z (ESI): 591.1 [M+1]⁺. Supercritical fluid chromatography (SFC): retention time = 3.89 min, UV = 254 nm. ¹HNMR (400 MHz, CD₃OD) δ 8.80 - 8.55 (m, 1H), 8.54 - 8.38 (m, 1H), 8.32 - 8.16 (m, 1H), 8.04 - 7.83 (m, 1H), 7.85 - 7.63 (m, 1H), 7.32 - 7.11 (m, 1H), 6.96 - 6.81 (m, 1H), 5.51 (d, J = 1.8 Hz, 2H), 2.79 (s, 3H), 2.17 (s, 3H), 2.12 (s, 3H), 1.83 (s, 3H).

### Compound 124-P4:

MS m/z (ESI): 591.1 [M+1]⁺. Supercritical fluid chromatography (SFC): retention time = 6.38 min, UV = 254 nm. ¹HNMR (400 MHz, CD₃OD) δ 8.61 (s, 1H), 8.46 (d, J = 2.3 Hz, 1H), 8.35 - 8.18 (m, 1H), 7.99 - 7.87 (m, 1H), 7.73 (td, J = 9.6, 2.3 Hz, 1H), 7.20 (dd, J = 11.1, 8.6 Hz, 1H), 6.87 (s, 1H), 5.52 (d, J *=* 1.7 Hz, 2H), 2.79 (s, 3H), 2.18 (s, 3H), 2.12 (s, 3H), 1.84 (s, 3H).

### Example 60 Synthesis of Compounds 125, 125-P1, 125-P2, 125-P3, and 125-P4

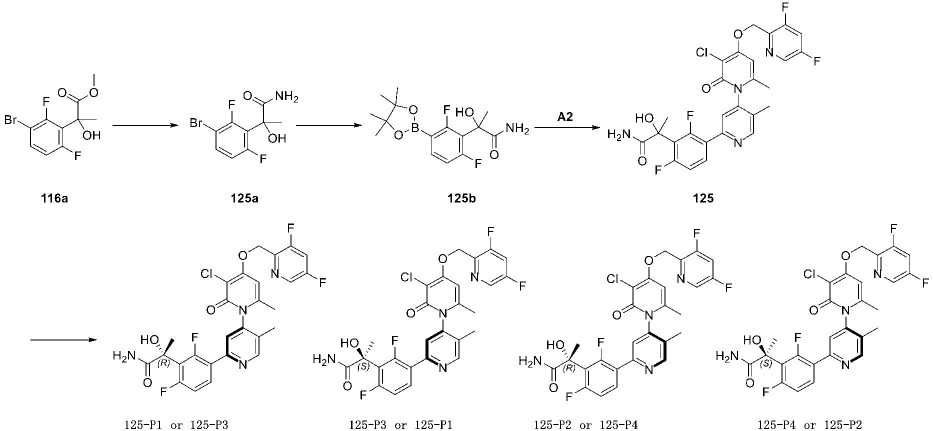

### Step 1: Synthesis of Compound 125a

Compound 116a (2.3 g, 7.8 mmol) was added to a solution of ammonia in ethanol (20 mL), and the reaction mixture was stirred at 80°C for 16 hours. After the reaction was completed, the reaction mixture was concentrated under reduced pressure. The residue was purified by silica gel column chromatography (petroleum ether/ethyl acetate = 1/0 to 10/1) to obtain compound 125a (1.5 g, yield: 61.54%). MS m/z (ESI):281.9 [M+1]⁺.

### Step 2: Synthesis of Compound 125b

1,1'-Bis(diphenylphosphino)ferrocene-palladium(II)dichloride dichloromethane complex (400 mg, 0.54 mmol) was added to a solution of compound 125a (1.5 g, 0.54 mmol), bis(pinacolato)diboron (2.06 g, 8.1 mmol), and potassium acetate (1.06 g, 10.8 mmol) in 1,4-dioxane (30 mL). The reaction mixture was stirred at 90°C for 16 hours. After the reaction was completed, the reaction mixture was filtered, and the filtrate was concentrated under reduced pressure to obtain compound 125b (1.5 g, crude product). MS m/z (ESI): 327.9 [M+1]⁺.

### Step 3: Synthesis of Compounds 125-P1, 125-P2, 125-P3, and 125-P4

Tetrakis(triphenylphosphine)palladium (529 mg, 0.45 mmol) was added to a mixed solution of compound 125b (1.5 g, 4.58 mmol), compound A2 (378 mg, 0.91 mmol), and sodium carbonate (972 mg, 9.17 mmol) in 1,4-dioxane and water (40 mL/4 mL). The reaction mixture was stirred at 90°C overnight. After the reaction was completed, the reaction mixture was concentrated under reduced pressure to remove the solvent, and the residue was purified by silica gel column chromatography (dichloromethane/methanol = 1/0 to 20/1) to obtain compound 125 (150 mg, yield: 28.6%). Compound 125 (150 mg) was then resolved by supercritical fluid chiral chromatography (equipment: SFC Thar prep 80, column: CHIRALPAK AD-H 250 mm × 20 mm, 5 µm, mobile phase: 40% MeOH (NH₄OH 0.2%): 60% CO₂, total flow rate: 40 g/min) to obtain compounds 125-P1 (31 mg, yield: 1.4%), 125-P2 (22.9 mg, yield: 1.2%), 125-P3 (31 mg, yield: 1.3%), and 125-P4 (36.6 mg, yield: 1.15%).

### Compound 125-P1:

MS m/z (ESI): 577.2 [M+1]⁺. Supercritical fluid chromatography (SFC): retention time = 4.12 min, UV = 214 nm. ¹HNMR (400 MHz, CD₃OD) δ 8.75 (s, 1H), 8.46 (d, *J* = 2.2 Hz, 1H), 7.89 (td, *J =* 8.6, 6.2 Hz, 1H), 7.73 (td, *J* = 9.6, 2.3 Hz, 1H), 7.65 (d, *J* = 1.7 Hz, 1H), 7.11 (t, *J* = 10.0 Hz, 1H), 6.81 (s, 1H), 5.50 (d, *J=* 1.3 Hz, 2H), 2.14 (s, 3H), 2.07 (s, 3H), 1.94 (d, *J* = 1.0 Hz, 3H).

### Compound 125-P2:

MS m/z (ESI): 577.2 [M+1]⁺. Supercritical fluid chromatography (SFC): retention time = 4.68 min, UV = 214 nm. ¹HNMR (400 MHz, CD₃OD) δ 8.75 (s, 1H), 8.46 (d, *J* = 2.3 Hz, 1H), 7.89 (td, *J =* 8.6, 6.1 Hz, 1H), 7.73 (td, *J* = 9.6, 2.3 Hz, 1H), 7.65 (d, *J= 1.8* Hz, 1H), 7.11 (t, *J= 9.6* Hz, 1H), 6.81 (s, 1H), 5.50 (d, *J=* 1.6 Hz, 2H), 2.14 (s, 3H), 2.07 (s, 3H), 1.94 (s, 3H).

### Compound 125-P3:

MS m/z (ESI): 577.2 [M+1]⁺. Supercritical fluid chromatography (SFC): retention time = 4.97 min, UV = 214 nm. ¹HNMR (400 MHz, CD₃OD) δ 8.75 (s, 1H), 8.46 (d, *J=* 2.3 Hz, 1H), 7.92 - 7.84 (m, 1H), 7.76 - 7.69 (m, 1H), 7.65 (d, *J=* 2.0 Hz, 1H), 7.14 - 7.04 (m, 1H), 6.81 (s, 1H), 5.50 (d, *J=* 1.7 Hz, 2H), 2.14 (s, 3H), 2.07 (s, 3H), 1.94 (s, 3H).

### Compound 125-P4:

MS m/z (ESI): 577.2 [M+1]⁺. Supercritical fluid chromatography (SFC): retention time = 5.78 min, UV = 214 nm. ¹HNMR (400 MHz, CD₃OD ) δ 8.75 (s, 1H), 8.46 (d, *J =* 2.3 Hz, 1H), 7.89 *(td, J=* 8.6, 6.1 Hz, 1H), 7.73 (td, *J* = 9.6, 2.3 Hz, 1H), 7.65 (d, *J=* 2.0 Hz, 1H), 7.14 - 7.06 (m, 1H), 6.81 (s, 1H), 5.50 (d, *J=* 1.6 Hz, 2H), 2.14 (s, 3H), 2.06 (s, 3H), 1.94 (dd, *J =* 4.0, 2.6 Hz, 3H).

### Example 61 Synthesis of Compounds 126, 126-P1, 126-P2, 126-P3, and 126-P4

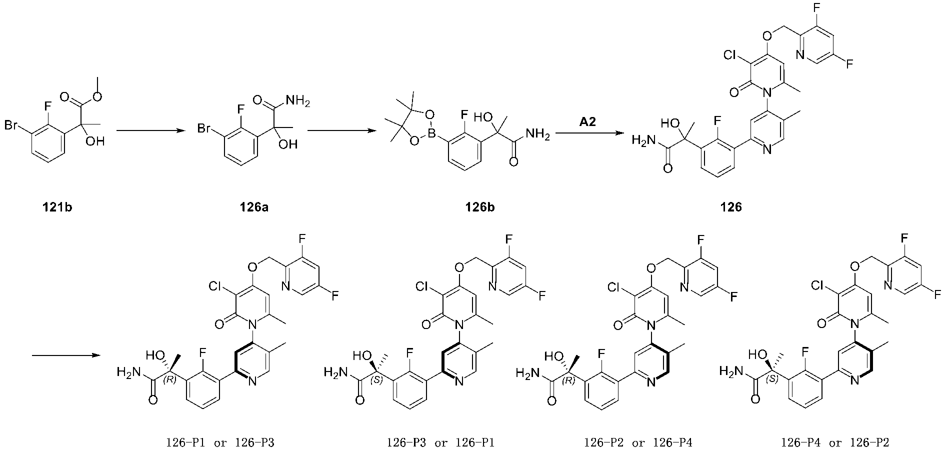

### Step 1: Synthesis of Compound 126a

Compound 121b (600 mg, 2.1 mmol) was added to a solution of ammonia in methanol (20 mL), and the reaction mixture was stirred in a sealed tube at 80°C for 16 hours. After the reaction was completed, the reaction mixture was concentrated under reduced pressure. The residue was purified by silica gel column chromatography (petroleum ether/ethyl acetate = 1/0 to 15/1) to obtain compound 126a (500 mg, yield: 75.3%). MS m/z (ESI): 264.0 [M+1]⁺.

### Step 2: Synthesis of Compound 126b

1,1'-Bis(diphenylphosphino)ferrocene-palladium(II)dichloride dichloromethane complex (55 mg, 0.07 mmol) was added to a solution of compound 126a (200 mg, 0.72 mmol), bis(pinacolato)diboron (290 mg, 1.1 mmol), and potassium acetate (149 mg, 1.5 mmol) in 1,4-dioxane (10 mL). The reaction mixture was stirred at 90°C for 16 hours. After the reaction was completed, the reaction mixture was filtered, and the filtrate was concentrated under reduced pressure to obtain compound 126b (250 mg, crude product). MS m/z (ESI): 309.9 [M+1]⁺.

### Step 4: Synthesis of compounds 126-P1, 126-P2, 126-P3, and 126-P4

Tetrakis(triphenylphosphine)palladium (93 mg, 0.08 mmol) was added to a mixed solution of compound 126b (250 mg, crude product), compound A2 (333 mg, 0.8 mmol), and sodium carbonate (171 mg, 1.6 mmol) in 1,4-dioxane and water (10 mL/1 mL). The reaction mixture was stirred at 80°C overnight. After the reaction was completed, the reaction mixture was concentrated under reduced pressure to remove the solvent. The residue was purified by silica gel column chromatography (dichloromethane/methanol = 1/0 to 10/1) to obtain compound 126 (200 mg). Compound 126 (200 mg) was then resolved by supercritical fluid chiral chromatography (equipment: SFC Thar prep 80, column: CHIRALPAK AD-H 250 mm × 20 mm, 5 µm, mobile phase: 40% EtOH (NH₄OH 0.2%): 60% CO₂, total flow rate: 40 g/min) to obtain white solid compounds 126-P1 (retention time: 3.61 minutes, 30 mg, yield: 15%) and 126-P4 (retention time: 5.71 minutes, 30 mg, yield: 15%), as well as a mixture of 126-P2 and 126-P3. The mixture of P2 and P3 was further resolved by supercritical fluid chiral chromatography (equipment: SFC Thar prep 80, column: CHIRALPAK OJ-H 250 mm × 20 mm, 5 µm, mobile phase: 40% IPA (NH₄OH 0.2%): 60% CO₂, total flow rate: 40 g/min) to obtain white solid compounds 126-P2 (retention time: 2.27 minutes, 30 mg, yield: 15%) and 126-P3 (retention time: 3.16 minutes, 30 mg, yield: 15%).

### Compound 126-P1:

MS m/z (ESI): 559.2 [M+1]⁺. Supercritical fluid chromatography (SFC): retention time = 3.61 min, UV = 254 nm. ¹H NMR (400 MHz, CD₃OD) δ 8.77 (s, 1H), 8.48 *(d, J= 2.3* Hz, 1H), 7.86 (dd, *J=* 10.4, 4.5 Hz, 1H), 7.73 (ddd, J= 14.7, 8.9, 2.1 Hz, 3H), 7.34 (t, *J =* 7.8 Hz, 1H), 6.83 (s, 1H), 5.52 (d, *J=* 1.8 Hz, 2H), 2.16 (s, 3H), 2.10 (s, 3H), 1.86 (s, 3H).

### Compound 126-P2:

MS m/z (ESI): 559.2 [M+1]⁺. Supercritical fluid chromatography (SFC): retention time = 2.27 min, UV = 254 nm. ¹H NMR (400 MHz, CD₃OD) δ 8.77 (s, 1H), 8.48 (d, *J= 2.3* Hz, 1H), 7.87 (td, *J=* 7.6, 1.6 Hz, 1H), 7.78 - 7.66 (m, 3H), 7.34 (t, *J= 7.8* Hz, 1H), 6.83 (s, 1H), 5.52 *(d, J=* 1.8 Hz, 2H), 2.16 (s, 3H), 2.10 (s, 3H), 1.86 (s, 3H).

### Compound 126-P3:

MS m/z (ESI): 559.2 [M+1]⁺. Supercritical fluid chromatography (SFC): retention time = 3.16 min, UV = 254 nm. ¹H NMR (400 MHz, CD₃OD) δ 8.77 (s, 1H), 8.48 (d, *J=* 2.3 Hz, 1H), 7.88 (td, *J* = 7.6, 1.6 Hz, 1H), 7.70 (d, *J =* 2.1 Hz, 3H), 7.35 (s, 1H), 6.83 (s, 1H), 5.52 (d, *J=* 1.8 Hz, 2H), 2.16 (s, 3H), 2.09 (s, 3H), 1.86 (s, 3H).

### Compound 126-P4:

MS m/z (ESI): 559.2 [M+1]⁺. Supercritical fluid chromatography (SFC): retention time = 5.71 min, UV = 254 nm. ¹H NMR (400 MHz, CD₃OD) δ 8.77 (s, 1H), 8.48 (d, *J= 2.3* Hz, 1H), 7.88 (td, *J=* 7.6, 1.6 Hz, 1H), 7.77 - 7.68 (m, 3H), 7.35 (t, *J= 7.8* Hz, 1H), 6.83 (s, 1H), 5.52 *(d, J=* 1.8 Hz, 2H), 2.16 (s, 3H), 2.09 (s, 3H), 1.86 (s, 3H).

### Example 62 Synthesis of Compounds 128, 128-P1, 128-P2, 128-P3, and 128-P4

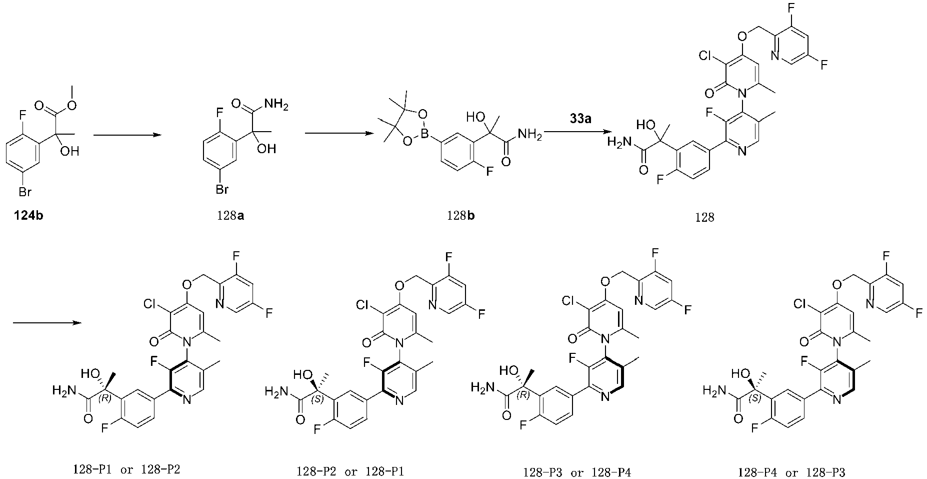

### Step 1: Synthesis of Compound 128a

Compound 124b (2.5 g, 9 mmol) was added to a solution of ammonia in methanol (20 mL), and the reaction mixture was stirred at 80°C for 16 hours. After the reaction was completed, the reaction mixture was concentrated under reduced pressure. The residue was purified by silica gel column chromatography (petroleum ether/ethyl acetate = 1/0 to 10/1) to obtain compound 128a (1.5 g, yield: 52.87%). MS m/z (ESI): 261.9 [M+1]⁺.

### Step 2: Synthesis of Compound 128b

1,1'-Bis(diphenylphosphino)ferrocene-palladium(II)dichloride dichloromethane complex (138 mg, 0.19 mmol) was added to a solution of compound 128a (500 mg, 1.91 mmol), bis(pinacolato)diboron (532.9 mg, 2.10 mmol), and potassium acetate (374.5 mg, 3.82 mmol) in 1,4-dioxane (30 mL). The reaction mixture was stirred at 90°C for 16 hours. After the reaction was completed, the reaction mixture was filtered, and the filtrate was concentrated under reduced pressure to obtain compound 128b (500 mg, crude product). MS m/z (ESI): 310.1 [M+1]⁺.

### Step 3: Synthesis of Compounds 128-P1, 128-P2, 128-P3, and 128-P4

Tetrakis(triphenylphosphine)palladium (186.9 mg, 0.16 mmol) was added to a mixed solution of compound 128b (500 mg, crude product), compound 33a (348 mg, 0.81 mmol), and sodium carbonate (343 mg, 3.24 mmol) in 1,4-dioxane and water (30 mL/3 mL). The reaction mixture was stirred at 90°C overnight. After the reaction was completed, the reaction mixture was concentrated under reduced pressure to remove the solvent. The residue was purified by silica gel column chromatography (dichloromethane/methanol = 1/0 to 20/1) to obtain compound 128 (150 mg). Compound 128 (150 mg) was then resolved by supercritical fluid chiral chromatography (equipment: SFC Thar prep 80, column: CHIRALPAK AD-H 250 mm × 20 mm, 5 µm, mobile phase: 40% MeOH (NH₄OH 0.2%): 60% CO₂, total flow rate: 40 g/min) to obtain compounds 128-P1 (20.3 mg, yield: 2.18%), 128-P2 (33.9 mg, yield: 3.63%), 128-P3 (35.2 mg, yield: 3.77%), and 128-P4 (23.9 mg, yield: 2.56%).

### Compound 128-P1:

MS m/z (ESI): 577.1 [M+1]⁺. Supercritical fluid chromatography (SFC): retention time = 3.56 min, UV = 254 nm. ¹HNMR (400 MHz CD₃OD) δ 8.61 (s, 1H), 8.46 (d, J = 2.3 Hz, 1H), 8.23 (d, J = 7.8 Hz, 1H), 8.01 - 7.86 (m, 1H), 7.81 - 7.63 (m, 1H), 7.22 (dd, J = 11.0, 8.6 Hz, 1H), 6.88 (s, 1H), 5.52 (d, J = 1.5 Hz, 2H), 2.17 (s, 3H), 2.12 (s, 3H), 1.85 (s, 3H).

### Compound 128-P2:

MS m/z (ESI): 577.1 [M+1]⁺. Supercritical fluid chromatography (SFC): retention time = 4.21 min, UV = 254 nm. ¹H NMR (400 MHz, CD₃OD) δ 8.61 (s, 1H), 8.45 (d, J = 2.3 Hz, 1H), 8.23 (d, J = 5.9 Hz, 1H), 7.90 (dd, J = 7.6, 3.3 Hz, 1H), 7.81 - 7.64 (m, 1H), 7.21 (dd, J = 11.1, 8.6 Hz, 1H), 6.86 (s, 1H), 5.51 (d, J = 1.7 Hz, 2H), 2.17 (s, 3H), 2.11 (s, 3H), 1.85 (s, 3H).

### Compound 128-P3:

MS m/z (ESI): 577.1 [M+1]⁺. Supercritical fluid chromatography (SFC): retention time = 6.58 min, UV = 254 nm. ¹HNMR (400 MHz, CD₃OD ) δ 8.61 (s, 1H), 8.46 (d, J = 2.3 Hz, 1H), 8.23 (dd, J = 7.5, 1.7 Hz, 1H), 7.92 (ddd, J = 8.5, 4.4, 2.1 Hz, 1H), 7.88 - 7.61 (m, 1H), 7.21 (dd, J = 11.1, 8.6 Hz, 1H), 6.87 (s, 1H), 5.52 (d, J = 1.8 Hz, 2H), 2.18 (s, 3H), 2.12 (s, 3H), 1.86 (s, 3H).

### Compound 128-P4:

MS m/z (ESI): 577.1 [M+1]⁺. Supercritical fluid chromatography (SFC): retention time = 7.61 min, UV = 254 nm. ¹HNMR (400 MHz, CD₃OD ) δ 8.61 (s, 1H), 8.45 (d, J = 2.3 Hz, 1H), 8.23 (d, J = 6.8 Hz, 1H), 7.92 (d, J = 4.1 Hz, 1H), 7.76 - 7.69 (m, 1H), 7.21 (dd, J= 11.0, 8.6 Hz, 1H), 6.87 (s, 1H), 5.51 (d, J = 1.5 Hz, 2H), 2.17 (s, 3H), 2.12 (s, 3H), 1.85 (s, 3H).

### Example 63 Synthesis of Compound 129

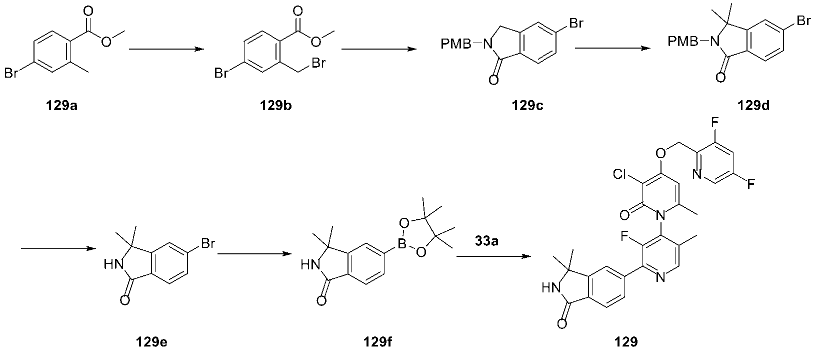

### Step 1: Synthesis of Compound 129b

Compound 129a (2.0 g, 8.8 mmol), *N*-bromosuccinimide (1.6 g, 8.8 mmol), and azobisisobutyronitrile (722 mg, 4.4 mmol) were sequentially added to a solution of carbon tetrachloride (20 mL). The reaction mixture was stirred at 80°C for 3 hours. After the reaction was completed, the reaction mixture was diluted with water (50 mL) and extracted with dichloromethane (50 mL × 2). The combined organic phases were washed with saturated brine (50 mL × 1), dried over anhydrous sodium sulfate, and filtered. The filtrate was concentrated under reduced pressure to obtain compound 129b (2.2 g, yield: 81.4%). MS m/z (ESI): 308.00 [M+1]⁺.

### Step 2: Synthesis of Compound 129c

129b (1.8 g, 5.9 mmol) and 4-methoxybenzylamine (890 mg, 6.5 mmol) were added to tetrahydrofuran (20 mL), and the reaction mixture was stirred at 80°C for 2 hours. After the reaction was completed, the reaction mixture was concentrated under reduced pressure, stirred with ethyl acetate (10 mL) at room temperature for 0.5 hours, filtered, and the filter cake was dried to obtain crude product 129c. The crude product was added with water (10 mL), stirred at room temperature for 0.5 hours, filtered, and the filter cake was dried to obtain compound 129c (700 mg, yield: 35.8%). MS m/z (ESI): 332.00 [M+1]⁺.

### Step 3: Synthesis of Compound 129d

Compound 129c (500 mg, 1.5 mmol) was added to tetrahydrofuran (10 mL), and sodium hydride (150 mg, 3.8 mmol) was added in batches at 0°C. The mixture was stirred at 0°C for 0.5 hours, and then iodomethane (640 mg, 4.5 mmol) was added. The reaction mixture was stirred at 70°C overnight. After the reaction was completed, the reaction mixture was quenched with saturated ammonium chloride solution (10 mL), diluted with water (20 mL), and extracted with ethyl acetate (20 mL × 2). The combined organic phases were washed with saturated brine (20 mL × 1), dried over anhydrous sodium sulfate, and filtered. The filtrate was concentrated under reduced pressure, and the residue was purified by silica gel column chromatography (petroleum ether/ethyl acetate = 2/1) to obtain compound 129d (400 mg, yield: 72.3%). MS m/z (ESI): 360.00 [M+1]⁺.

### Step 4: Synthesis of Compound 129e

Compound 129d (400 mg, 1.1 mmol) was added to a mixed solution of acetonitrile (8 mL) and water (2 mL). Ceric ammonium nitrate (1.84 g, 3.3 mmol) was added at 0°C, and the reaction mixture was stirred at 0°C for 1 hour. After the reaction was completed, the reaction mixture was diluted with water (20 mL) and extracted with ethyl acetate (20 mL × 2). The combined organic phases were washed with saturated brine (20 mL × 1), dried over anhydrous sodium sulfate, and filtered. The filtrate was concentrated under reduced pressure, and the residue was subjected to silica gel column chromatography (petroleum ether/ethyl acetate = 1/1) to obtain compound 129e (220 mg, yield: 91.7%). MS m/z (ESI): 240.00 [M+1]⁺.

### Step 5: Synthesis of Compound 129f

1,1'-Bis(diphenylphosphino)ferrocene-palladium(II)dichloride dichloromethane complex (82 mg, 0.1 mmol) was added to a solution of compound 129e (220 mg, 0.9 mmol), bis(pinacolato)diboron (303 mg, 1.2 mmol), and potassium acetate (225 mg, 2.3 mmol) in 1,4-dioxane (8 mL). The reaction mixture was stirred at 90°C for 0.5 hours. After the reaction was completed, the reaction mixture was directly concentrated under reduced pressure, and the residue was subjected to silica gel column chromatography (petroleum ether/ethyl acetate = 2/1) to obtain compound 129f (190 mg, yield: 73.6%). MS m/z (ESI): 288.10 [M+1]⁺.

### Step 6: Synthesis of Compound 129

129f (190 mg, 0.66 mmol), compound 33a (290 mg, 0.68 mmol), sodium carbonate (140 mg, 1.6 mmol), and tetrakis(triphenylphosphine)palladium (81 mg, 0.07 mmol) were sequentially added to a mixed solution of 1,4-dioxane (4 mL) and water (0.4 mL). The reaction mixture was stirred at 90°C for 3 hours. After the reaction was completed, the reaction mixture was concentrated under reduced pressure. The residue was purified by silica gel column chromatography (petroleum ether/ethyl acetate = 1/2 to dichloromethane/methanol = 30/1) to obtain crude product 129. The crude product was further purified by preparative HPLC (chromatographic column: YMC-Triart C18 30 × 250 mm, 10 µm; mobile phase: A: 0.1% ammonia water-water, B: acetonitrile; flow rate: 40 mL/min) to obtain compound 129 (48.74 mg, yield: 13.3%). MS m/z (ESI): 555.10 [M+1]⁺. ¹H NMR (400 MHz, DMSO-d₆) δ 8.76 (s, 1H), 8.72 (s, 1H), 8.59 (d, *J* = 2.4 Hz, 1H), 8.13 - 8.04 (m, 2H), 7.99 *(d, J=* 8.0 Hz, 1H), 7.74 (d, *J* = 8.0 Hz, 1H), 6.88 (s, 1H), 5.51 (s, 2H), 2.13 (s, 3H), 2.07 (s, 3H), 1.48 (s, 6H).

### Example 64 Synthesis of Compound 130

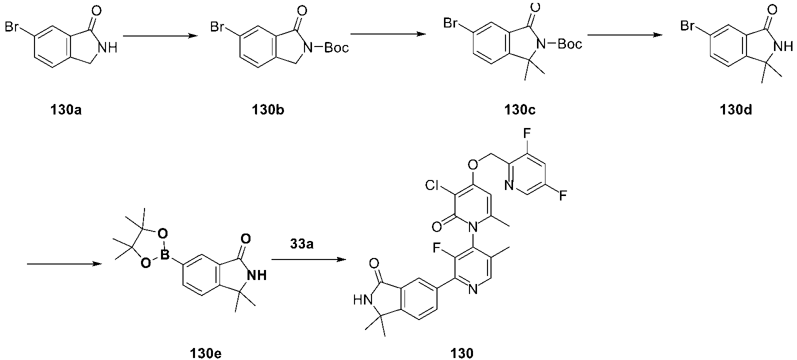

### Step 1: Synthesis of Compound 130b

4-Dimethylaminopyridine (260 mg, 2.13 mmol), triethylamine (2.58 g, 25.6 mmol), and di-tert-butyl dicarbonate (5.58 g, 25.6 mmol) were sequentially added to a solution of compound 130a (4.5 g, 21.3 mmol) in dichloromethane (60 mL). The reaction mixture was stirred at room temperature for 5 hours. After the reaction was completed, the reaction mixture was diluted with water (100 mL) and extracted with dichloromethane (40 mL × 2). The combined organic phases were washed with saturated brine (100 mL × 2), dried over anhydrous sodium sulfate, and filtered. The filtrate was concentrated under reduced pressure. The residue was purified by silica gel column chromatography (petroleum ether/ethyl acetate = 4/1) to obtain compound 130b (3.0 g, yield: 45.2%) as a white solid. MS m/z (ESI): 257.9 [M+1]⁺.

### Step 2: Synthesis of Compound 130c

At -78°C, a solution of sodium bis(trimethylsilyl)amide in tetrahydrofuran (2 M, 7.2 mL, 14.4 mmol) was added dropwise to a solution of compound 130b (3.0 g, 9.63 mmol) in tetrahydrofuran (30 mL). The mixture was stirred at -78°C for 1 hour, followed by the addition of iodomethane (2.73 g, 19.26 mmol). The reaction mixture was stirred at room temperature for 1 hour, then cooled back to -78°C. A solution of sodium bis(trimethylsilyl)amide in tetrahydrofuran (2 M, 7.2 mL, 14.4 mmol) was added dropwise, and the mixture was stirred at -78°C for 1 hour. Iodomethane (2.73 g, 19.26 mmol) was added, and the reaction mixture was stirred at room temperature for another 1 hour. After the reaction was completed, the reaction mixture was quenched with saturated aqueous ammonium chloride solution (10 mL), diluted with water (60 mL), and extracted with ethyl acetate (60 mL × 3). The combined organic phases were washed with saturated brine (80 mL × 2), dried over anhydrous sodium sulfate, and filtered. The filtrate was concentrated under reduced pressure, and the residue was separated by silica gel column chromatography (petroleum ether/ethyl acetate = 5/1) to obtain compound 130c (2.1 g, yield: 64.3%) as a pink solid. MS m/z (ESI): 339.9 [M+1]⁺.

### Step 3: Synthesis of Compound 130d

Trifluoroacetic acid (1.81 g, 15.9 mmol) was added to a solution of compound 130c (1.2 g, 3.53 mmol) in dichloromethane (12 mL). The reaction mixture was stirred at room temperature for 1 hour. After the reaction was completed, the reaction mixture was diluted with water (20 mL), adjusted to pH = 9 with saturated sodium carbonate solution, and extracted with dichloromethane (20 mL × 2). The combined organic phases were washed with saturated brine (15 mL × 2), dried over anhydrous sodium sulfate, and filtered. The filtrate was concentrated under reduced pressure, and the residue was purified by silica gel column chromatography (petroleum ether/ethyl acetate = 1/1) to obtain compound 130d (810 mg, yield: 96.0%). MS m/z (ESI): 240.1 [M+1]⁺.

### Step 4: Synthesis of Compound 130e

1,1'-Bis(diphenylphosphino)ferrocene-palladium(II)dichloride dichloromethane complex (169 mg, 0.2 mmol) was added to a solution of compound 130d (500 mg, 2.09 mmol), bis(pinacolato)diboron (1.59 g, 6.27 mmol), and potassium acetate (410 mg, 4.18 mmol) in 1,4-dioxane (10 mL). The reaction mixture was stirred at 90°C for 2 hours. After the reaction was completed, the reaction mixture was directly concentrated under reduced pressure. The residue was subjected to silica gel column chromatography (petroleum ether/ethyl acetate = 1/1 to 1/2) to obtain compound 130e (700 mg, crude product). MS m/z (ESI): 288.10 [M+1]⁺.

### Step 5: Synthesis of Compound 130

130e (200 mg, 0.69 mmol), compound 33a (320 mg, 0.76 mmol), sodium carbonate (144 mg, 1.67 mmol), and tetrakis(triphenylphosphine)palladium (92 mg, 0.07 mmol) were sequentially added to a mixed solution of 1,4-dioxane (6 mL) and water (1 mL). The reaction mixture was stirred at 90°C overnight. After the reaction was completed, the reaction mixture was concentrated under reduced pressure. The residue was purified by silica gel column chromatography (petroleum ether/ethyl acetate = 1/2 to dichloromethane/methanol = 30/1) to obtain crude product 130. The crude product was further purified by preparative HPLC (chromatographic column: YMC-Triart C18 30 × 250 mm, 10 µm; mobile phase: A: 0.1% ammonia water-water, B: acetonitrile; flow rate: 40 mL/min) to obtain compound 130 (51.34 mg, yield: 13.4%). MS m/z (ESI): 555.10 [M+1]⁺. ¹H NMR (400 MHz,DMSO-*d₆*) δ 8.77 (s, 1H), 8.72 (s, 1H), 8.60 (d, *J* = 2.4 Hz, 1H), 8.16 (d, *J* = 8.0 Hz, 1H), 8.13 - 8.06 (m, 2H), 7.79 (d, *J =* 8.0 Hz, 1H), 6.89 (s, 1H), 5.52 (s, 2H), 2.13 (s, 3H), 2.08 (s, 3H), 1.49 (s, 6H).

### Example 65 Synthesis of Compound 131

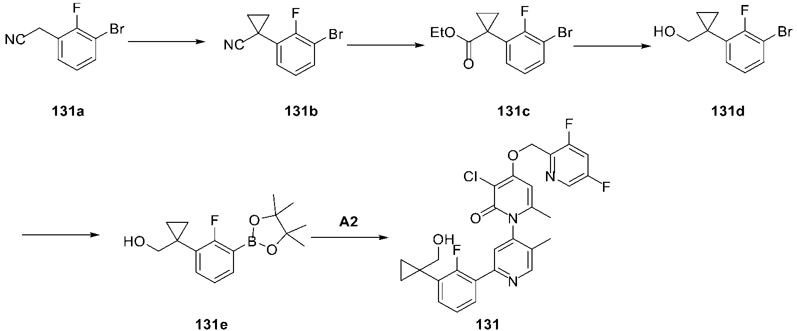

### Step 1: Synthesis of Compound 131b

At 0°C, sodium hydride (2.10 g, 52.6 mmol) was added to a solution of compound 131a (4.50 g, 21.0 mmol) and 1,2-dibromoethane (5.10 g, 27.3 mmol) in tetrahydrofuran (45 mL) in three batches. The reaction mixture was stirred at room temperature for 16 hours. After the reaction was completed, the reaction mixture was quenched by dropwise addition of isopropanol (6 mL), diluted with water (40 mL), and extracted with ethyl acetate (40 mL × 2). The combined organic phases were washed with saturated brine (40 mL × 1), dried over anhydrous sodium sulfate, and filtered. The filtrate was concentrated under reduced pressure, and the residue was subjected to silica gel column chromatography (petroleum ether/ethyl acetate = 20/1 to 10/1) to obtain compound 131b (4.8 g, yield: 95.2%). MS m/z (ESI): 241.95 [M+1]⁺.

### Step 2: Synthesis of Compound 131c

Compound 131b (4.80 g, 20.0 mmol) was dissolved in ethanol (80 mL), and concentrated sulfuric acid (12 mL) was added under ice bath conditions. The reaction mixture was stirred at 90°C for 48 hours. After the reaction was completed, the reaction mixture was concentrated under reduced pressure. The residue was added to ice water (50 mL) and extracted with ethyl acetate (50 mL × 2). The combined organic phases were washed with saturated aqueous sodium chloride solution (50 mL × 1), dried over anhydrous sodium sulfate, and filtered. The filtrate was concentrated under reduced pressure, and the residue was purified by silica gel column chromatography (petroleum ether/ethyl acetate = 20/1 to 10/1) to obtain compound 131c (4.4 g, yield: 76.9%). MS m/z (ESI): 286.95 [M+1]⁺.

### Step 3: Synthesis of Compound 131d

At -75°C, DIBAL-H (20 mL, 20.2 mmol, 1 M) was added dropwise to a solution of compound 131c (2.90 g, 10.1 mmol) in dichloromethane (30 mL), and the reaction mixture was stirred at -75°C for 2 hours. After the reaction was completed, the reaction mixture was quenched with water (6 mL), followed by the addition of anhydrous sodium sulfate and stirring for 5 minutes. The mixture was then filtered, and the filtrate was concentrated under reduced pressure. The resulting residue was purified by silica gel column chromatography (petroleum ether/ethyl acetate = 10/1-5/1) to obtain compound 131d (2.4 g, yield: 96.9%). MS m/z (ESI): 228.05 [M-17]⁺.

### Step 4: Synthesis of Compound 131e

1,1'-Bis(diphenylphosphino)ferrocene-palladium(II)dichloride dichloromethane complex (326 mg, 0.45 mmol) was added to a solution of compound 131d (1.10 g, 4.5 mmol), bis(pinacolato)diboron (3.42 g, 13.5 mmol), and potassium acetate (880 mg, 8.9 mmol) in 1,4-dioxane (20 mL). The reaction mixture was stirred at 90°C for 3 hours. After the reaction was completed, the reaction mixture was directly concentrated under reduced pressure. The residue was subjected to silica gel column chromatography (petroleum ether/ethyl acetate = 20/1 to 5/1) to obtain compound 131e (2 g, crude product). MS m/z (ESI): 275.10 [M-17]⁺.

### Step 5: Synthesis of Compound 131

Tetrakis(triphenylphosphine)palladium (84 mg, 0.07 mmol) was added to a mixed solution of compound 131e (300 mg, 0.73 mmol), compound A2 (426 mg, 1.46 mmol), and sodium carbonate (155 mg, 1.46 mmol) in 1,4-dioxane and water (5 mL/0.5 mL). The reaction mixture was stirred at 90°C for 16 hours. After the reaction was completed, the reaction mixture was concentrated under reduced pressure, and the resulting residue was purified by silica gel column chromatography (petroleum ether/ethyl acetate = 1/1 to 1/4) to obtain a crude product of compound 131, and the crude product was further purified by preparative HPLC (chromatographic column: YMC-Triart C1830 × 250 mm, 10 µm, mobile phase: A: 0.1% ammonia water-water, B: acetonitrile, flow rate: 40 mL/min) to obtain compound 131 (165.4 mg, yield: 41.9%). MS m/z (ESI): 542.05 [M+1]⁺. ¹H NMR (400 MHz, DMSO-d₆) δ 8.79 (s, 1H), 8.60 (d, *J=* 2.3 Hz, 1H), 8.16 - 8.05 (m, 1H), 7.79 (td, *J=* 7.6, 1.6 Hz, 1H), 7.73 (s, 1H), 7.42 (dd, *J=* 10.1, 4.3 Hz, 1H), 7.25 (t, *J =* 7.6 Hz, 1H), 6.80 (s, 1H), 5.48 (s, 2H), 4.67 (t, *J=* 5.9 Hz, 1H), 3.48 (d, *J= 5.9* Hz, 2H), 2.06 (s, 3H), 1.98 (s, 3H), 0.85 (t, *J* = 4.6 Hz, 2H), 0.74 (t, *J =* 4.4 Hz, 2H).

### Example 66 Synthesis of Compound 132

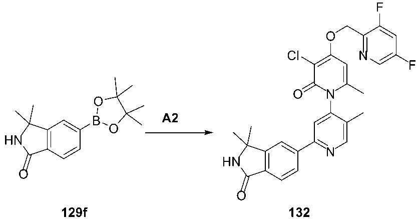

Tetrakis(triphenylphosphine)palladium (81.3 mg, 0.07 mmol) was added to a mixed solution of compound 129f (290 mg, 0.703 mmol), compound A2 (303 mg, 1.06 mmol), and sodium carbonate (149 mg, 1.41 mmol) in 1,4-dioxane and water (3 mL/0.3 mL). The reaction mixture was stirred at 90°C for 2 hours. After the reaction was completed, the reaction mixture was concentrated under reduced pressure, and the residue was purified by silica gel column chromatography (dichloromethane/methanol = 1/0 to 20/1) to obtain a crude product of compound 132. The crude product was further purified by preparative HPLC (chromatographic column: YMC-Triart C18 30 × 250 mm, 10 µm, mobile phase: A: 0.1% ammonia water-water, B: acetonitrile, flow rate: 40 mL/min) to obtain compound 132 (231.6 mg, yield: 79.9%). MS m/z (ESI): 537.05 [M+1]⁺. ¹H NMR (400 MHz, DMSO-d₆) δ 8.79 (s, 1H), 8.71 (s, 1H), 8.60 (s, 1H), 8.34 (s, 1H), 8.25 (d, *J*= 8.0 Hz, 1H), 8.14 - 8.07 (m, 2H), 7.69 (d, *J* = 8.0 Hz, 1H), 6.83 (s, 1H), 5.50 (s, 2H), 2.06 (s, 3H), 2.01 (s, 3H), 1.50 (s, 6H).

### Example 67 Synthesis of Compound 133

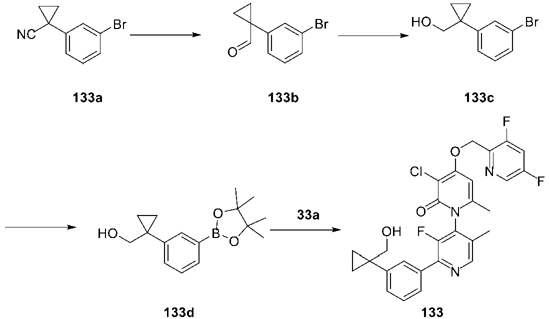

### Step 1: Synthesis of Compound 133b

At -78°C, a solution of diisobutylaluminum hydride in n-hexane (1.0 M, 14.4 mL, 14.4 mmol) was added to a solution of compound 133a (1.5 g, 6.78 mmol) in dichloromethane (25 mL). The reaction mixture was stirred at -78°C for 2 hours. After the reaction was completed, the reaction mixture was quenched with water (1.0 mL), and a small amount of anhydrous sodium sulfate was added. The temperature was gradually raised to room temperature, and the mixture was stirred for another 20 minutes. The mixture was filtered through diatomaceous earth to remove insoluble solids, and the filtrate was added with 40 mL of water and extracted with dichloromethane (20 mL × 2). The combined organic phases were washed with saturated brine (40 mL × 2), dried over anhydrous sodium sulfate, and filtered. The filtrate was concentrated under reduced pressure, and the residue was purified by silica gel column chromatography (petroleum ether/ethyl acetate = 10/1) to obtain compound 133b (1.3 g, yield: 85.6%). MS m/z (ESI): 227.00 [M+1]⁺.

### Step 2: Synthesis of Compound 133c

At 0°C, sodium borohydride (330 mg, 8.7 mmol) was added to a solution of compound 133b (1.3 g, 5.8 mmol) in methanol (20 mL) in batches. The reaction mixture was warmed to room temperature and stirred for 1 hour. After the reaction was completed, the reaction mixture was quenched with saturated aqueous ammonium chloride solution (10 mL), and the methanol was removed by concentrating under reduced pressure. The residue was diluted with water (30 mL) and extracted with ethyl acetate (15 mL × 3). The combined organic phases were washed with saturated brine (30 mL × 2), dried over anhydrous sodium sulfate, and filtered. The filtrate was concentrated under reduced pressure, and the residue was purified by silica gel column chromatography (petroleum ether/ethyl acetate = 10/1 to 5/1) to obtain compound 133c (1.0 g, yield: 76.3%). MS m/z (ESI): 211.00 [M+1]⁺.

### Step 3: Synthesis of Compound 133d

1,1'-Bis(diphenylphosphino)ferrocene-palladium(II)dichloride dichloromethane complex (179 mg, 0.22 mmol) was added to a solution of compound 133c (500 mg, 2.21 mmol), bis(pinacolato)diboron (1.68 g, 6.63 mmol), and potassium acetate (433 mg, 4.42 mmol) in 1,4-dioxane (10 mL). The reaction mixture was stirred at 90°C for 2 hours. After the reaction was completed, the reaction mixture was directly concentrated under reduced pressure. The residue was subjected to silica gel column chromatography (petroleum ether/ethyl acetate = 10/1 to 3/1) to obtain compound 133d (730 mg, crude product, containing a small amount of bis(pinacolato)diboron). MS m/z (ESI): 257.10 [M-17]⁺.

### Step 4: Synthesis of Compound 133

Tetrakis(triphenylphosphine)palladium (124.5 mg, 0.109 mmol) was added to a mixed solution of compound 133d (300 mg, 1.09 mmol), compound 33a (516 mg, 1.2 mmol), and sodium carbonate (235.2 mg, 2.73 mmol) in 1,4-dioxane and water (8 mL/1 mL). The reaction mixture was stirred at 90°C overnight. After the reaction was completed, the reaction mixture was concentrated under reduced pressure. The resulting residue was purified by silica gel column chromatography (petroleum ether/ethyl acetate = 1/2 to 0/1) to obtain crude product 133. The crude product was further purified by preparative HPLC (preparative column: YMC Triart C18 12 nm 10u, 30 × 250 mm; flow rate: 40 mL/min; mobile phase: A: water (0.1% ammonia water) B: preparative grade acetonitrile) to obtain compound 133 (176.52 mg, yield: 29.9%). MS m/z (ESI): = 542.05 [M+H]⁺. ¹H NMR (400 MHz,DMSO-*d₆*) δ 8.68 (s, 1H), 8.60 (d, *J =* 2.4 Hz, 1H), 8.13 - 8.06 (m, 1H), 7.88 (s, 1H), 7.73 (m, 1H), 7.46 - 7.40 (m, 2H), 6.88 (s, 1H), 5.52 (s, 2H), 4.70 (m,1H), 3.55 (d, *J =* 5.6 Hz, 2H), 2.11 (s, 3H), 2.06 (s, 3H), 0.87 (m, 2H), 0.78 (m, 2H).

### Example 68 Synthesis of Compound 134

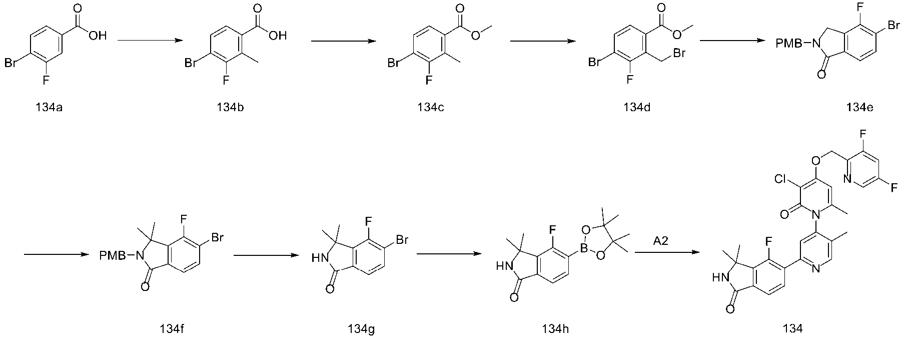

### Step 1: Synthesis of Compound 134b

At -70°C, LDA (48 mL, 95.89 mmol, 2 M) was slowly added dropwise to a solution of compound 134a (10.0 g, 45.66 mmol) in tetrahydrofuran (100 mL). The mixture was stirred at -70°C for 1 hour, followed by the addition of iodomethane (19.5 g, 136.98 mmol). The reaction mixture was gradually warmed to room temperature and stirred for 16 hours. After the reaction was completed, the reaction mixture was quenched with saturated aqueous ammonium chloride solution (200 mL) and extracted with ethyl acetate (200 mL × 2). The combined organic phases were washed with saturated brine (200 mL × 1), dried over anhydrous sodium sulfate, and filtered. The filtrate was concentrated under reduced pressure to obtain compound 134b (10.6 g, crude product). ¹H NMR (400 MHz, DMSO-d₆) δ 7.51 - 7.42 (m, 1H), 7.37 *(d, J=* 8.4 Hz, 1H), 2.41 (d, J= 2.7 Hz, 3H).

### Step 2: Synthesis of Compound 134c

Thionyl chloride (20 mL, 273.97 mmol) was added dropwise to a solution of compound 134b (10.6 g, 45.66 mmol) in methanol (100 mL) under an ice bath, and the reaction mixture was stirred at room temperature for 16 hours. After the reaction was completed, the reaction mixture was concentrated under reduced pressure. The residue was diluted with saturated aqueous sodium bicarbonate solution (100 mL) and extracted with ethyl acetate (100 mL × 3). The combined organic phases were dried over anhydrous sodium sulfate and filtered. The filtrate was concentrated under reduced pressure, and the residue was purified by silica gel column chromatography (petroleum ether/ethyl acetate = 20/1 to 10/1) to obtain compound 134c (10.0 g, crude product). ¹H NMR (400 MHz, DMSO-d₆) δ 7.67 - 7.60 (m, 1H), 7.54 (dd, *J=* 8.5, 1.0 Hz, 1H), 3.84 (s, 3H), 2.43 (d, *J=* 2.7 Hz, 3H).

### Step 3: Synthesis of Compound 134d

Azobisisobutyronitrile (3.3 g, 20.24 mmol) was added to a solution of compound 134c (10.0 g, 40.48 mmol) and N-bromosuccinimide (7.2 g, 40.48 mmol) in carbon tetrachloride (100 mL). The reaction mixture was stirred at 80°C for 7 hours. After the reaction was completed, the reaction mixture was diluted with water (100 mL) and extracted with dichloromethane (100 mL × 2). The combined organic phases were washed with saturated brine (100 mL × 1), dried over anhydrous sodium sulfate, and filtered. The filtrate was concentrated under reduced pressure to obtain compound 134d (6 g, crude product), which was directly used in the next step.

### Step 4: Synthesis of Compound 134e

A solution of 134d (6.0 g, crude product) and 4-methoxybenzylamine (2.5 g, 18.52 mmol) in tetrahydrofuran (60 mL) was stirred at 80°C for 3 hours. After the reaction was completed, the reaction mixture was concentrated under reduced pressure, and the residue was purified by silica gel column chromatography (petroleum ether/ethyl acetate = 10/1 to 3/1) to obtain compound 134e (2.7 g). MS m/z (ESI): [M+H]⁺ = 349.95.

### Step 5: Synthesis of Compound 134f

At -78°C, sodium bis(trimethylsilyl)amide (5 mL, 10.0 mmol, 2 M in THF) was added dropwise to a solution of compound 134e (2.2 g, 6.30 mmol) in tetrahydrofuran (20 mL). The mixture was stirred at -78°C for 1 hour, followed by the addition of iodomethane (1.8 g, 12.60 mmol). The reaction mixture was slowly warmed to room temperature and stirred for 1 hour, then cooled back to -78°C. Sodium bis(trimethylsilyl)amide (5 mL, 10.0 mmol, 2 M in THF) and iodomethane (1.8 g, 12.60 mmol) were added dropwise, and the mixture was returned to room temperature and stirred for another 1 hour after the addition was completed. After the reaction was completed, the reaction mixture was slowly added with saturated aqueous ammonium chloride solution (10 mL) for quenching, diluted with water (20 mL), and extracted with ethyl acetate (20 mL × 3). The combined organic phases were washed with saturated brine (20 mL × 1), dried over anhydrous sodium sulfate, and filtered. The filtrate was concentrated under reduced pressure, and the residue was subjected to silica gel column chromatography (petroleum ether/ethyl acetate = 20/1 to 5/1) to obtain compound 134f (1.2 g). MS m/z (ESI): [M+H]⁺ = 377.95.

### Step 6: Synthesis of Compound 134g

At 0°C, ceric ammonium nitrate (4.64 g, 8.76 mmol) was added to a mixed solution of compound 134f (1.10 g, 2.92 mmol) in acetonitrile and water (20 mL/10 mL). The reaction mixture was stirred at 0°C for 2 hours. After the reaction was completed, the reaction mixture was diluted with saturated brine (20 mL) and extracted with ethyl acetate (20 mL × 2). The combined organic phases were washed with saturated brine (20 mL × 1), dried over anhydrous sodium sulfate, and filtered. The filtrate was concentrated under reduced pressure, and the residue was purified by silica gel column chromatography (petroleum ether/ethyl acetate = 5/1 to 3/1) to obtain compound 134g (670 mg). MS m/z (ESI): [M+H]⁺ = 257.95.

### Step 7: Synthesis of compound 134h

1,1'-Bis(diphenylphosphino)ferrocene-palladium(II)dichloride dichloromethane complex (188 mg, 0.26 mmol) was added to a solution of compound 134g (670 mg, 2.61 mmol), bis(pinacolato)diboron (1.98 g, 7.82 mmol), and potassium acetate (511 mg, 5.22 mmol) in 1,4-dioxane (10 mL). The reaction mixture was stirred at 90°C for 16 hours. After the reaction was completed, the reaction mixture was concentrated under reduced pressure, and the residue was purified by silica gel column chromatography (petroleum ether/ethyl acetate = 3/1 to 0/1) to obtain compound 134h (400 mg). MS m/z (ESI): [M-81]⁺= 224.10.

### Step 8: Synthesis of Compound 134

Tetrakis(triphenylphosphine)palladium (84 mg, 0.07 mmol) was added to a mixed solution of compound A2 (300 mg, 0.73 mmol), compound 134h (196 mg, 0.87 mmol), and sodium carbonate (155 mg, 1.46 mmol) in 1,4-dioxane and water (5 mL/0.5 mL). The reaction mixture was stirred at 90°C for 3 hours. After the reaction was completed, the reaction mixture was concentrated under reduced pressure, and the residue was purified by silica gel column chromatography (petroleum ether/ethyl acetate = 1/1 to 0/1) to obtain a crude product of compound 134. The crude product of compound 134 was further purified by preparative HPLC (chromatographic column: YMC-Triart C18 30 × 250 mm, 12 nm 10u, mobile phase: A: 0.1% ammonia water-water, B: acetonitrile, flow rate: 70 mL/min) to obtain compound 134 (111.3 mg). MS m/z (ESI): [M+H]⁺ = 555.00. ¹H NMR (400 MHz, DMSO-d₆) δ 8.94 (s, 1H), 8.84 (s, 1H), 8.60 (d, *J* = 2.3 Hz, 1H), 8.18 - 7.99 (m, 2H), 7.84 (s, 1H), 7.59 (d, *J* = 7.8 Hz, 1H), 6.80 (s, 1H), 5.48 (s, 2H), 2.08 (s, 3H), 1.99 (s, 3H), 1.56 (s, 6H).

### Example 69 Synthesis of Compound 135

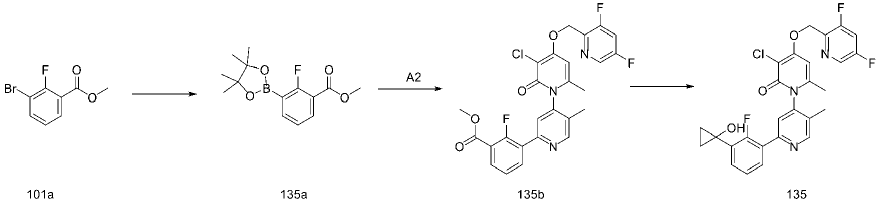

### Step 1: Synthesis of Compound 135a

1,1'-Bis(diphenylphosphino)ferrocene palladium(II)dichloride (249 mg, 0.343 mmol) was added to a solution of compound 101a (800 mg, 3.43 mmol), bis(pinacolato)diboron (1.74 g, 6.87 mmol), and potassium acetate (674 mg, 6.87 mmol) in 1,4-dioxane (15 mL). The reaction mixture was stirred at 90°C for 3 hours. After the reaction was completed, the reaction mixture was filtered through diatomaceous earth to remove insoluble materials. The filtrate was concentrated under reduced pressure, and the residue was purified by silica gel column chromatography (petroleum ether/ethyl acetate = 1/20 to 1/5) to obtain compound 135a (360 mg).

### Step 2: Synthesis of Compound 135b

Tetrakis(triphenylphosphine)palladium (70 mg, 0.06 mmol) was added to a mixed solution of compound 135a (339 mg, 1.21 mmol), compound A2 (250 mg, 0.61 mmol), and sodium carbonate (128 mg, 1.21 mmol) in 1,4-dioxane and water (5 mL/0.5 mL). The reaction mixture was stirred at 90°C for 3 hours. After the reaction was completed, the reaction mixture was filtered, and the filtrate was concentrated under reduced pressure. The residue was purified by silica gel column chromatography (petroleum ether/ethyl acetate = 1/2 to dichloromethane/methanol = 20/1) to obtain a crude product. The crude product was further purified by preparative HPLC (preparative column: YMC Triart C18 12 nm 10u, 50 × 250 mm; flow rate: 40 mL/min; column temperature: room temperature; mobile phase: A: water (0.1% ammonia water), B: preparative grade acetonitrile) to obtain compound 135b (210 mg). MS m/z (ESI): 529.95 [M+1]⁺.

### Step 3: Synthesis of Compound 135

Compound 135b (110 mg, 207 µmol) and tetraethyl titanate (88.5 mg, 311 µmol) were dissolved in anhydrous tetrahydrofuran (2.0 mL). At 0°C, ethyl boromagnesium bromide (0.21 mL, 623 µmol, 3 M) was added dropwise. After the dropwise addition was completed, the reaction mixture was stirred at 0°C for 20 minutes, then warmed to room temperature and stirred at room temperature for 16 hours. After the reaction was completed, the reaction mixture was quenched with aqueous ammonium chloride solution (1 mL) and extracted with ethyl acetate (5 mL × 3). The combined organic phases were washed with saturated brine (5 mL × 2), dried over anhydrous sodium sulfate, and filtered. The filtrate was concentrated under reduced pressure, and the residue was purified by silica gel column chromatography (petroleum ether/ethyl acetate = 1/2 to dichloromethane/methanol = 20/1) to obtain a crude product. The crude product was further purified by preparative HPLC (preparative column: YMC Triart C18 12 nm 10u, 50 × 250 mm; flow rate: 40 mL/min; column temperature: room temperature; mobile phase: A: water (0.1% ammonia water), B: preparative grade acetonitrile) to obtain compound 135 (5.2 mg). MS m/z (ESI): 528.00 [M+1]⁺. ¹H NMR (400 MHz, DMSO-d₆) δ 8.79 (s, 1H), 8.60 (s, 1H), 8.08 (t, *J=* 8.8 Hz, 1H), 7.82 (t, *J=* 7.2 Hz, 1H), 7.72 (s, 1H), 7.57 (t, *J=* 7.2 Hz, 1H), 7.28 (t, *J=* 7.6 Hz, 1H), 6.79 (s, 1H), 5.97 (s, 1H), 5.48 (s, 2H), 2.06 (s, 3H), 1.98 (s, 3H), 0.99 (s, 4H).

### Example 70 Synthesis of Compound 136

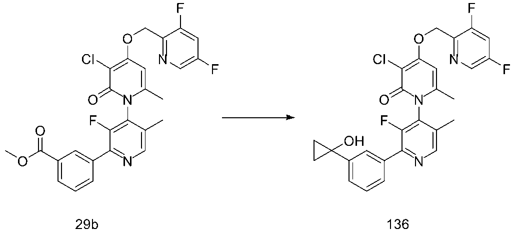

29b (50 mg, 0.095 mmol) and tetraethyl titanate (38 mg, 0.133 mmol) were added to anhydrous tetrahydrofuran (1 mL). Ethylmagnesium bromide (3 M in Et₂O, 0.1 mL) was added at 0°C. After the addition was completed, the reaction mixture was stirred overnight at room temperature. After the reaction was completed, saturated aqueous ammonium chloride solution (10 mL) was added to the reaction mixture, which was then extracted with ethyl acetate (20 mL × 2). The combined organic phases were dried over anhydrous sodium sulfate and filtered. The filtrate was concentrated under reduced pressure to obtain a crude product. The crude product was purified by preparative HPLC (preparative column: YMC Triart C18 12 nm 10u, 30 × 250 mm; flow rate: 40 mL/min; column temperature: room temperature; mobile phase: A: water (0.1% ammonia water) B: preparative grade acetonitrile) to obtain compound 136 (2.06 mg). MS m/z (ESI): 528.05 [M+1]⁺. ¹H NMR (400 MHz, CD₃OD-d₄) δ 8.60 (s, 1H), 8.46 (d, *J*= 2.0 Hz, 1H), 7.85 (s, 1H), 7.78 - 7.63 (m, 2H), 7.49 - 7.38 (m, 2H), 6.87 (s, 1H), 5.52 (d, *J=* 1.6 Hz, 2H), 2.17 (s, 3H), 2.13 (s, 3H), 1.24 -1.21 (m, 2H), 1.09 -1.06 (m, 2H).

### Example 71 Synthesis of Compound 137

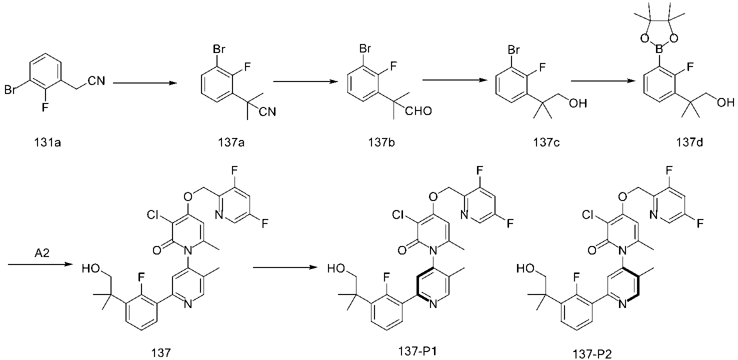

### Step 1: Synthesis of Compound 137a

At 0°C, a solution of 131a (2.0 g, 9.34 mmol) in N,N-dimethylformamide (10 mL) was added to a solution of sodium hydride (934 mg, 23.4 mmol, 60%) in N,N-dimethylformamide (10 mL). The mixture was stirred at 0°C for 30 minutes, followed by the dropwise addition of iodomethane (3.3 g, 23.4 mmol). The reaction mixture was stirred overnight at room temperature. After the reaction was completed, the reaction mixture was diluted with water (20 mL) and extracted with ethyl acetate (20 × 3). The combined organic phases were washed with saturated brine (10 mL × 2), dried over anhydrous sodium sulfate, and filtered. The filtrate was concentrated under reduced pressure, and the residue was purified by silica gel column chromatography (petroleum ether/ethyl acetate = 1/0 to 20/1) to obtain compound 137a (2.0 g). ¹H NMR (600 MHz, CDCl₃) δ 7.55 (m, 7.56-7.54, 1H), 7.46 (td, J= 7.8, 1.2 Hz, 1H), 7.06 (td, *J* = 7.8, 1.2 Hz, 1H), 1.81 (d, *J= 0.6* Hz, 6H).

### Step 2: Synthesis of Compound 137b

At -60°C, a solution of diisobutylaluminum hydride in tetrahydrofuran (1 M, 13.2 mL, 13.2 mmol) was slowly added dropwise to a solution of compound 137a (2.0 g, 8.26 mmol) in anhydrous dichloromethane (20 mL). The reaction mixture was slowly warmed to room temperature and stirred at room temperature for 3 hours. After the reaction was completed, the reaction mixture was cooled to 0°C, and dilute hydrochloric acid (1 M, 14 mL) was added dropwise to quench the reaction. The mixture was stirred at 0°C for 10 minutes, then extracted with ethyl acetate (30 mL × 3). The combined organic phases were washed with saturated brine (30 mL × 2), dried over anhydrous sodium sulfate, and filtered. The filtrate was concentrated under reduced pressure, and the residue was purified by silica gel column chromatography (petroleum ether/ethyl acetate = 1/0 to 20/1) to obtain compound 137b (1.9 g). ¹H NMR (600 MHz, CDCl₃) δ 9.64 (d, *J* = 4.8 Hz, 1H), 7.52 (m, 7.53-7.50, 1H), 7.26 (m, 7.27-7.25, 1H), 7.07 (t, *J =* 7.8 Hz, 1H), 1.46 (s, 6H).

### Step 3: Synthesis of Compound 137c

At 0°C, sodium borohydride (288 mg, 7.63 mmol) was added to a solution of compound 137b (1.7 g, 6.94 mmol) in anhydrous methanol (15 mL), and the reaction mixture was stirred at 0°C for 1 hour. After the reaction was completed, the reaction mixture was diluted with water (5 mL) and extracted with ethyl acetate (15 mL × 3). The combined organic phases were washed with saturated brine (10 mL × 2), dried over anhydrous sodium sulfate, and filtered. The filtrate was concentrated under reduced pressure, and the residue was purified by silica gel column chromatography (petroleum ether/ethyl acetate = 5/1) to obtain compound 137c (1.3 g). ¹H NMR (400 MHz, DMSO-d₆) δ 7.54 (m, 7.56-7.52, 1H), 7.35 (td, *J* = 7.6, 1.6 Hz, 1H), 7.08 (t, J= 8.0 Hz, 1H), 4.77 (s, 1H), 3.54 (s, 2H), 1.27 (d, *J =* 0.8 Hz, 6H).

### Step 4: Synthesis of Compound 137d

1,1'-Bis(diphenylphosphino)ferrocene palladium(II)dichloride (146 mg, 0.202 mmol) was added to a solution of compound 137c (500 mg, 2.02 mmol), bis(pinacolato)diboron (1.54 g, 6.07 mmol), and potassium acetate (397 mg, 4.04 mmol) in 1,4-dioxane (5 mL). The reaction mixture was stirred at 90°C for 3 hours. After the reaction was completed, the reaction mixture was filtered through diatomaceous earth, and the filtrate was concentrated under reduced pressure. The residue was purified by silica gel column chromatography (petroleum ether/ethyl acetate = 1/20 to 1/5) to obtain compound 137d (300 mg). MS m/z (ESI): 295.15 [M+1]⁺.

### Step 5: Synthesis of Compounds 137, 137-P1, and 137-P2

Tetrakis(triphenylphosphine)palladium (50.4 mg, 0.04 mmol) was added to a mixed solution of compound 137d (385 mg, 1.31 mmol), compound A2 (180 mg, 0.44 mmol), and sodium carbonate (92.6 mg, 0.87 mmol) in 1,4-dioxane and water (3 mL/0.3 mL). The reaction mixture was stirred overnight at 90°C. After the reaction was completed, the reaction mixture was concentrated under reduced pressure. The residue was purified by silica gel column chromatography (petroleum ether/ethyl acetate = 1/2 to dichloromethane/methanol = 20/1) to obtain a crude product. The crude product was further purified by preparative HPLC (preparative column: YMC Triart C18 12 nm 10u, 50 × 250 mm; flow rate: 40 mL/min; column temperature: room temperature; mobile phase: A: water (0.1% ammonia water), B: preparative grade acetonitrile) to obtain compound 137 (132 mg).

Compound 137 (125 mg, 0.23 mmol) was resolved by preparative chiral chromatography (preparative column: YMC CHIRALART Amylose-C NEO, 30 × 250 mm; flow rate: 30 mL/min; column temperature: room temperature; mobile phase: A: n-hexane B: isopropanol) to obtain compounds 137-P1 (50.9 mg) and 137-P2 (45.2 mg).

### Compound 137-P1:

MS m/z (ESI): 544.0 [M+1]⁺. Chiral HPLC: retention time = 8.434 min, UV = 254 nm. ¹H NMR (400 MHz, DMSO-d₆) δ 8.79 (s, 1H), 8.60 (d, *J* = 2.4 Hz, 1H), 8.09 (td, J= 9.6, 2.4 Hz, 1H), 7.74 *(d, J=* 7.2 Hz, 1H), 7.70 (d, *J* = 1.6 Hz, 1H), 7.43 *(t, J= 7.2* Hz, 1H), 7.25 *(t, J= 7.6* Hz, 1H), 6.80 (s, 1H), 5.48 (s, 2H), 4.73 (t, *J= 5.6* Hz, 1H), 3.60 (d, *J=* 2.8 Hz, 2H), 2.05 (s, 3H), 1.98 (s, 3H), 1.31 (s, 6H).

### Compound 137-P2:

MS m/z (ESI): 544.0 [M+1]⁺. Chiral HPLC: retention time = 13.848 min, UV = 254 nm. ¹H NMR (400 MHz, DMSO-d₆) δ 8.79 (s, 1H), 8.60 (d, *J= 2.4* Hz, 1H), 8.13 - 8.06 (m, 1H), 7.75-7.70 (m, 2H), 7.43 (dd, *J* = 7.6, 6.2 Hz, 1H), 7.25 (t, *J= 7.6* Hz, 1H), 6.80 (s, 1H), 5.48 (d, *J* = 1.2 Hz, 2H), 4.73 *(t, J= 5.6* Hz, 1H), 3.64 - 3.58 (m, 2H), 2.05 (s, 3H), 1.98 (s, 3H), 1.31 (s, 6H).

### Example 72 Synthesis of Compound 138

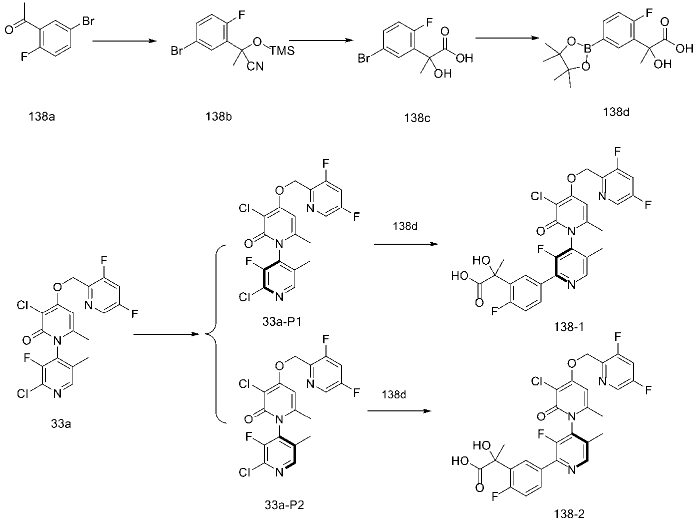

### Step 1: Synthesis of Compound 138b

Silver bis(trifluoromethanesulfonyl)imide (2.8 g, 7.40 mmol) and trimethylsilyl cyanide (7.3 g, 74.07 mmol) were sequentially added to a solution of compound 138a (8.0 g, 37.03 mmol) in toluene (80 mL). The reaction mixture was stirred at 60°C for 16 hours. After the reaction was completed, the reaction mixture was concentrated under reduced pressure, and the residue was purified by silica gel column chromatography (petroleum ether: ethyl acetate = 20:1 to 3:1) to obtain compound 138b (7.0 g).

### Step 2: Synthesis of Compound 138c

Concentrated hydrochloric acid (60 mL) was added to a solution of compound 138b (7.0 g, 28.80 mmol) in 1,4-dioxane (40 mL). The reaction mixture was stirred at 80°C for 16 hours. After the reaction was completed, the reaction mixture was diluted with water (50 mL), adjusted to pH = 9-10 with saturated aqueous sodium carbonate solution, and extracted with ethyl acetate (100 mL × 3). The aqueous phase was adjusted to pH = 3-4 with concentrated hydrochloric acid and extracted with ethyl acetate (100 mL × 1). The organic phase was dried over anhydrous sodium sulfate, filtered, and the filtrate was concentrated under reduced pressure to obtain compound 138c (6.0 g). MS m/z (ESI): 246.95 [M-17]⁺.

### Step 3: Synthesis of Compound 138d

1,1'-Bis(diphenylphosphino)ferrocene palladium(II)dichloride (166 mg, 0.23 mmol) was added to a solution of compound 138c (600 mg, 2.29 mmol), bis(pinacolato)diboron (1.75 g, 6.87 mmol), and potassium acetate (450 mg, 4.58 mmol) in 1,4-dioxane (10 mL). The reaction mixture was stirred at 90°C under a nitrogen atmosphere for 3 hours. After the reaction was completed, the reaction mixture was concentrated under reduced pressure, and the residue was purified by silica gel column chromatography (petroleum ether: ethyl acetate = 3:1-1:1 to dichloromethane: methanol = 1:0-10:1) to obtain compound 138d (380 mg).

### Step 4: Synthesis of Compounds 33a-P1 and 33a-P2

Compound 33a (1.5 g, 3.49 mmol) was resolved by preparative chiral chromatography (preparative column: YMC CHIRALART Cellulose-SC, 30 × 250 mm; flow rate: 40 mL/min; column temperature: room temperature; mobile phase: A: pure water (0.1% TFA) B: ACN) to obtain compounds 33a-P1 (550 mg) and 33a-P2 (580 mg). MS m/z (ESI): 429.90 [M+H]⁺.

### Compound 33a-P1:

MS m/z (ESI): 429.90 [M+1]⁺. Chiral HPLC: retention time = 11.52 min, UV = 220 nm.

### Compound 33a-P2:

MS m/z (ESI): 429.90 [M+1]⁺. Chiral HPLC: retention time = 21.68 min, UV = 220 nm.

### Step 5: Synthesis of Compounds 138-1 and 138-2

Tetrakis(triphenylphosphine)palladium (81 mg, 0.07 mmol) was added to a mixed solution of compound 33a-P1 (300 mg, 0.70 mmol), compound 138d (325 mg, 1.05 mmol), and sodium carbonate (148 mg, 1.40 mmol) in 1,4-dioxane and water (10 mL/2 mL). The reaction mixture was stirred at 90°C for 3 hours. After the reaction was completed, the reaction mixture was concentrated under reduced pressure. The residue was purified by silica gel column chromatography (dichloromethane: methanol = 1:0 to 5:1) to obtain a crude product. The crude product was further purified by preparative HPLC (preparation conditions: chromatographic column: YMC-Triart C18 30 × 250 mm, mobile phase: A: 0.1% hydrochloric acid-water, B: acetonitrile, flow rate: 40 mL/min) to obtain compound 138-1 (223.9 mg). MS m/z (ESI): 577.95 [M+H]⁺. ¹H NMR (400 MHz, DMSO-d₆) δ 8.68 (s, 1H), 8.60 (d, *J* = 2.3 Hz, 1H), 8.17 (d, *J=* 7.5 Hz, 1H), 8.13 - 8.04 (m, 1H), 7.89 - 7.75 (m, 1H), 7.22 (dd, *J=* 11.1, 8.6 Hz, 1H), 6.88 (s, 1H), 5.51 (s, 2H), 2.11 (s, 3H), 2.05 (s, 3H), 1.60 (s, 3H).

Tetrakis(triphenylphosphine)palladium (81 mg, 0.07 mmol) was added to a mixed solution of compound 33a-P2 (300 mg, 0.70 mmol), compound 138d (325 mg, 1.05 mmol), and sodium carbonate (148 mg, 1.40 mmol) in 1,4-dioxane and water (10 mL/2 mL). The reaction mixture was stirred at 90°C for 3 hours. After the reaction was completed, the reaction mixture was concentrated under reduced pressure. The residue was purified by silica gel column chromatography (dichloromethane: methanol = 1:0 to 5:1) to obtain a crude product. The crude product was further purified by preparative HPLC (preparation conditions: chromatographic column: YMC-Triart C18 30 × 250 mm, mobile phase: A: 0.1% hydrochloric acid-water, B: acetonitrile, flow rate: 40 mL/min) to obtain compound 138-2 (174.3 mg). MS m/z (ESI): 577.95 [M+H]⁺. ¹H NMR (400 MHz, DMSO-d₆) δ 8.69 (s, 1H), 8.60 (d, *J= 2.2* Hz, 1H), 8.24 (d, *J=* 7.4 Hz, 1H), 8.15 - 8.04 (m, 1H), 7.90 (s, 1H), 7.30 (dd, *J=* 10.9, 8.6 Hz, 1H), 6.88 (s, 1H), 5.51 (s, 2H), 2.11 (s, 3H), 2.05 (s, 3H), 1.63 (s, 3H).

### Example 73 Synthesis of Compound 139

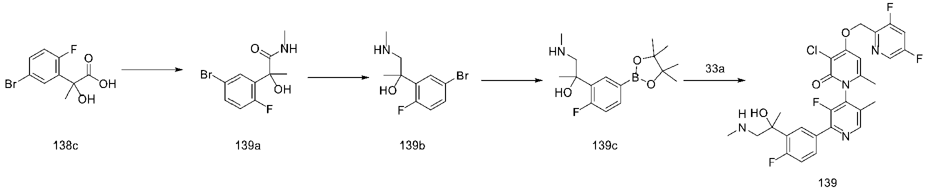

### Step 1: Synthesis of Compound 139a

Methylamine hydrochloride (1.03 g, 15.26 mmol), 1-(3-dimethylaminopropyl)-3-ethylcarbodiimide hydrochloride (2.2 g, 11.45 mmol), 1-hydroxybenzotriazole (1.55 g, 11.45 mmol), and triethylamine (3.85 g, 38.16 mmol) were sequentially added to a solution of compound 138c (2.0 g, 7.63 mmol) in N,N-dimethylformamide (20 mL). The reaction mixture was stirred at room temperature for 2 hours. After the reaction was completed, the reaction mixture was diluted with water (50 mL) and extracted with ethyl acetate (60 mL × 3). The combined organic phases were washed with saturated brine (60 mL × 2), dried over anhydrous sodium sulfate, and filtered. The filtrate was concentrated under reduced pressure. The residue was purified by silica gel column chromatography (petroleum ether: ethyl acetate = 2:1 to 1:1) to obtain compound 139a (2.0 g). MS m/z (ESI): 275.95 [M+H]⁺.

### Step 2: Synthesis of Compound 139b

Borane-methyl sulfide complex (6.54 mL, 65.40 mmol, 10 M) was added dropwise to a solution of compound 139a (1.8 g, 6.54 mmol) in tetrahydrofuran (20 mL), and the reaction mixture was stirred at 70°C for 3 hours. After the reaction was completed, the reaction mixture was slowly quenched by dropwise addition of methanol (20 mL), diluted with water (30 mL), and extracted with ethyl acetate (100 mL × 1). The aqueous phase was adjusted to pH = 9-10 with saturated aqueous sodium carbonate solution and extracted with ethyl acetate (100 mL × 2). The combined organic phases were dried over anhydrous sodium sulfate and filtered. The filtrate was concentrated under reduced pressure, and the residue was purified by silica gel column chromatography (dichloromethane: methanol = 1:0 to 10:1) to obtain compound 139b (1.0 g). MS m/z (ESI): 262.00 [M+H]⁺.

### Step 3: Synthesis of Compound 139c

139b (400 mg, 1.53 mmol), bis(pinacolato)diboron (584 mg, 2.30 mmol), 1,1'-bis(diphenylphosphino)ferrocene palladium(II)dichloride (111 mg, 0.15 mmol), and potassium acetate (300 mg, 3.06 mmol) were added to 1,4-dioxane (4 mL). The reaction mixture was stirred at 90°C for 2 hours. After the reaction was completed, the reaction mixture was directly used in the next step.

### Step 4: Synthesis of Compound 139

1,1'-Bis(diphenylphosphino)ferrocene-palladium(II)dichloride dichloromethane complex (54 mg, 0.07 mmol) was added to a mixed solution of compound 33a (320 mg, 0.74 mmol), compound 139c (4 mL of the previous reaction mixture), and potassium carbonate (206 mg, 1.49 mmol) in 1,4-dioxane and water (15 mL/3 mL). The reaction mixture was stirred at 90°C for 8 hours. After the reaction was completed, the reaction mixture was concentrated under reduced pressure. The residue was purified by silica gel column chromatography (dichloromethane: methanol = 1:0 to 5:1) to obtain a crude product. The crude product was further purified by preparative HPLC (preparation conditions: chromatographic column: YMC-Triart C18 30 × 250 mm, mobile phase: A: 0.1% hydrochloric acid-water, B: acetonitrile, flow rate: 40 mL/min) to obtain compound 139 (122.6 mg). MS m/z (ESI): 577.05 [M+H]⁺. ¹H NMR (400 MHz, DMSO-d₆) δ 8.70 (s, 1H), 8.60 (d, J=2.3 Hz, 1H), 8.31 (d, *J* = 7.8 Hz, 2H), 8.10 (td, J= 9.9, 2.3 Hz, 1H), 7.97 (s, 1H), 7.37 (t, J= 10.0 Hz, 1H), 6.91 (s, 1H), 6.50 (s, 1H), 5.52 (d, J= 1.1 Hz, 2H), 3.50 (s, 3H), 3.29 (s, 2H), 2.12 (s, 3H), 2.06 (d, *J* = 3.0 Hz, 3H), 1.63 (s, 3H).

### Example 74 Synthesis of Compound 140

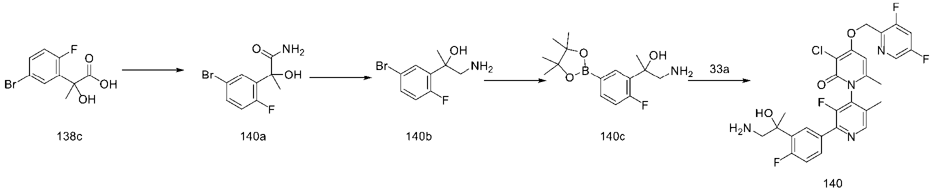

### Step 1: Synthesis of Compound 140a

Compound 138c (2.07 g, 7.88 mmol) was dissolved in N,N-dimethylformamide (20 mL), followed by the sequential addition of ammonium chloride (2.1 g, 39.43 mmol), 1-(3-dimethylaminopropyl)-3-ethylcarbodiimide hydrochloride (9.36 g, 47.28 mmol), 1-hydroxybenzotriazole (6.38 g, 47.28 mmol), and triethylamine (5.37 g, 63.04 mmol). The reaction mixture was stirred at 40°C for 2 hours. After the reaction was completed, the reaction mixture was diluted with water (50 mL) and extracted with ethyl acetate (60 mL × 3). The combined organic phases were washed with saturated brine (60 mL × 2), dried over anhydrous sodium sulfate, and filtered. The filtrate was concentrated under reduced pressure. The residue was purified by silica gel column chromatography (petroleum ether: ethyl acetate = 1:1 to 2:3) to obtain compound 140a (1.5 g). MS m/z (ESI): 263.95 [M+H]⁺.

### Step 2: Synthesis of Compound 140b

At 0°C, borane-methyl sulfide complex (5.3 mL, 53.6 mmol, 10 M) was added dropwise to a solution of compound 140a (1.4 g, 5.36 mmol) in tetrahydrofuran (10 mL). The reaction mixture was stirred at 70°C for 2 hours. After the reaction was completed, the reaction mixture was slowly quenched by dropwise addition of methanol (20 mL), diluted with water (30 mL), adjusted to pH = 2 with 1 M dilute hydrochloric acid, and extracted with ethyl acetate (60 mL × 2). The aqueous phase was adjusted to pH = 9 with saturated aqueous sodium carbonate solution, extracted with ethyl acetate (40 mL × 3), and the combined organic phases were washed with saturated brine (60 mL × 2), dried over anhydrous sodium sulfate, and filtered. The filtrate was concentrated under reduced pressure to obtain compound 140b (800 mg). MS m/z (ESI): 248.00 [M+H]⁺.

### Step 3: Synthesis of Compound 140c

140c (400 mg, 1.6 mmol), bis(pinacolato)diboron (616 mg, 2.4 mmol), 1,1'-bis(diphenylphosphino)ferrocene-palladium(II)dichloride dichloromethane complex (130 mg, 0.16 mmol), and potassium acetate (313 mg, 3.2 mmol) were added to 1,4-dioxane (6 mL). The reaction mixture was stirred at 90°C for 24 hours. After the reaction was completed, the reaction mixture was cooled to room temperature and directly used in the next step. MS m/z (ESI): 296.10 [M+H]⁺.

### Step 4: Synthesis of Compound 140

1,1'-Bis(diphenylphosphino)ferrocene-palladium(II)dichloride dichloromethane complex (45.7 mg, 0.056 mmol) was added to a mixed solution of compound 33a (240 mg, 0.56 mmol), compound 140c (6 mL of the previous reaction mixture), and potassium carbonate (154 mg, 1.12 mmol) in 1,4-dioxane and water (15 mL/3 mL). The reaction mixture was stirred at 90°C for 15 hours. After the reaction was completed, the reaction mixture was concentrated under reduced pressure. The residue was purified by silica gel column chromatography (dichloromethane: methanol = 20:1 to dichloromethane: methanol: triethylamine = 10:1:0.1) to obtain a crude product. The crude product was further purified by preparative HPLC (preparation conditions: chromatographic column: YMC-Triart C18 30 × 250 mm, mobile phase: A: 0.1% hydrochloric acid-water, B: acetonitrile, flow rate: 40 mL/min) to obtain compound 140 (35.6 mg). MS m/z (ESI): 563.05 [M+H]⁺. ¹H NMR (400 MHz, DMSO-d₆) δ 8.67 (s, 1H), 8.58 (d, *J= 2.4* Hz, 1H), 8.32 - 8.26 (m, 1H), 8.07 (m, 1H), 8.00 - 7.78 (m, 4H), 7.41 - 7.30 (m, 1H), 6.88 (s, 1H), 6.30 (d, *J =* 7.2 Hz, 1H), 5.49 (s, 2H), 3.12 *(d, J=* 5.2 Hz, 2H), 2.10 (s, 3H), 2.03 (d, *J=* 4.8 Hz, 3H), 1.57 (s, 3H).

### Example 75 Synthesis of Compound 141

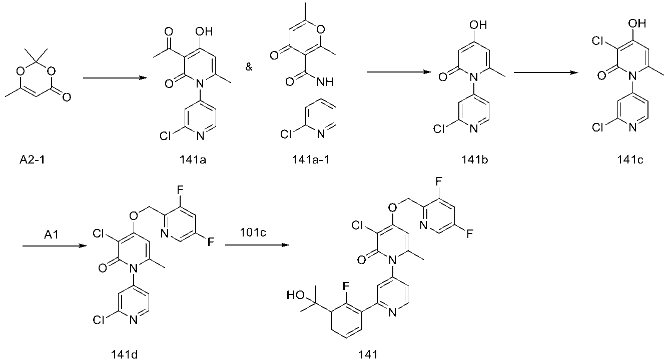

### Step 1: Synthesis of Compound 141a

Compound A2-1 (20.5 g, 144 mmol) was added to a solution of compound 2-chloro-4-aminopyridine (5.0 g, 38.9 mmol) in N,N-dimethylacetamide (25.0 mL). The reaction mixture was stirred at 120°C for 2 hours until the reaction was completed. The reaction mixture was cooled to 50 to 60°C. Water (75.0 mL) was added, and the mixture was stirred for 30 minutes and filtered. The filter cake was washed with ice water (75 mL) and dried to obtain compound 141a (7.0 g, crude product, containing a portion of 141a-1). MS m/z (ESI): 279.00 [M+1]⁺.

### Step 2: Synthesis of Compound 141b

A solution of hydrogen chloride in isopropanol (28 mL, 4 M) was added to a solution of compound 141a (7.0 g, crude product, containing a portion of 141a-1) in water (70 mL). The reaction mixture was stirred at 90°C for 16 hours. After the reaction was completed, the reaction mixture was cooled to room temperature and filtered. The filter cake was added to a mixed solution of water and isopropanol (30 mL/6 mL) and stirred for 30 minutes. The mixture was filtered, and the filter cake was concentrated under reduced pressure to remove the solvent. The resulting residue was added to concentrated sulfuric acid (10 mL, 98%) and reacted at 120°C for 2 hours. After the reaction was completed, the reaction mixture was cooled to room temperature and diluted with ice water (50 mL). The temperature of the reaction mixture was maintained at 10 to 20°C, and the pH was adjusted to 9 using saturated sodium hydroxide solution (200 mL). The pH was then adjusted to 3 with saturated citric acid (30 mL). The mixture was filtered, and the filter cake was added to a mixed solution of NN-dimethylformamide and water (25 mL/25 mL) and stirred for 30 minutes, and then filtrated. The filter cake was dried to obtain compound 141b (1.7 g). MS m/z (ESI): 237.05 [M+1]⁺.

### Step 3: Synthesis of Compound 141c

N-Chlorosuccinimide (973 mg, 7.29 mmol) was added to a solution of compound 141b (1.5 g, 6.34 mmol) and *p-*toluenesulfonic acid (54.0 mg, 317 µmol) in N,N-dimethylformamide (15 mL). The reaction mixture was stirred at 70°C for 4 hours. After the reaction was completed, the reaction mixture was cooled to room temperature. Water (30 mL) was added, and the mixture was stirred for 30 minutes, and then filtrated. The filter cake was added to ethyl acetate (8 mL), stirred at room temperature for 30 minutes, and filtered. The filter cake was dried to obtain compound 141c (1.16 g). MS m/z (ESI): 270.95 [M+1]⁺.

### Step 4: Synthesis of Compound 141d

Compound 141c (660 mg, 2.43 mmol) was added to a solution of potassium carbonate (673 mg, 4.87 mmol), 18-crown-6 (64.0 mg, 0.24 mmol), and compound A1 (398 mg, 2.43 mmol) in N,N-dimethylformamide (3.0 mL). The reaction mixture was stirred at 55°C for 16 hours. After the reaction was completed, the reaction mixture was cooled to room temperature. Water (12 mL) was added, and the mixture was stirred for 30 minutes and then filtrated. The filter cake was added to ethyl acetate (7.0 mL), stirred at room temperature for 30 minutes, and filtered. The filter cake was dried to obtain compound 141d (390 mg, yield: 40.2%). MS m/z (ESI): 397.95 [M+1]⁺.

### Step 5: Synthesis of Compound 141

Tetrakis(triphenylphosphine)palladium (29.0 mg, 0.025 mmol) was added to a mixed solution of compound 101c (105 mg, 0.376 mmol), compound 141d (100 mg, 0.251 mmol), and sodium carbonate (53.0 mg, 0.52 mmol) in 1,4-dioxane and water (2.5 mL/0.5 mL). The reaction mixture was stirred at 90°C for 1.5 hours. After the reaction was completed, the reaction mixture was concentrated under reduced pressure, and the residue was purified by silica gel column chromatography (petroleum ether/ethyl acetate = 1/10 to 2/1) to obtain a crude product. The crude product was further purified by preparative HPLC (preparative column: YMC Triart C18, 30 × 250 mm; flow rate: 40 mL/min; column temperature: room temperature; mobile phase: A: water (0.1% ammonia water-water), B: preparative grade acetonitrile) to obtain compound 141 (64.2 mg). MS m/z (ESI): 516.00 [M+1]⁺. ¹H NMR (400 MHz,DMSO-*d₆*) δ 8.89 (d, *J=* 5.2 Hz, 1H), 8.60 (d, *J=* 2.4 Hz, 1H), 8.09 (td, J= 9.6, 2.4 Hz, 1H), 7.75 (dd, *J=* 14.8, 7.2 Hz, 3H), 7.46 (dd, *J= 5.2,* 1.6 Hz, 1H), 7.30 (t, *J=* 7.6 Hz, 1H), 6.75 (s, 1H), 5.48 (d, *J=* 1.2 Hz, 2H), 5.34 (s, 1H), 2.02 (s, 3H), 1.53 (s, 6H).

### Example 76 Synthesis of Compound 142

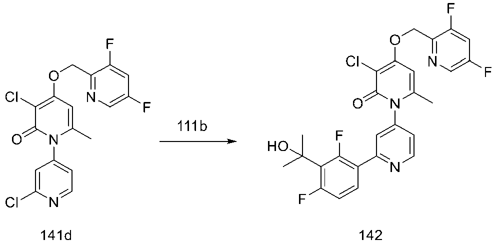

1,1'-Bis(diphenylphosphino)ferrocene-palladium(II)dichloride dichloromethane complex (20.4 mg, 0.025 mmol) was added to a mixed solution of compound 141d (100 mg, 0.25 mmol), compound 111b (201 mg, 0.67 mmol), and potassium carbonate (86 mg, 0.62 mmol) in 1,4-dioxane and water (5 mL/1 mL). The reaction mixture was stirred at 90°C for 2 hours. After the reaction was completed, the reaction mixture was diluted with water (10 mL) and extracted with ethyl acetate (10 mL × 3). The combined organic phases were washed with saturated brine (30 mL × 2), dried over anhydrous sodium sulfate, and filtered. The filtrate was concentrated under reduced pressure, and the residue was purified by silica gel column chromatography (dichloromethane: methanol = 40:1 to 25:1) to obtain a crude product. The crude product was further purified by preparative HPLC (chromatographic column: YMC-Triart C18 30 × 250 mm, mobile phase: A: 0.1% ammonia water-water, B: acetonitrile; flow rate: 40 mL/min) to obtain compound 142 (53.48 mg). MS m/z (ESI): 534.00 [M+H]⁺. ¹H NMR (400 MHz, DMSO-d₆) δ 8.88 (d, *J= 5.2* Hz, 1H), 8.59 (d, *J= 2.4* Hz, 1H), 8.08 (m, 1H), 7.87 - 7.77 (m, 1H), 7.74 (s, 1H), 7.46 (m, 1H), 7.15 (m,1H), 6.75 (s, 1H), 5.47 (s, 2H), 5.32 (s, 1H), 2.02 (s, 3H), 1.63 (s, 6H).

### Example 77 Synthesis of Compound 143

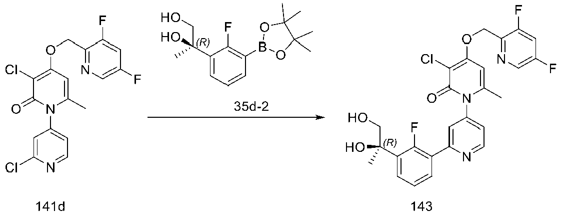

Tetrakis(triphenylphosphine)palladium (45 mg, 0.04 mmol) was added to a mixed solution of compound 141d (150 mg, 0.37 mmol), compound 35d-2 (225 mg, 0.75 mmol), and sodium carbonate (80 mg, 0.75 mmol) in 1,4-dioxane and water (5 mL/0.5 mL). The reaction mixture was stirred at 90°C for 2 hours. After the reaction was completed, the reaction mixture was concentrated under reduced pressure. The resulting residue was purified by silica gel column chromatography (petroleum ether: ethyl acetate = 1:1 to 1:4) to obtain a crude product. The crude product was further purified by preparative HPLC (preparation conditions: preparative column: YMC Triart C18 12 nm 10u, 30 × 250 mm; number: none; flow rate: 40 mL/min; column temperature: room temperature; mobile phase: A: water (0.1% ammonia water) B: preparative grade acetonitrile) to obtain compound 143 (85.1 mg). MS m/z (ESI): 531.95 [M+H]⁺. ¹H NMR (400 MHz, DMSO-d₆) δ 8.89 (d, J= 5.2 Hz, 1H), 8.60 (d, *J= 2.3* Hz, 1H), 8.16 - 8.02 (m, 1H), 7.88 - 7.67 (m, 3H), 7.46 (dd, *J= 5.2,* 1.8 Hz, 1H), 7.30 (t, J= 7.7 Hz, 1H), 6.75 (s, 1H), 5.48 (s, 2H), 5.16 (s, 1H), 4.67 (s, 1H), 3.73 - 3.52 (m, 2H), 2.02 (s, 3H), 1.46 (d, *J* = 3.3 Hz, 3H).

### Example 78 Synthesis of Compound 144

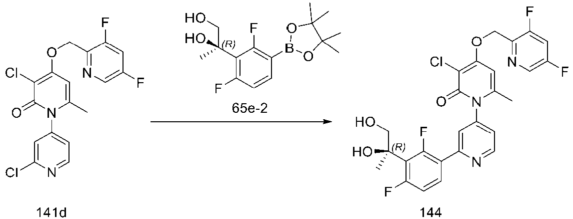

Tetrakis(triphenylphosphine)palladium (45 mg, 0.04 mmol) was added to a mixed solution of compound 141d (150 mg, 0.37 mmol), compound 65e-2 (235 mg, 0.75 mmol), and sodium carbonate (80 mg, 0.75 mmol) in 1,4-dioxane and water (5 mL/0.5 mL). The reaction mixture was stirred at 90°C for 2 hours. After the reaction was completed, the reaction mixture was concentrated under reduced pressure. The residue was purified by silica gel column chromatography (dichloromethane: methanol = 1:0 to 10:1) to obtain a crude product. The crude product was further purified by preparative HPLC (preparation conditions: preparative column: YMC Triart C18 12 nm 10u, 30 × 250 mm; number: none; flow rate: 40 mL/min; column temperature: room temperature; mobile phase: A: water (0.1% ammonia water) B: preparative grade acetonitrile) to obtain compound 144 (55.6 mg). MS m/z (ESI): 549.95 [M+H]⁺. ¹H NMR (400 MHz, DMSO-d₆) δ 8.88 (d, J= 5.2 Hz, 1H), 8.60 (d, *J* = 2.3 Hz, 1H), 8.22 - 8.02 (m, 1H), 7.82 (dd, *J* = 14.8, 6.8 Hz, 1H), 7.73 (s, 1H), 7.46 (dd, *J* = 5.2, 1.8 Hz, 1H), 7.14 (dd, *J* = 11.3, 9.1 Hz, 1H), 6.75 (s, 1H), 5.47 (d, *J = 1.2* Hz, 2H), 5.15 (s, 1H), 4.87 (s, 1H), 3.63 (ddd, *J* = 22.7, 10.8, 5.0 Hz, 2H), 2.02 (s, 3H), 1.59 (s, 3H).

### Example 79 Synthesis of Compound 145

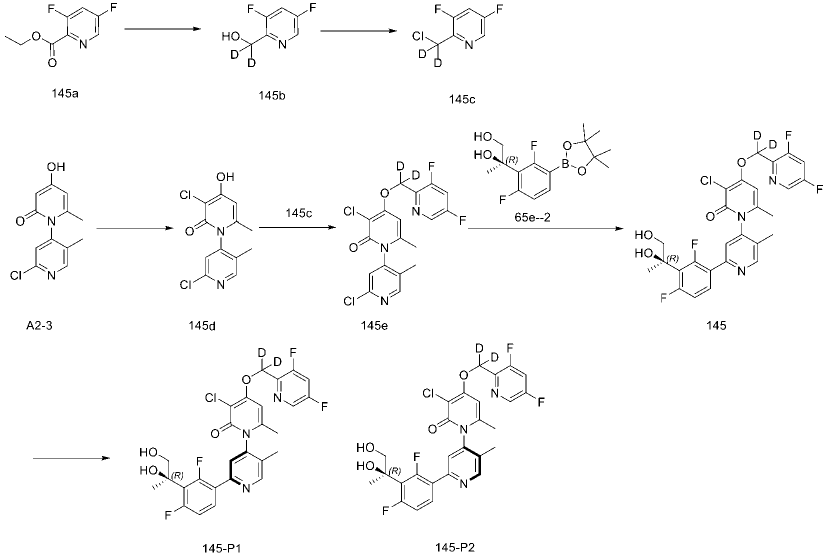

### Step 1: Synthesis of Compound 145b

At 0°C, lithium aluminum deuteride (1.35 g, 32 mmol) was added to a solution of compound 145a (5 g, 26.7 mmol) in tetrahydrofuran (80 mL). The reaction mixture was stirred at 0°C for 3 hours. After the reaction was completed, the reaction mixture was quenched sequentially with water (1.5 mL), 15% sodium hydroxide solution (1.5 mL), and water (4.5 mL), then filtered. The filtrate was concentrated under reduced pressure, and the residue was purified by silica gel column chromatography (petroleum ether/ethyl acetate = 1/0 to 4/1) to obtain compound 145b (1.6 g). MS m/z (ESI): 147.9 [M+1]⁺.

### Step 2: Synthesis of Compound 145c

At 0°C, thionyl chloride (485.2 mg, 4.08 mmol) and N,N-dimethylformamide (25 mg, 0.34 mmol) were sequentially added to a solution of compound 145b (500 mg, 3.4 mmol) in dichloromethane (15 mL). The reaction mixture was stirred at room temperature for 2 hours. After the reaction was completed, the reaction mixture was concentrated under reduced pressure to obtain compound 145c (450 mg).

### Step 3: Synthesis of Compound 145d

NCS (22.3 g, 167 mmol) was added to a solution of compound A2-3 (40 g, 159 mmol) in acetonitrile (200 mL). The reaction mixture was stirred under reflux for 3 hours. After the reaction was completed, the reaction mixture was concentrated under reduced pressure to remove acetonitrile. Water (120 mL) was added to the residue, and the mixture was stirred for 30 minutes and then filtrated. The filter cake was washed with water (100 mL) and dried to obtain compound 145d (36 g). MS m/z (ESI): 285.0 [M+H]⁺.

### Step 4: Synthesis of Compound 145e

Compound 145d (6.56 g, 23.03 mmol), compound 145c (5.70 g, 34.54 mmol), potassium carbonate (6.36 g, 46.06 mmol), and 18-crown-6 (608 mg, 2.30 mmol) were added to N,N-dimethylformamide (33 mL). The reaction mixture was heated to 55°C and stirred for 16 hours. After the reaction was completed, the reaction mixture was diluted with water (130 mL), stirred for 30 minutes, and filtered. The filter cake was rinsed with water (30 mL), collected, stirred with methanol (65 mL) at 70°C for 30 minutes, and then filtered. The filter cake was collected and dried to obtain compound 145e (7.2 g). MS m/z (ESI): 413.95 [M+1]⁺.

### Step 5: Synthesis of Compounds 145, 145-P1, and 145-P2

Compound 145e (2.00 g, 4.84 mmol), compound 65e-2 (3.04 g, 9.68 mmol), 1,1'-bis(diphenylphosphino)ferrocene-palladium(II)dichloride dichloromethane complex (395 mg, 0.48 mmol), and potassium carbonate (1.33 g, 9.68 mmol) were added to a mixed solution of 1,4-dioxane and water (50 mL/10 mL). The reaction mixture was stirred at 90°C for 3 hours. After the reaction was completed, the reaction mixture was concentrated under reduced pressure to remove the solvent. The residue was purified by silica gel column chromatography (dichloromethane: methanol = 1:0 to 15:1) to obtain a crude product. The crude product was further purified by preparative HPLC (preparation conditions: preparative column: YMC Triart C18 12 nm 10u, 30 × 250 mm; number: none; flow rate: 40 mL/min; column temperature: room temperature; mobile phase: A: water (0.1% ammonia water) B: preparative grade acetonitrile) to obtain compound 145 (820 mg). Compound 145 (820 mg) was then resolved by preparative chiral chromatography (preparative column: YMC CHIRALART Amylose-C NEO, 30 × 250 mm; flow rate: 30 mL/min; column temperature: room temperature; mobile phase: A: n-hexane B: isopropanol) to obtain compounds 145-P1 (280.9 mg) and 145-P2 (136.3 mg).

### Compound 145-P1:

MS m/z (ESI): 565.95 [M+1]⁺. Chiral HPLC: retention time = 17.54 min, UV = 220 nm. ¹H NMR (400 MHz, DMSO-d₆) δ 8.79 (s, 1H), 8.60 (d, *J* = 2.3 Hz, 1H), 8.09 (td, *J* = 9.4, 2.2 Hz, 1H), 7.82 (dd, *J* = 14.5, 8.5 Hz, 1H), 7.66 (s, 1H), 7.12 (dd, *J* = 11.4, 9.2 Hz, 1H), 6.79 (s, 1H), 5.12 (s, 2H), 3.63 (dd, *J* = 26.3, 10.8 Hz, 2H), 2.05 (s, 3H), 1.97 (s, 3H), 1.59 (s, 3H).

### Compound 145-P2:

MS m/z (ESI): 565.95 [M+1]⁺. Chiral HPLC: retention time = 21.11 min, UV = 220 nm. ¹H NMR (400 MHz, DMSO-d₆) δ 8.78 (s, 1H), 8.60 (d, *J* = 2.2 Hz, 1H), 8.09 (td, *J* = 9.8, 2.3 Hz, 1H), 7.81 (dd, *J=* 14.5, 8.5 Hz, 1H), 7.66 (s, 1H), 7.19 - 7.01 (m, 1H), 6.79 (s, 1H), 5.13 (s, 1H), 4.86 (s, 1H), 3.63 (q, *J=* 10.7 Hz, 2H), 2.05 (s, 3H), 1.98 (s, 3H), 1.58 (s, 3H).

### Example 80 Synthesis of Compound 146

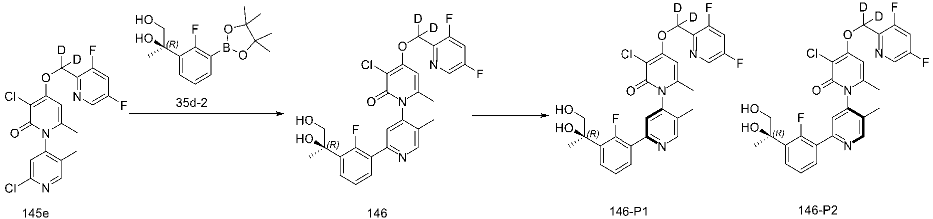

At room temperature, compound 35d-2 (1.4 g, 3.38 mmol), compound 145e (4.8 g, crude product, content converted to about 6.7 mmol), potassium carbonate (931 mg, 6.7 mmol), and 1,1'-bis(diphenylphosphino)ferrocene-palladium(II)dichloride dichloromethane complex (280 mg, 0.34 mmol) were added to a mixed solution of 1,4-dioxane and water (35 mL/7 mL). The reaction mixture was stirred at 70°C for 2 hours. After the reaction was completed, the reaction mixture was diluted with water (50 mL) and extracted with ethyl acetate (40 mL × 3). The combined organic phases were washed with saturated brine (40 mL × 2), dried over anhydrous sodium sulfate, and filtered. The filtrate was concentrated under reduced pressure, and the residue was purified by silica gel column chromatography (dichloromethane: methanol = 40:1 to 20:1) to obtain a crude product. The crude product was further purified by preparative HPLC (chromatographic column: YMC-Triart C18 30 × 250 mm, mobile phase: A: 0.1% ammonia water-water, B: acetonitrile; flow rate: 40 mL/min) to obtain compound 146 (1.0 g). Compound 146 (1.0 g) was then resolved by preparative chiral chromatography (preparative column: CHIRAL ART Amylose-C NEO, 30 × 250 mm, flow rate: 30 mL/min; column temperature: room temperature; mobile phase: A: n-hexane, B: ethanol) to obtain compounds 146-P1 (440 mg) and 146-P2 (343.0 mg).

### Compound 146-P1:

MS m/z (ESI): 548.05 [M+1]⁺. Chiral HPLC: retention time = 14.18 min, UV = 220 nm. ¹H NMR (400 MHz, DMSO-d₆) δ 8.79 (s, 1H), 8.60 (d, J= 2.4 Hz, 1H), 8.09 (m, 1H), 7.81 - 7.67 (m, 3H), 7.29 (t, *J= 7.6* Hz, 1H), 6.80 (d, *J=* 0.8 Hz, 1H), 3.64 - 3.56 (m, 2H), 2.05 (s, 3H), 1.98 (s, 3H), 1.46 (s, 3H).

### Compound 146-P2:

MS m/z (ESI): 548.05 [M+1]⁺. Chiral HPLC: retention time = 19.29 min, UV = 220 nm. ¹H NMR (400 MHz, DMSO-d₆) δ 8.79 (s, 1H), 8.60 (d, *J= 2.4* Hz, 1H), 8.09 (m,1H), 7.83 - 7.68 (m, 3H), 7.29 (t, *J=* 7.6 Hz, 1H), 6.79 (s, 1H), 5.15 (s, 1H), 4.68 (m, 1H), 3.61 (m, 2H), 2.05 (s, 3H), 1.98 (s, 3H), 1.45 (s, 3H).

### Example 81 Synthesis of Compound 147

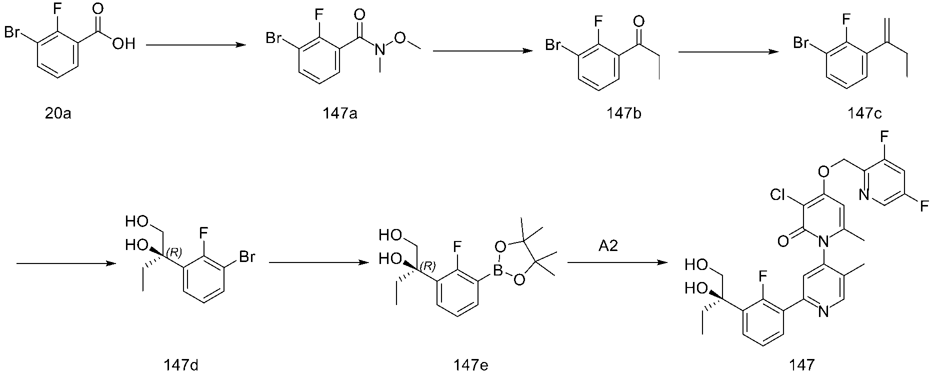

### Step 1: Synthesis of Compound 147a

Compound 20a (5.50 g, 25.11 mmol), *N,O*-dimethylhydroxylamine hydrochloride (3.65 g, 37.67 mmol), 2-(7-azabenzotriazol-1-yl)-N,N,N;N'-tetramethyluronium hexafluorophosphate (14.31 g, 37.67 mmol), and *N*,*N-*diisopropylethylamine (9.72 g, 75.33 mmol) were dissolved in dichloromethane (100 mL). The reaction mixture was stirred at room temperature for 2 hours. After the reaction was completed, the reaction mixture was diluted with water (100 mL) and extracted with dichloromethane (200 mL × 3). The combined organic phases were concentrated under reduced pressure. The residue was purified by silica gel column chromatography (petroleum ether: ethyl acetate = 5:1 to 4:1) to obtain compound 147a (6.4 g). MS m/z (ESI): 261.95 [M+H]⁺.

### Step 2: Synthesis of Compound 147b

At -78°C, ethylmagnesium bromide (6.67 mL, 20.00 mmol, 3 M in THF) was added dropwise to a solution of compound 147a (2.60 g, 10.00 mmol) in THF (30 mL). The reaction mixture was stirred at room temperature for 16 hours. After the reaction was completed, the reaction mixture was cooled to 0°C, and 2 M hydrochloric acid (2 mL) was added dropwise to quench. The mixture was diluted with water (30 mL), and the pH was adjusted to 1 with 2 M hydrochloric acid. The mixture was extracted with ethyl acetate (50 mL × 3). The combined organic phases were concentrated under reduced pressure, and the residue was purified by silica gel column chromatography (petroleum ether: ethyl acetate = 5:1) to obtain compound 147b (1.1 g). MS m/z (ESI): 230.95 [M+H]⁺.

Step 3: Synthesis of Compound 147c

At 0°C, a solution of potassium tert-butoxide (643 mg, 5.74 mmol) in tetrahydrofuran (5 mL) was added dropwise to a solution of methyltriphenylphosphonium bromide (2.05 g, 5.74 mmol) in tetrahydrofuran (15 mL). The mixture was stirred for 1 hour, followed by the dropwise addition of a solution of 147b (1.10 g, 4.78 mmol) in tetrahydrofuran (5 mL). The reaction mixture was stirred at room temperature for 16 hours. After the reaction was completed, the reaction mixture was quenched by dropwise addition of saturated aqueous ammonium chloride solution (20 mL), diluted with water (30 mL), and extracted with ethyl acetate (50 mL × 3). The combined organic phases were concentrated under reduced pressure, and the residue was purified by silica gel column chromatography (petroleum ether) to obtain compound 147c (720 mg). ¹H NMR (400 MHz, DMSO-d₆) δ 7.70 - 7.55 (m, 1H), 7.39 - 7.26 (m, 1H), 7.14 (t, *J* = 7.8 Hz, 1H), 5.30 (s, 1H), 5.15 (s, 1H), 2.42 (q, *J* = 7.3 Hz, 2H), 0.97 (t, *J = 7.4* Hz, 3H).

### Step 4: Synthesis of Compound 147d

Compound 147c (720 mg, 3.14 mmol) was dissolved in a mixed solution of tert-butanol and water (10 mL/10 mL). Potassium carbonate (1.30 g, 9.43 mmol), potassium ferricyanide (3.10 g, 9.43 mmol), potassium osmate monohydrate (6 mg, 0.016 mmol), and hydroquinidine 1,4-phthalazinediyl diether (25 mg, 0.032 mmol) were sequentially added. The reaction mixture was stirred at room temperature for 24 hours. After the reaction was completed, the reaction mixture was quenched with saturated aqueous sodium sulfite solution (20 mL), then diluted with water (30 mL), and extracted with ethyl acetate (50 mL × 3). The combined organic phases were concentrated under reduced pressure, and the residue was purified by silica gel column chromatography (dichloromethane: methanol = 1:0 to 20:1) to obtain compound 147d (720 mg). ¹H NMR (400 MHz, DMSO-d₆) δ 7.62-7.57 (m, 2H), 7.12 (t, *J=* 7.9 Hz, 1H), 4.99 (s, 1H), 4.69 (t, *J=* 6.0 Hz, 1H), 3.67 (ddd, *J=* 10.8, 6.4, 1.4 Hz, 1H), 3.55 (dd, *J=* 10.9, 5.5 Hz, 1H), 1.92 - 1.62 (m, 2H), 0.64 (t, *J=* 7.4 Hz, 3H).

### Step 5: Synthesis of Compound 147e

Compound 147d (700 mg, 2.67 mmol), bis(pinacolato)diboron (2.03 g, 8.01 mmol), 1,1'-bis(diphenylphosphino)ferrocene-palladium(II)dichloride dichloromethane complex (217 mg, 0.26 mmol), and potassium acetate (523 mg, 5.34 mmol) were added to 1,4-dioxane (10 mL). The reaction mixture was stirred at 90°C for 2 hours. After the reaction was completed, the reaction mixture was concentrated to dryness under reduced pressure. The residue was purified by silica gel column chromatography (petroleum ether: ethyl acetate = 4:1 to 1:2) to obtain compound 147e (1.5 g, crude product, theoretical 830 mg). MS m/z (ESI): 328.10 [M+NH₄]⁺.

### Step 6: Synthesis of Compound 147

Compound A2 (200 mg, 0.48 mmol), compound 147e (302 mg, crude product), potassium carbonate (134 mg, 0.97 mmol), and 1,1'-bis(diphenylphosphino)ferrocene-palladium(II)dichloride dichloromethane complex (39 mg, 0.05 mmol) were sequentially added to a solution of 1,4-dioxane and water (5 mL/1 mL). The reaction mixture was stirred at 90°C for 2 hours. After the reaction was completed, the reaction mixture was concentrated under reduced pressure. The residue was purified by silica gel column chromatography (dichloromethane: methanol = 1:0 to 10:1) to obtain a crude product. The crude product was further purified by preparative HPLC (preparation conditions: preparative column: YMC Triart C18 12 nm 10u, 30 × 250 mm; flow rate: 40 mL/min; column temperature: room temperature; mobile phase: A: water (0.1% ammonia water) B: preparative grade acetonitrile) to obtain compound 147 (98.4 mg). MS m/z (ESI): 560.05 [M+H]⁺. ¹H NMR (400 MHz, DMSO-d₆) δ 8.79 (s, 1H), 8.60 (d, J= 2.3 Hz, 1H), 8.20 - 8.03 (m, 1H), 7.77 (dd, *J* = 14.4, 7.1 Hz, 1H), 7.70 (d, *J* = 9.1 Hz, 2H), 7.29 (t, *J* = 7.7 Hz, 1H), 6.80 (s, 1H), 5.48 (s, 2H), 4.91 (s, 1H), 4.64 (t, *J=* 6.0 Hz, 1H), 3.72 (dd, *J =* 16.9, 10.2 Hz, 1H), 3.60 (dt, *J=* 10.5, 5.1 Hz, 1H), 2.04 (d, *J* = 7.6 Hz, 3H), 1.98 (s, 3H), 1.95 - 1.85 (m, 1H), 1.81 - 1.71 (m, 1H), 0.73 - 0.63 (m, 3H).

### Example 82 Synthesis of Compound 148

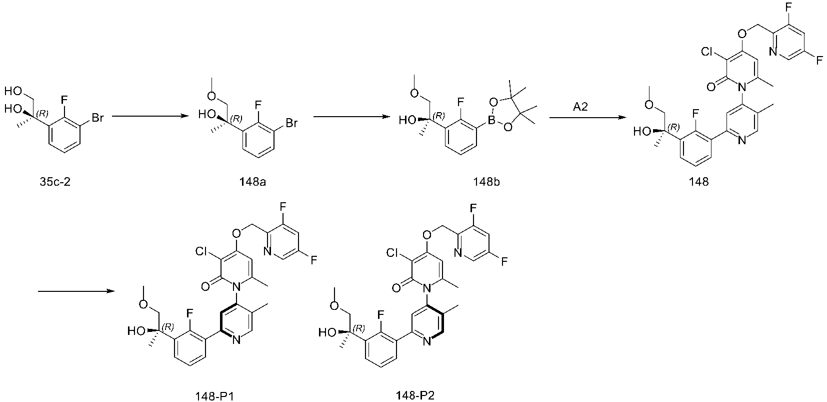

### Step 1: Synthesis of Compound 148a

Iodomethane (600 mg, 4.23 mmol) was added to a solution of compound 35c-2 (700 mg, 2.82 mmol) and cesium carbonate (1.38 g, 4.23 mmol) in N,N-dimethylformamide (10 mL). The reaction mixture was stirred at room temperature for 16 hours. After the reaction was completed, the reaction mixture was diluted with water (20 mL) and extracted with ethyl acetate (30 mL × 3). The combined organic phases were concentrated under reduced pressure, and the residue was purified by silica gel column chromatography (petroleum ether: ethyl acetate = 5:1 to 3:1) to obtain compound 148a (450 mg). ¹H NMR (600 MHz, DMSO-d₆) δ 7.59 (dt, *J* = 14.2, 7.5 Hz, 2H), 7.13 (t, *J=* 7.9 Hz, 1H), 5.47 (s, 1H), 3.57 (d, *J = 9.8* Hz, 1H), 3.47 (d, *J = 9.8* Hz, 1H), 3.22 (s, 3H), 1.42 (s, 3H).

### Step 2: Synthesis of Compound 148b

Compound 148a (450 mg, 1.71 mmol), bis(pinacolato)diboron (651 mg, 2.56 mmol), 1,1'-bis(diphenylphosphino)ferrocene-palladium(II)dichloride dichloromethane complex (139 mg, 0.17 mmol), and potassium acetate (335 mg, 3.42 mmol) were added to 1,4-dioxane (10 mL). The reaction mixture was stirred at 90°C for 2 hours. After the reaction was completed, the reaction mixture was cooled to room temperature and directly used in the next step.

### Step 3: Synthesis of Compounds 148, 148-P1, and 148-P2

Compound A2 (360 mg, 0.87 mmol), compound 148b (10 mL of the previous reaction mixture), potassium carbonate (236 mg, 1.71 mmol), and 1,1'-bis(diphenylphosphino)ferrocene-palladium(II)dichloride dichloromethane complex (73 mg, 0.09 mmol) were added to a mixed solution of 1,4-dioxane and water (10 mL/2 mL). The reaction mixture was stirred at 90°C for 2 hours. After the reaction was completed, the reaction mixture was concentrated under reduced pressure. The residue was purified by preparative HPLC (preparation conditions: preparative column: YMC Triart C18 12 nm 10u, 30 × 250 mm; flow rate: 40 mL/min; column temperature: room temperature; mobile phase: A: water (0.1% ammonia water), B: preparative grade acetonitrile) to obtain compound 148 (146.8 mg). Compound 148 (146.8 mg) was then resolved by preparative chiral chromatography (preparative column: YMC CHIRALART cellulose-SC, 30 × 250 mm; flow rate: 30 mL/min; column temperature: room temperature; mobile phase: B: methanol) to obtain compounds 148-P1 (12.7 mg) and 148-P2 (43.7 mg).

### Compound 148-P1:

MS m/z (ESI): 560.15 [M+1]⁺. Chiral HPLC: retention time = 9.69 min, UV = 220 nm. ¹H NMR (400 MHz, DMSO-d₆) δ 8.79 (s, 1H), 8.60 (d, *J= 2.3* Hz, 1H), 8.09 (td, *J = 9.9,* 2.4 Hz, 1H), 7.78 (td, *J =* 7.5, 1.7 Hz, 1H), 7.74 - 7.67 (m, 2H), 7.29 (t, *J =* 7.7 Hz, 1H), 6.80 (s, 1H), 5.48 (d, *J =* 1.2 Hz, 2H), 5.37 (s, 1H), 3.57 (dd, *J =* 25.6, 10.3 Hz, 2H), 3.24 (s, 3H), 2.05 (s, 3H), 1.98 (s, 3H), 1.48 (s, 3H).

### Compound 148-P2:

MS m/z (ESI): 560.15 [M+1]⁺. Chiral HPLC: retention time = 14.35 min, UV = 220 nm. ¹H NMR (400 MHz, DMSO-d₆) δ 8.79 (s, 1H), 8.60 (d, *J=* 2.4 Hz, 1H), 8.09 (ddd, *J* = 9.9, 9.0, 2.4 Hz, 1H), 7.79 (td, *J* = 7.5, 1.8 Hz, 1H), 7.75 - 7.64 (m, 2H), 7.29 (t, *J* = 7.7 Hz, 1H), 6.80 (s, 1H), 5.48 (d, *J =* 1.5 Hz, 2H), 5.40 (s, 1H), 3.57 (dd, *J* = 30.9, 10.8 Hz, 2H), 3.23 (s, 3H), 2.05 (s, 3H), 1.98 (s, 3H), 1.47 (s, 3H).

### Example 82 Synthesis of Compound 149

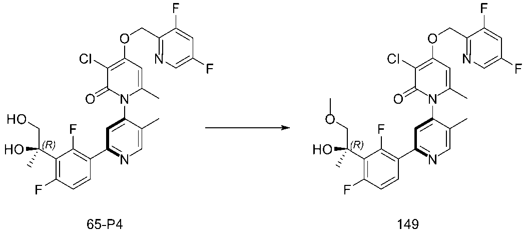

Cesium carbonate (49 mg, 0.15 mmol) and iodomethane (21 mg, 0.15 mmol) were sequentially added to a solution of compound 65-P4 (56 mg, 0.10 mmol) in N,N-dimethylformamide (2 mL). The reaction mixture was stirred at room temperature for 16 hours. After the reaction was completed, the reaction mixture was diluted with water (10 mL) and extracted with ethyl acetate (10 mL × 3). The combined organic phases were washed with saturated brine (20 mL × 1), dried over anhydrous sodium sulfate, and filtered. The filtrate was concentrated under reduced pressure. The residue was purified by preparative HPLC (preparation conditions: preparative column: YMC Triart C18 12 nm 10u, 30 × 250 mm; flow rate: 40 mL/min; column temperature: room temperature; mobile phase: A: water (0.1% ammonia water) B: preparative grade acetonitrile) to obtain compound 149 (10.3 mg). MS m/z (ESI): 578.00 [M+H]⁺. ¹H NMR (400 MHz, DMSO-d₆) δ 8.78 (s, 1H), 8.60 (d, *J=* 2.3 Hz, 1H), 8.20 - 8.01 (m, 1H), 7.82 (dd, *J =* 14.6, 8.5 Hz, 1H), 7.66 (d, *J* = 1.5 Hz, 1H), 7.13 (dd, *J* = 11.2, 9.1 Hz, 1H), 6.79 (s, 1H), 5.47 (s, 2H), 3.59 (q, J = 9.3 Hz, 2H), 3.25 (s, 3H), 2.05 (s, 3H), 1.97 (s, 3H), 1.61 (s, 3H).

### Example 83 Synthesis of Compound 150

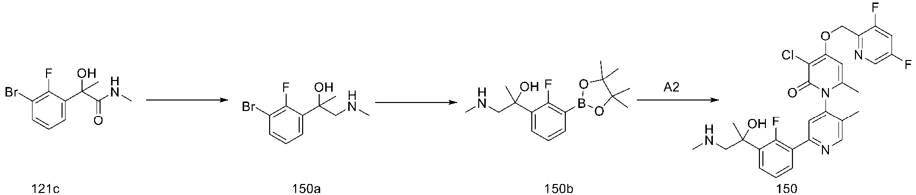

### Step 1: Synthesis of Compound 150a

Borane-methyl sulfide complex (10.36 mL, 10.36 mmol, 10 M) was added dropwise to a solution of compound 121c (2.85 g, 10.36 mmol) in tetrahydrofuran (40 mL), and the reaction mixture was stirred at 70°C for 4 hours. After the reaction was completed, the reaction mixture was slowly quenched by dropwise addition of methanol (20 mL), diluted with water (30 mL), and extracted with ethyl acetate (100 mL × 1). The aqueous phase was adjusted to pH = 9-10 with saturated aqueous sodium carbonate solution and extracted with ethyl acetate (100 mL × 2). The combined organic phases were dried over anhydrous sodium sulfate and filtered. The filtrate was concentrated under reduced pressure, and the residue was purified by silica gel column chromatography (dichloromethane: methanol: triethylamine = 1:0:0 to 10:1:1%) to obtain compound 150a (1.9 g). MS m/z (ESI): 262.00 [M+H]⁺.

### Step 2: Synthesis of Compound 150b

150a (1.90 g, 7.28 mmol), bis(pinacolato)diboron (2.77 g, 10.92 mmol), 1,1'-bis(diphenylphosphino)ferrocene-palladium(II)dichloride dichloromethane complex (596 mg, 0.73 mmol), and potassium acetate (1.43 g, 14.56 mmol) were added to 1,4-dioxane (40 mL). The reaction mixture was stirred at 90°C for 3 hours. After the reaction was completed, the reaction mixture was cooled to room temperature and directly used in the next step.

### Step 3: Synthesis of Compound 150

Compound A2 (1.56 g, 3.84 mmol), compound 150b (40 mL of the previous reaction mixture), potassium carbonate (1.06 g, 7.68 mmol), and 1,1'-bis(diphenylphosphino)ferrocene-palladium(II)dichloride dichloromethane complex (324 mg, 0.40 mmol) were added to a mixed solution of 1,4-dioxane and water (20 mL/5 mL). The reaction mixture was stirred at 90°C for 5 hours. After the reaction was completed, the reaction mixture was concentrated under reduced pressure. The residue was diluted with water (40 mL), and the pH was adjusted to 2-3 with 1 M HCl. The mixture was extracted with ethyl acetate (100 mL × 1). The aqueous phase was adjusted to pH = 9-10 with saturated aqueous sodium carbonate solution and extracted again with ethyl acetate (100 mL × 1). The resulting organic phase was concentrated under reduced pressure, and the residue was purified by preparative HPLC (preparation conditions: chromatographic column: YMC-Triart C18 30 × 250 mm; mobile phase: A: 0.1% hydrochloric acid-water, B: acetonitrile; flow rate: 40 mL/min) to obtain compound 150 (549.8 mg). MS m/z (ESI): 559.20 [M+H]⁺. ¹H NMR (400 MHz, DMSO-d₆) δ 8.83 (s, 1H), 8.74 (s, 1H), 8.60 (d, *J=* 2.2 Hz, 1H), 8.35 (d, *J=* 55.4 Hz, 1H), 8.16 - 8.06 (m, 1H), 7.88 (dt, *J=* 14.0, 7.0 Hz, 1H), 7.81 - 7.70 (m, 2H), 7.39 (td, *J = 7.7,* 2.8 Hz, 1H), 6.83 (d, *J* = 2.6 Hz, 1H), 5.48 (s, 2H), 3.44 - 3.20 (m, 2H), 2.50 (d, *J =* 1.5 Hz, 3H), 2.07 (s, 3H), 1.99 (s, 3H), 1.61 (s, 3H).

### Example 84 Synthesis of Compound 151

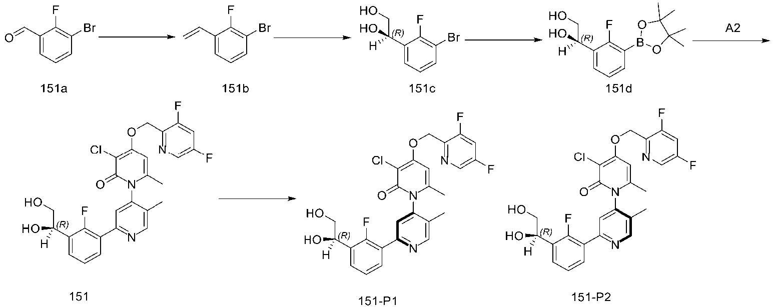

### Step 1: Synthesis of Compound 151b

At 0°C, a solution of potassium tert-butoxide (51.7 mL, 1 M) in tetrahydrofuran was added to a solution of methyltriphenylphosphonium bromide (18.48 g, 51.7 mmol) in tetrahydrofuran (20 mL). After stirring the mixture for 30 minutes, a solution of compound 151a (7 g, 34.5 mmol) in tetrahydrofuran (10 mL) was added dropwise at 0°C. The reaction mixture was stirred at room temperature for 2 hours. After the reaction was completed, the reaction mixture was diluted with water (50 mL) and extracted with ethyl acetate (100 mL × 3). The combined organic phases were washed with saturated aqueous sodium chloride solution (100 mL × 2), dried over anhydrous sodium sulfate, and filtered. The filtrate was concentrated under reduced pressure, and the residue was purified by silica gel column chromatography (petroleum ether: ethyl acetate = 1:0) to obtain compound 151b (4.2 g). ¹H NMR (400 MHz, CDCl₃) δ 7.43 (dd, J = 13.6, 6.8 Hz, 2H), 6.99 (t, J = 8.0 Hz, 1H), 6.85 (dd, J = 17.6, 11.2 Hz, 1H), 5.84 (d, J = 17.6 Hz, 1H), 5.42 (d, J = 11.2 Hz, 1H).

### Step 2: Synthesis of Compound 151c

Compound 151b (2.50 g, 12.4 mmol), hydroquinidine 1,4-phthalazinediyl diether (48.4 mg, 0.062 mmol), potassium osmate dihydrate (45.8 mg, 0.12 mmol), potassium ferricyanide hexahydrate (12.3 g, 37.3 mmol), and potassium carbonate (5.16 g, 37.3 mmol) were sequentially added to a mixed solution of tert-butanol and water (25.0 mL/8.3 mL). The reaction mixture was stirred at room temperature for 58 hours. After the reaction was completed, saturated sodium sulfite (100 mL) was added to the reaction mixture, followed by extraction with ethyl acetate (50 mL × 3). The combined organic phases were dried over anhydrous sodium sulfate and filtered. The filtrate was concentrated under reduced pressure, and the residue was purified by silica gel column chromatography (petroleum ether/ethyl acetate = 1/5 to 1/1) to obtain compound 151c (2.08 g). ¹H NMR (400 MHz, DMSO-d₆) δ 7.61 - 7.54 (m, 1H), 7.49 (t, J = 7.2 Hz, 1H), 7.15 (t, J = 7.6 Hz, 1H), 5.52 (d, J = 4.8 Hz, 1H), 4.89 - 4.79 (m, 2H), 3.46 (t, J = 5.6 Hz, 2H).

### Step 3: Synthesis of Compound 151d

Compound 151c (2.08 g, 8.85 mmol), bis(pinacolato)diboron (4.05 g, 15.9 mmol), potassium acetate (1.74 g, 17.7 mmol), and 1,1'-bis(diphenylphosphino)ferrocene palladium(II)dichloride (647 mg, 0.89 mmol) were sequentially added to 1,4-dioxane (20 mL). The reaction mixture was stirred at 90°C for 2 hours until the reaction was completed. The reaction mixture was filtered through diatomaceous earth to remove insoluble materials, and the filtrate was concentrated under reduced pressure. The residue was purified by silica gel column chromatography (petroleum ether/ethyl acetate = 1/5 to 1/1) to obtain compound 151d (1.3 g).

### Step 4: Synthesis of Compounds 151, 151-P1, and 151-P2

Compound A2 (400 mg, 0.97 mmol), compound 151d (547.31 mg, 1.94 mmol), sodium carbonate (206 mg, 1.94 mmol), and tetrakis(triphenylphosphine)palladium (112 mg, 0.097 mmol) were added to a mixture of 1,4-dioxane and water (10 mL/2.0 mL). The reaction mixture was stirred at 90°C for 2 hours. After the reaction was completed, the reaction mixture was filtered through diatomaceous earth to remove insoluble matter, and the filtrate was concentrated under reduced pressure. The residue was purified by silica gel column chromatography (petroleum ether/ethyl acetate = 1/10 to 2/1) to obtain a crude product. The crude product was further purified by preparative HPLC (preparative column: YMC Triart C18, 30 × 250 mm; flow rate: 40 mL/min; column temperature: room temperature; mobile phase: A: water (0.1% ammonia water-water) B: preparative grade acetonitrile) to obtain compound 151 (380 mg). Compound 151 (380 mg) was then resolved by chiral preparative chromatography (preparative column: YMC CHIRALART Amylose-C NEO, 30 × 250 mm; flow rate: 30 mL/min; column temperature: room temperature; mobile phase: A: n-hexane, B: isopropanol) to obtain compounds 151-P1 (144 mg) and 151-P2 (149.8 mg).

### Compound 151-P1:

MS m/z (ESI): 532.10 [M+1]⁺. Chiral HPLC: retention time = 14.71 min, UV = 220 nm. ¹H NMR (400 MHz, DMSO-d₆) δ 8.80 (s, 1H), 8.60 (d, J = 2.0 Hz, 1H), 8.13 - 8.05 (m, 1H), 7.81 (t, *J* = 7.2 Hz, 1H), 7.71 (s, 1H), 7.59 (t, J = 7.2 Hz, 1H), 7.32 (t, J = 7.6 Hz, 1H), 6.80 (s, 1H), 5.48 (s, 3H), 4.93-4.85 (m, 2H), 3.55 (dd, J=10.8, 4.0 Hz, 1H), 3.46 (dd, J = 11.2, 7.2 Hz, 1H), 2.05 (s, 3H), 1.98 (s, 3H).

### Compound 151-P2:

MS m/z (ESI): 532.10 [M+1]⁺. Chiral HPLC: retention time = 20.09 min, UV = 220 nm. ¹H NMR (400 MHz, DMSO-d₆) δ 8.78 (s, 1H), 8.58 (d, J = 2.0 Hz, 1H), 8.07 (td, J = 10.0, 2.4 Hz, 1H), 7.80 (td, J = 7.6, 1.6 Hz, 1H), 7.70 (s, 1H), 7.57 (t, J = 7.2 Hz, 1H), 7.30 (t, J = 7.6 Hz, 1H), 6.78 (s, 1H), 5.46 (s, 3H), 4.91-4.83 (m, 2H), 3.53 (dd, J = 11.2, 4.4 Hz, 1H), 3.44 (dd, J = 11.2, 7.2 Hz, 1H), 2.04 (s, 3H), 1.97 (s, 3H).

### Example 85 Synthesis of Compound 153

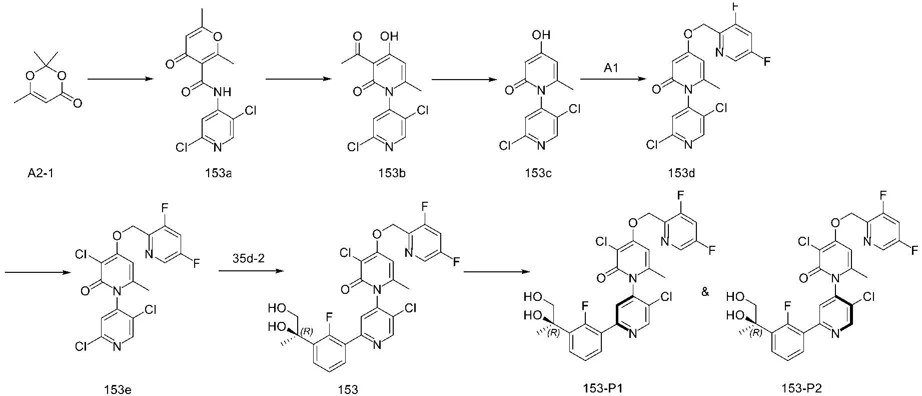

### Step 1: Synthesis of Compound 153a

2,5-Dichloro-4-aminopyridine (2 g, 12.27 mmol) was added to a solution of compound A2-1 (6.45 g, 45.40 mmol) in N,N-dimethylacetamide (20 mL), and the reaction mixture was stirred at 120°C for 4 hours. After the reaction was completed, the reaction mixture was cooled to 50 to 60°C, diluted with water (30 mL), and further cooled to room temperature and stirred for 30 minutes. The mixture was filtered, and the filter cake was rinsed with water (30 mL). The filter cake was collected and dried to obtain compound 152a (3.8 g). MS m/z (ESI): 312.95 [M+H]⁺.

### Step 2: Synthesis of Compound 153b

Compound 153a (3.8 g, 12.14 mmol) was added to a 350 mL glass sealed tube, followed by the sequential addition of an isopropanol solution of hydrochloric acid (4 M, 60 mL) and water (40 mL). The reaction mixture was stirred at 100°C overnight. After the reaction was completed, the reaction mixture was concentrated under reduced pressure to remove isopropanol. The remaining mixture was filtered, and the filter cake was added with a mixed solution of water and isopropanol (20 mL/4 mL), stirred, and then filtered. The collected filter cake was dried to obtain compound 153b (2.6 g). MS m/z (ESI): 312.90 [M+H]⁺.

### Step 3: Synthesis of Compound 153c

Compound 153b (2.6 g, 8.30 mmol) was dissolved in concentrated sulfuric acid (5 mL), and the reaction mixture was stirred at 120°C for 2.5 hours. After the reaction was completed, the reaction mixture was cooled to room temperature and diluted with water (20 mL) under an ice bath. The pH was adjusted to 9 with 30% aqueous sodium hydroxide solution, then the pH was further adjusted to 3 with saturated aqueous citric acid solution. The mixture was filtered, and the filter cake was rinsed with water (20 mL). The collected filter cake was added with ethyl acetate (26 mL) and stirred for 30 minutes. The mixture was filtered, and the collected filter cake was dried to obtain compound 153c (1.95 g). MS m/z (ESI): 270.90 [M+H]⁺.

### Step 4: Synthesis of Compound 153d

Compound 153c (1.75 g, 6.46 mmol), compound A1 (1.27 g, 7.75 mmol), potassium carbonate (1.79 g, 12.92 mmol), and 18-crown-6 (0.17 g, 0.65 mmol) were sequentially added to N,N-dimethylformamide (17 mL). The reaction mixture was stirred at 55°C for 2 hours. After the reaction was completed, the reaction mixture was diluted with water (40 mL) and extracted with ethyl acetate (60 mL × 3). The combined organic phases were washed with saturated brine (60 mL × 1), and the organic phase was concentrated under reduced pressure. The residue was purified by silica gel column chromatography (petroleum ether: ethyl acetate = 3:1 to 1:2) to obtain compound 153d (2.69 g). MS m/z (ESI): 397.95 [M+H]⁺.

### Step 5: Synthesis of Compound 153e

N-Chlorosuccinimide (0.91 g, 6.78 mmol) was added to a solution of compound 153d (2.69 g, 6.78 mmol) and *p-*toluenesulfonic acid (0.058 g, 0.34 mmol) in N,N-dimethylformamide (S0, 27 mL). The reaction mixture was stirred at 75°C for 1 hour. After the reaction was completed, the reaction mixture was diluted with water (50 mL), extracted with ethyl acetate (60 mL × 3), and the combined organic phases were washed with saturated brine (100 mL × 1) and concentrated under reduced pressure. The residue was purified by silica gel column chromatography (petroleum ether: ethyl acetate = 3:1 to 1:3) to obtain compound 153e (2.21 g). MS m/z (ESI): 431.90 [M+H]⁺.

### Step 6: Synthesis of Compounds 153, 153-P1, and 153-P2

Compound 153e (400 mg, 0.92 mmol), compound 35d-2 (544.90 mg, 1.84 mmol), tetrakis(triphenylphosphine)palladium (106.31 mg, 0.092 mmol), and sodium carbonate (195.02 mg, 1.84 mmol) were sequentially added to a mixed solution of 1,4-dioxane and water (5 mL/1 mL). The reaction mixture was stirred at 90°C for 1.5 hours. After the reaction was completed, the reaction mixture was concentrated under reduced pressure, and the residue was purified by silica gel column chromatography (dichloromethane: methanol = 1:0 to 10:1) to obtain a crude product. The crude product was further purified by preparative HPLC (preparation conditions: chromatographic column: YMC Triart C18 50 × 250 mm; mobile phase: A: 0.1% ammonia water-water, B: acetonitrile, flow rate: 70 mL/min) to obtain compound 153, resulting in a total of 432 mg of compound 153 after combining with the small-scale synthesis batch. Compound 153 (432 mg) was then resolved by chiral preparative chromatography (preparative column: YMC CHIRALART cellulose-SC, 30 × 250 mm; flow rate: 40 mL/min; column temperature: room temperature; mobile phase: A: water, B: acetonitrile) to obtain compounds 153-P1 (155.1 mg) and 153-P2 (142.5 mg).

### Compound 153-P1:

MS m/z (ESI): 566.05 [M+1]⁺. Chiral HPLC: retention time = 16.95 min, UV = 220 nm. ¹H NMR (400 MHz, DMSO-d₆) δ 9.04 (s, 1H), 8.60 (d, *J* = 2.2 Hz, 1H), 8.17 - 8.06 (m, 1H), 8.04 (s, 1H), 7.77 (dd, *J=* 12.4, 7.1 Hz, 2H), 7.32 (t, *J =* 7.7 Hz, 1H), 6.83 (s, 1H), 5.50 (s, 2H), 5.19 (s, 1H), 4.70 (t, *J=* 6.1 Hz, 1H), 3.61 (d, *J =* 6.0 Hz, 2H), 2.02 (s, 3H), 1.46 (s, 3H).

### Compound 153-P2:

MS m/z (ESI): 566.05 [M+1]⁺. Chiral HPLC: retention time = 22.10 min, UV = 220 nm. ¹H NMR (400 MHz, DMSO-d₆) δ 9.04 (s, 1H), 8.60 (d, *J =* 2.3 Hz, 1H), 8.09 (td, *J=* 9.6, 2.3 Hz, 1H), 8.04 (d, *J* = 1.3 Hz, 1H), 7.77 (dt, *J=* 11.1, 5.4 Hz, 2H), 7.32 (t, *J* = 7.7 Hz, 1H), 6.83 (s, 1H), 5.50 (s, 2H), 5.19 (s, 1H), 4.70 (t, J= 6.1 Hz, 1H), 3.61 (ddd, *J* = 24.3, 11.1, 6.2 Hz, 2H), 2.02 (s, 3H), 1.46 (s, 3H).

### Example 86 Synthesis of Compound 154

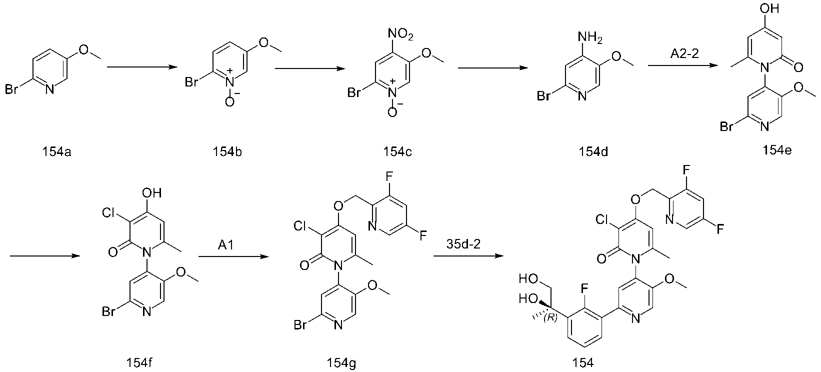

### Step 1: Synthesis of Compound 154b

At room temperature, m-chloroperoxybenzoic acid (9.72 g, 47.86 mmol, content of 85%) was added to a solution of compound 154b (5 g, 26.59 mmol) in 1,2-dichloroethane (50 mL). The reaction mixture was stirred at 60°C overnight. After the reaction was completed, the reaction mixture was quenched with saturated aqueous sodium sulfite solution (20 mL), diluted with water (50 mL), and adjusted to pH = 9-10 by adding solid sodium bicarbonate. The mixture was extracted with dichloromethane (50 mL × 3), and the combined organic phases were washed with saturated brine (50 mL), dried over anhydrous sodium sulfate, and filtered. The filtrate was concentrated under reduced pressure to obtain compound 154b (4.6 g). MS m/z (ESI): 203.90 [M+1]⁺.

### Step 2: Synthesis of Compound 154c

At room temperature, concentrated sulfuric acid (27.6 g, 281.40 mmol) and nitric acid (21.15 g, 218.18 mmol, purity 65%) were sequentially added to compound 154b (4.6 g, 22.55 mmol). The reaction mixture was stirred overnight at 60°C. After the reaction was completed, the reaction mixture was cooled to 0°C. The reaction mixture was slowly added dropwise to water (200 mL), resulting in the precipitation of a large amount of yellow solid. The mixture was filtered, and the filter cake was rinsed with water (20 mL). The collected filter cake was dried to obtain compound 154c (3.6 g). MS m/z (ESI): 248.90 [M+1]⁺.

### Step 3: Synthesis of Compound 154d

At room temperature, compound 154c (3.6 g, 14.46 mmol), iron powder (8.08 g, 144.60 mmol), and ammonium chloride (7.73 g, 144.60 mmol) were sequentially added to a mixed solution of ethanol and water (60 mL/18 mL). The reaction mixture was stirred at 75°C for 3 hours. After the reaction was completed, the reaction mixture was filtered. Water (40 mL) was added to the filtrate, which was then extracted with dichloromethane (40 mL × 3). The combined organic phases were washed with saturated brine (50 mL), dried over anhydrous sodium sulfate, and filtered. The filtrate was concentrated under reduced pressure to obtain compound 154d (2.7 g). MS m/z (ESI): 204.95 [M+1]⁺.

### Step 4: Synthesis of Compound 154e

At room temperature, compound 154d (2.2 g, 10.84 mmol) and compound A2-2 (3.99 g, 21.68 mmol) were added to 1,4-dioxane (30 mL). The mixture was stirred at 90°C for 1 hour. After the complete disappearance of the starting materials, concentrated sulfuric acid (1.06 g, 10.84 mmol) was added, and the reaction mixture was stirred at 90°C for another 0.5 hours. After the reaction was completed, the reaction mixture was cooled to room temperature and concentrated under reduced pressure to remove the solvent. The residue was then diluted with water (100 mL), and the mixture was stirred at room temperature for 0.5 hours and filtered. The filter cake was rinsed with water (10 mL), and the collected filter cake was dried to obtain compound 154e (2.1 g). MS m/z (ESI): 310.90 [M+1]⁺.

### Step 5: Synthesis of Compound 154f

At room temperature, compound 154e (1.9 g, 6.11 mmol), N-chlorosuccinimide (0.98 g, 7.33 mmol), and *p-*toluenesulfonic acid (0.11 g, 0.61 mmol) were added to N,N-dimethylformamide (20 mL). The reaction mixture was stirred at 60°C for 1 hour. After the reaction was completed, the reaction mixture was cooled to room temperature, diluted with water (100 mL), resulting in the precipitation of a large amount of solid. The mixture was filtered, and the filter cake was rinsed with water (10 mL). The collected filter cake was dried to obtain compound 154f (1.7 g). MS m/z (ESI): 346.85 [M+1]⁺.

### Step 6: Synthesis of Compound 154g

At room temperature, compound 154f (1.5 g, 4.82 mmol), compound A1 (0.87 g, 5.30 mmol), and potassium carbonate (1.33 g, 9.64 mmol) were sequentially added to *N,N*-dimethylformamide (30 mL). The reaction mixture was stirred at 60°C for 1 hour. After the reaction was completed, the reaction mixture was diluted with water (150 mL), resulting in the precipitation of a large amount of solid. The mixture was filtered, and the filter cake was rinsed with water (20 mL). The collected filter cake was added to ethyl acetate (10 mL), stirred at room temperature for 10 minutes, and then filtered. The filter cake was rinsed with a mixed solvent of ethyl acetate and petroleum ether (1 mL/1 mL). The collected filter cake was dried to obtain compound 154g (1.0 g). MS m/z (ESI): 473.90 [M+1]⁺.

### Step 7: Synthesis of Compound 154

At room temperature, compound 154g (200 mg, 0.42 mmol), compound 35d-2 (0.50 g, 1.68 mmol), 1,1'-bis(diphenylphosphino)ferrocene-palladium(II)dichloride dichloromethane complex (33 mg, 0.042 mmol), and potassium carbonate (153 mg, 1.06 mmol) were sequentially added to a mixed solution of 1,4-dioxane and water (5 mL/1 mL). The reaction mixture was stirred at 85°C for 2 hours. After the reaction was completed, the reaction mixture was diluted with water (20 mL) and extracted with ethyl acetate (20 mL × 3). The combined organic phases were washed with saturated brine (20 mL), dried over anhydrous sodium sulfate, and filtered. The filtrate was concentrated under reduced pressure, and the residue was purified by silica gel column chromatography (PE: EA = 1:3) to obtain a crude product. The crude product was further purified by preparative HPLC (preparative column: YMC Triart C18 12 nm 10u, 30 × 250mm, flow rate: 40 mL/min, column temperature: room temperature, mobile phase: A: water (0.1% ammonia water), B: preparative grade acetonitrile) to obtain compound 154 (168.2 mg). MS m/z (ESI): 562.10 [M+1]⁺. ¹H NMR (400 MHz, DMSO-d₆) δ 8.71 (s, 1H), 8.56 (d, J = 2.4 Hz, 1H), 8.05 (td, J = 9.6, 2.4 Hz, 1H), 7.80 - 7.61 (m, 3H), 7.24 (t, J = 7.6 Hz, 1H), 6.69 (s, 1H), 5.44 (s, 2H), 5.10 (d, J = 3.6 Hz, 1H), 4.64 (td, J = 6.0, 2.8 Hz, 1H), 3.91 (s, 3H), 3.64 - 3.48 (m, 2H), 1.96 (s, 3H), 1.41 (d, J = 2.8 Hz, 3H).

### Example 87 Synthesis of Compound 155

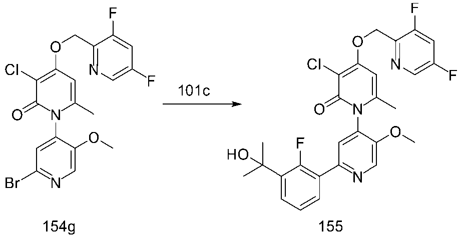

At room temperature, compound 154g (200 mg, 0.42 mmol), compound 101c (0.21 g, 0.76 mmol), 1,1'-bis(diphenylphosphino)ferrocene-palladium(II)dichloride dichloromethane complex (34 mg, 0.042 mmol), and potassium carbonate (150 mg, 1.05 mmol) were sequentially added to a mixed solution of 1,4-dioxane and water (5 mL/1 mL). The reaction mixture was stirred at 85°C for 2 hours. After the reaction was completed, the reaction mixture was diluted with water (20 mL) and extracted with ethyl acetate (20 mL × 3). The combined organic phases were washed with saturated brine (20 mL), dried over anhydrous sodium sulfate, and filtered. The filtrate was concentrated under reduced pressure, and the residue was purified by silica gel column chromatography (PE: EA = 1:3) to obtain a crude product. The crude product was further purified by preparative HPLC (preparative column: YMC Triart C18 12 nm 10u, 30 × 250mm, flow rate: 40 mL/min, column temperature: room temperature, mobile phase: A: water (0.1% ammonia water), B: preparative grade acetonitrile) to obtain compound 155 (128.2 mg). MS m/z (ESI): 546.10 [M+1]⁺. ¹H NMR (400 MHz, DMSO-d₆) δ 8.70 (s, 1H), 8.55 (d, J = 2.4 Hz, 1H), 8.14 - 7.94 (m, 1H), 7.76 - 7.59 (m, 3H), 7.23 (t, J = 7.6 Hz, 1H), 6.68 (s, 1H), 5.43 (s, 2H), 5.27 (s, 1H), 3.90 (s, 3H), 1.95 (s, 3H), 1.48 (s, 6H).

### Example 88 Synthesis of Compound 156

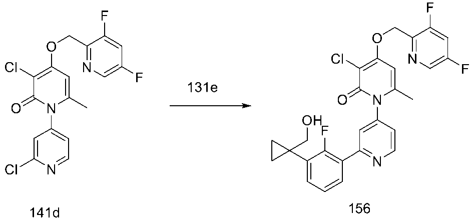

Compound 141d (150 mg, 0.38 mmol), compound 131e (166.53 mg, 0.57 mmol), tetrakis(triphenylphosphine)palladium (43.91 mg, 0.038 mmol), and sodium carbonate (80.55 mg, 0.76 mmol) were sequentially added to a mixed solution of 1,4-dioxane and water (6 mL/1.2 mL). The reaction mixture was stirred at 90°C for 1.5 hours. After the reaction was completed, the reaction mixture was concentrated under reduced pressure, and the residue was purified by silica gel column chromatography (dichloromethane: methanol = 1:0 to 10:1) to obtain a crude product. The crude product was further purified by preparative HPLC (preparation conditions: chromatographic column: YMC Triart C18 50 × 250 mm; mobile phase: A: 0.1% ammonia water-water, B: acetonitrile, flow rate: 70 mL/min) to obtain compound 156 (120 mg). MS m/z (ESI): 528.10 [M+H]⁺. ¹H NMR (400 MHz, DMSO-d₆) δ 8.89 (d, *J =* 5.2 Hz, 1H), 8.60 (d, *J* = 2.2 Hz, 1H), 8.09 (td, *J* = 9.9, 2.3 Hz, 1H), 7.81 (dd, *J=* 9.4, 1.5 Hz, 2H), 7.58 - 7.37 (m, 2H), 7.26 (t, *J=* 7.6 Hz, 1H), 6.76 (s, 1H), 5.48 (s, 2H), 4.68 (t, *J=* 5.9 Hz, 1H), 3.48 (d, *J=* 5.8 Hz, 2H), 2.03 (s, 3H), 0.86 (s, 2H), 0.76 (d, *J=* 10.2 Hz, 2H).

### Example 89 Synthesis of Compound 35

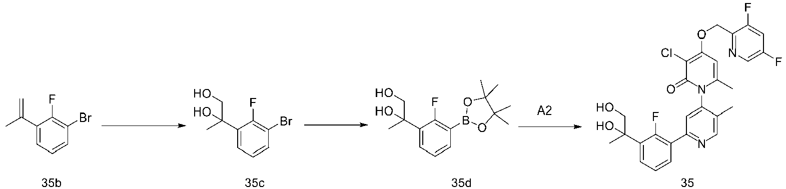

### Step 1: Synthesis of Compound 35c

Compound 35b (1.3 g, 6.04 mmol), potassium osmate dihydrate (1.78 g, 4.83 mmol), and N-methylmorpholine N-oxide (1.42 g, 12.08 mmol) were sequentially added to a mixed solution of tert-butanol and tetrahydrofuran (6 mL/18 mL). The reaction mixture was stirred at room temperature for 7 hours. After the reaction was completed, the reaction mixture was quenched with saturated aqueous sodium sulfite solution (50 mL) and extracted with ethyl acetate (50 mL × 3). The combined organic phases were concentrated under reduced pressure, and the residue was purified by silica gel column chromatography (dichloromethane: methanol = 1:0 to 10:1) to obtain compound 35c (0.77 g).

### Step 2: Synthesis of Compound 35d

Compound 35c (770 mg, 3.09 mmol), bis(pinacolato)diboron (1.18 g, 4.63 mmol), 1,1'-bis(diphenylphosphino)ferrocene-palladium(II)dichloride dichloromethane complex (252.34 mg, 0.31 mmol), and potassium acetate (606.51 mg, 6.18 mmol) were sequentially added to 1,4-dioxane (40 mL). The reaction mixture was stirred at 90°C for 4 hours. After the reaction was completed, the reaction mixture was concentrated under reduced pressure. The residue was subjected to silica gel column chromatography (petroleum ether: ethyl acetate = 3:1 to 1:4) for preliminary purification to obtain compound 35d (1.1 g, crude product, purity: 50%). MS m/z (ESI): 314.10 [M+18]⁺.

### Step 3: Synthesis of Compound 35

Compound A2 (150 mg, 0.36 mmol), compound 35d (319.83 mg, 0.54 mmol, purity: 50%), tetrakis(triphenylphosphine)palladium (41.60 mg, 0.036 mmol), and sodium carbonate (76.31 mg, 0.72 mmol) were sequentially added to a mixed solution of 1,4-dioxane and water (4 mL/0.8 mL). The reaction mixture was stirred at 90°C for 2 hours. After the reaction was completed, the reaction mixture was concentrated under reduced pressure, and the residue was purified by silica gel column chromatography (dichloromethane: methanol = 1:0 to 10:1) to obtain a crude product. The crude product was further purified by preparative HPLC (preparation conditions: chromatographic column: YMC Triart C18 50 × 250 mm; mobile phase: A: 0.1% ammonia water-water, B: acetonitrile, flow rate: 70 mL/min) to obtain compound 35 (114.3 mg). MS m/z (ESI): 546.10 [M+H]⁺. ¹H NMR (400 MHz, DMSO-d₆) δ 8.79 (s, 1H), 8.60 (d, *J =* 2.3 Hz, 1H), 8.09 (td, *J* = 9.7, 2.3 Hz, 1H), 7.84 - 7.66 (m, 3H), 7.29 (t, *J* = 7.7 Hz, 1H), 6.80 (s, 1H), 5.48 (s, 2H), 5.16 (d, *J* = 2.7 Hz, 1H), 4.68 (t, *J* = 5.7 Hz, 1H), 3.72 - 3.48 (m, 2H), 2.05 (s, 3H), 1.98 (s, 3H), 1.45 (s, 3H).

### Example 90 Synthesis of Compounds 157, 157-P1, and 157-P2

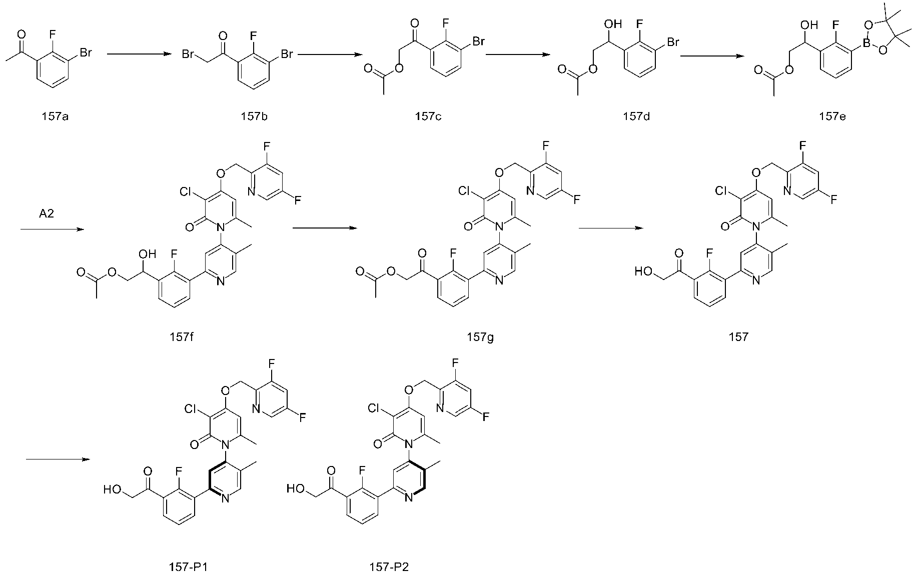

### Step 1: Synthesis of Compound 157b

At room temperature, bromine (1.93 g, 12.10 mmol) was added dropwise to a solution of compound 157a (2.5 g, 11.52 mmol) in acetic acid (25 mL), and the reaction mixture was stirred overnight at room temperature. After the reaction was completed, the reaction mixture was diluted with water (50 mL) and extracted with ethyl acetate (50 mL × 2). The combined organic phases were washed with saturated brine (50 mL × 1), dried over anhydrous sodium sulfate, and filtered. The filtrate was concentrated under reduced pressure to obtain compound 157b (3.5 g, crude product).

### Step 2: Synthesis of Compound 157c

At room temperature, compound 157b (3.4 g, crude product), potassium acetate (3.38 g, 34.47 mmol), and potassium iodide (1.91 g, 11.49 mmol) were sequentially added to N,N-dimethylformamide (50 mL), and the reaction mixture was stirred at room temperature for 1 hour. After the reaction was completed, water (200 mL) was added to the reaction mixture for dilution, followed by extraction with ethyl acetate (50 mL × 2). The combined organic phases were washed with saturated brine (50 mL × 1), dried over anhydrous sodium sulfate, and filtered. The filtrate was concentrated under reduced pressure, and the residue was subjected to silica gel column chromatography (petroleum ether: ethyl acetate = 3: 1 to 1:1) to obtain compound 157c (2.4 g). MS m/z (ESI): 276.90 [M+1]⁺.

### Step 3: Synthesis of Compound 157d

At 0°C, sodium borohydride (0.33 g, 8.73 mmol) was added to a solution of compound 157c (2.4 g, 8.73 mmol) in methanol (30 mL), and the reaction mixture was stirred at room temperature for 0.5 hours. After the reaction was completed, the reaction mixture was quenched with 1 N hydrochloric acid and concentrated under reduced pressure. The residue was purified by silica gel column chromatography (petroleum ether: ethyl acetate = 6:1 to 3:1) to obtain compound 157d (1.7 g) as a colorless oil. MS m/z (ESI): 258.95 [M-17]⁺.

### Step 4: Synthesis of Compound 157e

At room temperature, bis(pinacolato)diboron (2.81 g, 11.05 mmol), potassium acetate (1.21 g, 12.28 mmol), and 1,1'-bis(diphenylphosphino)ferrocene-palladium(II)dichloride dichloromethane complex (0.50 g, 0.61 mmol) were sequentially added to a solution of compound 157d (1.7 g, 6.14 mmol) in 1,4-dioxane (40 mL). The reaction mixture was stirred at 90°C under a nitrogen atmosphere for 2 hours. After the reaction was completed, the reaction mixture was directly concentrated under reduced pressure, and the residue was purified by silica gel column chromatography (petroleum ether: ethyl acetate = 5:1 to 3:1) to obtain compound 157e (1.7 g). MS m/z (ESI): 307.05 [M-17]⁺.

### Step 5: Synthesis of Compound 157f

At room temperature, compound 157e (1.7 g, 5.24 mmol), compound A2 (1.08 g, 2.62 mmol), sodium carbonate (0.83 g, 7.86 mmol), and tetrakis(triphenylphosphine)palladium (0.61 g, 0.52 mmol) were sequentially added to a mixed solution of 1,4-dioxane and water (30 mL/5 mL). The reaction mixture was stirred at 90°C under a nitrogen atmosphere for 2 hours. After the reaction was completed, the reaction mixture was concentrated under reduced pressure, and the residue was purified by silica gel column chromatography (dichloromethane: methanol = 30:1 to 10:1) to obtain compound 157f (1.4 g). MS m/z (ESI): 574.10 [M+1]⁺.

### Step 6: Synthesis of Compound 157g

At room temperature, compound 157f (300 mg, 0.52 mmol) and IBX (0.58 g, 2.08 mmol) were added to acetonitrile (10 mL), and the reaction mixture was stirred at 80°C for 1 hour. After the reaction was completed, the reaction mixture was diluted with water (20 mL) and extracted with ethyl acetate (20 mL × 3). The combined organic phases were washed with saturated brine (20 mL), dried over anhydrous sodium sulfate, and filtered. The filtrate was concentrated under reduced pressure to obtain compound 157g (300 mg, crude product). MS m/z (ESI): 572.10 [M+1]⁺.

### Step 7: Synthesis of Compounds 157, 157-P1, and 157-P2

At room temperature, compound 157g (200 mg, 0.35 mmol) and 1 N hydrochloric acid (2 mL, 2 mmol) were added to methanol (2 mL). The reaction mixture was stirred at 60°C for 1 hour. After the reaction was completed, the reaction mixture was diluted with water (20 mL) and extracted with ethyl acetate (20 mL × 3). The combined organic phases were washed with saturated brine (20 mL), dried over anhydrous sodium sulfate, and filtered. The filtrate was concentrated under reduced pressure, and the residue was purified by silica gel column chromatography (petroleum ether: ethyl acetate = 1:0/1:3) to obtain a crude product of compound 157. The crude product was further purified by preparative HPLC (preparative column: YMC Triart C18 12 nm 10u, 30 × 250 mm; flow rate: 40 mL/min; column temperature: room temperature; mobile phase: A: water (0.1% acetic acid), B: preparative grade acetonitrile) to obtain compound 157 (90 mg). Compound 157 (90 mg) was then resolved by chiral preparative chromatography (preparative column: YMC CHIRALART cellulose-SC, 30 × 250 mm; flow rate: 40 mL/min; column temperature: room temperature; mobile phase: A: water, B: acetonitrile) to obtain compounds 157-P1 (27.1 mg) and 157-P2 (26.4 mg).

### Compound 157-P1:

MS m/z (ESI): 530.15 [M+1]⁺. Chiral HPLC: retention time = 16.04 min, UV = 220 nm. ¹H NMR (400 MHz, DMSO-d₆) δ 8.81 (s, 1H), 8.58 (d, J = 2.4 Hz, 1H), 8.18 - 8.02 (m, 2H), 7.90 (dd, J = 10.0, 4.0 Hz, 1H), 7.77 (s, 1H), 7.46 (t, J = 7.6 Hz, 1H), 6.79 (s, 1H), 5.46 (s, 2H), 5.25 (t, J = 6.0 Hz, 1H), 4.64 (dd, J = 6.0, 2.4 Hz, 2H), 2.05 (s, 3H), 1.97 (s, 3H).

### Compound 157-P2:

MS m/z (ESI): 530.15 [M+1]⁺. Chiral HPLC: retention time = 20.84 min, UV = 220 nm. ¹H NMR (400 MHz, DMSO-d₆) δ 8.81 (s, 1H), 8.58 (s, 1H), 8.10 (dt, J = 18.8, 8.4 Hz, 2H), 7.89 (t, J = 7.2 Hz, 1H), 7.77 (s, 1H), 7.47 (t, J = 7.6 Hz, 1H), 6.79 (s, 1H), 5.46 (s, 2H), 5.25 (t, J = 6.0 Hz, 1H), 4.64 (d, J = 3.6 Hz, 2H), 2.05 (s, 3H), 1.97 (s, 3H).

### Example 91 Synthesis of Compounds 158, 158-P1, and 158-P2

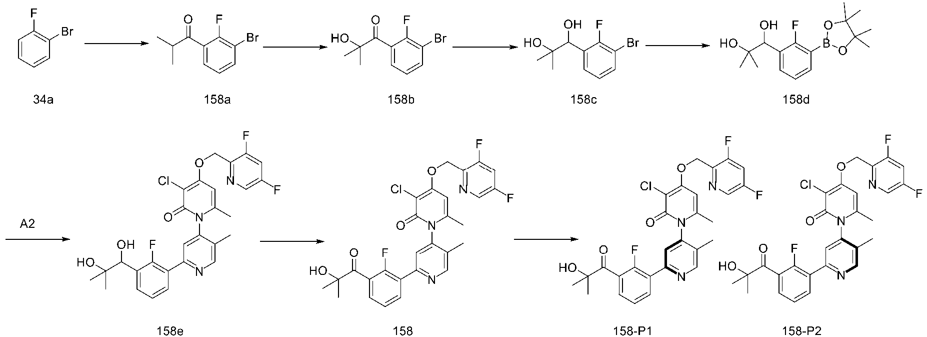

### Step 1: Synthesis of compound 158a

At -78°C, lithium diisopropylamide (21.43 mL, 42.86 mmol) was slowly added dropwise to a solution of compound 34a (5 g, 28.57 mmol) in tetrahydrofuran (50 mL). The reaction mixture was stirred at -78°C for 2 hours, followed by the addition of *N*-methoxy-*N*,2-dimethylpropanamide (4.12 g, 31.43 mmol, CAS: 113778-69-1). The reaction mixture was then stirred for another 1 hour. After the reaction was completed, the reaction mixture was quenched with saturated aqueous ammonium chloride solution (50 mL) and extracted with ethyl acetate (50 mL × 3). The combined organic phases were washed with saturated brine (50 mL × 1), dried over anhydrous sodium sulfate, and filtered. The filtrate was concentrated under reduced pressure, and the residue was purified by silica gel column chromatography (petroleum ether: ethyl acetate = 5:1 to 3:1) to obtain compound 158a (3.5 g). MS m/z (ESI): 244.95 [M+1]⁺.

### Step 2: Synthesis of Compound 158b

At room temperature, compound 158a (3.5 g, 14.28 mmol) and N-bromosuccinimide (0.51 g, 2.86 mmol) were sequentially added to dimethyl sulfoxide (50 mL), and the reaction mixture was stirred at 100°C overnight. After the reaction was completed, the reaction mixture was diluted with water (200 mL) and extracted with ethyl acetate (50 mL × 3). The combined organic phases were washed with saturated brine (50 mL × 1), dried over anhydrous sodium sulfate, and filtered. The filtrate was concentrated under reduced pressure, and the residue was purified by silica gel column chromatography (petroleum ether: ethyl acetate = 3:1 to 2:1) to obtain compound 158b (2.2 g). MS m/z (ESI): 260.95 [M+1]⁺.

### Step 3: Synthesis of Compound 158c

Sodium borohydride (0.23 g, 6.13 mmol) was added to a solution of compound 158b (1.6 g, 6.13 mmol) in methanol (30 mL) at room temperature, and the reaction mixture was stirred for 0.5 hours at room temperature. After the reaction was completed, 1 N hydrochloric acid was added to the reaction mixture until no obvious bubbles were generated. The reaction mixture was concentrated under reduced pressure, and the residue was purified by silica gel column chromatography (petroleum ether: ethyl acetate = 6:1 to 3:1) to obtain compound 158c (1.3 g).

### Step 4: Synthesis of Compound 158d

At room temperature, compound 158c (1.3 g, 4.94 mmol), bis(pinacolato)diboron (2.01 g, 7.90 mmol), potassium acetate (0.97 g, 9.88 mmol), and 1,1'-bis(diphenylphosphino)ferrocene-palladium(II)dichloride dichloromethane complex (0.40 g, 0.49 mmol) were added to 1,4-dioxane (30 mL). The reaction mixture was stirred at 90°C for 2 hours. After the reaction was completed, the reaction mixture was concentrated under reduced pressure. The residue was purified by silica gel column chromatography (petroleum ether: ethyl acetate = 3:1 to 2:1) to obtain a crude product (1.0 g). The crude product was purified by preparative HPLC (preparative column: YMC Triart C18, 12 nm, 10 u, 30 × 250 mm; flow rate: 40 mL/min; column temperature: room temperature; mobile phase: A: water (0.1% trifluoroacetic acid), B: preparative grade acetonitrile) to obtain compound 158d (100mg). MS m/z (ESI): 328.15 [M+18]⁺.

### Step 5: Synthesis of Compound 158e

At room temperature, compound 158d (100 mg, 0.32 mmol), compound A2 (0.13 g, 0.32 mmol), sodium carbonate (0.068 g, 0.64 mmol), and tetrakis(triphenylphosphine)palladium (0.037 g, 0.032 mmol) were sequentially added to a mixed solution of 1,4-dioxane and water (4 mL/0.5 mL). The reaction mixture was stirred at 90°C for 2 hours. After the reaction was completed, the reaction mixture was concentrated under reduced pressure, and the residue was purified by silica gel column chromatography (dichloromethane: methanol = 30:1 to 20:1) to obtain compound 158e (200 mg, crude product). MS m/z (ESI): 560.10 [M+1]⁺.

### Step 6: Synthesis of Compounds 158, 158-P1, and 158-P2

At room temperature, compound 158e (200 mg, 0.36 mmol) and IBX (20 g, 0.72 mmol) were sequentially added to acetonitrile (8 mL), and the reaction mixture was stirred at 80°C for 1 hour. After the reaction was completed, the reaction mixture was diluted with water (20 mL) and extracted with ethyl acetate (20 mL × 3). The combined organic phases were washed with saturated brine (20 mL × 1), dried over anhydrous sodium sulfate, and filtered. The filtrate was concentrated under reduced pressure, and the residue was purified by silica gel column chromatography (PE: EA = 1:0 to 1:3) to obtain a crude product. The crude product was further purified by preparative HPLC (preparative column: YMC Triart C18 12 nm 10u, 30 × 250mm, flow rate: 40 mL/min, column temperature: room temperature, mobile phase: A: water (0.1% TFA), B: preparative grade acetonitrile) to obtain compound 158 (80 mg). 80 mg of compound 158 was then resolved by preparative chiral chromatography (preparative column: YMC CHIRALART cellulose-SC, 30 × 250 mm, flow rate: 30 mL/min, column temperature: room temperature, mobile phase: A: n-hexane, B: isopropanol) to obtain compounds 158-P1 (36.8 mg) and 158-P2 (34.6 mg).

### Compound 158-P1:

MS m/z (ESI): 558.15 [M+1]⁺. Chiral HPLC: retention time = 8.63 min, UV = 220 nm. ¹H NMR (400 MHz, DMSO-d₆) δ 8.78 (s, 1H), 8.55 (d, J = 2.4 Hz, 1H), 8.10 - 7.91 (m, 2H), 7.71 (s, 1H), 7.54 (t, J = 6.0 Hz, 1H), 7.35 (t, J = 7.6 Hz, 1H), 6.75 (s, 1H), 5.49 (s, 1H), 5.43 (s, 2H), 2.02 (s, 3H), 1.93 (s, 3H), 1.33 (s, 6H).

### Compound 158-P2:

MS m/z (ESI): 558.15 [M+1]⁺. Chiral HPLC: retention time = 12.83 min, UV = 220 nm.

¹H NMR (400 MHz, DMSO-d₆) δ 8.78 (s, 1H), 8.55 (d, J = 2.4 Hz, 1H), 8.04-8.00 (m, 2H), 7.71 (s, 1H), 7.54 (t, J = 6.4 Hz, 1H), 7.35 (t, J = 7.6 Hz, 1H), 6.75 (s, 1H), 5.49 (s, 1H), 5.43 (s, 2H), 2.02 (s, 3H), 1.93 (s, 3H), 1.33 (s, 6H).

### Example 92 Synthesis of Compound 163

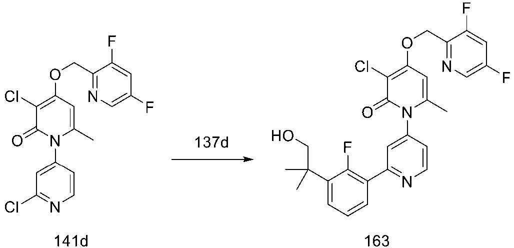

Compound 141d (150 mg, 0.38 mmol), compound 137d (167.68 mg, 0.57 mmol), tetrakis(triphenylphosphine)palladium (43.91 mg, 0.038 mmol), and sodium carbonate (80.55 mg, 0.76 mmol) were sequentially added to a mixed solution of 1,4-dioxane and water (6 mL/1.2 mL). The reaction mixture was stirred at 90°C for 2 hours. After the reaction was completed, the reaction mixture was concentrated under reduced pressure. The residue was purified by silica gel column chromatography (dichloromethane: methanol = 1:0 to 10:1) to obtain a crude product of compound 163 (160 mg). The crude product was further purified by preparative HPLC (chromatographic column: YMC Triart C18 50 × 250 mm; mobile phase: A: 0.1% ammonia water-HCl, B: acetonitrile, flow rate: 70 mL/min) to obtain compound 163 (59 mg). MS m/z (ESI): 530.15 [M+H]⁺. ¹H NMR (400 MHz, DMSO-d₆) δ 8.91 (d, *J =* 5.2 Hz, 1H), 8.60 (d, *J* = 2.1 Hz, 1H), 8.32 - 8.00 (m, 1H), 7.81 (s, 1H), 7.73 (t, *J= 7.2* Hz, 1H), 7.62 - 7.37 (m, 2H), 7.27 (t, *J=* 7.7 Hz, 1H), 6.77 (s, 1H), 5.46-5.48 (m, 3H), 3.72 - 3.41 (m, 2H), 2.03 (s, 3H), 1.32 (s, 6H).

### Biological Evaluation

### Test Example 1. In Vitro Activity Assay of p38 MAPK/MK2

The inhibitory effect of the compound on p38 MAPK/MK2 was evaluated using the Z-LYTE kinase assay kit (Thermo, PV3177). The test compound was dissolved in DMSO to obtain a 10 mM stock solution and stored at -20°C for future use. The initial concentration of the compound was 10 µM, with 1% DMSO, 5-fold serial dilution, 8 concentrations, and duplicate wells. The reaction buffer consisted of 50 mM HEPES pH 7.5, 10 mM MgCl₂, 0.01% Brij-35, and 1 mM EGTA, which was used to prepare 2x active p38a/inactive MK2/Ser/Thr 4 mixture. The final 10 µL reaction system was performed in a 384-well plate (Corning, 4514), containing 500 ng/mL inactive MK2 (Abcam, 79910), 8 ng/mL active p38a (Carna, 04-152), and 2 µM Ser/Thr 4. After a 1-hour reaction at 20°C, Development Reagent A diluted 2048-fold was added to each well, followed by incubation at room temperature for 1 hour. The reaction was terminated by adding 5 µL of stop buffer, and the plate was read using a microplate reader (Ex. 400 nm, Em. 445 nm; Ex. 400 nm, Em. 520 nm). The concentration-effect curve was fitted using GraphPad Prism 8 software, and the compound concentration resulting in 50% inhibition, IC₅₀, was calculated. The results are shown in Table 1.

**Table 1**

| Compound | p38 MAPK/MK2 IC₅₀ (nM) |
|---|---|
| Compound 1 | 58.59 |
| Compound 2 | 62.40 |
| Compound 3 | 21.66 |
| Compound 4 | 50.48 |
| Compound 5 | 90.30 |
| Compound 7 | 87.55 |
| Compound 10 | 60.49 |
| Compound 11 | 14.57 |
| Compound 17 | 87.86 |
| Compound 20 | 1.03 |
| Compound 20-P2 | 0.27 |
| Compound 21-P2 | 0.51 |
| Compound 32 | 3.65 |
| Compound 29-P1 | 4.86 |
| Compound 30-P1 | 0.16 |
| Compound 31-P1 | 2.79 |
| Compound 32-P1 | 0.51 |
| Compound 33-P1 | 0.056 |
| Compound 35 | 2.39 |
| Compound 35-P1 | 1.44 |
| Compound 35-P2 | 0.71 |
| Compound 37-1 | 4.66 |
| Compound 50-P1 | 0.60 |
| Compound 50-P2 | 0.48 |
| Compound 51-P1 | 1.83 |
| Compound 51-P2 | 0.71 |
| Compound 65-1 | 1.27 |
| Compound 65-2 | 1.86 |
| Compound 101 | 0.93 |
| Compound 102 | 0.15 |
| Compound 37-P1 | 8.752 |
| Compound 52-P1 | 0.12 |
| Compound 53-1 | 1.20 |
| Compound 53-2 | 3.02 |
| Compound 62-P1 | 0.35 |
| Compound 62-P3 | 0.058 |
| Compound 65-P2 | 0.40 |
| Compound 65-P4 | 0.59 |
| Compound 70-P1 | 0.38 |
| Compound 87-P1 | 8.54 |
| Compound 88-P2 | 2.11 |
| Compound 106-P1 | 7.73 |
| Compound 107-P1 | 4.99 |
| Compound 110-1 | 1.94 |
| Compound 110-P1 | 0.70 |
| Compound 111-P2 | 0.22 |
| Compound 112-P1 | 0.13 |
| Compound 113-P1 | 0.09 |
| Compound 115-P1 | 0.14 |
| Compound 115-P2 | 0.70 |
| Compound 116-P2 | 2.26 |
| Compound 116-P4 | 0.14 |
| Compound 121-P1 | 6.81 |
| Compound 121-P2 | 2.831 |
| Compound 124-P2 | 0.10 |
| Compound 125-P1 | 0.41 |
| Compound 125-P3 | 0.28 |
| Compound 126-P1 | 1.68 |
| Compound 126-P3 | 0.28 |
| Compound 128-P1 | 0.15 |
| Compound 129 | 19.94 |
| Compound 134 | 39.88 |
| Compound 135 | 12.96 |
| Compound 136 | 26.24 |
| Compound 137 | 11.67 |
| Compound 138-1 | 1.21 |
| Compound 139 | 53 |
| Compound 141 | 0.794 |
| Compound 142 | 3.373 |
| Compound 143 | 18.13 |
| Compound 144 | 12.56 |
| Compound 145-P1 | 1.191 |
| Compound 146-P1 | 1.425 |
| Compound 147 | 7.942 |
| Compound 148-P1 | 15.89 |
| Compound 149 | 2.253 |
| Compound 150 | 8.59 |
| Compound 151-P1 | 1.56 |
| Compound 154 | 20.62 |
| Compound 155 | 3.49 |
| Compound 156 | 0.336 |

As can be seen from Table 1, the compounds of the present disclosure exhibit good inhibitory activity on p38 MAPK/MK2.

### Test Example 2. In Vitro Activity Assay of p38 MAPK/MK5

The inhibitory effect of the compound on p38 MAPK/MK5 was evaluated using the Z-LYTE kinase assay kit (Thermo, PV3177). The test compound was dissolved in DMSO to obtain a 10 mM stock solution and stored at -20°C for future use. The initial concentration of the compound was 10 µM, with 1% DMSO, 5-fold serial dilution, 8 concentrations, and duplicate wells. The reaction buffer consisted of 50 mM HEPES pH 7.5, 10 mM MgCl₂, 0.01% Brij-35, and 1 mM EGTA, which was used to prepare 2x active p38a/inactive MK5/Ser/Thr 4 mixture. The final 10 µL reaction system was performed in a 384-well plate (Corning, 4514), containing 10 µg/mL inactive MK5 (Abcam, 217826), 1 ng/mL active p38a (Carna, 04-152), and 2 µM Ser/Thr 4. After a 4-hour reaction at 20°C, Development Reagent A diluted 2048-fold was added to each well, followed by incubation at room temperature for 1 hour. The reaction was terminated by adding 5 µL of stop buffer, and the plate was read using a microplate reader (Ex. 400 nm, Em. 445 nm; Ex. 400 nm, Em. 520 nm). The concentration-effect curve was fitted using GraphPad Prism 8 software, and the compound concentration resulting in 50% inhibition, IC₅₀, was calculated. The results are shown in Table 2.

**Table 2**

| Compound | p38 MAPK/MK5 IC₅₀ (nM) |
|---|---|
| Compound 1 | 6077 |
| Compound 2 | 3050 |
| Compound 3 | > 10000 |
| Compound 4 | 6592 |
| Compound 32 | 6250 |
| Compound 30-P1 | 5664 |
| Compound 31-P1 | 4342 |
| Compound 32-P1 | 1654 |
| Compound 33-P1 | 1654 |
| Compound 35-P1 | 4675 |
| Compound 65-P4 | > 20000 |
| Compound 51-P2 | 4491 |

As can be seen from Table 2, the inhibitory activity of the compounds of the present disclosure on p38 MAPK/MK5 is greater than 1.6 µM. This further demonstrates that the compounds of the present disclosure exhibit good selectivity to p38 MAPK/MK2.

### Test Example 3. In Vitro Activity Assay of p38 MAPK/ATF2

The inhibitory effect of the compound on p38a-catalyzed ATF2 was evaluated by the HTRF method. The test compound was dissolved in DMSO to obtain a 10 mM stock solution and stored at -20°C for future use. The initial concentration of the compound was 10 µM, with 0.25% DMSO, 5-fold serial dilution, 8 concentrations, and duplicate wells. The reaction buffer, consisting of 40 mM Tris pH 7.5, 20 mM MgCl₂, 0.1 mg/mL BSA, and 50 µM DTT, was used to prepare 3.5 x p38a (MAPK14, Carna Biosciences, 04-152) protein working solution, 3.5 x Human ATF2 Protein (Sino Biological, 11599-H20B) working solution, and 3.5 x ATP working solution. The reaction was terminated with 10 mM EDTA. The final 14 ilL reaction system was performed in a 96-well plate (cisbio, 66PL96025), containing 0.29 ng/µL p38a, 0.29 µM Human ATF2 Protein, and 25 µM ATP. After reacting at 20°C for 35 min, the pre-prepared antibody solution (cibio, 63ADK015PEG, Phospho-ATF2 Eu Cryptate antibody, Phospho-ATF2 d2 antibody, diluted 40-fold with Detection buffer) was added to each well and incubated overnight at room temperature. The samples were then measured using a microplate reader (HTRF compatible reader). The concentration-effect curve was fitted using GraphPad Prism 8 software, and the compound concentration resulting in 50% inhibition, IC₅₀, was calculated. The results are shown in Table 3.

**Table 3**

| Compound | p38 MAPK/ATF2 IC₅₀ (nM) |
|---|---|
| Compound 1 | > 20000 |
| Compound 2 | 10335 |
| Compound 4 | > 20000 |
| Compound 29-P1 | 1148 |
| Compound 31-P1 | 1851 |
| Compound 32-P1 | 3653 |
| Compound 65-P4 | 4600 |

As can be seen from Table 3, the inhibitory activity of the compounds of the present disclosure on p38 MAPK/ATF2 is greater than 1.1 µM. This further demonstrates that the compounds of the present disclosure exhibit good selectivity to p38 MAPK/MK2.

### Test Example 4. In vitro TNF-α activity assay in human PBMC cell supernatant

The inhibitory effect of the compound on TNF-α in the supernatant of human PBMC cells was evaluated using an ELISA detection kit (Beyotime, PI518). The test compound was dissolved in DMSO to obtain a 10 mM stock solution and stored at -20°C for future use. The initial concentration of the compound was 2 µM, followed by 5-fold serial dilution to obtain 6 concentrations. Cells were plated in duplicate, and ELISA was performed in single wells. The final concentration of DMSO was 0.4%. Alternatively, the initial concentration of the compound, dilution factor, number of concentration gradients, and replicates could be adjusted based on the specific screening conditions.

Fresh human peripheral blood mononuclear cells (PBMCs) (Sailybio) were seeded at a density of 2 × 10⁵ cells per well in a 96-well plate (Corning, 3599), with each well containing 100 µL of RPMI-1640 (Gibco#A1049101) + 10% FBS (Gibco, 10099141C), and incubated overnight at 37°C under 5% CO₂. Test compounds were added to the 96-well culture plate at a volume of 25 ilL per well. After 1 hour, 5 µL of LPS was added to achieve a final concentration of 100 ng/mL. No LPS and compound were added to the negative control wells, and no compound was added to the positive control wells. The mixture was further incubated for 24 hours at 37°C under 5% CO₂. Subsequently, the cell culture supernatant was collected by centrifugation at 500 rcf for 8 minutes. The concentration of TNF-α was measured according to the operation manual of the ELISA kit. The concentration-effect curve was fitted using GraphPad Prism 8 software, and the compound concentration resulting in 50% inhibition, IC₅₀, was calculated. The results are shown in Table 4.

**Table 4**

| Compound | p38 MAPK/TNF-α IC₅₀ (nM) |
|---|---|
| Compound 2 | 89.01 |
| Compound 4 | 96.65 |
| Compound 11 | 18.38 |
| Compound 20 | 8.68 |
| Compound 20-P2 | 4.20 |
| Compound 32 | 2.89 |
| Compound 30-P1 | 8.68 |
| Compound 31-P1 | 3.79 |
| Compound 32-P1 | 3.21 |
| Compound 33-P1 | 2.93 |
| Compound 35-P1 | 1.90 |
| Compound 65-P4 | 4.50 |
| Compound 50-P1 | 2.82 |
| Compound 111-P2 | 1.43 |
| Compound 70-P1 | 11.24 |
| Compound 112-P1 | 0.44 |
| Compound 113-P1 | 1.70 |
| Compound 124-P1 | 3.59 |
| Compound 137 | 0.84 |
| Compound 141 | 14.05 |
| Compound 145-P1 | 8.51 |
| Compound 146-P1 | 3.38 |
| Compound 151-P1 | 12.92 |
| Compound C | 26.37 |

As can be seen from Table 4, the compounds of the present disclosure exhibit good inhibitory effects on TNF-α in human PBMC cells.

### Test Example 5. Evaluation of In Vitro Liver Microsome Stability

The reference compound used in this experiment was Ketanserin, and the final concentration of microsomes in the experimental system was 0.5 mg/mL. The PBS buffer was a 50 mM K₂HPO₄ buffer. The concentration of the test compound was 1 µM. The microsomes were added to PBS, and the control/test compounds were respectively added to the corresponding reaction systems, mixed thoroughly, and pre-incubated in a 37°C water bath for 5 minutes. The reaction was initiated by adding 20 mM NADPH solution under 37°C water bath conditions (for the No NADPH samples, an equal volume of PBS solution was used as a substitute for the 20 mM NADPH solution). At the reaction time points of 0 min, 10 min, 30 min, 60 min, and 90 min, 30 µL of reaction samples were taken from each reaction system (for the No NADPH samples, sampling was performed at 0 min and 90 min), and the reaction was immediately terminated by adding 300 µL of the internal standard acetonitrile solution. After centrifugation at 4000 rpm, the supernatant was taken and mixed thoroughly with an equal volume of pure water. The samples at each time point were analyzed by LC-MS/MS (AB Triple Quard 5500) to determine the compound content, and the T_{1/2} was calculated. The results are shown in Table 5:

**Table 5**

| Example | Mouse liver microsomes | Rat liver microsomes | Human liver microsomes |
|---|---|---|---|
| | Half-life T_{1/2} (min) | Half-life T_{1/2} (min) | Half-life T_{1/2} (min) |
| Compound 1 | > 279.57 | >279.57 | >279.57 |
| Compound 2 | 237.42 | 250.40 | 248.43 |
| Compound 4 | 175.65 | >279.57 | >279.57 |
| Compound 11 | 253.50 | 227.44 | >279.57 |
| Compound 20-P2 | > 279.57 | >279.57 | >279.57 |
| Compound 29-P1 | > 279.57 | >279.57 | >279.57 |
| Compound 31-P1 | 107.23 | 165.30 | >279.57 |
| Compound 35-P1 | > 279.57 | >279.57 | >279.57 |
| Compound 65-P4 | > 279.57 | >279.57 | >279.57 |
| Compound 50-P1 | > 279.57 | >279.57 | >279.57 |
| Compound 111-P2 | > 279.57 | >279.57 | >279.57 |
| Compound 70-P1 | / | / | > 279.57 |
| Compound 112-P1 | / | / | > 279.57 |
| Compound 113-P1 | / | / | > 279.57 |
| Compound 88-P2 | / | / | > 279.57 |
| Compound 124-P1 | / | / | > 279.57 |
| Compound 124-P2 | / | / | > 279.57 |
| Compound 128-P1 | / | / | > 279.57 |
| Compound 128-P2 | / | / | > 279.57 |
| Compound 141 | > 279.57 | 150.73 | |
| Compound 145-P1 | > 279.57 | >279.57 | >279.57 |
| Compound 146-P1 | 137.2 | >279.57 | >279.57 |

As can be seen from Table 5, the compounds of the present disclosure exhibit good *in vitro* liver microsomal stability (human/mouse/rat).

### Test Example 6. Pharmacokinetic Evaluation in Mice

The compound was weighed and dissolved in a mixed solvent of DMAC: Solutol: PBS = 1:1:8. After intravenous/gastric administration to mice, whole blood (30 µL) was collected at 0.083, 0.25, 0.5, 1, 2, 4, 7, and 24 hours. The samples were anticoagulated with EDTA-K2, immediately centrifuged at 4000 rpm for 5 minutes at 4°C, and the supernatant was frozen in a -80°C refrigerator. Plasma sample processing: The samples were precipitated with an internal standard-containing CH₃CN precipitant, followed by centrifugation at 12700 rpm for 10 min. The supernatant was collected and analyzed by LC-MS/MS (AB Triple Quard 5500) to obtain the plasma drug concentration. The parameters were calculated using a non-compartmental model in Winnolin version 8.1. The results are shown in Table 6:

**Table 6**

| | | | | | | |
|---|---|---|---|---|---|---|
| Compound 29-P1 | Intravenous injection group 1 mg/kg | Initial concentration | Half-life | Area under curve | Volume of distribution | Clearance |
| | | C₀ (ng/mL) | T_{1/2} (h) | AUC₀₋₂₄ₕ (hr*ng/mL) | Vd (L/kg) | CL (mL/hr/kg ) |
| | | 1452.00 | 2.57 | 1388.42 | 1.83 | 621.55 |
| | Intragastric administration group 5 mg/kg | Maximum concentration | Half-life | Area under curve | Bioavailability | |
| | | Cₘₐₓ (ng/mL) | T_{1/2} (h) | AUC₀₋₂₄ₕ (hr*ng/mL) | F% | |
| | | 1382.80 | 3.37 | 6492.29 | 80.63 | |
| Compound 30-P1 | Intravenous injection group 1 mg/kg | Initial concentration | Half-life | Area under curve | Volume of distribution | Clearance |
| | | C₀ (ng/mL) | T_{1/2} (h) | AUC₀₋₂₄ₕ (hr*ng/mL) | Vd (L/kg) | CL (mL/hr/kg ) |
| | | 2409.73 | 0.47 | 1099.97 | 0.41 | 913.46 |
| | Intragastric administration group 5 mg/kg | Maximum concentration | Half-life | Area under curve | Bioavailability | |
| | | Cₘₐₓ (ng/mL) | T_{1/2} (h) | AUC₀₋₂₄ₕ (hr*ng/mL) | F% | |
| | | 1572.97 | 3.31 | 2637.05 | 48.03 | |
| Compound 31-P1 | Intravenous injection group 1 mg/kg | Initial concentration | Half-life | Area under curve | Volume of distribution | Clearance |
| | | C₀ (ng/mL) | T_{1/2} (h) | AUC₀₋₂₄ₕ (hr*ng/mL) | Vd (L/kg) | CL (mL/hr/kg ) |
| | | 2149.53 | 0.57 | 1358.11 | 0.47 | 739.42 |
| | Intragastric administration group 5 mg/kg | Maximum concentration | Half-life | Area under curve | Bioavailability | |
| | | Cₘₐₓ (ng/mL) | T_{1/2} (h) | AUC₀₋₂₄ₕ (hr*ng/mL) | F% | |
| | | 979.25 | 3.24 | 2072.58 | 30.52 | |
| Compound 32-P1 | Intravenous injection group 1 mg/kg | Initial concentration | Half-life | Area under curve | Volume of distribution | Clearance |
| | | C₀ (ng/mL) | T_{1/2} (h) | AUC₀₋₂₄ₕ (hr*ng/mL) | Vd (L/kg) | CL (mL/hr/kg ) |
| | | 1793.13 | 0.35 | 841.90 | 0.49 | 1265.82 |
| | Intragastric administration group 5 mg/kg | Maximum concentration | Half-life | Area under curve | Bioavailability | |
| | | Cₘₐₓ (ng/mL) | T_{1/2} (h) | AUC₀₋₂₄ₕ (hr*ng/mL) | F% | |
| | | 1609.73 | 1.54 | 2351.94 | 57.89 | |
| Compound 33-P1 | Intravenous injection group 1 mg/kg | Initial concentration | Half-life | Area under curve | Volume of distribution | Clearance |
| | | C₀ (ng/mL) | T_{1/2} (h) | AUC₀₋₂₄ₕ (hr*ng/mL) | Vd (L/kg) | CL (mL/hr/kg ) |
| | | 3199.47 | 0.98 | 1579.66 | 0.32 | 649.41 |
| | Intragastric administration group 5 mg/kg | Maximum concentration | Half-life | Area under curve | Bioavailability | |
| | | Cₘₐₓ (ng/mL) | T_{1/2} (h) | AUC₀₋₂₄ₕ (hr*ng/mL) | F% | |
| | | 3665.17 | 1.99 | 2802.23 | 36.88 | |

As can be seen from Table 6, within the administered concentration dose and detection time range, the compounds of the present disclosure exhibit high *in vivo* exposure, showing good *in vivo* pharmacokinetic properties of the compounds of the present disclosure.

Test Example 7. Experiment on the Inhibitory Effect of Plasma TNF-α in LPS-Induced Balbc Mouse Inflammatory Model

Balb/c male mice were randomly divided into groups (n=4). At 0 hours, animals in Group 1 and Group 2 were orally administered with Vehicle (PO, 10 mL/kg), while animals in Groups 3, 4, 5, 6, and 7 were intragastrically administered with the compound at the required doses. Animals in Group 1 were injected with saline (ip, 10 mL/kg) 4.5 hours after administration, while the remaining animals were stimulated with LPS (ip, 10 mL/kg, 0.2 mpk) 4.5 hours after administration. All animals were euthanized by CO₂ 6 hours after administration, and blood was collected from the heart. The blood sample was anticoagulated with EDTA-K2, placed in wet ice, and then centrifuged at 4000 rpm for 5 min to separate the plasma, which was subsequently frozen at low temperature for future analysis. The plasma TNFα protein levels were detected by ELISA, and the percentage inhibition relative to the control group was calculated, as shown in FIG. 1. 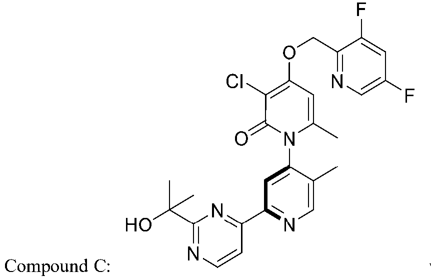 was prepared according to the method described in Example 1 of WO2021195475A1. Conclusion: The results in FIG. 1 show that the compounds of the present disclosure exhibit significant inhibitory effects on TNFα in mouse plasma.

The embodiments of the technical solution of the present disclosure are exemplarily illustrated. It should be understood that the scope of protection of the present disclosure is not limited to the above embodiments. Any modifications, equivalent substitutions, and improvements, *etc.* made by those skilled in the art within the spirit and principles of the present disclosure shall be encompassed within the scope of protection of the claims of the present disclosure.

## Claims

1. A compound represented by formula (I) or a pharmaceutically acceptable salt thereof, 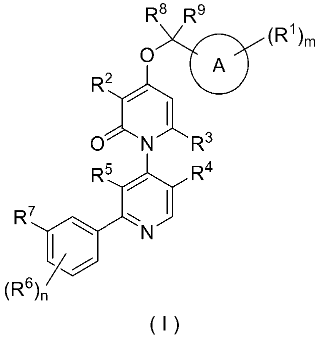 wherein
ring A is phenyl or 5- to 6-membered heteroaryl;
each R¹ is the same or different, and independently selected from the group consisting of H, halogen, C₁₋₆ alkyl, C₁₋₆ haloalkyl, C₁₋₆ alkoxy, C₁₋₆ haloalkoxy, C₁₋₆ hydroxyalkyl, cyano, hydroxyl, amino, nitro, and 3- to 8-membered cycloalkyl;
R² is selected from the group consisting of H, halogen, and C₁₋₆ alkyl;
R³ is selected from the group consisting of H, halogen, C₁₋₆ alkyl, and C₁₋₆ haloalkyl;
R⁴ is selected from the group consisting of H, halogen, cyano, hydroxyl, amino, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
R⁵ is selected from the group consisting of H, halogen, and C₁₋₆ alkyl;
each R⁶ is the same or different, and independently selected from the group consisting of H, halogen, cyano, hydroxyl, amino, C₁₋₆ alkyl, C₁₋₆ haloalkyl, C₁₋₆ alkoxy, C₁₋₆ haloalkoxy, C₁₋₆ hydroxyalkyl, -C(O)OR^{e}, -C(O)NR^{g}R^{h}, -C(O)R^{f}, -S(O)₂R^{f}, and 3- to 8-membered cycloalkyl;
R⁷ is selected from the group consisting of H, halogen, 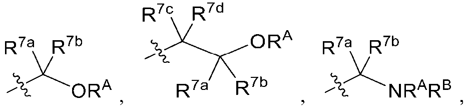 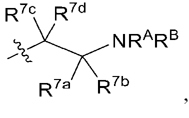 -C(O)OR^{e}, -C(O)NR^{g}R^{h}, -C(O)R^{f}, and -S(O)₂R^{f};
R^{A} and R^{B} are the same or different, and each independently selected from the group consisting of H, C₁₋₆ alkyl, or C₁₋₆ haloalkyl;
R^{7a} and R^{7b} are the same or different, and each independently selected from the group consisting of H, D, C₁₋₆ alkyl, C₁₋₆ haloalkyl, C₁₋₆ hydroxyalkyl, -C(O)OR^{e}, -C(O)NR^{g}R^{h}, -C(O)R^{f}, -S(O)₂R^{f}, and 3- to 8-membered cycloalkyl;
alternatively, R^{7a} and R^{7b} together with the carbon to which they are attached form a 3- to 8-membered cycloalkyl or 3- to 8-membered heterocyclyl ring, wherein the 3- to 8-membered cycloalkyl or 3- to 8-membered heterocyclyl is optionally substituted by one or more substituents selected from the group consisting of halogen and C₁₋₆ alkyl;
R^{7c} and R^{7d} are the same or different, and each independently selected from the group consisting of H, D, halogen, cyano, hydroxyl, amino, oxo (=O), C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
alternatively, R^{7c} and R^{7d} together with the carbon to which they are attached form a 3- to 8-membered cycloalkyl or 3- to 8-membered heterocyclyl ring, wherein the 3- to 8-membered cycloalkyl or 3- to 8-membered heterocyclyl is optionally substituted by one or more substituents selected from the group consisting of halogen and C₁₋₆ alkyl;
R^{e} is selected from the group consisting of H, C₁₋₆ alkyl, C₁₋₆ haloalkyl, and 3- to 8-membered cycloalkyl;
R^{g} and R^{h} are the same or different, and each independently selected from the group consisting of H, C₁₋₆ alkyl, hydroxyl, C₁₋₆ alkoxy, and 3- to 8-membered cycloalkyl, wherein the C₁₋₆ alkyl, C₁₋₆ alkoxy, and 3- to 8-membered cycloalkyl are optionally substituted by one or more substituents selected from the group consisting of halogen, cyano, hydroxyl, amino, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
alternatively, R^{g} and R^{h} together with the N to which they are attached form a 3- to 6-membered heterocyclyl ring, wherein the 3- to 6-membered heterocyclyl is optionally substituted by one or more substituents selected from the group consisting of halogen, hydroxyl, C₁₋₆ alkyl, and C₁₋₆ haloalkyl;
R^{f} is selected from the group consisting of H, C₁₋₆ alkyl, C₁₋₆ haloalkyl, C₁₋₆ hydroxyalkyl, and 3- to 8-membered cycloalkyl; alternatively, R⁶ and R⁷ together with the atom to which they are attached form a 3- to 8-membered heterocyclyl ring, wherein the 3- to 8-membered heterocyclyl is optionally substituted by one or more substituents selected from the group consisting of hydroxyl, oxo, C₁₋₆ alkoxy, C₁₋₆ alkyl, C₁₋₆ haloalkyl, and C₁₋₆ haloalkoxy;
R⁸ and R⁹ are the same or different, and each independently selected from the group consisting of H, D, halogen, C₁₋₆ alkyl, and C₁₋₆ haloalkyl;
alternatively, R⁸ and R⁹ together with the atom to which they are attached form a 3- to 6-membered cycloalkyl ring, wherein the 3- to 6-membered cycloalkyl is optionally substituted by one or more substituents selected from the group consisting of halogen, hydroxyl, C₁₋₆ alkyl, and C₁₋₆ haloalkyl;
n is selected from the group consisting of 0, 1, 2, 3, and 4;
m is selected from the group consisting of 0, 1, 2, 3, 4, and 5.

2. The compound or the pharmaceutically acceptable salt thereof according to claim 1, wherein R⁷ is selected from the group consisting of H, halogen, 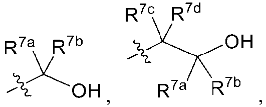 -C(O)OR^{e}, -C(O)NR^{g}R^{h}, -C(O)R^{f}, or -S(O)₂R^{f};
R^{7a} and R^{7b} are the same or different, and each independently selected from the group consisting of H, D, C₁₋₆ alkyl, C₁₋₆ haloalkyl, C₁₋₆ hydroxyalkyl, and 3- to 8-membered cycloalkyl;
alternatively, R^{7a} and R^{7b} together with the carbon to which they are attached form a 3- to 8-membered cycloalkyl or 3- to 8-membered heterocyclyl ring, wherein the 3- to 8-membered cycloalkyl or 3- to 8-membered heterocyclyl is optionally substituted by one or more substituents selected from the group consisting of halogen and C₁₋₆ alkyl;
R^{7c} and R^{7d} are the same or different, and each independently selected from the group consisting of H, D (deuterium), halogen, cyano, hydroxyl, amino, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
alternatively, R^{7c} and R^{7d} together with the carbon to which they are attached form a 3- to 8-membered cycloalkyl or 3- to 8-membered heterocyclyl ring, wherein the 3- to 8-membered cycloalkyl or 3- to 8-membered heterocyclyl is optionally substituted by one or more substituents selected from the group consisting of halogen and C₁₋₆ alkyl;
R^{e} is selected from the group consisting of H, C₁₋₆ alkyl, C₁₋₆ haloalkyl, and 3- to 8-membered cycloalkyl;
R^{g} and R^{h} are the same or different, and each independently selected from the group consisting of H, C₁₋₆ alkyl, hydroxyl, C₁₋₆ alkoxy, and 3- to 8-membered cycloalkyl, wherein the C₁₋₆ alkyl, C₁₋₆ alkoxy, and 3- to 8-membered cycloalkyl are optionally substituted by one or more substituents selected from the group consisting of halogen, cyano, hydroxyl, amino, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
alternatively, R^{g} and R^{h} together with the N to which they are attached form a 3- to 6-membered heterocyclyl ring, wherein the 3- to 6-membered heterocyclyl is optionally substituted by one or more substituents selected from the group consisting of halogen, hydroxyl, C₁₋₆ alkyl, and C₁₋₆ haloalkyl;
R^{f} is selected from the group consisting of H, C₁₋₆ alkyl, C₁₋₆ haloalkyl, C₁₋₆ hydroxyalkyl, and 3- to 8-membered cycloalkyl.

3. The compound or the pharmaceutically acceptable salt thereof according to claim 1, wherein R⁶ and R⁷ together with the atom to which they are attached form a 3- to 8-membered heterocyclyl ring, and the 3- to 8-membered heterocyclyl is optionally substituted by one or more substituents selected from the group consisting of hydroxyl, oxo, C₁₋₆ alkyl, and C₁₋₆ haloalkyl.

4. The compound or the pharmaceutically acceptable salt thereof according to claim 1, wherein ring A is pyridyl.

5. The compound or the pharmaceutically acceptable salt thereof according to any one of claims 1 to 4, wherein the compound or the pharmaceutically acceptable salt thereof is a compound represented by formula (II) or a pharmaceutically acceptable salt thereof, 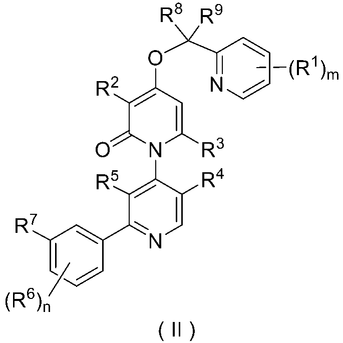 wherein
R¹, R², R³, R⁴, R⁵, R⁶, R⁷, R⁸, R⁹, n, and m are as defined in claims 1 to 4.

6. The compound or the pharmaceutically acceptable salt thereof according to any one of claims 1 to 5, wherein the compound or the pharmaceutically acceptable salt thereof is a compound represented by formula (II-1) or formula (II-2) or a pharmaceutically acceptable salt thereof, 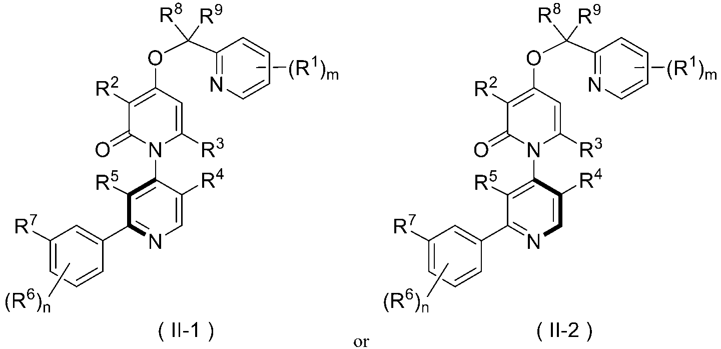 wherein
R¹, R², R³, R⁴, R⁵, R⁶, R⁷, R⁸, R⁹, n, and m are as defined in claims 1 to 5.

7. The compound or the pharmaceutically acceptable salt thereof according to any one of claims 1 to 6, selected from the group consisting of any one of the following schemes:
scheme I:
each R⁶ is the same or different, and independently selected from the group consisting of H, halogen, cyano, hydroxyl, amino, C₁₋₆ alkyl, C₁₋₆ haloalkyl, C₁₋₆ alkoxy, C₁₋₆ haloalkoxy, C₁₋₆ hydroxyalkyl, -C(O)NR^{g}R^{h}, -S(O)₂R^{f}, and 3- to 8-membered cycloalkyl; R^{g} and R^{h} are the same or different, and each independently selected from the group consisting of H, C₁₋₆ alkyl, C₁₋₆ haloalkyl, and 3- to 8-membered cycloalkyl; R^{f} is selected from the group consisting of C₁₋₆ alkyl, C₁₋₆ haloalkyl, C₁₋₆ hydroxyalkyl, and 3- to 8-membered cycloalkyl;
R⁷ is 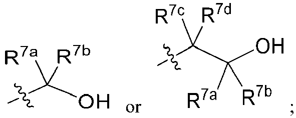 R^{7a} and R^{7b} are the same or different, and each independently selected from the group consisting of H, D, C₁₋₆ alkyl, C₁₋₆ haloalkyl, C₁₋₆ hydroxyalkyl, and 3- to 8-membered cycloalkyl;
R^{7c} and R^{7d} are the same or different, and each independently selected from the group consisting of H, D, halogen, cyano, hydroxyl, amino, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
scheme II:
each R⁶ is the same or different, and independently selected from the group consisting of H, halogen, cyano, hydroxyl, amino, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
R⁷ is selected from the group consisting of -C(O)OR^{e}, -C(O)NR^{g}R^{h}, -C(O)R^{f}, and -S(O)₂R^{f};
R^{e} is H or C₁₋₆ alkyl;
R^{g} and R^{h} are the same or different, and each independently selected from the group consisting of H, C₁₋₆ alkyl, hydroxyl, C₁₋₆ alkoxy, and 3- to 8-membered cycloalkyl, wherein the C₁₋₆ alkyl, C₁₋₆ alkoxy, and 3- to 8-membered cycloalkyl are optionally substituted by one or more substituents selected from the group consisting of halogen, cyano, hydroxyl, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl;
alternatively, R^{g} and R^{h} together with the N to which they are attached form a 3- to 6-membered heterocyclyl ring, wherein the 3- to 6-membered heterocyclyl is optionally substituted by one or more substituents selected from the group consisting of halogen, hydroxyl, C₁₋₆ alkyl, and C₁₋₆ haloalkyl;
R^{f} is selected from the group consisting of C₁₋₆ alkyl, C₁₋₆ haloalkyl, C₁₋₆ hydroxyalkyl, and 3- to 8-membered cycloalkyl;
preferably,
each R⁶ is the same or different, and independently selected from the group consisting of H and halogen;
R⁷ is selected from the group consisting of -C(O)(CH₂)pOH, -C(O)NR^{g}R^{h}, and -S(O)₂R^{f}; p is a positive integer from 0 to 5;
R^{g} and R^{h} are the same or different, and each independently selected from the group consisting of H, hydroxyl, C₁₋₆ alkyl, and C₁₋₆ alkoxy, wherein the C₁₋₆ alkyl and C₁₋₆ alkoxy are optionally substituted by one or more substituents selected from the group consisting of halogen and hydroxyl;
alternatively, R^{g} and R^{h} together with the N to which they are attached form a 3- to 6-membered heterocyclyl ring, wherein the 3- to 6-membered heterocyclyl is optionally substituted by one or more substituents selected from the group consisting of halogen, hydroxyl, and C₁₋₆ alkyl;
R^{f} is C₁₋₆ alkyl or 3- to 8-membered cycloalkyl;
scheme III:
R⁶ is selected from the group consisting of -C(O)(CH₂)pOH, -C(O)NR^{g}R^{h}, and -S(O)₂R^{f}; p is a positive integer from 0 to 5; n is 1;
R^{g} and R^{h} are the same or different, and each independently selected from the group consisting of H, hydroxyl, C₁₋₆ alkyl, and C₁₋₆ alkoxy, wherein the C₁₋₆ alkyl and C₁₋₆ alkoxy are optionally substituted by one or more substituents selected from the group consisting of halogen and hydroxyl;
alternatively, R^{g} and R^{h} together with the N to which they are attached form a 3- to 6-membered heterocyclyl ring, wherein the 3- to 6-membered heterocyclyl is optionally substituted by one or more substituents selected from the group consisting of halogen, hydroxyl, and C₁₋₆ alkyl;
R^{f} is C₁₋₆ alkyl or 3- to 8-membered cycloalkyl;
R⁷ is H or halogen.

8. The compound or the pharmaceutically acceptable salt thereof according to any one of claims 1 to 7, wherein R⁷ is selected from the group consisting of

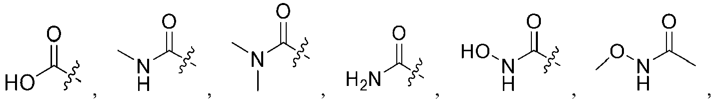

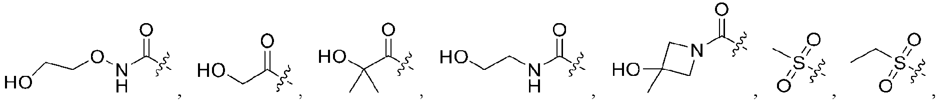

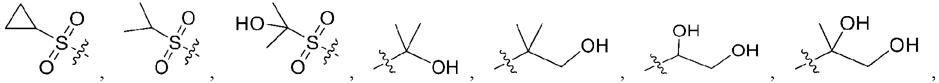

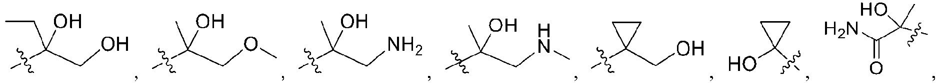

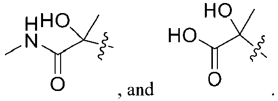

9. The compound or the pharmaceutically acceptable salt thereof according to any one of claims 1 to 8, wherein each R⁶ is the same or different, and independently selected from the group consisting of H, halogen, cyano, hydroxyl, amino, C₁₋₆ alkyl, C₁₋₆ haloalkyl, -C(O)NR^{g}R^{h}, -S(O)₂R^{f}, and 3- to 8-membered cycloalkyl; R^{g} and R^{h} are the same or different, and each is independently H or C₁₋₆ alkyl; R^{f} is selected from the group consisting of C₁₋₆ alkyl and 3- to 8-membered cycloalkyl;

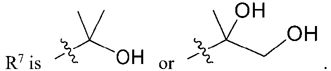

10. The compound or the pharmaceutically acceptable salt thereof according to any one of claims 1 to 9, wherein 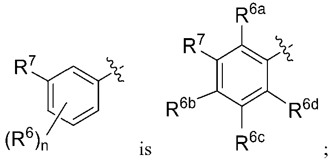 R^{6a}, R^{6b}, R^{6c}, and R^{6d} are the same or different, and each independently selected from the group consisting of H, halogen, cyano, hydroxyl, C₁₋₆ alkyl, C₁₋₆ haloalkyl, C₁₋₆ alkoxy, C₁₋₆ haloalkoxy, C₁₋₆ hydroxyalkyl, - C(O)OH, -C(O)NR^{g}R^{h}, -S(O)₂R^{f}, and 3- to 8-membered cycloalkyl; R^{g} and R^{h} are the same or different, and each independently selected from the group consisting of H, C₁₋₆ alkyl, C₁₋₆ haloalkyl, and 3- to 8-membered cycloalkyl; R^{f} is selected from the group consisting of C₁₋₆ alkyl, C₁₋₆ haloalkyl, C₁₋₆ hydroxyalkyl, and 3- to 8-membered cycloalkyl; preferably, R^{6a} and R^{6b} are the same or different, and each independently selected from the group consisting of H, halogen, cyano, hydroxyl, C₁₋₆ alkyl, C₁₋₆ haloalkyl, C₁₋₆ alkoxy, C₁₋₆ haloalkoxy, C₁₋₆ hydroxyalkyl, and 3- to 8-membered cycloalkyl; R^{6c} and R^{6d} are the same or different, and each is independently H, halogen, or C₁₋₆ alkyl;
R⁷ is as defined in claims 1-9.

11. The compound or the pharmaceutically acceptable salt thereof according to any one of claims 1 to 10, wherein R² is halogen or C₁₋₆ alkyl; preferably, R² is Cl, Br, F, or methyl.

12. The compound or the pharmaceutically acceptable salt thereof according to any one of claims 1 to 11, wherein R³ is C₁₋₆ alkyl or C₁₋₆ haloalkyl; preferably, R³ is methyl.

13. The compound or the pharmaceutically acceptable salt thereof according to any one of claims 1 to 12, wherein R⁴ is selected from the group consisting of H, halogen, C₁₋₆ alkyl, C₁₋₆ alkoxy, and C₁₋₆ haloalkyl.

14. The compound or the pharmaceutically acceptable salt thereof according to any one of claims 1 to 13, wherein R⁵ is H, halogen, or C₁₋₆ alkyl; preferably, R⁵ is H, F, or methyl.

15. The compound or the pharmaceutically acceptable salt thereof according to any one of claims 1 to 14, wherein R⁸ and R⁹ are the same or different, and each is independently H or D.

16. The compound or the pharmaceutically acceptable salt thereof according to any one of claims 1 to 15, wherein each R¹ is the same or different, and independently selected from the group consisting of H, halogen, C₁₋₆ alkyl, and C₁₋₆ haloalkyl; preferably, R¹ is H or halogen.

17. The compound or the pharmaceutically acceptable salt thereof according to any one of claims 1 to 16, wherein the compound is selected from the group consisting of any one of the following compounds,

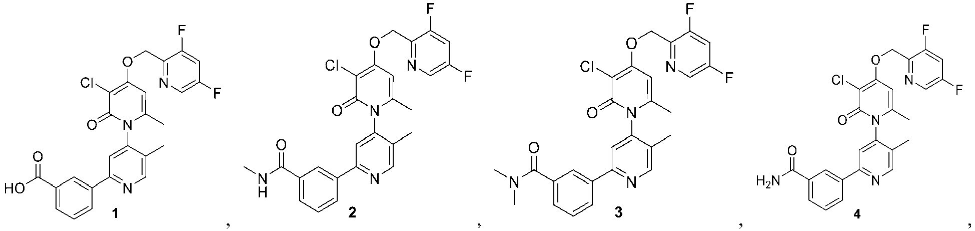

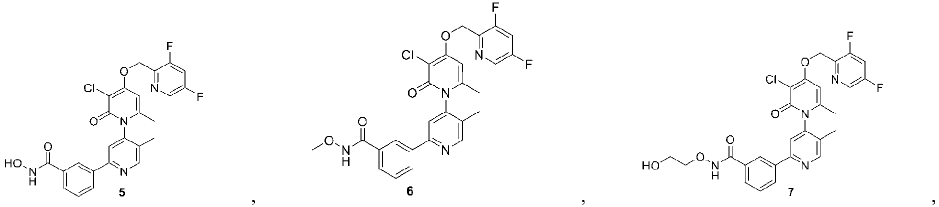

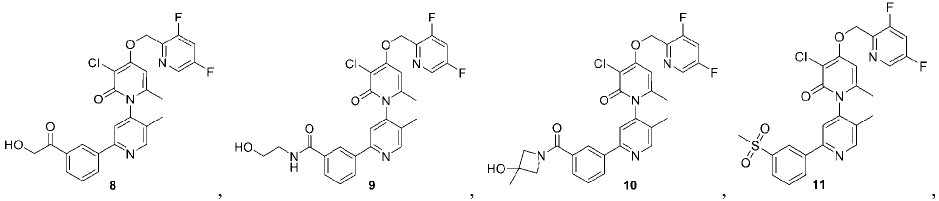

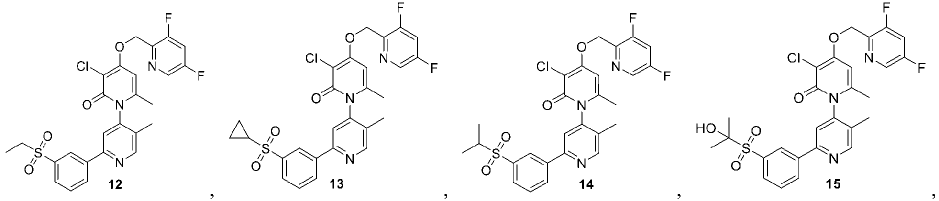

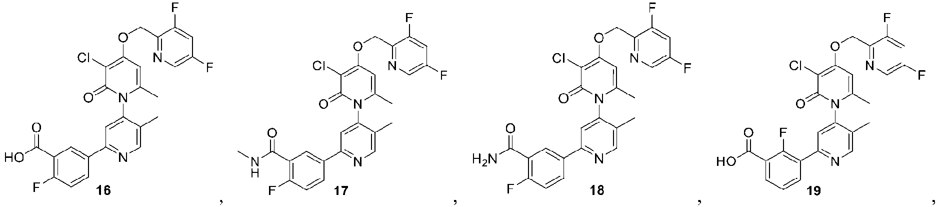

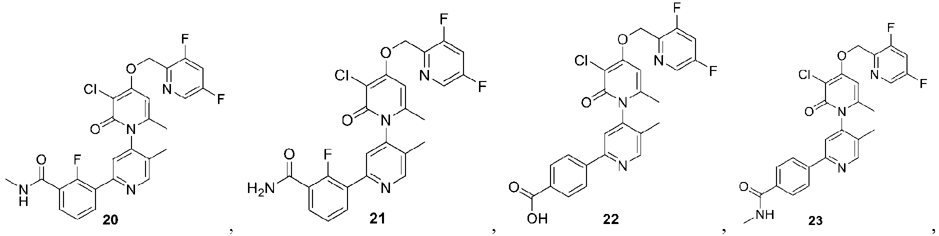

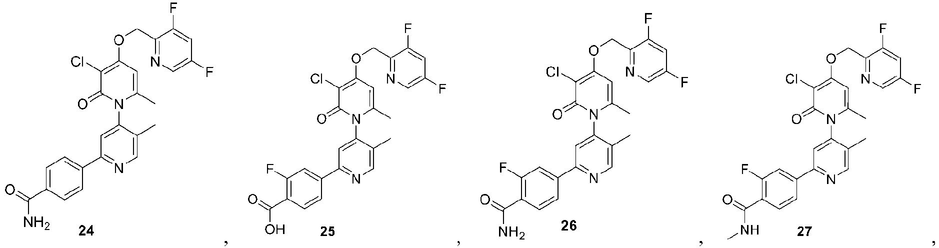

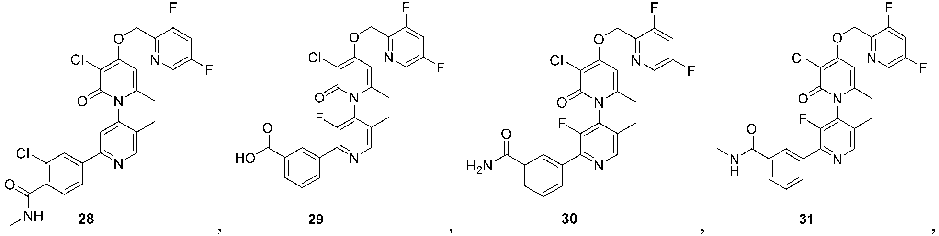

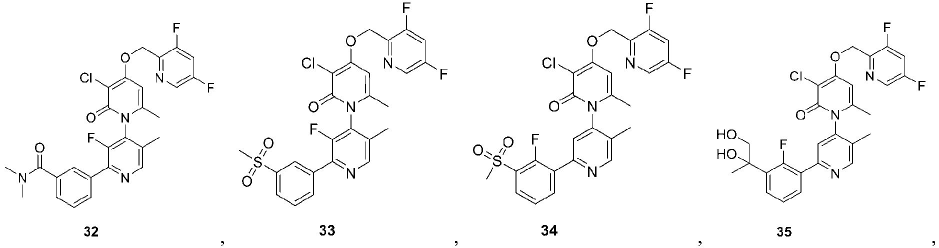

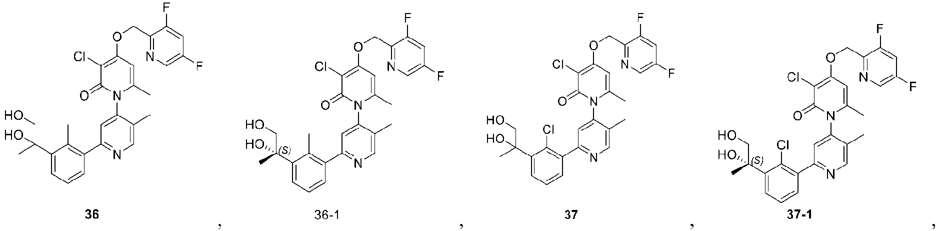

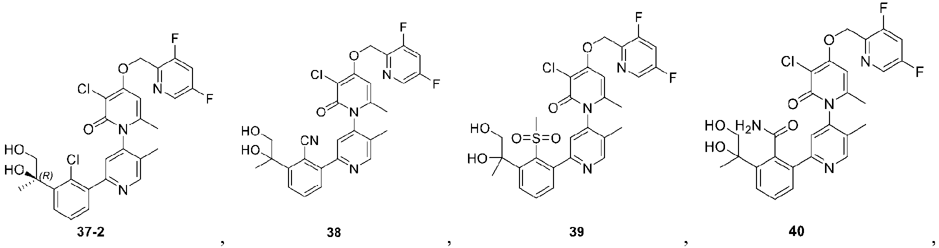

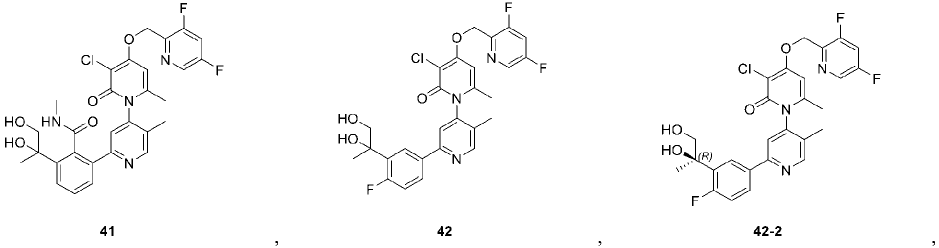

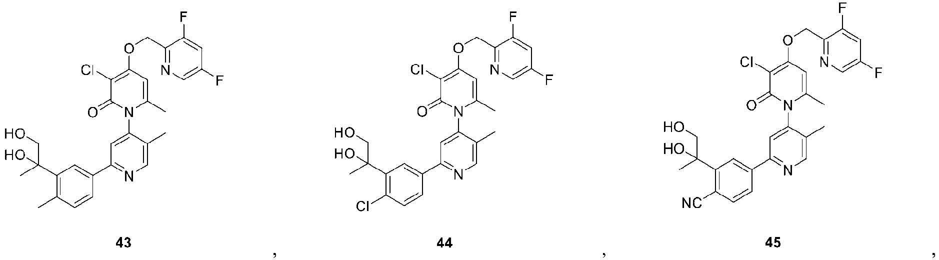

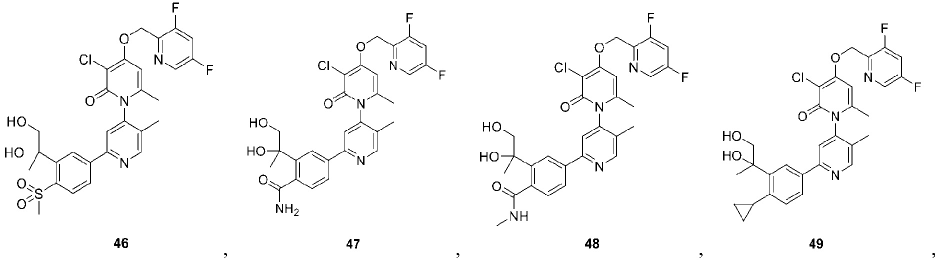

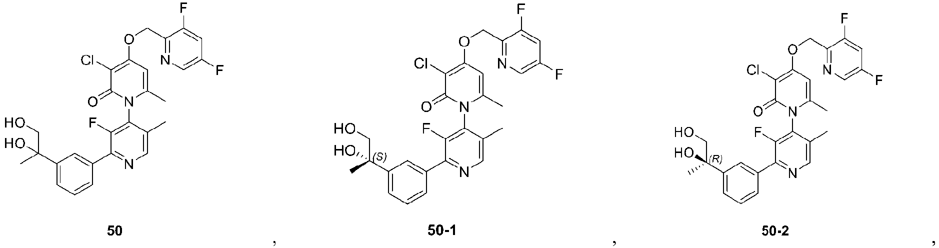

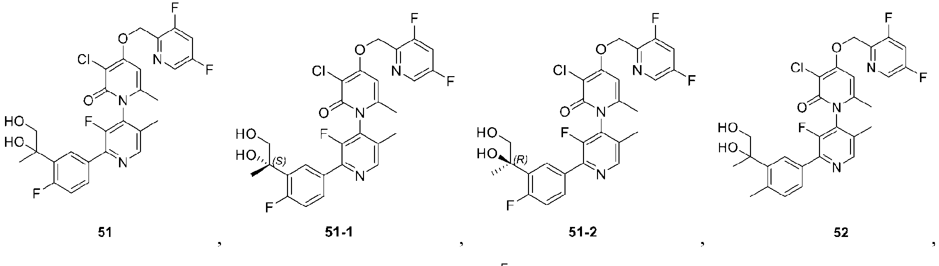

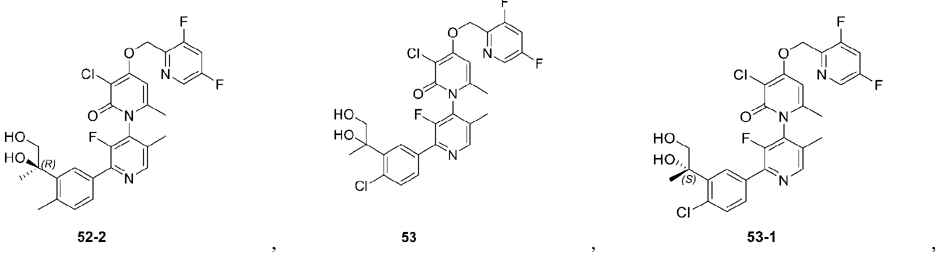

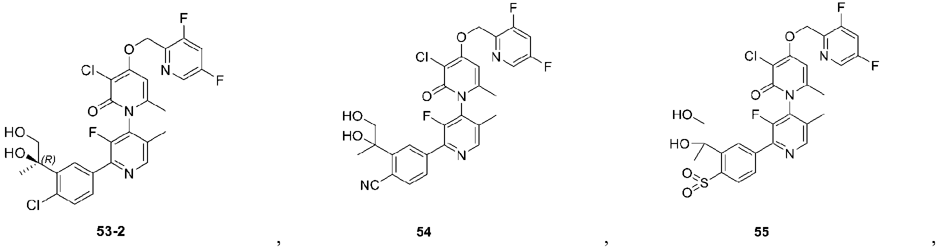

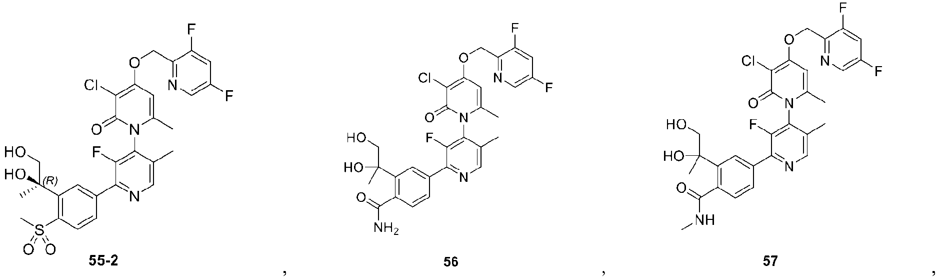

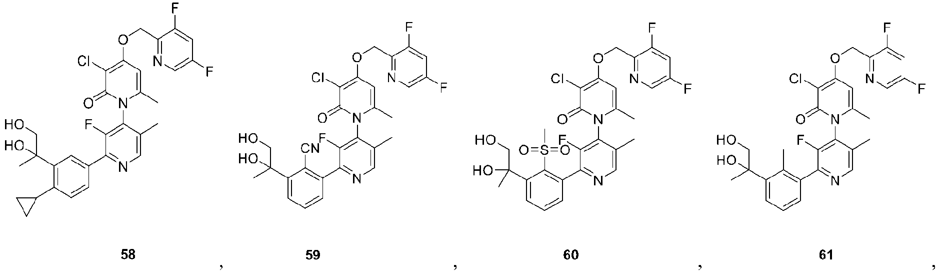

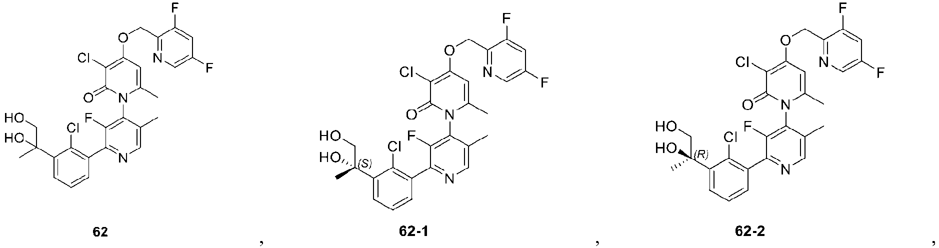

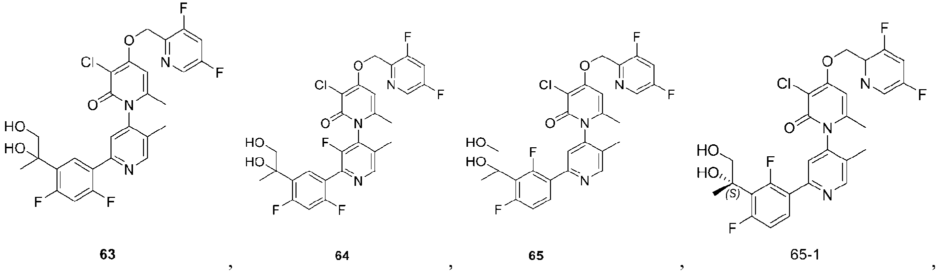

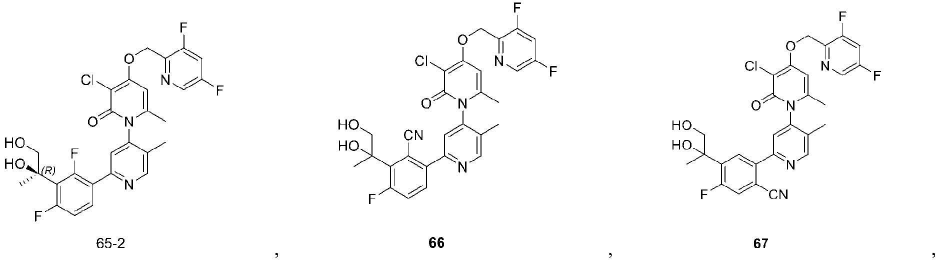

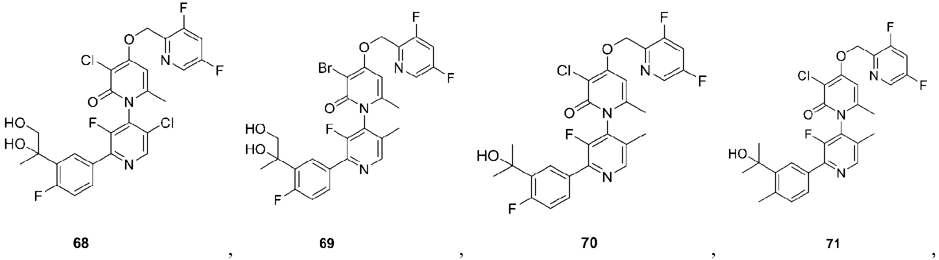

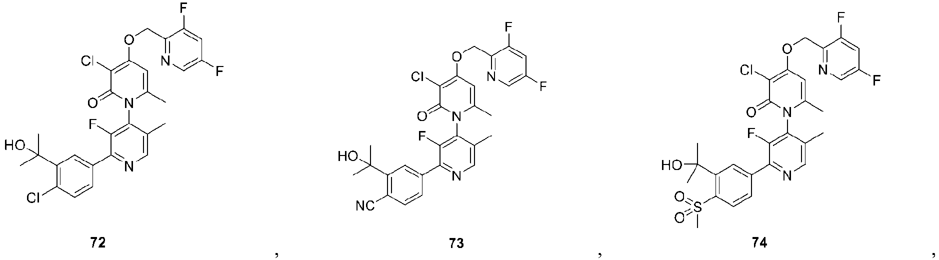

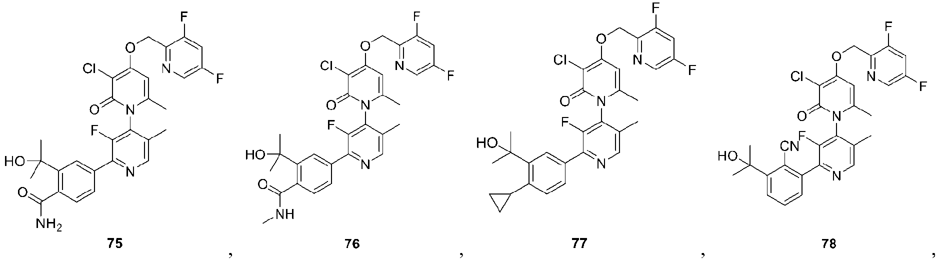

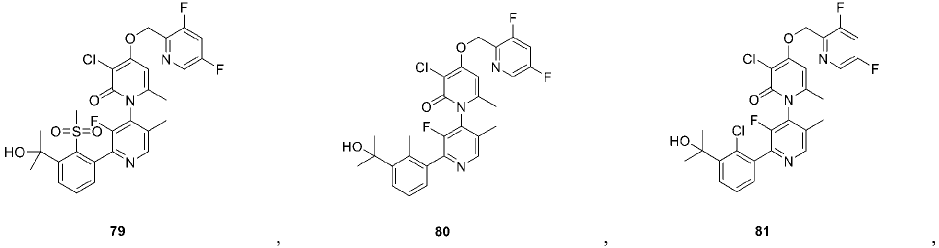

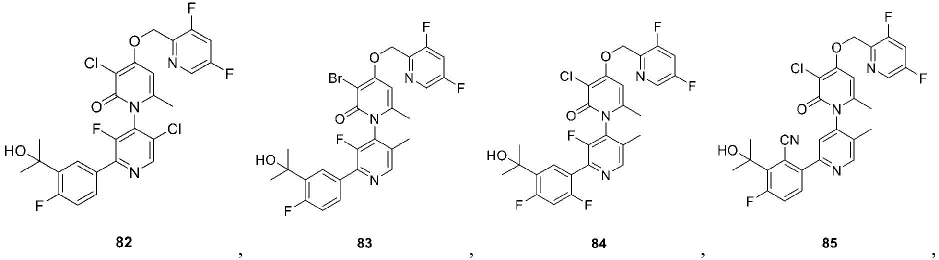

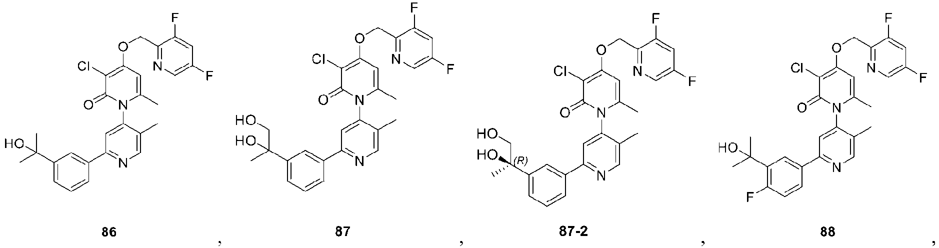

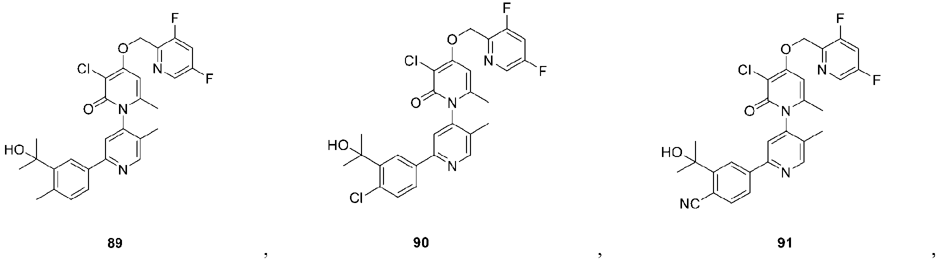

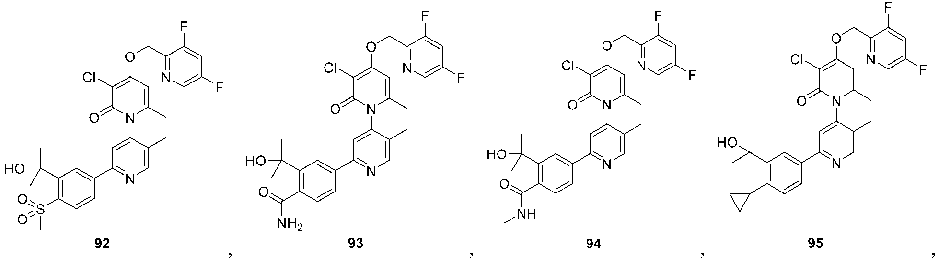

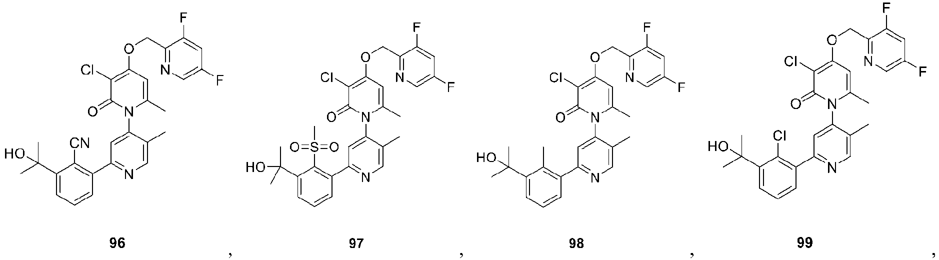

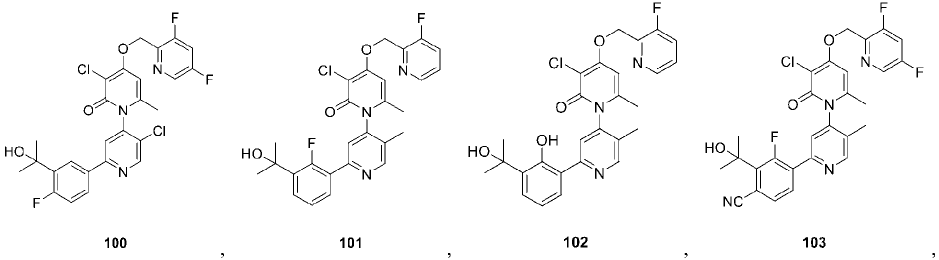

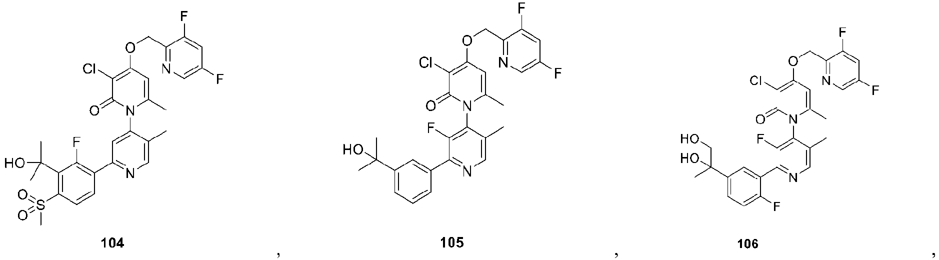

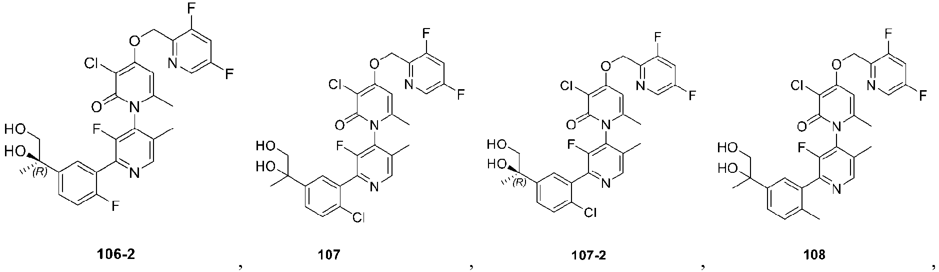

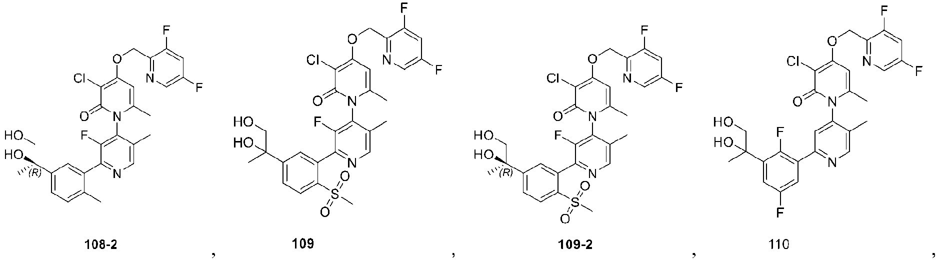

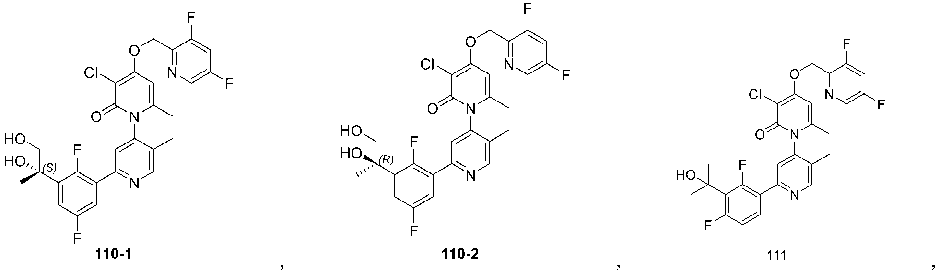

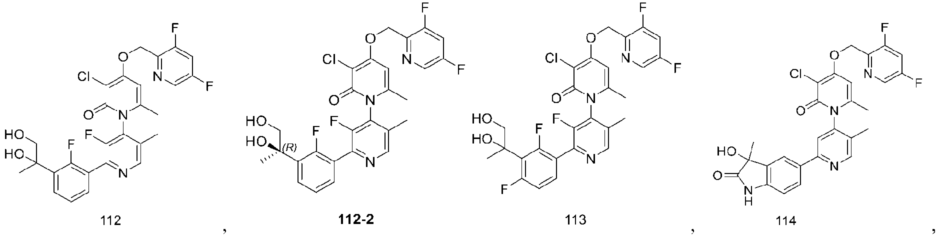

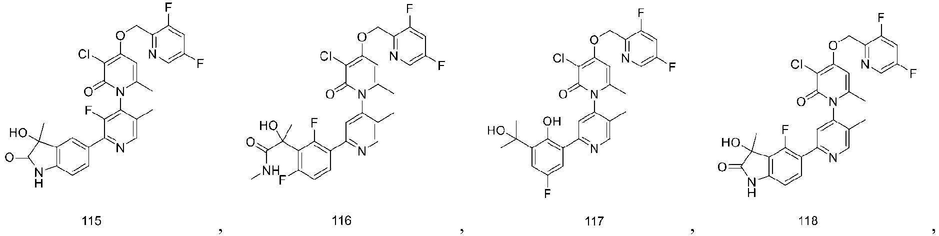

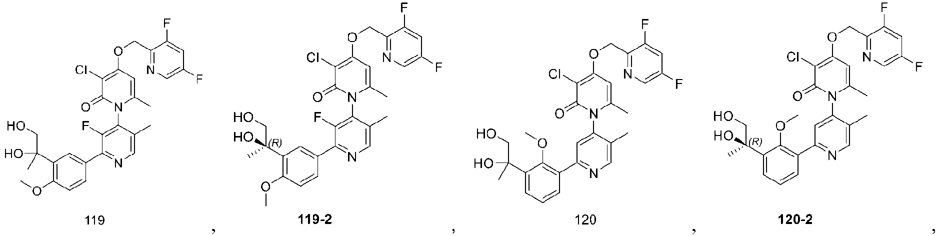

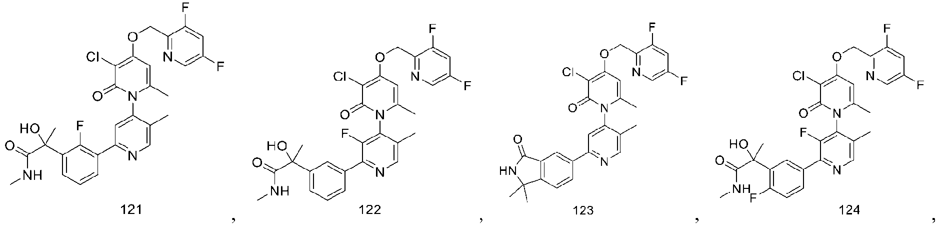

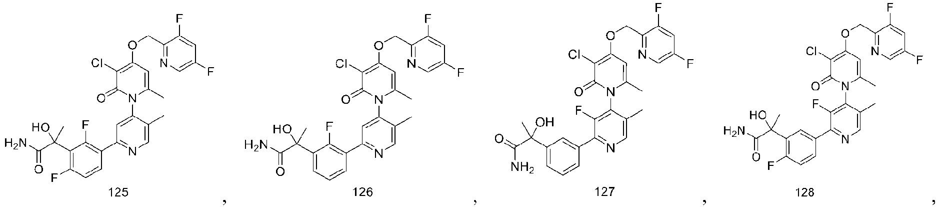

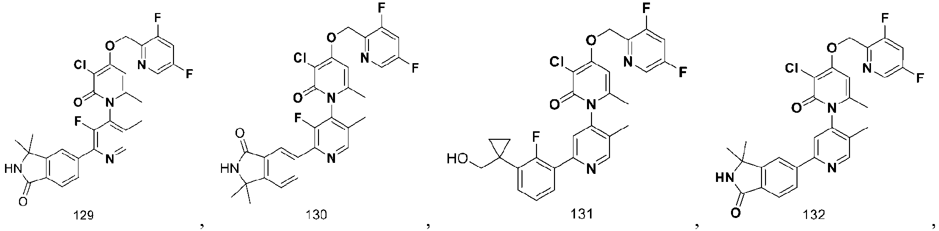

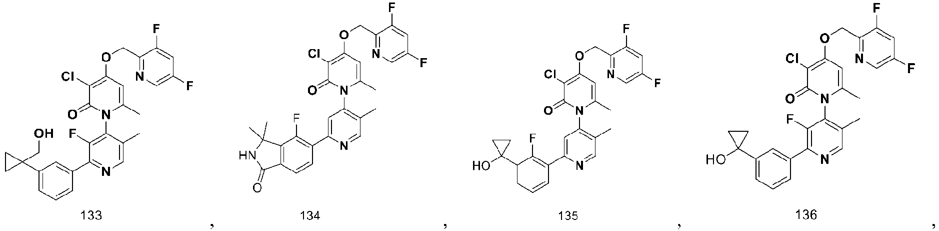

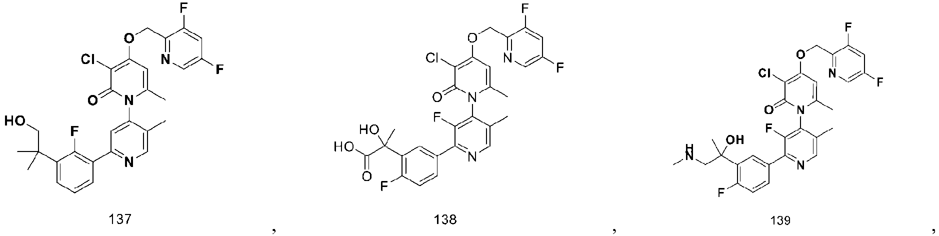

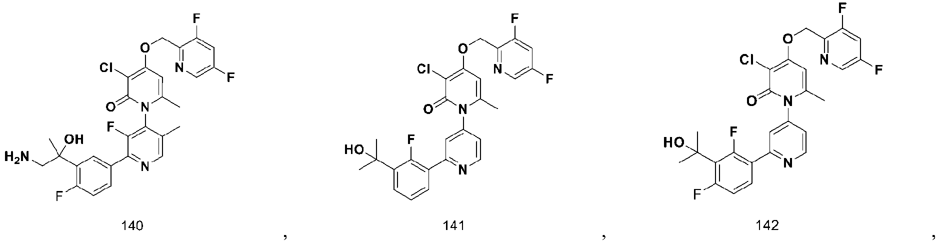

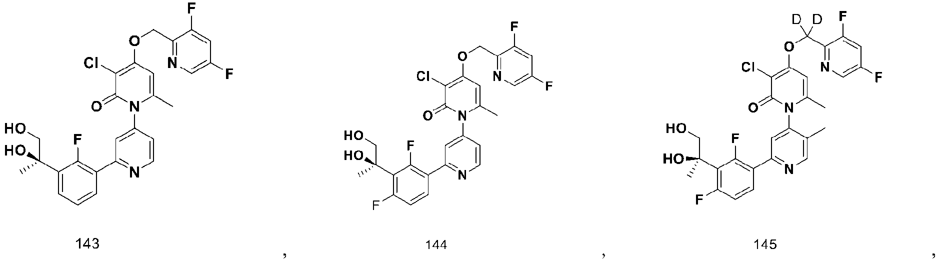

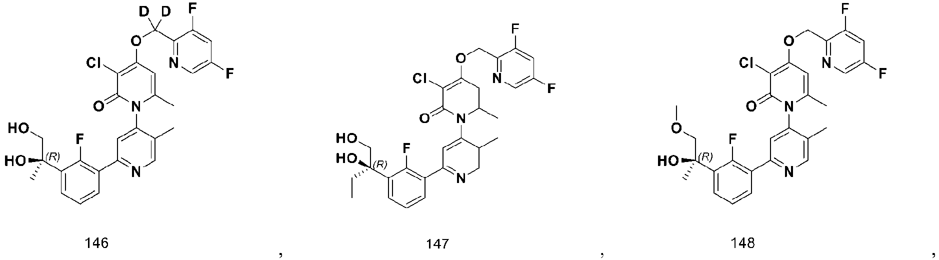

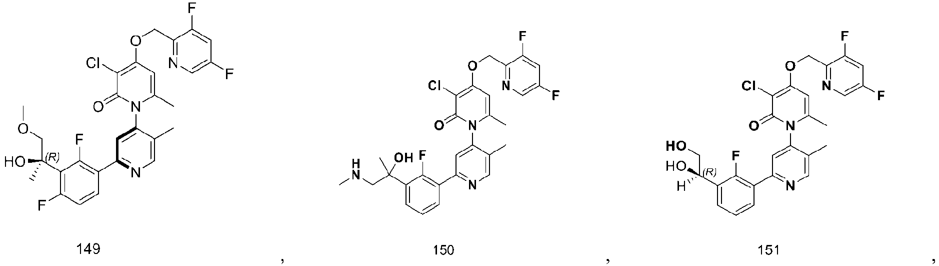

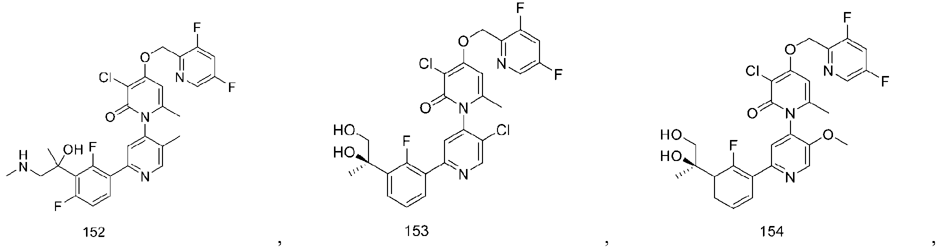

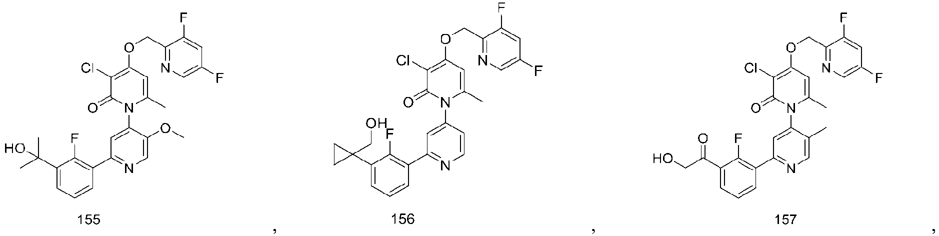

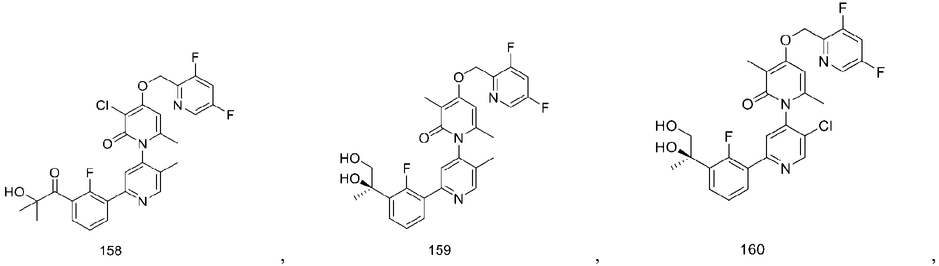

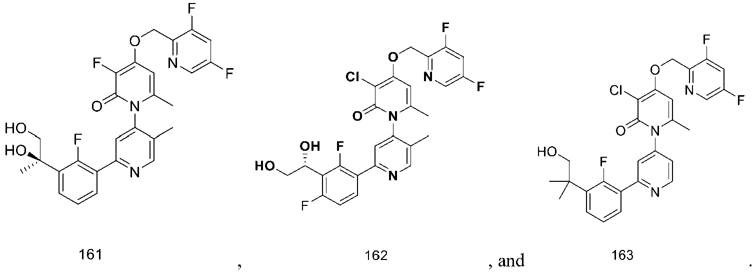

18. The compound or the pharmaceutically acceptable salt thereof according to any one of claims 1 to 17, wherein the compound is selected from the group consisting of any one of the following compounds, 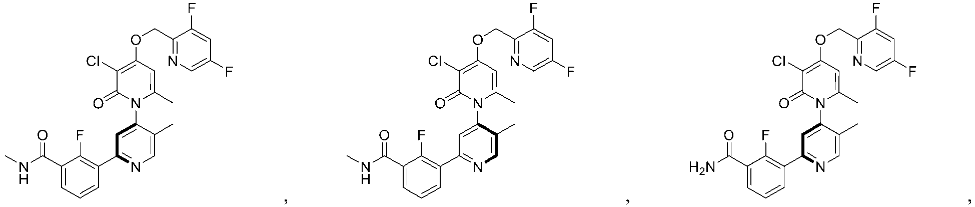 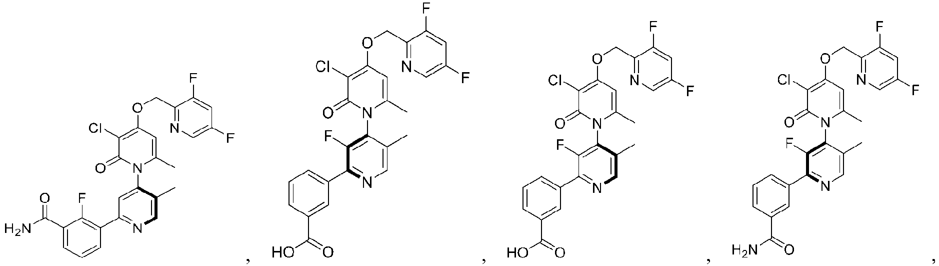 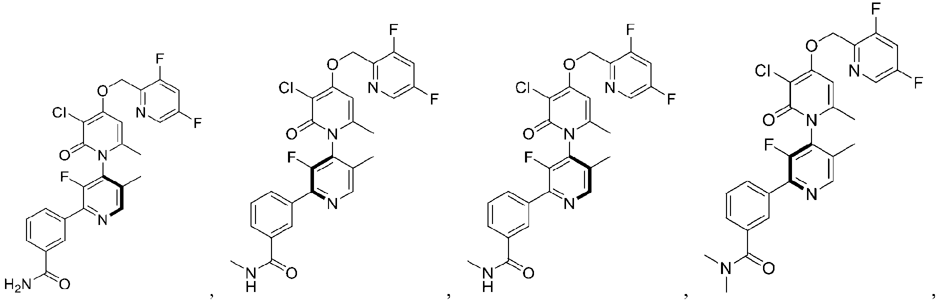 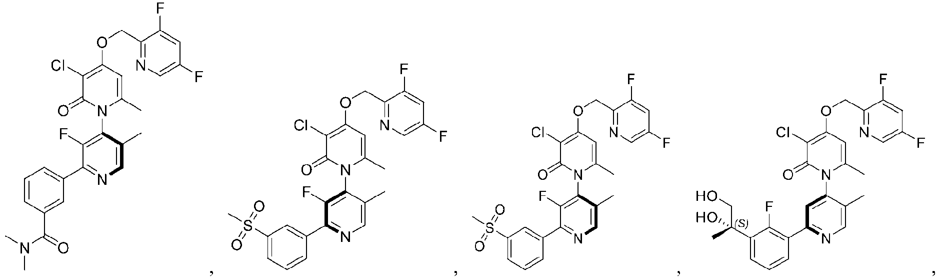 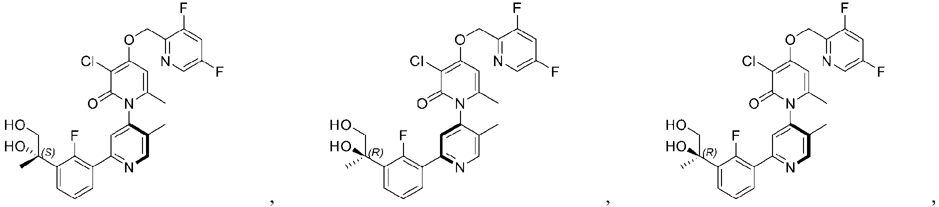 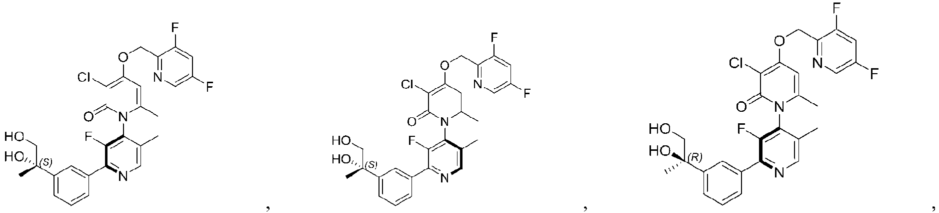 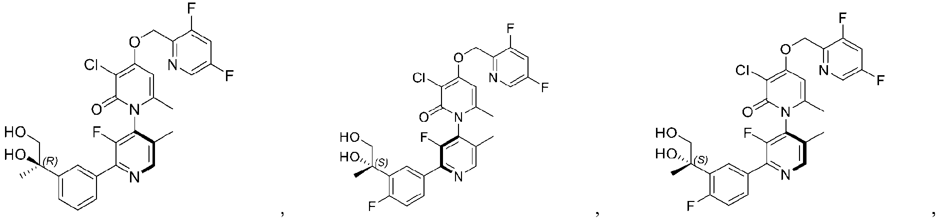 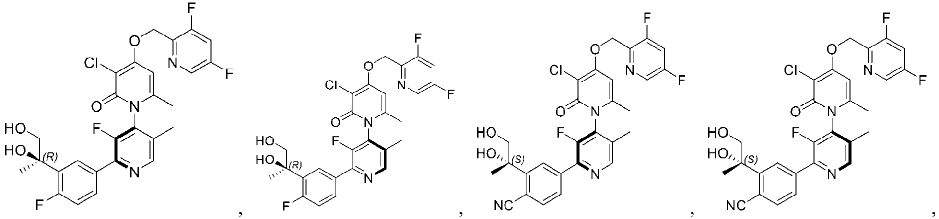 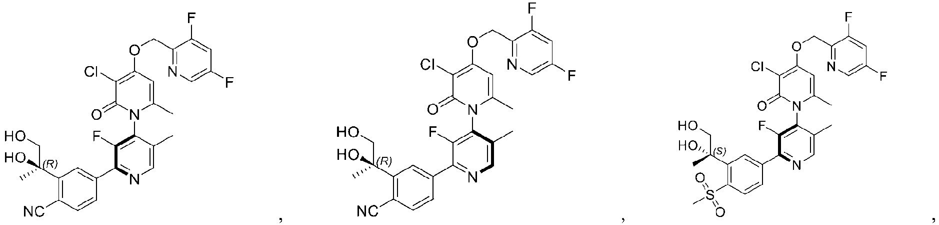 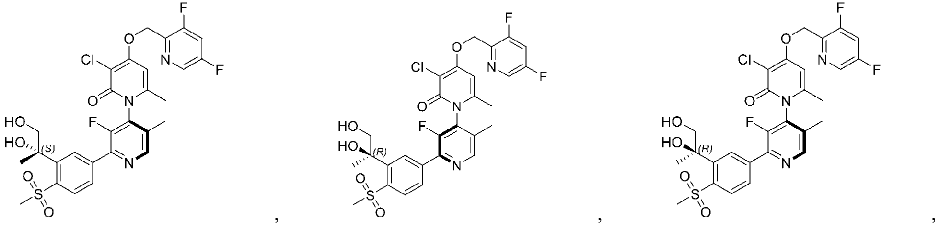 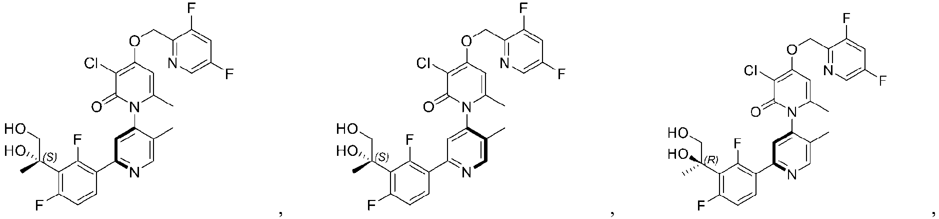 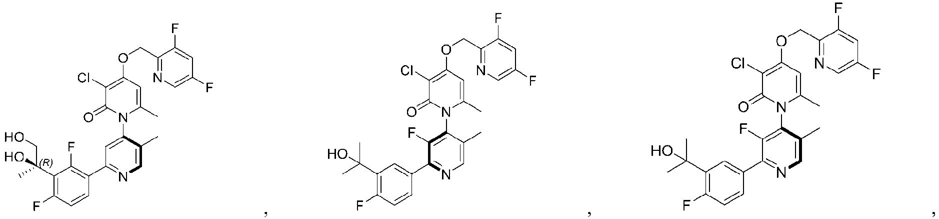 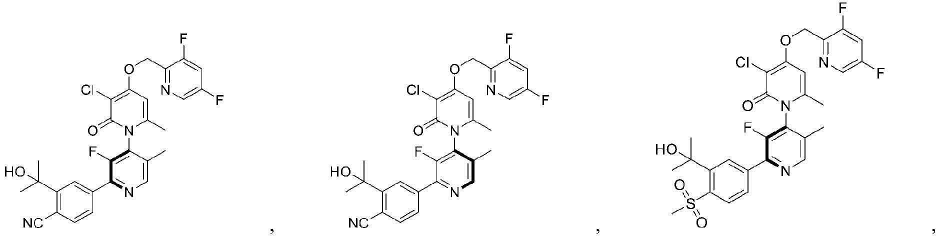 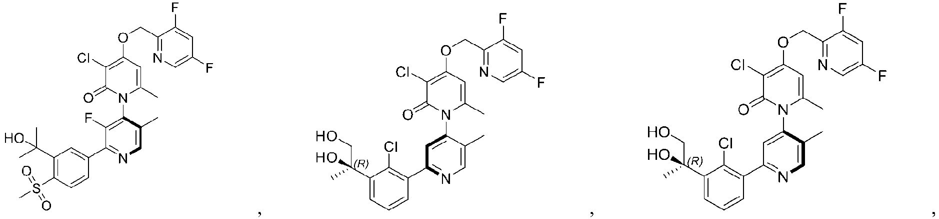 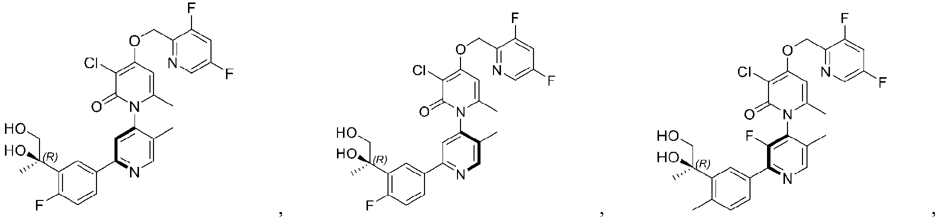 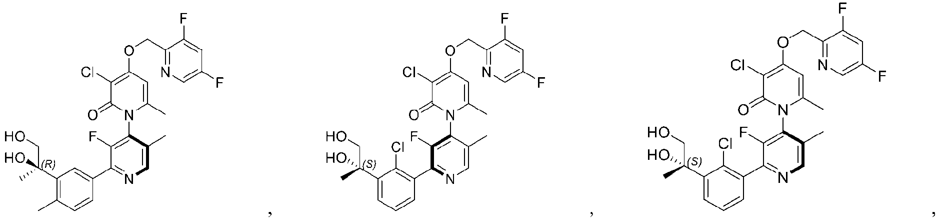 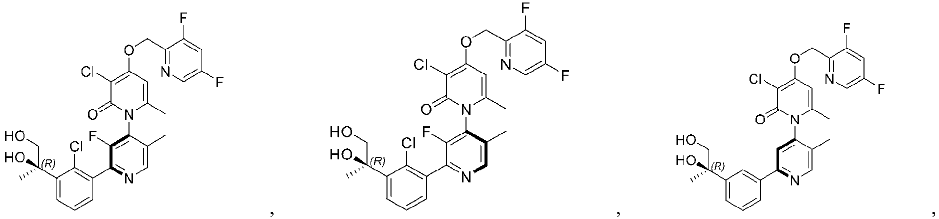 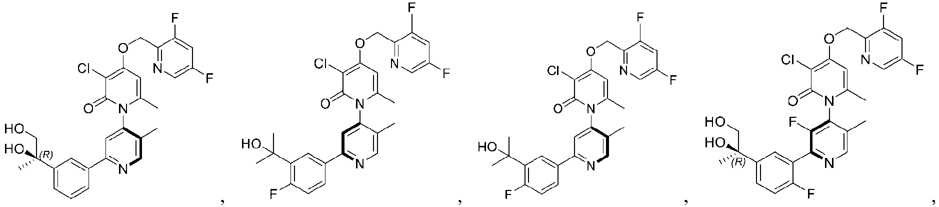 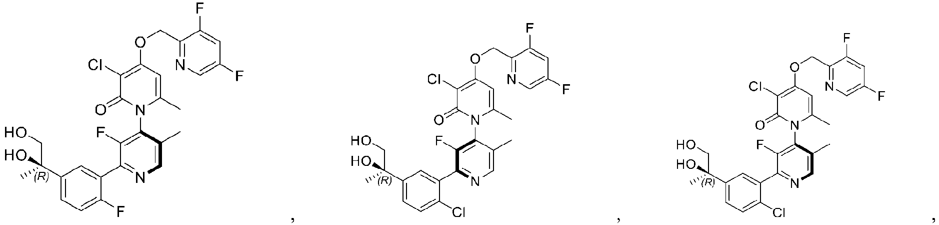 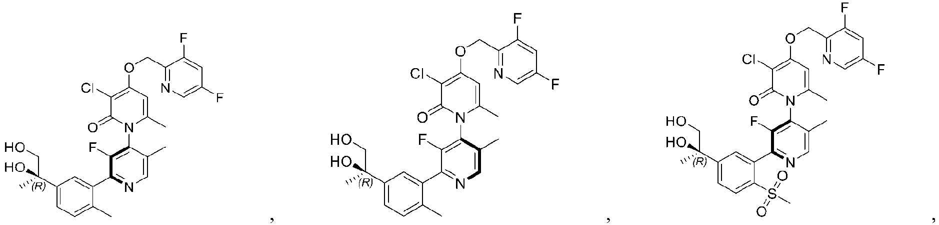 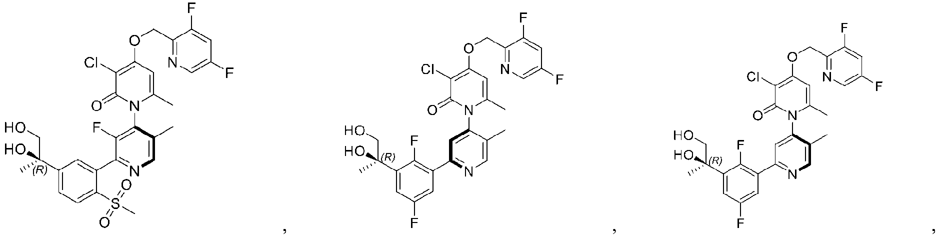 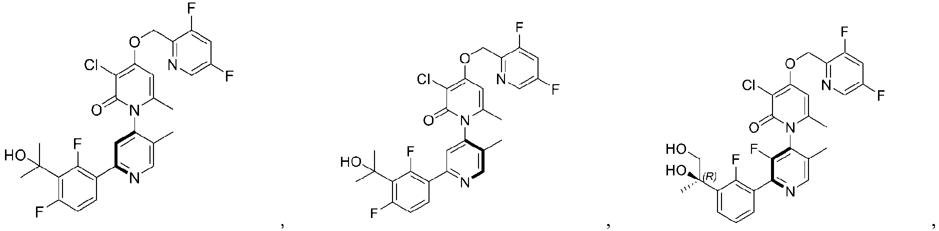 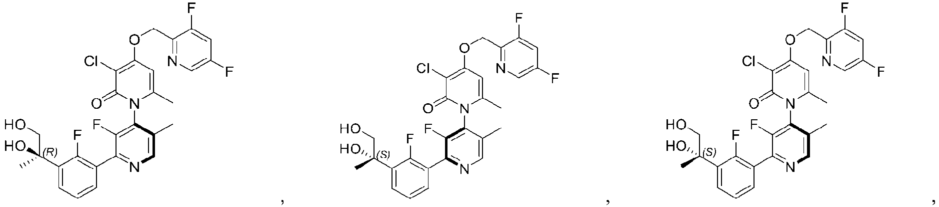 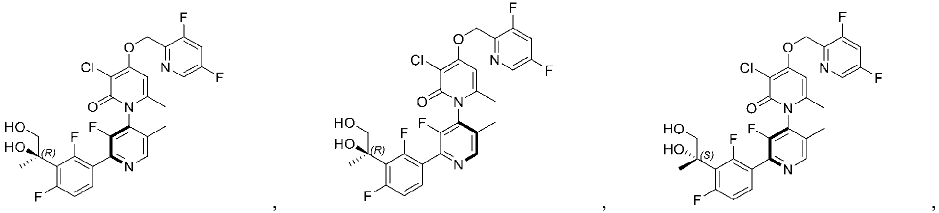 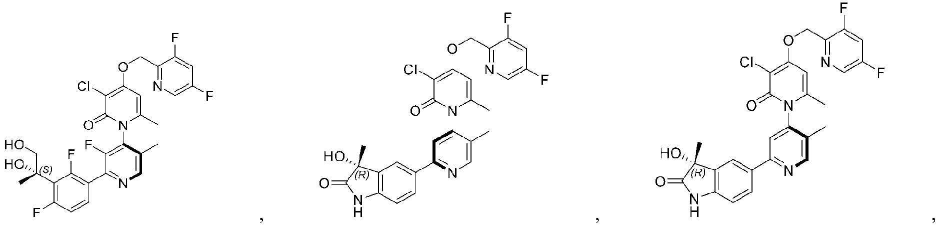 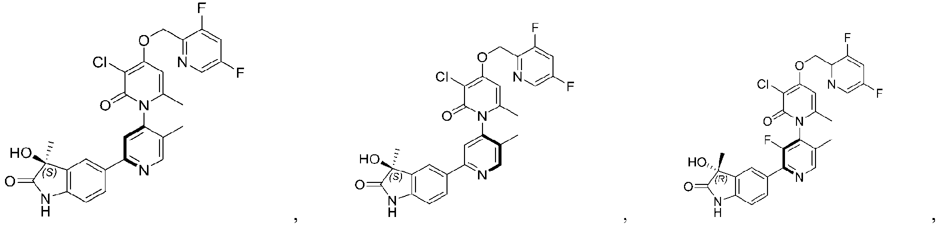 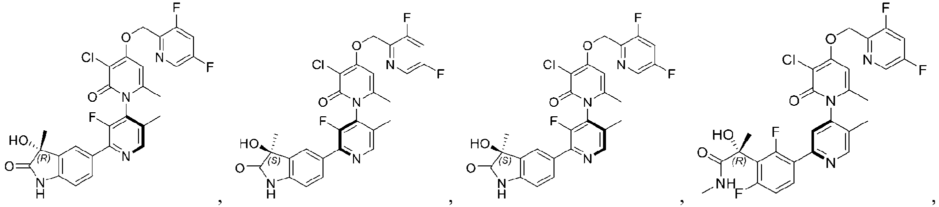 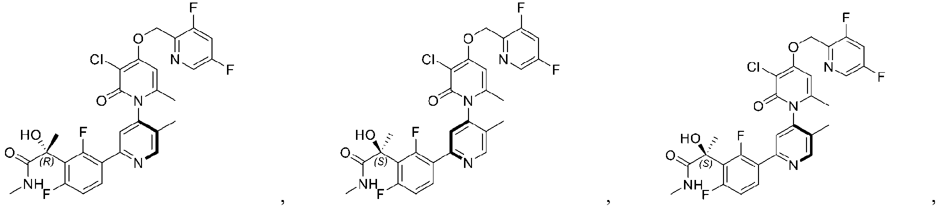 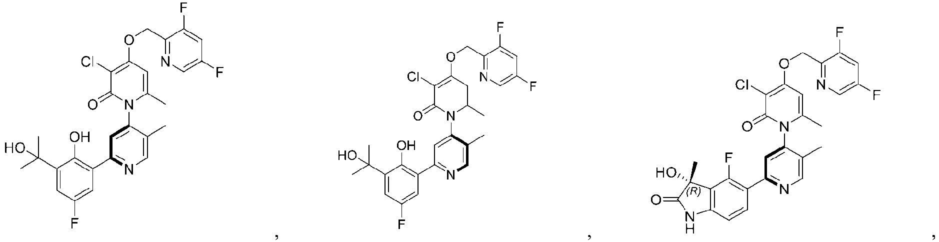 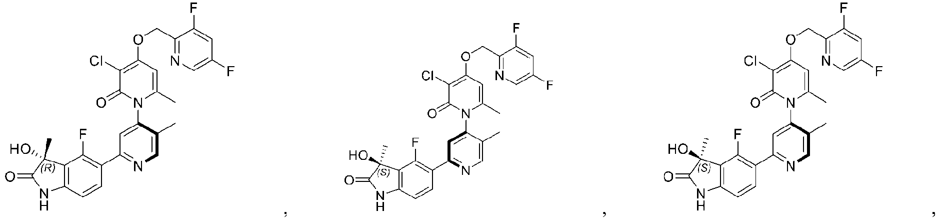 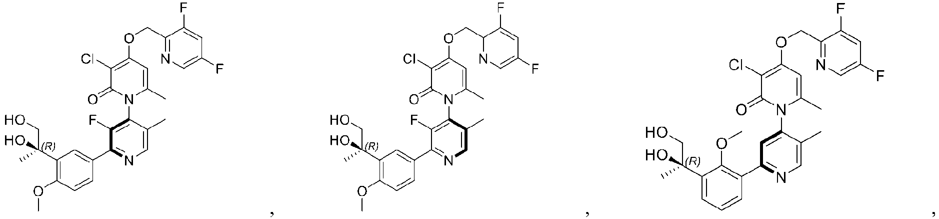 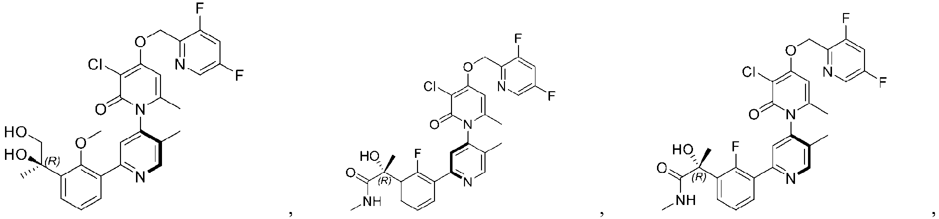 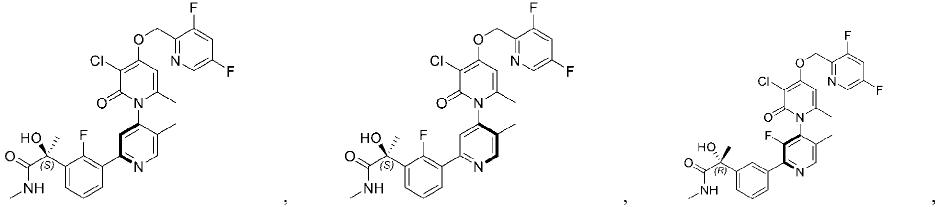 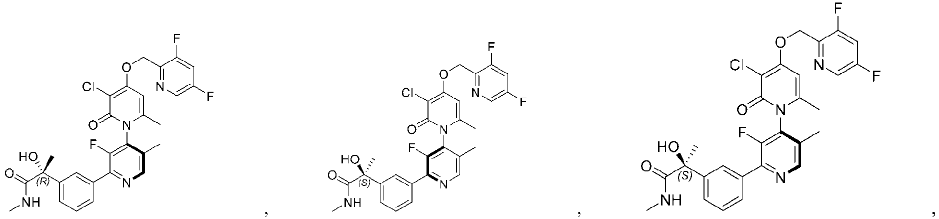 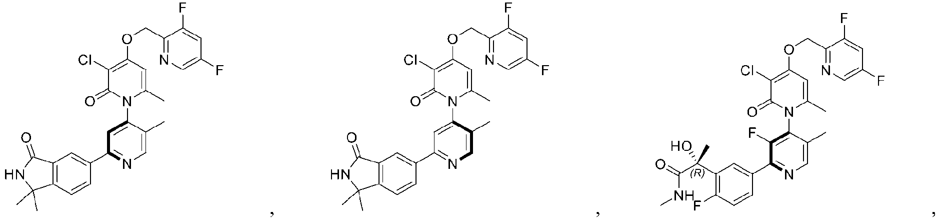 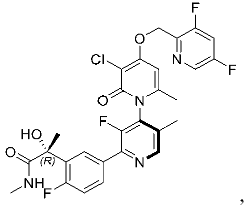 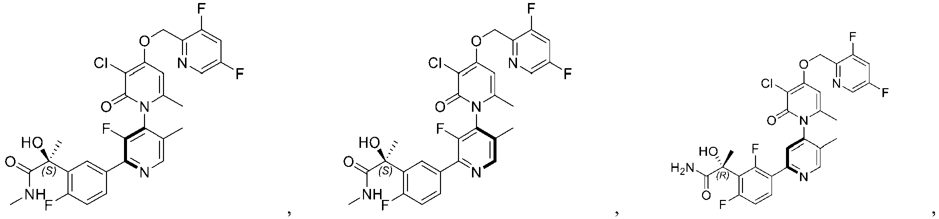 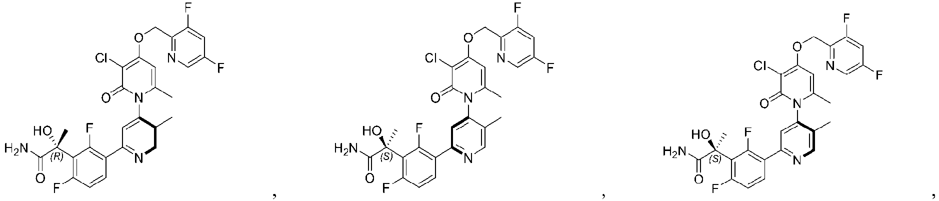 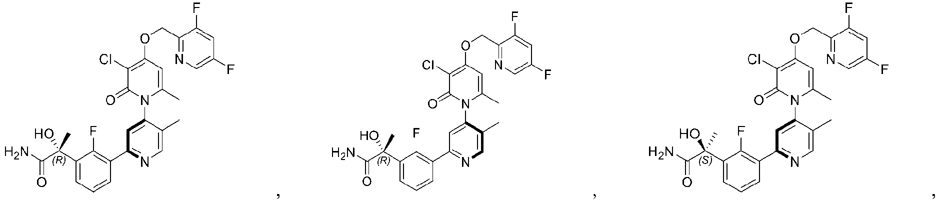 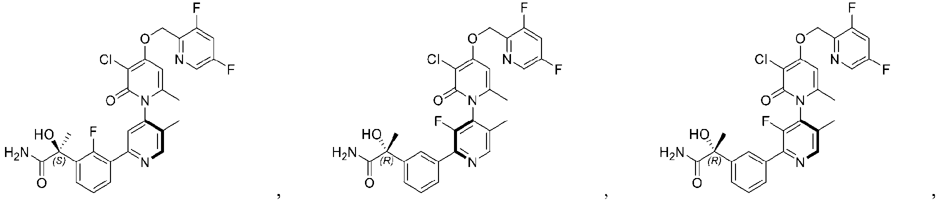 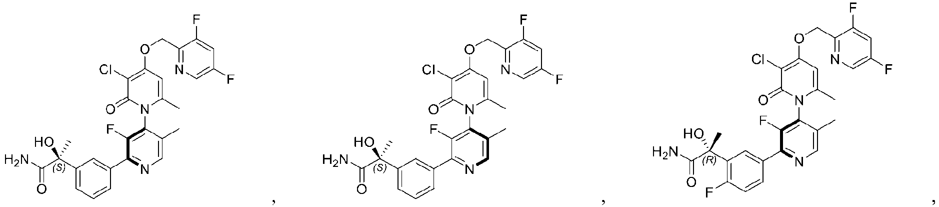 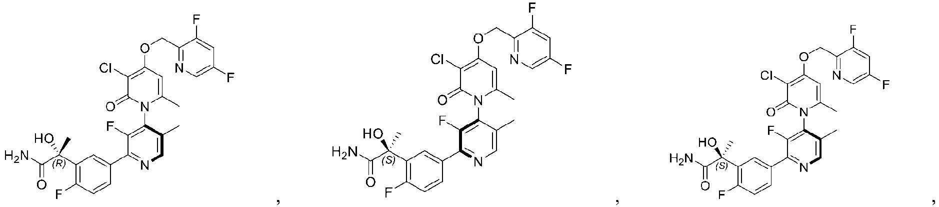 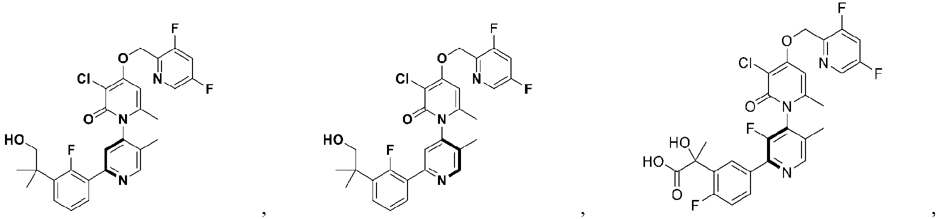 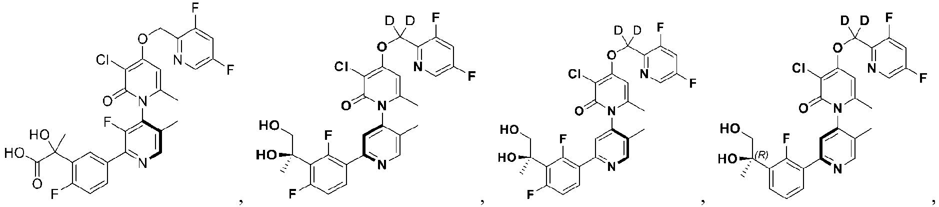 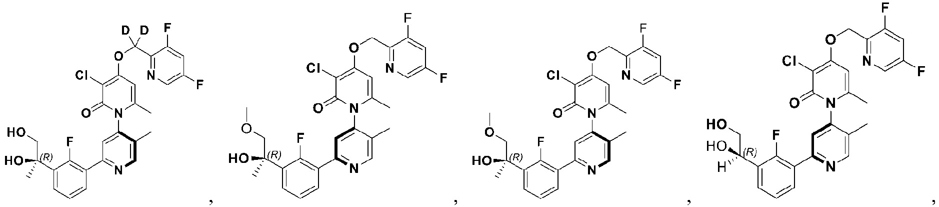 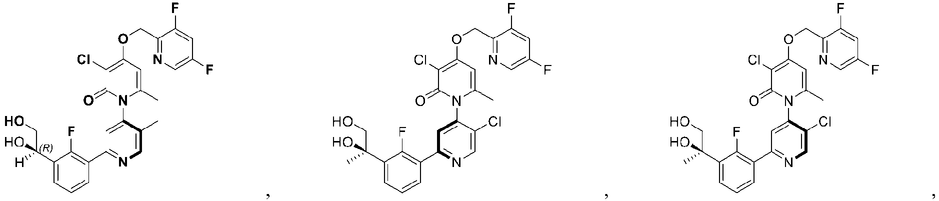 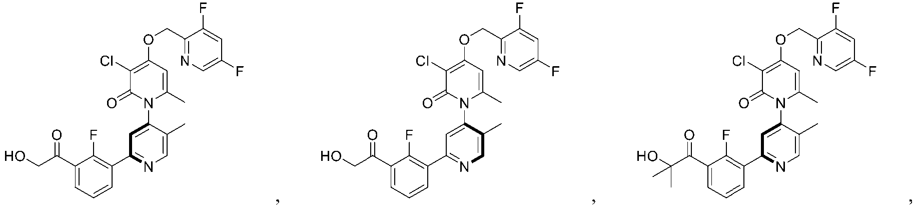 and

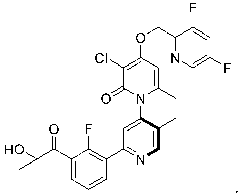

19. A method for preparing a compound represented by formula (I) or a pharmaceutically acceptable salt thereof, comprising the following steps: 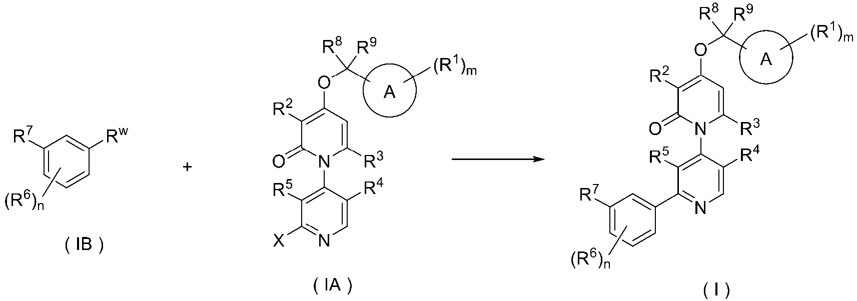
carrying out a coupling reaction on a compound represented by formula (IA) and a compound represented by formula (IB) in the presence of a catalyst to obtain the compound represented by formula (I) or a pharmaceutically acceptable salt thereof,
wherein X is halogen; preferably Cl;
R^{w} is -B(OH)₂ or 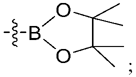
ring A, R¹, R², R³, R⁴, R⁵, R⁶, R⁷, R⁸, R⁹, n, and m are as defined in claim 1.

20. A pharmaceutical composition, comprising at least one therapeutically effective amount of the compound or the pharmaceutically acceptable salt thereof as defined in any one of claims 1 to 18, and one or more pharmaceutically acceptable excipients.

21. A use of the compound or the pharmaceutically acceptable salt thereof as defined in any one of claims 1 to 18, or the pharmaceutical composition as defined in claim 20 in the manufacture of a medicament of a p38 kinase inhibitor.

22. A use of the compound or the pharmaceutically acceptable salt thereof as defined in any one of claims 1 to 18, or the pharmaceutical composition as defined in claim 20 in the manufacture of a medicament for preventing and/or treating a disease mediated by p38 kinase; preferably, the disease mediated by p38 kinase is a disease associated with p38 MAPK/MK2 pathway.

23. A use of the compound or the pharmaceutically acceptable salt thereof as defined in any one of claims 1 to 18, or the pharmaceutical composition as defined in claim 20 in the manufacture of a medicament for the prevention and/or treatment of autoimmune diseases, inflammatory diseases, cardiovascular diseases, central nervous system diseases, and cancer; preferably, in the manufacture of a medicament for the prevention and/or treatment of arthritis, psoriasis, systemic lupus erythematosus, diabetes, leukemia, lymphoma, atherosclerosis, and Alzheimer's disease.
